# EUROPÄISCHE PATENTSCHRIFT

(11) **EP 2 649 045 B1**
(45) Veröffentlichungstag und Bekanntmachung des Hinweises auf die Patenterteilung: **27.05.2015**
(21) Anmeldenummer: 11805433.7
(22) Anmeldetag: 05.12.2011
(51) Int. Cl.: C07C 231/02, C07C 233/24

(54) **SUBSTITUIERTE 1-BENZYLCYCLOALKYLCARBONSÄUREN UND IHRE VERWENDUNG**
SUBSTITUTED 1-BENZYLCYCLOALKYLCARBOXLIC ACIDS AND USE THEREOF
ACIDES CARBOXYLIQUES 1-BENZYLCYCLOALKYLE SUBSTITUÉS ET LEUR UTILISATION

(30) Priorität: 07.12.2010 DE 102010062544; 07.04.2011 DE 102011006974
(43) Veröffentlichungstag der Anmeldung: 16.10.2013
(73) Patentinhaber: Bayer Intellectual Property GmbH, 40789 Monheim am Rhein (DE)
(72) Erfinder: LAMPE, Thomas, 40545 Düsseldorf (DE); HAHN, Michael, G., 40764 Langenfeld (DE); STASCH, Johannes-Peter, 42651 Solingen (DE); SCHLEMMER, Karl-Heinz, 42113 Wuppertal (DE); WUNDER, Frank, 42117 Wuppertal (DE); EL SHEIKH, Sherif, 45219 Essen (DE); LI, Volkhart Min-Jian, 42553 Velbert (DE); BECKER, Eva-Maria, 42327 Wuppertal (DE); STOLL, Friederike, 40215 Düsseldorf (DE); KNORR, Andreas, 40699 Erkrath (DE); KOLKHOF, Peter, 42113 Wuppertal (DE); WOLTERING, Elisabeth, 40723 Hilden (DE)
(74) Vertreter: BIP Patents
(86) Internationale Anmeldenummer: PCT/EP2011/071747
(87) Internationale Veröffentlichungsnummer: WO 2012/076466

(56) Entgegenhaltungen:
- WO-A1-2009/127338

## Beschreibung

Die vorliegende Anmeldung betrifft neue substituierte 1-Benzylcycloalkylcarbonsäure-Derivate, Verfahren zu ihrer Herstellung, ihre Verwendung zur Behandlung und/oder Prävention von Krankheiten sowie ihre Verwendung zur Herstellung von Arzneimitteln zur Behandlung und/oder Prävention von Krankheiten, insbesondere zur Behandlung und/oder Prävention kardiovaskulärer Erkrankungen.

Eines der wichtigsten zellulären Übertragungssysteme in Säugerzellen ist das cyclische Guanosinmonophosphat (cGMP). Zusammen mit Stickstoffmonoxid (NO), das aus dem Endothel freigesetzt wird und hormonelle und mechanische Signale überträgt, bildet es das NO/cGMP-System. Die Guanylatcyclasen katalysieren die Biosynthese von cGMP aus Guanosintriphosphat (GTP). Die bisher bekannten Vertreter dieser Familie lassen sich sowohl nach strukturellen Merkmalen als auch nach der Art der Liganden in zwei Gruppen aufteilen: Die partikulären, durch natriuretische Peptide stimulierbaren Guanylatcyclasen und die löslichen, durch NO stimulierbaren Guanylatcyclasen. Die löslichen Guanylatcyclasen bestehen aus zwei Untereinheiten und enthalten höchstwahrscheinlich ein Häm pro Heterodimer, das ein Teil des regulatorischen Zentrums ist. Dieses hat eine zentrale Bedeutung für den Aktivierungsmechanismus. NO kann an das Eisenatom des Häms binden und so die Aktivität des Enzyms deutlich erhöhen. Hämfreie Präparationen lassen sich hingegen nicht durch NO stimulieren. Auch Kohlenmonoxid (CO) ist in der Lage, am Eisen-Zentralatom des Häms anzugreifen, wobei die Stimulierung durch CO deutlich geringer ist als die durch NO.

Durch die Bildung von cGMP und der daraus resultierenden Regulation von Phosphodiesterasen, Ionenkanälen und Proteinkinasen spielt die Guanylatcyclase eine entscheidende Rolle bei unterschiedlichen physiologischen Prozessen, insbesondere bei der Relaxation und Proliferation glatter Muskelzellen, der Plättchenaggregation und -adhäsion und der neuronalen Signalübertragung sowie bei Erkrankungen, welche auf einer Störung der vorstehend genannten Vorgänge beruhen. Unter pathophysiologischen Bedingungen kann das NO/cGMP-System supprimiert sein, was zum Beispiel zu Bluthochdruck, einer Plättchenaktivierung, einer vermehrten Zellproliferation, endothelialer Dysfunktion, Atherosklerose, Angina pectoris, Herzinsuffizienz, Thrombosen, Schlaganfall und Myokardinfarkt führen kann.

Eine auf die Beeinflussung des cGMP-Signalweges in Organismen abzielende NO-unabhängige Behandlungsmöglichkeit für derartige Erkrankungen ist aufgrund der zu erwartenden hohen Effizienz und geringen Nebenwirkungen ein vielversprechender Ansatz.

Zur therapeutischen Stimulation der löslichen Guanylatcyclase wurden bisher ausschließlich Verbindungen wie organische Nitrate verwendet, deren Wirkung auf NO beruht. Dieses wird durch Biokonversion gebildet und aktiviert die lösliche Guanylatcyclase durch Angriffe am Eisen-Zentralatom des Häms. Neben den Nebenwirkungen gehört die Toleranzentwicklung zu den entscheidenden Nachteilen dieser Behandlungsweise [O.V. Evgenov et al., Nature Rev. Drug Disc. 5 (2006), 755].

In den letzten Jahren wurden Substanzen identifiziert, die die lösliche Guanylatcyclase direkt, d.h. ohne vorherige Freisetzung von NO stimulieren. Mit dem Indazolderivat YC-1 wurde erstmals ein NO-unabhängiger, jedoch Häm-abhängiger Stimulator der sGC beschrieben [Evgenov et al., *ibid*.]. Ausgehend von YC-1 wurden weitere Substanzen gefunden, welche eine höhere Potenz als YC-1 besitzen und keine relevante Hemmung von Phosphodiesterasen (PDE) aufweisen. Dies führte zur Identifizierung der Pyrazolopyridin-Derivate BAY 41-2272, BAY 41-8543 und BAY 63-2521. Diese Verbindungen bilden gemeinsam mit den kürzlich publizierten, strukturell diversen Substanzen CMF-1571 und A-350619 die neue Klasse der sGC-Stimulatoren [Evgenov et al., *ibid*.]. Gemeinsames Charakteristikum dieser Substanzklasse ist eine NO-unabhängige und selektive Aktivierung der hämhaltigen sGC. Darüber hinaus zeigen die sGC-Stimulatoren in Kombination mit NO einen synergistischen Effekt auf die sGC-Aktivierung, welcher auf einer Stabilisierung des Nitrosyl-Häm-Komplexes basiert. Die genaue Bindungsstelle der sGC-Stimulatoren an der sGC ist bis heute Gegenstand der Diskussion. Entfernt man von der löslichen Guanylatcyclase die HämGruppe, zeigt das Enzym immer noch eine nachweisbare katalytische Basalaktivität, d.h. es wird nach wie vor cGMP gebildet. Die verbleibende katalytische Basalaktivität des Häm-freien Enzyms ist durch keinen der vorstehend genannten Stimulatoren stimulierbar [Evgenov et al., *ibid*.]*.*

Darüber hinaus wurden NO- und Häm-unabhängige sGC-Aktivatoren, mit BAY 58-2667 als Prototyp dieser Klasse, identifiziert. Gemeinsame Charakteristika dieser Substanzen sind, dass sie in Kombination mit NO nur einen additiven Effekt auf die Enzymaktivierung ausüben, und dass die Aktivierung des oxidierten oder hämfreien Enzyms im Vergleich zum hämhaltigen Enzym deutlich stärker ist [Evgenov et al., *ibid*.; J.P. Stasch et al., Br. J. Pharmacol. 136 (2002), 773; J.P. Stasch et al., J. Clin. Invest. 116 (2006), 2552]. Spektroskopische Untersuchungen lassen erkennen, dass BAY 58-2667 die oxidierte Hämgruppe verdrängt, die durch Schwächung der Eisen-Histidin-Bindung nur schwach an der sGC gebunden ist. Auch wurde gezeigt, dass das charakteristische sGC-Hämbindungsmotiv Tyr-x-Ser-x-Arg sowohl für die Interaktion der negativ geladenen Propionsäuren der Hämgruppe als auch für die Wirkung von BAY 58-2667 zwingend erforderlich ist. Vor diesem Hintergrund wird angenommen, dass die Bindungsstelle von BAY 58-2667 an der sGC identisch zur Bindungsstelle der Hämgruppe ist [J.P. Stasch et al., J. Clin. Invest. 116 (2006), 2552].

Die in der vorliegenden Erfindung beschriebenen Verbindungen sind nun ebenfalls in der Lage, die Häm-freie Form der löslichen Guanylatcyclase zu aktivieren. Dies wird auch dadurch belegt, dass diese neuartigen Aktivatoren einerseits am Häm-haltigen Enzym keine synergistische Wirkung mit NO zeigen und andererseits sich ihre Wirkung nicht durch den Häm-abhängigen Inhibitor der löslichen Guanylatcyclase, 1*H*-1,2,4-Oxadiazolo[4,3-a]chinoxalin-1-on (ODQ), blockieren lässt, sondern durch diesen sogar potenziert wird [vgl. O.V. Evgenov et al., Nature Rev. Drug Disc. 5 (2006), 755; J.P. Stasch et al., J. Clin. Invest. 116 (2006), 2552].

Aufgabe der vorliegenden Erfindung war somit die Bereitstellung neuer Verbindungen, welche in der oben beschriebenen Weise als Aktivatoren der löslichen Guanylatcyclase wirken und als solche insbesondere zur Behandlung und zur Prävention kardiovaskulärer Erkrankungen eingesetzt werden können.

In WO 00/64888-A1, EP 1 216 980-A1, EP 1 285 908-A1, EP 1 348 698-A1, EP 1 375 472-A1, EP 1 452 521-A1 und US 2005/0234066-A1 werden verschiedene Arylalkancarbonsäure-Derivate als PPAR-Agonisten zur Behandlung von Diabetes, Dyslipidämie, Arteriosklerose, Obesitas und anderen Erkrankungen beschrieben. Weiterhin sind substituierte Arylalkancarbonsäuren aus EP 1 312 601-A1 und EP 1 431 267-A1 als PGE₂-Rezeptorantagonisten zur Behandlung beispielsweise von urologischen Erkrankungen, Schmerzzuständen, der Alzheimer'schen Krankheit und Krebs bekannt. In WO 2009/067493-A2 werden 3,5-disubstituierte Phenylessigsäure-Derivate als Wirkstoffe zur Behandlung der Alzheimer'schen Krankheit beansprucht. In WO 2009/127338-A1 und WO 2010/102717-A1 werden oxo-heterocyclisch substituierte Carbonsäure-Derivate offenbart, die als Aktivatoren der löslichen Guanylatcyclase wirken.

Gegenstand der vorliegenden Erfindung sind Verbindungen der allgemeinen Formel (I) 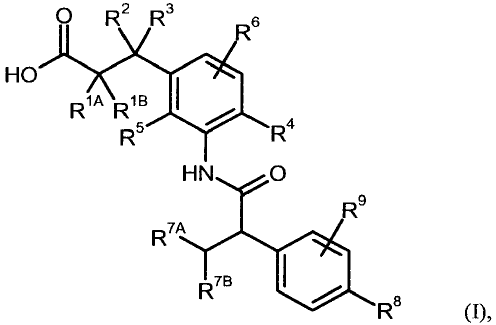 in welcher
- R^{1A} und R^{1A}: miteinander verknüpft sind und gemeinsam mit dem Kohlenstoffatom, an das sie gebunden sind, eine Cycloalkyl-Gruppe der Formel 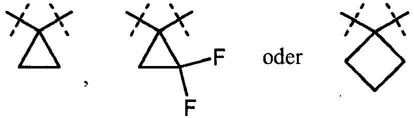 bilden,
- R²: für Wasserstoff, Methyl, Ethyl, Vinyl, Hydroxy, Methoxy, Trideuteromethoxy, Trifluormethoxy, Ethoxy oder Cyclopropyloxy steht,
- R³: für Wasserstoff, Methyl, Ethyl, Isopropyl oder Cyclopropyl steht,
- R⁴: für Wasserstoff, Fluor, Chlor, Brom, Cyano, Methyl, Trifluormethyl, Ethyl, Isopropyl, Cyclopropyl, Cyclobutyl, Methoxy oder Trifluormethoxy steht,
- R⁵: für Wasserstoff, Fluor, Chlor, Methyl, Trifluormethyl oder Trifluormethoxy steht,
- R⁶: für Wasserstoff, Fluor, Chlor, Methyl, Trifluormethyl oder Trifluormethoxy steht,
- R^{7A}: für Methyl oder Ethyl steht,
- R^{7B}: für Trifluormethyl steht,
oder
- R^{7A} und R^{7B}: miteinander verknüpft sind und gemeinsam mit dem Kohlenstoffatom, an das sie gebunden sind, einen optional difluor-substituierten Cyclopentyl-Ring der Formel 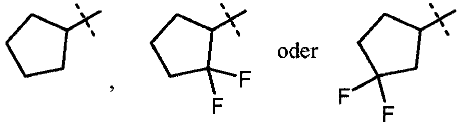 bilden,
- R⁸: für Fluor, Chlor, Brom, Nitro, Cyano, Trifluormethoxy, Acetyl, 2-Cyanovinyl, (C₁-C₄)-Alkyl, (C₂-C₄)-Alkenyl, Cyclopropyl oder Cyclobutyl steht, wobei (C₁-C₄)-Alkyl und (C₂-C₄)-Alkenyl bis zu dreifach mit Fluor
und
Cyclopropyl und Cyclobutyl bis zu zweifach mit Fluor substituiert sein können,
und
- R⁹: für Wasserstoff, Fluor, Chlor, Methyl, Trifluormethyl, Ethyl, Methoxy oder Trifluormethoxy steht,
sowie ihre Salze, Solvate und Solvate der Salze.

Erfindungsgemäße Verbindungen sind die Verbindungen der Formel (I) und deren Salze, Solvate und Solvate der Salze, die von Formel (I) umfassten Verbindungen der nachfolgend genannten Formeln und deren Salze, Solvate und Solvate der Salze sowie die von Formel (I) umfassten, nachfolgend als Ausführungsbeispiele genannten Verbindungen und deren Salze, Solvate und Solvate der Salze, soweit es sich bei den von Formel (I) umfassten, nachfolgend genannten Verbindungen nicht bereits um Salze, Solvate und Solvate der Salze handelt.

Als Salze sind im Rahmen der vorliegenden Erfindung physiologisch unbedenkliche Salze der erfindungsgemäßen Verbindungen bevorzugt. Umfasst sind auch Salze, die für pharmazeutische Anwendungen selbst nicht geeignet sind, jedoch beispielsweise für die Isolierung, Reinigung oder Lagerung der erfindungsgemäßen Verbindungen verwendet werden können.

Physiologisch unbedenkliche Salze der erfindungsgemäßen Verbindungen umfassen insbesondere die Salze üblicher Basen, wie beispielhaft und vorzugsweise Alkalimetall-Salze (z.B. Natrium- und Kaliumsalze), Erdalkali-Salze (z.B. Calcium- und Magnesiumsalze) und Ammonium-Salze, abgeleitet von Ammoniak oder organischen Aminen mit 1 bis 16 C-Atomen, wie beispielhaft und vorzugsweise Ethylamin, Diethylamin, Triethylamin, *N,N*-Diisopropylethylamin, Monoethanolamin, Diethanolamin, Triethanolamin, Dimethylaminoethanol, Diethylaminoethanol, Procain, Dicyclohexylamin, Dibenzylamin, *N*-Methylpiperidin, *N*-Methylmorpholin, Arginin, Lysin und 1,2-Ethylendiamin.

Als Solvate werden im Rahmen der Erfindung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt. Als Solvate sind im Rahmen der vorliegenden Erfindung Hydrate bevorzugt.

Die erfindungsgemäßen Verbindungen können in Abhängigkeit von ihrer Struktur in unterschiedlichen stereoisomeren Formen existieren, d.h. in Gestalt von Konfigurationsisomeren oder gegebenenfalls auch als Konformationsisomere (Enantiomere und/oder Diastereomere, einschließlich solcher bei Atropisomeren). Die vorliegende Erfindung umfasst deshalb die Enantiomere und Diastereomere und ihre jeweiligen Mischungen. Aus solchen Mischungen von Enantiomeren und/ oder Diastereomeren lassen sich die stereoisomer einheitlichen Bestandteile in bekannter Weise isolieren; vorzugsweise werden hierfür chromatographische Verfahren verwendet, insbesondere die HPLC-Chromatographie an achiraler bzw. chiraler Phase.

Sofern die erfindungsgemäßen Verbindungen in tautomeren Formen vorkommen können, umfasst die vorliegende Erfindung sämtliche tautomere Formen.

Die vorliegende Erfindung umfasst auch alle geeigneten isotopischen Varianten der erfindungsgemäßen Verbindungen. Unter einer isotopischen Variante einer erfindungsgemäßen Verbindung wird hierbei eine Verbindung verstanden, in welcher mindestens ein Atom innerhalb der erfindungsgemäßen Verbindung gegen ein anderes Atom der gleichen Ordnungszahl, jedoch mit einer anderen Atommasse als der gewöhnlich oder überwiegend in der Natur vorkommenden Atommasse ausgetauscht ist. Beispiele für Isotope, die in eine erfindungsgemäße Verbindung inkorporiert werden können, sind solche von Wasserstoff, Kohlenstoff, Stickstoff, Sauerstoff, Phosphor, Schwefel, Fluor, Chlor, Brom und Iod, wie ²H (Deuterium), ³H (Tritium), ¹³C, ¹⁴C, ¹⁵N, ¹⁷O, ¹⁸O, ³²P, ³³P, ³³S, ³⁴S, ³⁵S, ³⁶S, ¹⁸F, ³⁶Cl, ⁸²Br, ¹²³I, ¹²⁴I, ¹²⁹I und ¹³¹I. Bestimmte isotopische Varianten einer erfindungsgemäßen Verbindung, wie insbesondere solche, bei denen ein oder mehrere radioaktive Isotope inkorporiert sind, können von Nutzen sein beispielsweise für die Untersuchung des Wirkmechanismus oder der Wirkstoff-Verteilung im Körper; aufgrund der vergleichsweise leichten Herstell- und Detektierbarkeit sind hierfür insbesondere mit ³H- oder ¹⁴C-Isotopen markierte Verbindungen geeignet. Darüber hinaus kann der Einbau von Isotopen, wie beispielsweise von Deuterium, zu bestimmten therapeutischen Vorteilen als Folge einer größeren metabolischen Stabilität der Verbindung führen, wie beispielsweise zu einer Verlängenmg der Halbwertszeit im Körper oder zu einer Reduktion der erforderlichen Wirkdosis; solche Modifikationen der erfindungsgemäßen Verbindungen können daher gegebenenfalls auch eine bevorzugte Ausführungsform der vorliegenden Erfindung darstellen. Isotopische Varianten der erfindungsgemäßen Verbindungen können nach allgemein gebräuchlichen, dem Fachmann bekannten Verfahren hergestellt werden, so beispielsweise nach den weiter unten beschriebenen Methoden und den bei den Ausführungsbeispielen wiedergegebenen Vorschriften, indem hierbei entsprechende isotopische Modifikationen der jeweiligen Reagentien und/oder Ausgangsverbindungen eingesetzt werden.

Außerdem umfasst die vorliegende Erfindung auch Prodrugs der erfindungsgemäßen Verbindungen. Der Begriff "Prodrugs" bezeichnet hierbei Verbindungen, welche selbst biologisch aktiv oder inaktiv sein können, jedoch während ihrer Verweilzeit im Körper auf beispielsweise metabolischem oder hydrolytischem Wege zu erfindungsgemäßen Verbindungen umgesetzt werden.

Insbesondere umfasst die vorliegende Erfindung als Prodrugs hydrolysierbare Ester-Derivate der erfindungsgemäßen Carbonsäuren der Formel (I). Hierunter werden Ester verstanden, die in physiologischen Medien, unter den Bedingungen der im weiteren beschriebenen biologischen Tests und insbesondere *in vivo* auf enzymatischem oder chemischem Wege zu den freien Carbonsäuren, als den biologisch hauptsächlich aktiven Verbindungen, hydrolysiert werden können. Als solche Ester werden (C₁-C₄)-Alkylester, in welchen die Alkylgmppe geradkettig oder verzweigt sein kann, bevorzugt. Besonders bevorzugt sind Methyl-, Ethyl- oder *tert*.-Butylester.

Im Rahmen der vorliegenden Erfindung haben die Substituenten, soweit nicht anders spezifiziert, die folgende Bedeutung:
(C₁-C₄)Alkyl steht im Rahmen der Erfindung für einen geradkettigen oder verzweigten Alkylrest mit 1 bis 4 Kohlenstoffatomen. Beispielhaft und vorzugsweise seien genannt: Methyl, Ethyl, *n*-Pro-pyl, Isopropyl, *n*-Butyl, *iso*-Butyl, *sec*.-Butyl und *tert*.-Butyl.
(C₂₋C₄)-Alk.enyl und (C₂-C₃)-Alkenyl stehen im Rahmen der Erfindung für einen geradkettigen oder verzweigten Alkenylrest mit einer Doppelbindung und 2 bis 4 bzw. 2 oder 3 Kohlenstoffatomen. Bevorzugt ist ein geradkettiger oder verzweigter Alkenylrest mit 2 oder 3 Kohlenstoffatomen. Beispielhaft und vorzugsweise seien genannt: Vinyl, Allyl, *n*-Prop-1-en-1-yl, Isopropenyl, *n*-But-1-en-1-yl, *n*-But-2-en-1-yl, *n*-But-3-en-1-yl, 2-Methylprop-1-en-1-yl und 2-Methylprop-2-en-1-yl.

Im Rahmen der vorliegenden Erfindung gilt, dass für alle Reste, die mehrfach auftreten, deren Bedeutung unabhängig voneinander ist. Wenn Reste in den erfindungsgemäßen Verbindungen substituiert sind, können die Reste, soweit nicht anders spezifiziert, ein- oder mehrfach substituiert sein. Eine Substitution mit einem oder zwei oder drei gleichen oder verschiedenen Substituenten ist bevorzugt. Besonders bevorzugt ist die Substitution mit einem oder zwei gleichen oder verschiedenen Substituenten.

Bevorzugt im Rahmen der vorliegenden Erfindung sind Verbindungen der Formel (I), in welcher
- R^{1A} und R^{1B}: miteinander verknüpft sind und gemeinsam mit dem Kohlenstoffatom, an das sie gebunden sind, eine Cycloalkyl-Gruppe der Formel 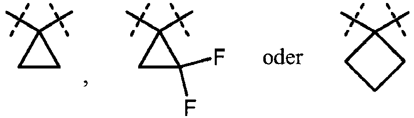 bilden,
- R²: für Wasserstoff, Methyl, Ethyl, Hydroxy, Methoxy, Trideuteromethoxy, Ethoxy oder Cyclopropyloxy steht,
- R³: für Wasserstoff, Methyl oder Ethyl steht,
- R⁴: für Wasserstoff, Fluor, Chlor, Methyl oder Cyclopropyl steht,
- R⁵: für Wasserstoff, Fluor, Chlor oder Methyl steht,
- R⁶: für Wasserstoff, Fluor, Chlor oder Methyl steht,
- R^{7A}: für Methyl steht,
- R^{7B}: für Trifluormethyl steht,
oder
- R^{7A} und R^{7B}: miteinander verknüpft sind und gemeinsam mit dem Kohlenstoffatom, an das sie gebunden sind, einen difluor-substituierten Cyclopentyl-Ring der Formel 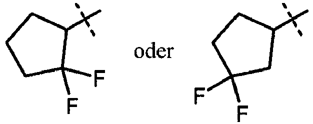 bilden,
- R⁸: für Fluor, Chlor, Acetyl, 2-Cyanovinyl, (C₁-C₄)-Alkyl, (C₂-C₃)-Alkenyl, Cyclopropyl oder Cyclobutyl steht, wobei (C₁-C₄)-Alkyl und (C₂-C₃)-Alkenyl bis zu dreifach mit Fluor substituiert sein können,
und
- R⁹: für Wasserstoff, Fluor, Chlor, Methyl, Trifluormethyl oder Methoxy steht,
sowie ihre Salze, Solvate und Solvate der Salze.

Eine besondere Ausführungsform der vorliegenden Erfindung umfasst Verbindungen der Formel (I), in welcher
- R^{1A} und R^{1B}: miteinander verknüpft sind und gemeinsam mit dem Kohlenstoffatom, an das sie gebunden sind, einen optional difluor-substituierten Cyclopropyl-Ring der Formel 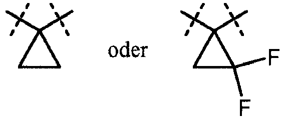 bilden,
- R²: für Wasserstoff, Methyl oder Ethyl steht,
und
- R³: für Wasserstoff steht,
sowie ihre Salze, Solvate und Solvate der Salze.

Eine weitere besondere Ausführungsform der vorliegenden Erfindung umfasst Verbindungen der Formel (I), in welcher
- R^{1A} und R^{1B}: miteinander verknüpft sind und gemeinsam mit dem Kohlenstoffatom, an das sie gebunden sind, einen Cyclopropyl- oder Cyclobutyl-Ring der Formel 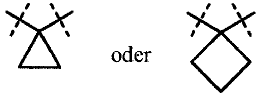 bilden,
- R²: für Hydroxy, Methoxy, Trideuteromethoxy oder Ethoxy steht,
und
- R³: für Wasserstoff steht,
sowie ihre Salze, Solvate und Solvate der Salze.

Eine weitere besondere Ausführungsform der vorliegenden Erfindung umfasst Verbindungen der Formel (I), in welcher
- R⁴: für Wasserstoff, Fluor oder Chlor steht,
sowie ihre Salze, Solvate und Solvate der Salze.

Eine weitere besondere Ausführungsform der vorliegenden Erfindung umfasst Verbindungen der Formel (I), in welcher
- R⁵: für Wasserstoff oder Fluor steht
und
- R⁶: für Wasserstoff steht,
sowie ihre Salze, Solvate und Solvate der Salze.

Eine weitere besondere Ausführungsform der vorliegenden Erfindung umfasst Verbindungen der Formel (I), in welcher
- R^{7A}: für Methyl steht
und
- R^{7B}: für Trifluormethyl steht,
sowie ihre Salze, Solvate und Solvate der Salze.

Eine weitere besondere Ausführungsform der vorliegenden Erfindung umfasst Verbindungen der Formel (I), in welcher
- R^{7A} und R^{7B}: miteinander verknüpft sind und gemeinsam mit dem Kohlenstoffatom, an das sie gebunden sind, einen difluor-substituierten Cyclopentyl-Ring der Formel 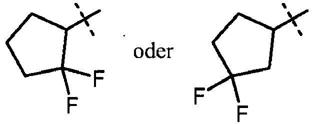 bilden,
sowie ihre Salze, Solvate und Solvate der Salze.

Eine weitere besondere Ausführungsform der vorliegenden Erfindung umfasst Verbindungen der Formel (I), in welcher
- R⁸: für Chlor, (C₁-C₄)-Alkyl, (C₂-C₃)-Alkenyl oder Cyclopropyl steht, wobei (C₁-C₄)-Alkyl und (C₂-C₃)-Alkenyl bis zu dreifach mit Fluor substituiert sein können,
sowie ihre Salze, Solvate und Solvate der Salze.

Eine weitere besondere Ausführungsform der vorliegenden Erfindung umfasst Verbindungen der Formel (I), in welcher
- R⁹: für Wasserstoff, Fluor, Chlor oder Methoxy steht,
sowie ihre Salze, Solvate und Solvate der Salze.

Besonders bevorzugt im Rahmen der vorliegenden Erfindung sind Verbindungen der Formel (I), in welcher
- R^{1A} und R^{1B}: miteinander verknüpft sind und gemeinsam mit dem Kohlenstoffatom, an das sie gebunden sind, einen optional difluor-substituierten Cyclopropyl-Ring der Formel 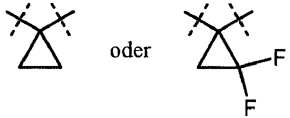 bilden,
- R²: für Wasserstoff oder Ethyl steht,
- R³: für Wasserstoff steht,
- R⁴: für Wasserstoff, Fluor oder Chlor steht,
- R⁵: für Wasserstoff oder Fluor steht,
- R⁶: für Wasserstoff steht,
- R^{7A}: für Methyl steht,
- R^{7B}: für Trifluormethyl steht,
oder
- R^{7A} und R^{7B}: miteinander verknüpft sind und gemeinsam mit dem Kohlenstoffatom, an das sie gebunden sind, einen difluor-substituierten Cyclopentyl-Ring der Formel 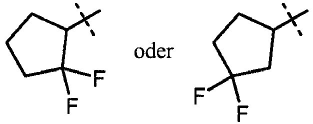 bilden,
- R⁸: für Chlor, Methyl, Trifluormethyl, Ethyl, 1,1-Difluorethyl, 2,2,2-Trifluorethyl, Isopropyl, *tert*.-Butyl, 1,1,1-Trifluor-2-methylpropan-2-yl, Vinyl, 2,2-Difluorvinyl oder Cyclopropyl steht,
und
- R⁹: für Wasserstoff, Fluor, Chlor oder Methoxy steht,
sowie ihre Salze, Solvate und Solvate der Salze.

Besonders bevorzugt im Rahmen der vorliegenden Erfindung sind auch Verbindungen der Formel (I), in welcher
- R^{1A} und R^{1B}: miteinander verknüpft sind und gemeinsam mit dem Kohlenstoffatom, an das sie gebunden sind, einen Cyclopropyl- oder Cyclobutyl-Ring der Formel 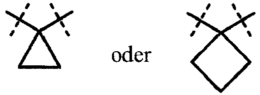 bilden,
- R²: für Hydroxy, Methoxy, Trideuteromethoxy, Ethoxy oder Cyclopropyloxy steht,
- R³: für Wasserstoff steht,
- R⁴: für Wasserstoff, Fluor oder Chlor steht,
- R⁵: für Wasserstoff oder Fluor steht,
- R⁶: für Wasserstoff steht,
- R^{7A}: für Methyl steht,
- R^{7B}: für Trifluormethyl steht,
oder
- R^{7A} und R^{7B}: miteinander verknüpft sind und gemeinsam mit dem Kohlenstoffatom, an das sie gebunden sind, einen difluor-substituierten Cyclopentyl-Ring der Formel 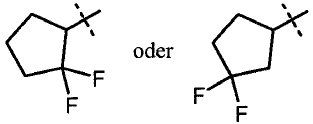 bilden,
- R⁸: für Chlor, Methyl, Trifluormethyl, Ethyl, 1,1-Difluorethyl, 2,2,2-Trifluorethyl, Isopropyl, *tert*.-Butyl, 1,1,1-Trifluor-2-methylpropan-2-yl, Vinyl, 2,2-Difluorvinyl oder Cyclopropyl steht,
und
- R⁹: für Wasserstoff, Fluor, Chlor oder Methoxy steht,
sowie ihre Salze, Solvate und Solvate der Salze.

Von besonderer Bedeutung im Rahmen der vorliegenden Erfindung sind Verbindungen der Formel (I-A) 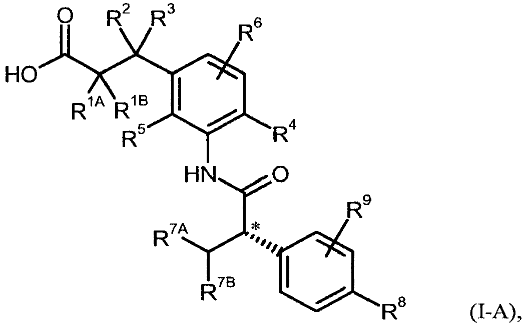 in welcher das mit * gekennzeichnete C-Atom der Phenylacetamid-Gruppierung die abgebildete S-Konfiguration aufweist
und
die Reste R^{1A}, R^{1B}, R², R³, R⁴, R⁵, R⁶, R^{7A}, R^{7B}, R⁸ und R⁹ jeweils die oben angegebenen Bedeutungen haben,
sowie ihre Salze, Solvate und Solvate der Salze.

Die in den jeweiligen Kombinationen bzw. bevorzugten Kombinationen von Resten im einzelnen angegebenen Reste-Definitionen werden unabhängig von den jeweiligen angegebenen Kombinationen der Reste beliebig auch durch Reste-Definitionen anderer Kombinationen ersetzt. Ganz besonders bevorzugt sind Kombinationen von zwei oder mehreren der oben genannten Vorzugsbereiche.

Weiterer Gegenstand der Erfindung ist ein Verfahren zur Herstellung der erfindungsgemäßen Verbindungen der Formel (I), dadurch gekennzeichnet, dass man eine Carbonsäure der Formel (II) 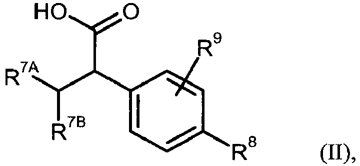 in welcher R^{7A}, R^{7B}, R⁸ und R⁹ die oben angegebenen Bedeutungen haben,

in einem inerten Lösungsmittel mit Hilfe eines Kondensationsmittels oder über die Zwischenstufe des entsprechenden Carbonsäurechlorids in Gegenwart einer Base mit einem Amin der Formel (III) 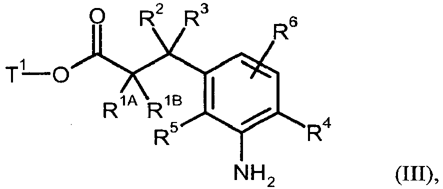 in welcher R^{1A}, R^{1B}, R², R³, R⁴, R⁵ und R⁶ die oben angegebenen Bedeutungen haben
und
- T¹: für (C₁-C₄)-Alkyl oder Benzyl steht,
zu einem Carbonsäureamid der Formel (IV) 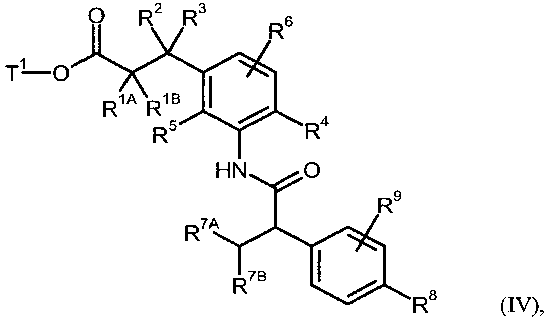 in welcher R^{1A}, R^{1B}, R², R³, R⁴, R⁵, R⁶, R^{7A} , R^{7B}, R⁸, R⁹ und T¹ die oben angegebenen Bedeutungen haben,
kuppelt und anschließend den Ester-Rest T¹ durch basische oder saure Solvolyse oder im Fall, dass T¹ für Benzyl steht, auch durch Hydrogenolyse unter Erhalt der Carbonsäure der Formel (I) abspaltet
und gegebenenfalls die Verbindungen der Formel (I) nach dem Fachmann bekannten Methoden in ihre Enantiomere und/oder Diastereomere trennt und/oder mit den entsprechenden (i) Lösungsmitteln und/oder (ii) Basen zu ihren Solvaten, Salzen und/oder Solvaten der Salze umsetzt.

Inerte Lösungsmittel für den Verfahrensschritt (II) + (III) → (IV) [Amid-Kupplung] sind beispielsweise Ether wie Diethylether, *tert*.-Butyl-methylether, Tetrahydrofuran, 1,4-Dioxan, Glykoldimethylether oder Diethylenglykoldimethylether, Kohlenwasserstoffe wie Benzol, Toluol, Xylol, Hexan, Cyclohexan oder Erdölfraktionen, Halogenkohlenwasserstoffe wie Dichlormethan, Trichlormethan, Tetrachlonnethan, 1,2-Dichlorethan, Trichlorethylen oder Chlorbenzol, oder andere Lösungsmittel wie Aceton, Acetonitril, Ethylacetat, Pyridin, Dimethylsulfoxid (DMSO), *N,N-*Dimethylformamid (DMF), *N,N'*-Dimethylpropylenharnstoff (DMPU) oder *N*-Methylpyrrolidinon (NMP). Ebenso ist es möglich, Gemische der genannten Lösungsmittel einzusetzen. Bevorzugt werden Dichlormethan, Tetrahydrofuran, Dimethylformamid oder Gemische dieser Lösungsmittel verwendet.

Als Kondensationsmittel für diese Kupplungsreaktion eignen sich beispielsweise Carbodiimide wie *N,N'*-Diethyl-, *N,N'*-Dipropyl-, *N,N'*-Diisopropyl-, *N,N'*-Dicyclohexylcarbodiimid. (DCC) oder *N-*(3-Dimethylaminoisopropyl)-*N'*-ethylcarbodiimid-Hydrochlorid (EDC), Phosgen-Derivate wie *N,N'*-Carbonyldiimidazol (CD1) oder Isobutylchlorformiat, 1,2-Oxazolium-Verbindungen wie 2-Ethyl-5-phenyl-1,2-oxazolium-3-sulfat oder 2-*tert*.-Butyl-5-methylisoxazolium-perchlorat, Acylamino-Verbindungen wie 2-Ethoxy-1-ethoxycarbonyl-1,2-dihydrochinolin, α-Chlorenamine wie 1-Chlor-2-methyl-1-dimethylamino-1-propen, Phosphor-Verbindungen wie Propanphosphonsäureanhydrid, Cyanophosphonsäurediethylester, Bis-(2-oxo-3-oxazolidinyl)-phosphorylchlorid, Benzotriazol-1-yloxy-tris(dimethylamino)phosphonium-hexafluorophosphat oder Benzotriazol-1-yloxytris(pyrrolidino)phosphonium-hexafluorophosphat (PyBOP), oder Uronium-Verbindungen wie *O*-(Beazotriazol-1-yl)-*N,N,N',N'*-tetramethyluronium-tetrafluoroborat (TBTU), *O*-(Benzotriazol-1-yl)-*N,N,N',N'*-tetramethyluronium-hexafluorophosphat (HBTU), 2-(2-Oxo-1-(2*H*)-pyridyl)-1,1,3,3-tetramethyluronium-tetrafluoroborat (TPTU), *O*-(7-Azabenzotriazol-1-yl)-*N,N,N',N'*-tetramethyl-uronium-hexafluorophosphat (HATU) oder *O*-(1*H*-6-Chlorbenzotriazol-1-yl)-1,1,3,3-tetramethyl-uronium-tetrafluoroborat (TCTU), gegebenenfalls in Kombination mit weiteren Hilfsstoffen wie 1-Hydroxybenzotriazol (HOBt) oder *N*-Hydroxysuccinimid (HOSu), sowie als Basen Alkalicarbonate, z.B. Natrium- oder Kaliumcarbonat, oder tertiäre Aminbasen wie Triethylamin, *N*-Methylmorpholin, *N*-Methylpiperidin, *N,N-*Diisopropylethylamin, Pyridin oder 4-*N,N-*Dimethylaminopyridin. Bevorzugt eingesetzt werden *O*-(7-Azabenzotriazol-1-yl)-*N,N,N'*,*N'*-tetramethyluronium-hexafluorophosphat (HATU) in Kombination mit Pyridin oder *N,N-*Diisopropylethylamin, oder *N*-(3-Dimethylaminoisopropyl)-*N'*-ethylcarbodümid-Hydrochlorid (EDC) in Verbindung mit 1-Hydroxybenzotriazol (HOBt) und Triethylamin, oder 1-Chlor-2-methyl-1-dimethylamino-1-propen zusammen mit Pyridin.

Die Reaktion (II) + (III) → (IV) wird in der Regel in einem Temperaturbereich von 0°C bis +60°C, bevorzugt bei +10°C bis +40°C durchgeführt.

Bei Einsatz eines der Verbindung (II) entsprechenden Carbonsäurechlorids wird die Kupplung mit der Amin-Komponente (III) in Gegenwart einer üblichen organischen Hilfsbase wie Triethylamin, *N*-Methylmorpholin, *N*-Methylpiperidin, *N,N*-Diisopropylethylamin, Pyridin, 4-*N,N*-Dimethylaminopyridin, 1,8-Diazabicyclo[5.4.0]undec-7-en (DBU) oder 1,5-Diazabicyclo[4.3.0]non-5-en (DBN) durchgeführt. Bevorzugt wird Triethylamin oder *N,N*-Diisopropylethylamin verwendet.

Die Umsetzung des Amins (III) mit dem Carbonsäurechlorid erfolgt im Allgemeinen in einem Temperaturbereich von -20°C bis +60°C, bevorzugt im Bereich von -10°C bis +30°C.

Die Herstellung der Carbonsäurechloride selbst geschieht auf übliche Weise durch Behandlung der Carbonsäure (II) mit Thionylchlorid oder Oxalylchlorid.

Die Abspaltung der Ester-Gruppe T¹ im Verfahrensschritt (IV) → (I) wird nach üblichen Methoden durchgeführt, indem man den Ester in inerten Lösungsmitteln mit Säuren oder Basen behandelt, wobei bei letzterer Variante das zunächst entstehende Salz durch Behandeln mit Säure in die freie Carbonsäure überführt wird. Im Falle der *tert*.-Butylester erfolgt die Esterspaltung vorzugsweise mit Säuren. Benzylester werden bevorzugt durch Hydrogenolyse (Hydrierung) in Gegenwart eines geeigneten Katalysators, wie beispielsweise Palladium auf Aktivkohle, abgespalten.

Als inerte Lösungsmittel eignen sich für diese Reaktionen Wasser oder die für eine Esterspaltung üblichen organischen Lösungsmittel. Hierzu gehören bevorzugt Alkohole wie Methanol, Ethanol, n-Propanol, Isopropanol, n-Butanol oder *tert*.-Butanol, oder Ether wie Diethylether, Tetrahydrofuran, Dioxan oder Glykoldimethylether, oder andere Lösungsmittel wie Aceton, Dichlormethan, Dimethylformamid oder Dimethylsulfoxid. Ebenso ist es möglich, Gemische der genannten Lösungsmittel einzusetzen. Im Falle einer basischen Ester-Hydrolyse werden bevorzugt Gemische von Wasser mit Dioxan, Tetrahydrofuran, Methanol und/oder Ethanol eingesetzt. Im Falle der Umsetzung mit Trifluoressigsäure wird bevorzugt Dichlormethan und im Falle der Umsetzung mit Chlorwasserstoff bevorzugt Tetrahydrofuran, Diethylether, Dioxan oder Wasser verwendet.

Als Basen sind die üblichen anorganischen Basen geeignet. Hierzu gehören insbesondere Alkali- oder Erdalkalihydroxide wie beispielsweise Lithium-, Natrium-, Kalium- oder Bariumhydroxid, oder Alkali- oder Erdalkalicarbonate wie Natrium-, Kalium- oder Calciumcarbonat. Bevorzugt sind Lithium-, Natrium- oder Kaliumhydroxid.

Als Säuren eignen sich für die Esterspaltung im Allgemeinen Schwefelsäure, Chlorwasserstoff/ Salzsäure, Bromwasserstoff/Bromwasserstoffsäure, Phosphorsäure, Essigsäure, Trifluoressigsäure, Toluolsulfonsäure, Methansulfonsäure oder Trifluormethansulfonsäure oder deren Gemische gegebenenfalls unter Zusatz von Wasser. Bevorzugt sind Chlorwasserstoff oder Trifluoressigsäure im Falle der *tert*.-Butylester und Salzsäure im Falle der Methylester.

Die Esterspaltung erfolgt im Allgemeinen in einem Temperaturbereich von -20°C bis +100°C, bevorzugt bei 0°C bis +60°C.

Die Intermediate der Formel (II) können beispielsweise dadurch hergestellt werden, dass man
[A] einen Carbonsäureester der Formel (V) 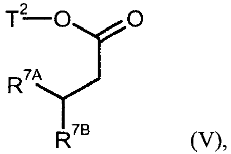 in welcher R^{7A} und R^{7B} die oben angegebenen Bedeutungen haben
   und
   - T²: für (C₁-C₄)-Alkyl oder Benzyl steht,
   in einem inerten Lösungsmittel zunächst mit Hilfe einer Base deprotoniert und dann in Gegenwart eines geeigneten Palladium-Katalysators mit einem Phenylbromid der Formel (VI) 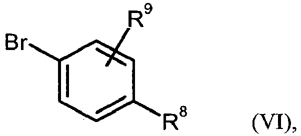 in welcher R⁸ und R⁹ die oben angegebenen Bedeutungen haben,
   zu einer Verbindung der Formel (VII) 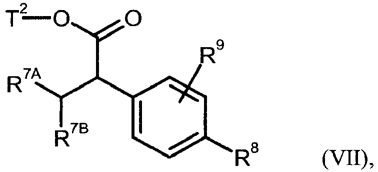 in welcher R^{7A}, R^{7B}, R⁸, R⁹ und T² die oben angegebenen Bedeutungen haben, aryliert
   oder
[B] einen Phenylessigsäureester der Formel (VIII) 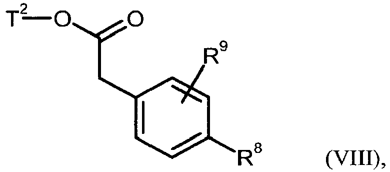 in welcher R⁸ und R⁹ die oben angegebenen Bedeutungen haben
   und
   - T²: für (C₁-C₄)-Alkyl oder Benzyl steht,
   in einem inerten Lösungsmittel in Gegenwart einer Base mit einer Verbindung der Formel (IX) 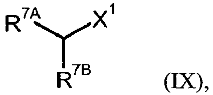 in welcher R^{7A} und R^{7B} die oben angegebenen Bedeutungen haben
   und
   - X¹: für eine geeignete Abgangsgruppe wie beispielsweise Brom oder Iod steht,
   zu der Verbindung der Formel (VII) 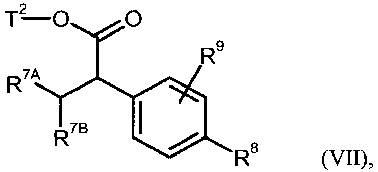 in welcher R^{7A}, R^{7B}, R⁸, R⁹ und T² die oben angegebenen Bedeutungen haben, alkyliert
und jeweils nachfolgend den Ester-Rest T² durch basische oder saure Solvolyse oder im Fall, dass T² für Benzyl steht, auch durch Hydrogenolyse unter Erhalt der Carbonsäure (II) abspaltet.

Die Arylierungsreaktion im Verfahrensschritt (V) + (VI) → (VII) wird vorzugsweise in Toluol oder Toluol/Tetrahydrofuran-Gemischen in einem Temperaturbereich von +20°C bis +100°C durchgeführt. Als Base zur Deprotonierung des Esters (V) wird hierbei bevorzugt Lithium-bis(trimethylsilyl)amid eingesetzt. Geeignete Palladium-Katalysatoren sind beispielsweise Palladium(II)-acetat oder Tris(dibenzylidenaceton)-dipalladium, jeweils in Kombination mit einem elektronenreichen, sterisch anspruchsvollen Phosphin-Liganden wie 2-Dicyclohexylphosphino-2'-(*N,N*-di-methylamino)biphenyl oder 2-Di-*tert*.-butylphosphino-2'-(*N,N*-dimethylamino)biphenyl [vgl. z.B. W.A. Moradi, S.L. Buchwald, J. Am. Chem. Soc. 123, 7996-8002 (2001)].

Inerte Lösungsmittel für die Alkylierungsreaktion (VIII) + (IX) → (VII) sind beispielsweise Ether wie Diethylether, Methyl-*tert*.-butylether, Dioxan, Tetrahydrofuran, Glykoldimethylether oder Diethylenglykoldimethylether, Kohlenwasserstoffe wie Benzol, Toluol, Xylol, Hexan, Cyclohexan oder Erdölfraktionen, oder dipolar-aprotische Lösungsmittel wie *N,N*-Dimethylformamid (DMF), Dimethylsulfoxid (DMSO), *N,N'*-Dimethylpropylenharnstoff (DMPU) oder *N*-Methylpyrrolidinon (NMP). Ebenso ist es möglich, Gemische der genannten Lösungsmittel einzusetzen. Bevorzugt werden Tetrahydrofuran, Dimethylformamid oder Gemische hiervon verwendet.

Als Base für den Verfahrensschritt (VIII) + (IX) → (VII) eignen sich übliche starke anorganische oder organische Basen. Hierzu gehören insbesondere Alkalialkoholate wie Natrium- oder Kaliummethanolat, Natrium- oder Kaliumethanolat oder Natrium- oder Kalium-*tert*.-butylat, Alkalihydride wie Natrium- oder Kaliumhydrid, oder Amide wie Lithium-, Natrium- oder Kalium-bis(trimethylsilyl)amid oder Lithiumdiisopropylamid. Bevorzugt wird Kalium-*tert*.-butylat, Natriumhydrid oder Lithiumdiisopropylamid verwendet.

Die Umsetzung (VIII) + (IX) → (VII) wird im Allgemeinen in einem Temperaturbereich von -80°C bis +40°C, bevorzugt bei -20°C bis +20°C durchgeführt.

Die Abspaltung der Ester-Gruppe T² im Verfahrensschritt (VII) → (II) erfolgt auf analoge Weise wie zuvor für den Ester-Rest T¹ beschrieben.

### Intermediate der Formel (II-A)

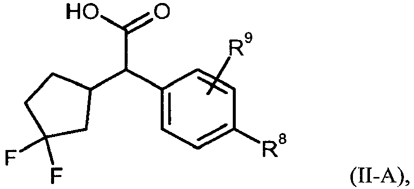

in welcher R⁸ und R⁹ die oben angegebenen Bedeutungen haben,
können alternativ auch dadurch hergestellt werden, dass man den Phenylessigsäureester der Formel (VIII) 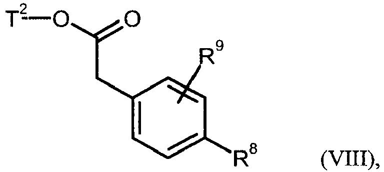 in welcher R⁸, R⁹ und T² die oben angegebenen Bedeutungen haben,
zunächst durch baseninduzierte Addition an 2-Cyclopenten-1-on zu einer Verbindung der Formel (X) 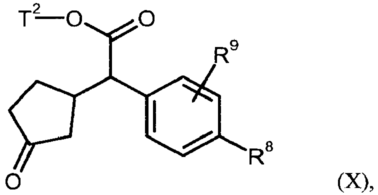 in welcher R⁸, R⁹ und T² die oben angegebenen Bedeutungen haben,
umsetzt, diese anschließend mit 1,1'-[(Trifluor-λ⁴-sulfanyl)imino]bis(2-methoxyethan) unter Bortrifluorid-Katalyse zu einer Verbindung der Formel (VII-A) 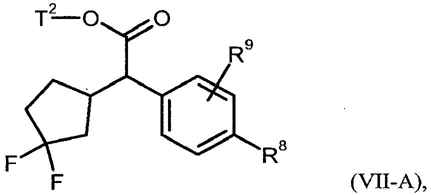 in welcher R⁸, R⁹ und T² die oben angegebenen Bedeutungen haben,
fluoriert und nachfolgend wiederum die Ester-Gruppe T² unter Erhalt der Carbonsäure (II-A) abspaltet.

Im Verfahrensschritt (VIII) → (X) wird zur Deprotonierung des Esters (VIII) bevorzugt eine Amid-Base wie Lithiumdiisopropylamid oder Lithium-bis(trimethylsilyl)amid verwendet. Zur Deoxy-Fluorierung in der Transformation (X) → (VII-A) können an Stelle des oben genannten 1,1'-[(Trifluor-X⁴-sulfanyl)imino]bis(2-methoxyethan) ("Desoxofluor") gegebenenfalls auch andere bekannte Fluorierungsagentien, wie Diethylaminoschwefeltrifluorid (DAST) oder Morpholinoschwefeltrifluorid (Morpho-DAST), eingesetzt werden [zur Reaktionssequenz (VIII) → (X) → (VII-A) vgl. z.B. T. Mase et al., J. Org. Chem. 66 (20), 6775-6786 (2001)].

Die Intermediate der Formel (III) können in Abhängigkeit von der Natur des Restes R² beispielsweise dadurch hergestellt werden, dass man einen Carbonsäureester der Formel (XI) 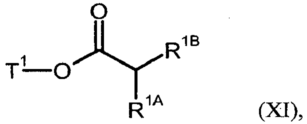 in welcher R^{1A}, R^{1B} und T¹ die oben angegebenen Bedeutungen haben,
in einem inerten Lösungsmittel nach α-Deprotonierung entweder
[C] mit einer 3-Brombenzyl-Verbindung der Formel (XII) 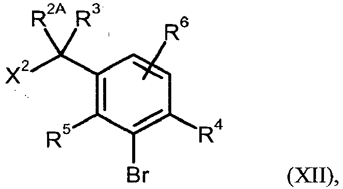 in welcher R³, R⁴, R⁵ und R⁶ die oben angegebenen Bedeutungen haben,
   - R^{2A}: für Wasserstoff, Methyl, Ethyl oder Vinyl steht
   und
   - X²: für eine geeignete Abgangsgruppe wie Chlor, Brom, Iod, Mesylat, Triflat oder Tosylat steht,
   zu einer Verbindung der Formel (XIII) 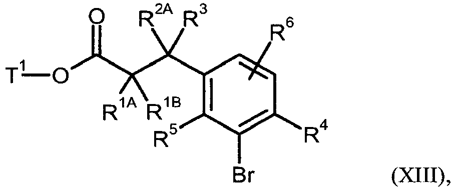 in welcher R^{1A}, R^{1B}, R^{2A}, R³, R⁴, R⁵, R⁶ und T¹ die oben angegebenen Bedeutungen haben,
   alkyliert, anschließend mit Benzylamin in Gegenwart einer Base und eines Palladium-Katalysators zu einer Verbindung der Formel (XIV) 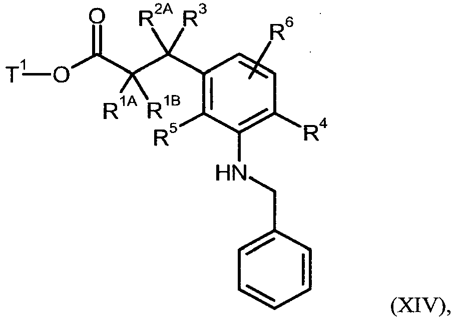 in welcher R^{1A}, R^{1B}, R^{2A}, R³, R⁴, R⁵, R⁶ und T¹ die oben angegebenen Bedeutungen haben,
   umsetzt und die N-Benzylgruppe dann durch Hydrogenolyse unter Erhalt eines 3-Amino-phenyl-Derivats der Formel (III-A) 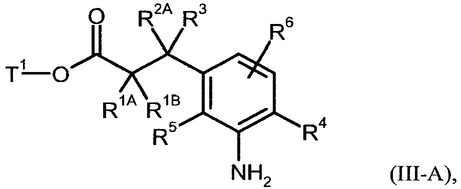 in welcher R^{1A}, R^{1B}, R^{2A}, R³, R⁴, R⁵, R⁶ und T¹ die oben angegebenen Bedeutungen haben, entfernt
   oder
[D] mit einer 3-Brombenzoyl-Verbindung der Formel (XV) 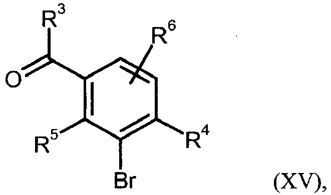 in welcher R³, R⁴, R⁵ und R⁶ die oben angegebenen Bedeutungen haben,
   zu einer Verbindung der Formel (XVI) 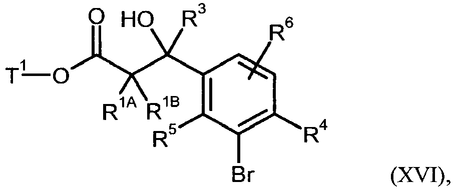 in welcher R^{1A}, R^{1B}, R³, R⁴, R⁵, R⁶ und T¹ die oben angegebenen Bedeutungen haben,
   umsetzt, diese nachfolgend, falls gewünscht, mit einer Verbindung der Formel (XVII)

   R¹⁰-X³ (XVII),

   in welcher
   - R¹⁰: für Methyl, Trideuteromethyl, Trifluormethyl, Ethyl oder Cyclopropyl steht
   und
   - X³: für eine geeignete Abgangsgruppe wie Chlor, Brom, Iod, Mesylat, Triflat oder Tosylat steht,
   in Gegenwart einer Base zu einer Verbindung der Formel (XVIII) 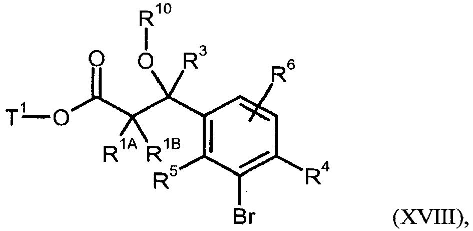 in welcher R^{1A}, R^{1B}, R³, R⁴, R⁵, R⁶, R¹⁰ und T¹ die oben angegebenen Bedeutungen haben,
   alkyliert, die Verbindung der Formel (XVI) bzw. (XVIII) dann in Analogie zur unter [C] beschriebenen Reaktionssequenz mit Benzylamin unter Palladium-Katalyse zu einer Verbindung der Formel (XIX) 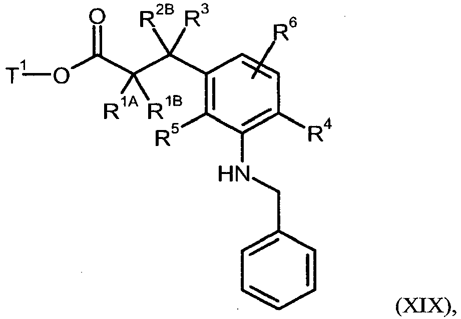 in welcher R^{1A}, R^{1B}, R³, R⁴, R⁵, R⁶ und T¹ die oben angegebenen Bedeutungen haben
   und
   - R^{2B}: für Hydroxy, Methoxy, Trideuteromethoxy, Trifluormethoxy, Ethoxy oder Cyclopropyloxy steht,
   umsetzt und schließlich die N-Benzylgruppe durch Hydrogenolyse unter Erhalt eines 3-Aminophenyl-Derivats der Formel (III-B) 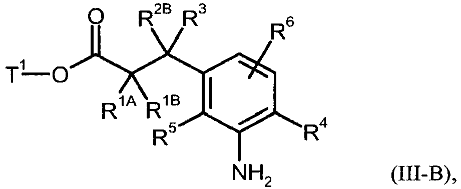 in welcher R^{1A}, R^{1B}, R^{2B}, R³, R⁴, R⁵, R⁶ und T¹ die oben angegebenen Bedeutungen haben,
   entfernt.

Zur α-Deprotonierung des Carbonsäureesters (XI) bei den Umsetzungen (XI) + (XII) → (XIII) und (XI) + (XV) → (XVI) eignen sich insbesondere nicht-nukleophile, starke Basen wie beispielsweise Natrium- oder Kalium-*tert*.-butylat, Natrium- oder Kaliumlaydrid, Lithiumdiisopropylamid oder Lithium-, Natrium- oder Kalium-bis(trimethylsilyl)amid; bevorzugt wird Lithiumdiisopropylamid verwendet. Als inertes Lösungsmittel werden bei diesen Reaktionen vorzugsweise Ether wie Diethylether, Diisopropylether, Methyl-*tert*.-butylether, Tetrahydrofuran, Glykoldimethylether oder Diethylenglykoldimethylether eingesetzt. Die Umsetzungen erfolgen üblicherweise in einem Temperaturbereich von -80°C bis +25°C.

Für die Transformationen (XIII) → (XIV) sowie (XVI) bzw. (XVIII) → (XIX) [Buchwald-Hartwig-Kupplung mit Benzylamin] wird bevorzugt Tris(dibenzylidenaceton)dipalladium(0) als Katalysator in Verbindung mit (±)-2,2'-Bis(d,iphenylphosphino)-1,1'-binaphthyl als Phosphin-Liganden und Natrium- oder Kalium-*tert*.-butylat als Base verwendet [vgl. z.B. J. P. Wolfe und S. L. Buchwald, Organic Syntheses, Coll. Vol. 10, 423 (2004), Vol. 78, 23 (2002)].

Die hydrogenolytische Entfernung der *N*-Benzylgruppe in den Verfahrensschritten (XIV) → (III-A) und (XIX) → (III-B) wird in der Regel unter einer stationären Wasserstoffatmosphäre bei normalem Druck durchgeführt. Als Katalysator wird hierbei bevorzugt Palladium auf Aktivkohle (als Trägermaterial) eingesetzt.

Als Base für die Alkylierungsreaktion (XVI) + (XVII) → (XVIII) eignen sich gleichfalls übliche starke, nicht-nukleophile Basen wie Natrium- oder Kalium-*tert*.-butylat, Natrium- oder Kaliumhydrid, Lithium-, Natrium- oder Kalium-bis(trimethylsilyl)amid oder Lithiumdiisopropylamid; bevorzugt wird hier Natriumhydrid verwendet. Als inerte Lösungsmittel für diese Reaktion sind insbesondere Ether, wie Diethylether, Diisopropylether, Methyl-*tert*.-butylether, Tetrahydrofuran, Glykoldimethylether oder Diethylenglykoldimethylether, oder dipolar-aprotische Solventien, wie *N,N*-Dimethylformamid (DMF), Dimethylsulfoxid (DMSO), *N*-Methylpyrrolidinon (NMP) oder *N,N'*-Dimethylpropylenharnstoff (DMPU), geeignet; bevorzugt wird *N,N-*Dimethylfortnamid eingesetzt. Die Umsetzung erfolgt in der Regel in einem Temperaturbereich von -20°C bis +40°C.

Die zuvor beschriebenen Reaktionen können bei normalem, bei erhöhtem oder bei erniedrigtem Druck durchgeführt werden (z.B. im Bereich von 0.5 bis 5 bar); im Allgemeinen arbeitet man jeweils bei Normaldruck.

Eine Trennung der erfindungsgemäßen Verbindungen in die entsprechenden Enantiomere und/ oder Diastereomere kann gegebenenfalls, je nach Zweckmäßigkeit, auch bereits auf der Stufe der Verbindungen (II), (III), (IV), (VII), (XIII), (XIV), (XVI), (XVIII) oder (XIX) erfolgen, welche dann in separierter Form entsprechend den zuvor beschriebenen Verfahrenssequenzen weiter umgesetzt werden. Eine solche Auftrennung der Stereoisomeren läßt sich nach üblichen, dem Fachmann bekannten Methoden durchführen. Vorzugsweise werden chromatographische Verfahren an achiralen bzw. chiralen Trennphasen angewandt; im Falle von Carbonsäuren als Zwischen- oder Endprodukten kann alternativ auch eine Trennung über diastereomere Salze erfolgen.

Die Verbindungen der Formeln (V), (VI), (VIII), (IX), (XI), (XII), (XV) und (XVII) sind entweder kommerziell erhältlich oder als solche in der Literatur beschrieben, oder sie können auf für den Fachmann offenkundigem Wege in Analogie zu in der Literatur publizierten Methoden hergestellt werden. Zahlreiche detaillierte Vorschriften sowie Literaturangaben zur Herstellung der Ausgangsmaterialien befinden sich auch im Experimentellen Teil im Abschnitt zur Herstellung der Ausgangsverbindungen und Intermediate.

Die Herstellung der erfindungsgemäßen Verbindungen kann durch die folgenden Reaktionsschemata beispielhaft veranschaulicht werden:

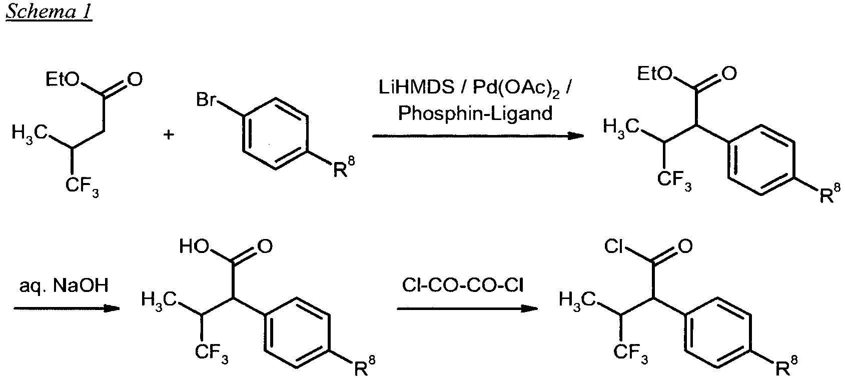

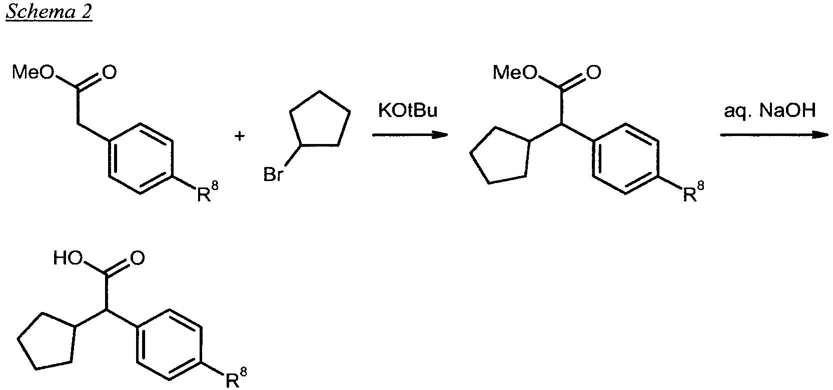

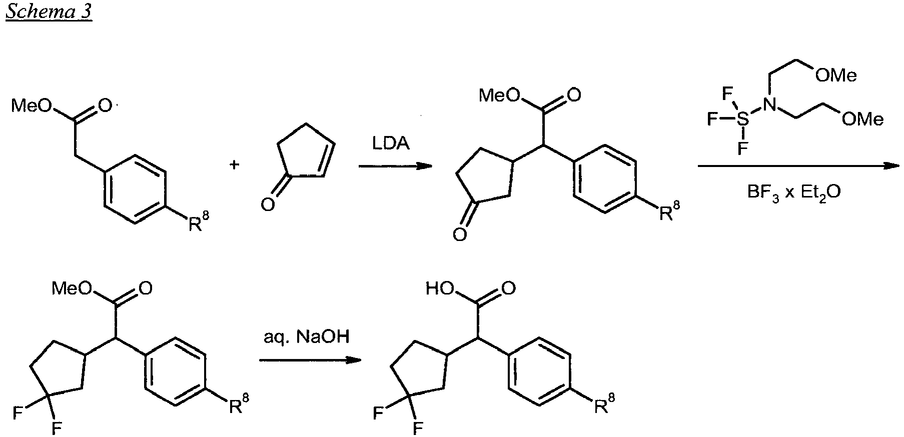

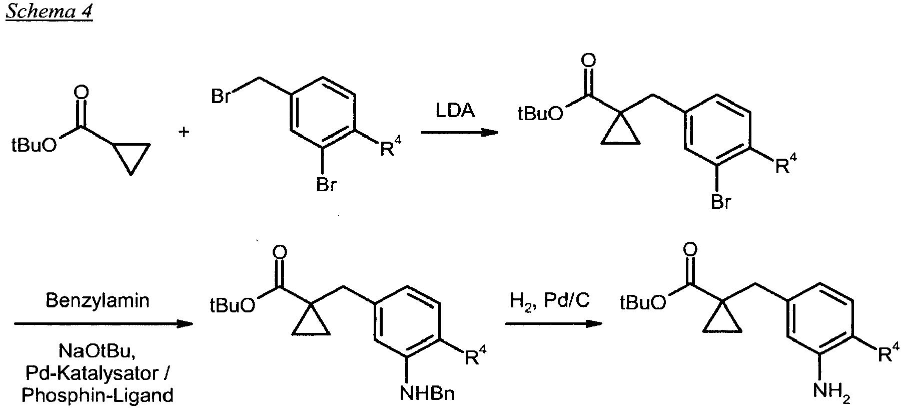

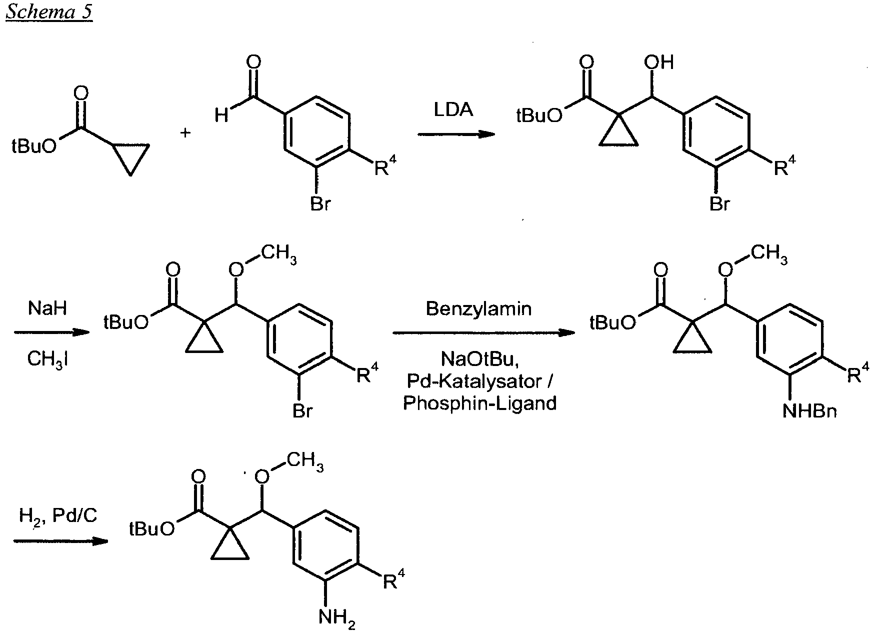

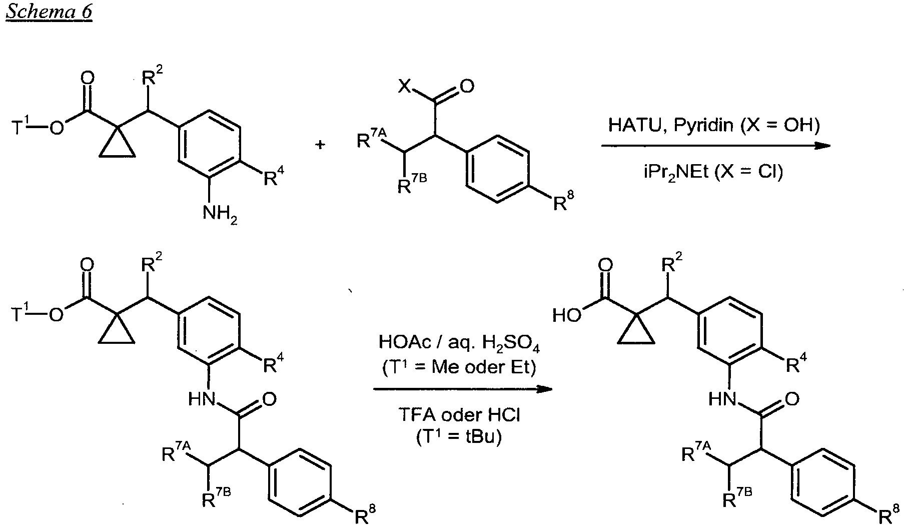

Die erfindungsgemäßen Verbindungen besitzen wertvolle pharmakologische Eigenschaften und können zur Vorbeugung und Behandlung von Erkrankungen bei Menschen und Tieren verwendet werden.

Die erfindungsgemäßen Verbindungen stellen potente Aktivatoren der löslichen Guanylatcyclase dar. Sie führten zu einer Gefäßrelaxation, zu einer Thrombozytenaggregationshemmung und zu einer Blutdrucksenkung sowie zu einer Steigerung des koronaren Blutflusses. Diese Wirkungen sind über eine direkte, Härn-unabhängige Aktivierung der löslichen Guanylatcyclase und einen intrazellulären cGMP-Anstieg vermittelt.

Darüber hinaus verfügen die erfindungsgemäßen Verbindungen über gute pharmakokinetische Eigenschaften, insbesondere bezüglich ihrer Bioverfügbarkeit und/oder Wirkdauer nach intravenöser oder oraler Gabe.

Die erfindungsgemäßen Verbindungen eignen sich in besonderem Maße zur Behandlung und/oder Prävention von kardiovaskulären, pulmonalen, thromboembolischen und fibrotischen Erkrankungen.

Die erfindungsgemäßen Verbindungen können daher in Arzneimitteln eingesetzt werden zur Behandlung und/oder Prävention von kardiovaskulären Erkrankungen, wie beispielsweise Bluthochdruck (Hypertonie), Herzinsuffizienz, koronare Herzerkrankung, stabile und instabile Angina pectoris, pulmonale arterielle Hypertonie (PAH) und andere Formen der pulmonalen Hypertonie (PH), renale Hypertonie, periphere und kardiale Gefäßerkrankungen, Arrhythmien, Rhythmusstörungen der Vorhöfe und der Kammern sowie Überleitungsstörungen wie beispielsweise atrioventrikuläre Blockaden des Grades I-III, supraventrikuläre Tachyarrhythmie, Vorhofflimmern, Vorhofflattern, Kammerflimmern, Kammerflattern, ventrikuläre Tachyarrhythmie, Torsade de pointes-Tachykardie, Extrasystolen des Vorhofs und des Ventrikels, AV-junktionale Extrasystolen, Sick-Sinus-Syndrom, Synkopen, AV-Knoten-Reentry-Tachykardie, Wolff-Parkinson-White-Syndrom, akutes Koronarsyndrom (ACS), autoimmune Herzerkrankungen (Perikarditis, Endokarditis, Valvolitis, Aortitis, Kardiomyopathien), Boxerkardiomyopathie, Aneurysmen, Schock wie kardiogener Schock, septischer Schock und anaphylaktischer Schock, ferner zur Behandlung und/oder Prävention von thromboembolischen Erkrankungen und Ischämien, wie myokardiale Ischämie, Myokardinfarkt, Hirnschlag, Herzhypertrophie, transistorische und ischämische Attacken, Präeklampsie, entzündliche kardiovaskuläre Erkrankungen, Spasmen der Koronararterien und peripherer Arterien, Ödembildung wie beispielsweise pulmonales Ödem, Hirnödem, renales Ödem oder Herzinsuffizienz-bedingtes Ödem, periphere Durchblutungsstörungen, Reperfusionsschäden, arterielle und venöse Thrombosen, Mikroalbuminurie, Herzmuskelschwäclve, endotheliale Dysfunktion, mikro- und makrovaskuläre Schädigungen (Vaskulitis), zur Verhinderung von Restenosen beispielsweise nach Thrombolyse-Therapien, percutan-transluminalen Angioplastien (PTA), percutan-transluminalen Koronarangioplastien (PTCA), Herztransplantationen und Bypass-Operationen, sowie zur Behandlung und/oder Prävention von Arteriosklerose.

Im Sinne der vorliegenden Erfindung umfasst der Begriff Herzinsuffizienz sowohl akute als auch chronische Erscheinungsformen der Herzinsuffizienz wie auch spezifische oder verwandte Krankheitsformen hiervon, wie akute dekompensierte Herzinsuffizienz, Rechtsherzinsuffizienz, Linksherzinsuffizienz, Globalinsuffizienz, ischämische Kardiomyopathie, dilatative Kardiomyopathie, hypertrophe Kardiomyopathie, idiopathische Kardiomyopathie, angeborene Herzfehler, Herzklappenfehler, Herzinsuffizienz bei Herzklappenfehlern, Mitralklappenstenose, Mitralklappeninsuffizienz, Aortenklappenstenose, Aortenklappeninsuffizienz, Trikuspidalstenose, Trikuspidalinsuffizienz, Pulmonalklappenstenose, Pulmonalklappeninsuffizienz, kombinierte Herzklappenfehler, Herzmuskelentzündung (Myokarditis), chronische Myokarditis, akute Myokarditis, virale Myokarditis, diabetische Herzinsuffizienz, alkoholtoxische Kardiomyopathie, kardiale Speichererkrankungen sowie diastolische und systolische Herzinsuffizienz.

Weiterhin können die erfindungsgemäßen Verbindungen zur Behandlung und/oder Prävention von primärem und sekundärem Raynaud-Phänomen, Mikrozirkulationsstörungen, Claudicatio, Tinnitus, peripheren und autonomen Neuropathien, diabetischen Mikroangiopathien, diabetischer Retinopathie, diabetischen Geschwüren an den Extremitäten, Gangrän, CREST-Syndrom, Erythematose, Onychomykose sowie von rheumatischen Erkrankungen verwendet werden.

Die erfindungsgemäßen Verbindungen können darüber hinaus zur Verhinderung von ischämie- und/oder reperfusionsbedingten Schädigungen von Organen oder Geweben sowie als Zusatzstoffe für Perfusions- und Konservierungslösungen von Organen, Organteilen, Geweben oder Gewebeteilen menschlichen oder tierischen Ursprungs, insbesondere bei chirurgischen Eingriffen oder im Bereich der Transplantationsmedizin, Verwendung finden.

Die erfindungsgemäßen Verbindungen eignen sich außerdem zur Behandlung und/oder Prävention von Nierenerkrankungen, insbesondere von Niereninsuffizienz und Nierenversagen. Im Sinne der vorliegenden Erfindung umfassen die Begriffe Niereninsuffizienz und Nierenversagen sowohl akute als auch chronische Erscheinungsformen hiervon wie auch diesen zugrundeliegende oder verwandte Nierenerkrankungen, wie renale Hypoperfusion, intradialytische Hypotonie, obstruktive Uropathie, Glomerulopathien, Glomerulonephritis, akute Glomerulonephritis, Glomerulosklerose, tubulointerstitielle Erkrankungen, nephropathische Erkrankungen wie primäre und angeborene Nierenerkrankung, Nierenentzündung, immunologische Nierenerkrankungen wie Nierentransplantat-Abstoßung und Immunkomplex-induzierte Nierenerkrankungen, durch toxische Substanzen induzierte Nephropathie, Kontrastmittel-induzierte Nephropathie, diabetische und nicht-diabetische Nephropathie, Pyelonephritis, Nierenzysten, Nephrosklerose, hypertensive Nephrosklerose und nephrotisches Syndrom, welche diagnostisch beispielsweise durch abnorm verminderte Kreatinin- und/oder Wasser-Ausscheidung, abnorm erhöhte Blutkonzentrationen von Harnstoff, Stickstoff, Kalium und/oder Kreatinin, veränderte Aktivität von Nierenenzymen wie z.B. Glutamylsynthetase, veränderte Urinosmolarität oder Urinmenge, erhöhte Mikroalbuminurie, Makroalbuminurie, Läsionen an Glomerula und Arteriolen, tubuläre Dilatation, Hyperphosphatämie und/oder die Notwendigkeit zur Dialyse charakterisiert werden können. Die vorliegende Erfindung umfasst auch die Verwendung der erfindungsgemäßen Verbindungen zur Behandlung und/oder Prävention von Folgeerscheinungen einer Niereninsuffizienz, wie beispielsweise Hypertonie, Lungenödem, Herzinsuffizienz, Urämie, Anämie, Elektrolytstörungen (z.B. Hyperkalämie, Hyponaträmie) und Störungen im Knochen- und Kohlenhydrat-Metabolismus.

Darüber hinaus sind die erfindungsgemäßen Verbindungen zur Behandlung und/oder Prävention von Erkrankungen des Urogenitalsystems geeignet, wie beispielsweise von überaktiver Blase, Blasenentleerungsstörungen, unterem Harnwegssyndrom (LUTS), Inkontinenz, benigner Prostatahyperplasie (BPH), erektiler Dysfunktion und weiblicher sexueller Dysfunktion.

Die erfindungsgemäßen Verbindungen eignen sich auch zur Behandlung und/oder Prävention von asthmatischen Erkrankungen, chronisch-obstruktiven Atemwegserkrankungen (COPD), des akuten Atemwegssyndroms (ARDS) und der akuten Lungenschädigung (ALI), alpha-1-Antitrypsin-Defizienz (AATD), Lungenfibrose, Lungenemphysem (z.B. durch Zigarettenrauch induziertes Lungenemphysem) und zystischer Fibrose (CF) sowie von pulmonaler arterieller Hypertonie (PAH) und anderen Formen der pulmonalen Hypertonie (PH), einschließlich der mit Linksherzerkrankung, HIV, Sichelzellanämie, Thromboembolien, Sarkoidose, COPD oder Lungenfibrose assoziierten pulmonalen Hypertonie.

Die in der vorliegenden Erfindung beschriebenen Verbindungen stellen auch Wirkstoffe zur Bekämpfung von Krankheiten im Zentralnervensystem dar, die durch Störungen des NO/cGMP-Systems gekennzeichnet sind. Insbesondere sind sie geeignet zur Verbesserung der Wahrnehmung, Konzentrationsleistung, Lernleistung oder Gedächtnisleistung nach kognitiven Störungen, wie sie insbesondere bei Situationen/Krankheiten/Syndromen auftreten wie "Mild cognitive impairment", altersassozüerten Lern- und Gedächtnisstörungen, altersassoziierten Gedächtnisverlusten, vaskulärer Demenz, Schädel-Hirn-Trauma, Schlaganfall, Demenz, die nach Schlaganfallen auftritt ("post stroke dementia"), post-traumatischem Schädel-Hirn-Trauma, allgemeinen Konzentrationsstörungen, Konzentrationsstörungen bei Kindern mit Lern- und Gedächtnisproblemen, Alzheimer'scher Krankheit, Demenz mit Lewy-Körperchen, Demenz mit Degeneration der Frontallappen einschliesslich des Pick's-Syndroms, Parkinson'scher Krankheit, progressiver nuclear palsy, Demenz mit corticobasaler Degeneration, Amyolateralsklerose (ALS), Huntington'scher Krankheit, Multipler Sklerose, Thalamischer Degeneration, Creutzfeld-Jacob-Demenz, HIV-Demenz, Schizophrenie mit Demenz oder Korsakoff-Psychose. Sie eignen sich auch zur Behandlung von Erkrankungen des Zentralnervensystems wie Angst-, Spannungs- und Depressionszuständen, zentral-nervös bedingten Sexualdysfunktionen und Schlafstörungen sowie zur Regulierung krankhafter Störungen der Nahrungs-, Genuss- und Suchtmittelaufnahme.

Weiterhin eignen sich die erfindungsgemäßen Verbindungen auch zur Regulation der cerebralen Durchblutung und stellen wirkungsvolle Mittel zur Bekämpfung von Migräne dar. Auch eignen sie sich zur Prophylaxe und Bekämpfung der Folgen cerebraler Infarktgeschehen (Apoplexia cerebri) wie Schlaganfall, cerebraler Ischämien und des Schädel-Hirn-Traumas. Ebenso können die erfindungsgemäßen Verbindungen zur Bekämpfung von Schmerzzuständen eingesetzt werden.

Zudem besitzen die erfindungsgemäßen Verbindungen anti-inflammatorische Wirkung und können daher als entzündungshemmende Mittel zur Behandlung und/oder Prävention von Sepsis, multiplem Organversagen, entzündlichen Erkrankungen der Niere, rheumatoiden Erkrankungen, entzündlichen Hauterkrankungen und entzündlichen Augenerkrankungen eingesetzt werden.

Die erfindungsgemäßen Verbindungen sind ferner zur Behandlung und/oder Prävention von fibrotischen Erkrankungen der inneren Organe, wie beispielsweise der Lunge, des Herzens, der Niere, des Knochenmarks und insbesondere der Leber, sowie von dermatologischen Fibrosen und fibrotischen Erkrankungen des Auges geeignet. Im Sinne der vorliegenden Erfindung umfasst der Begriff fibrotische Erkrankungen insbesondere solche Erkrankungen wie Leberfibrose, Leberzirrhose, Lungenfibrose, Endomyokardfibrose, Nephropathie, Glomerulonephritis, interstitielle Nierenfibrose, fibrotische Schäden in Folge von Diabetes, Knochenmarksfibrose und ähnliche fibrotische Erkrankungen, Sklerodermie, Morphaea, Keloide, hypertrophe Narbenbildung, Naevi, diabetische Retinopathie, proliferative Vitroretinopathie und Erkrankungen des Bindegewebes (z.B. Sarkoidose). Die erfindungsgemäßen Verbindungen können ebenso verwendet werden zur Förderung der Wundheilung, zur Bekämpfung postoperativer Narbenbildung, z.B. nach Glaukom-Operationen, und zu kosmetischen Zwecken bei alternder oder verhornender Haut.

Aufgrund ihres Wirkprofils eignen sich die erfindungsgemäßen Verbindungen insbesondere zur Behandlung und/oder Prävention kardiovaskulärer Erkrankungen wie Herzinsuffizienz, Angina pectoris, Hypertonie und pulmonale Hypertonie, sowie von thromboembolischen Erkrankungen und Ischämien, Gefäßerkrankungen, Mikrozirkulationsstörungen, Niereninsuffizienz, fibrotischen Erkrankungen und Arteriosklerose.

Weiterer Gegenstand der vorliegenden Erfindung ist die Verwendung der erfindungsgemäßen Verbindungen zur Behandlung und/oder Prävention von Erkrankungen, insbesondere der zuvor genannten Erkrankungen.

Weiterer Gegenstand der vorliegenden Erfindung ist die Verwendung der erfindungsgemäßen Verbindungen zur Herstellung eines Arzneimittels zur Behandlung und/oder Prävention von Erkrankungen, insbesondere der zuvor genannten Erkrankungen.

Weiterer Gegenstand der vorliegenden Erfindung ist die Verwendung der erfindungsgemäßen Verbindungen in einem Verfahren zur Behandlung und/oder Prävention von Erkrankungen, insbesondere der zuvor genannten Erkrankungen.

Weiterer Gegenstand der vorliegenden Erfindung ist ein Verfahren zur Behandlung und/oder Prävention von Erkrankungen, insbesondere der zuvor genannten Erkrankungen, unter Verwendung einer wirksamen Menge von mindestens einer der erfindungsgemäßen Verbindungen.

Die erfindungsgemäßen Verbindungen können allein oder bei Bedarf in Kombination mit anderen Wirkstoffen eingesetzt werden. Weiterer Gegenstand der vorliegenden Erfindung sind Arzneimittel, enthaltend mindestens eine der erfindungsgemäßen Verbindungen und einen oder mehrere weitere Wirkstoffe, insbesondere zur Behandlung und/oder Prävention der zuvor genannten Erkrankungen. Als geeignete Kombinationswirkstoffe seien beispielhaft und vorzugsweise genannt:
- organische Nitrate und NO-Donatoren, wie beispielsweise Natriumnitroprussid, Nitroglycerin, Isosorbidmononitrat, Isosorbiddinitrat, Molsidomin oder SIN-1, sowie inhalatives NO;
- Verbindungen, die den Abbau von cyclischem Guanosinmonophosphat (cGMP) inhibieren, wie beispielsweise Inhibitoren der Phosphodiesterasen (PDE) 1, 2 und/oder 5, insbesondere PDE 5-Inhibitoren wie Sildenafil, Vardenafil und Tadalafil;
- NO-unabhängige, jedoch Häm-abhängige Stimulatoren der Guanylatcyclase, wie insbesondere Riociguat sowie die in WO 00/06568, WO 00/06569, WO 02/42301 und WO 03/095451 beschriebenen Verbindungen;
- antithrombotisch wirkende Mittel, beispielhaft und vorzugsweise aus der Gruppe der Thrombozytenaggregationshemmer, der Antikoagulantien oder der profibrinolytischen Substanzen;
- den Blutdruck senkende Wirkstoffe, beispielhaft und vorzugsweise aus der Gruppe der Calcium-Antagonisten, Angiotensin AII-Antagonisten, ACE-Hemmer, Endothelin-Antagonisten, Renin-Inhibitoren, alpha-Rezeptoren-Blocker, beta-Rezeptoren-Blocker, Mineralocorticoid-Rezeptor-Antagonisten sowie der Diuretika; und/oder
- den Fettstoffwechsel verändernde Wirkstoffe, beispielhaft und vorzugsweise aus der Gruppe der Thyroidrezeptor-Agonisten, Cholesterinsynthese-Inhibitoren wie beispielhaft und vorzugsweise HMG-CoA-Reduktase- oder Squalensynthese-Inhibitoren, der ACAT-Inhibitoren, CETP-Inhibitoren, MTP-Inhibitoren, PPAR-alpha-, PPAR-gamma- und/oder PPAR-delta-Agonisten, Cholesterin-Absorptionshemmer, Lipase-Inhibitoren, polymeren Gallensäureadsorber, Gallensäure-Reabsorptionshemmer und Lipoprotein(a)-Antagonisten.

Unter antithrombotisch wirkenden Mittel werden vorzugsweise Verbindungen aus der Gruppe der Thrombozytenaggregationshemmer, der Antikoagulantien oder der profibrinolytischen Substanzen verstanden.

Bei einer bevorzugten Ausführungsform der Erfindung werden die erfindungsgemäßen Verbindungen in Kombination mit einem Thrombozytenaggregationshemmer, wie beispielhaft und vorzugsweise Aspirin, Clopidogrel, Ticlopidin oder Dipyridamol, verabreicht.

Bei einer bevorzugten Ausführungsform der Erfindung werden die erfindungsgemäßen Verbindungen in Kombination mit einem Thrombin-Inhibitor, wie beispielhaft und vorzugsweise Ximelagatran, Melagatran, Dabigatran, Bivalirudin oder Clexane, verabreicht.

Bei einer bevorzugten Ausführungsform der Erfindung werden die erfindungsgemäßen Verbindungen in Kombination mit einem GPIIb/IIIa-Antagonisten, wie beispielhaft und vorzugsweise Tirofiban oder Abciximab, verabreicht.

Bei einer bevorzugten Ausführungsform der Erfindung werden die erfindungsgemäßen Verbindungen in Kombination mit einem Faktor Xa-Inhibitor, wie beispielhaft und vorzugsweise Rivaroxaban, Apixaban, Fidexaban, Razaxaban, Fondaparinux, Idraparinux, DU-176b, PMD-3112, YM-150, KFA-1982, EMD-503982, MCM-17, MLN-1021, DX 9065a, DPC 906, JTV 803, SSR-126512 oder SSR-128428, verabreicht.

Bei einer bevorzugten Ausführungsform der Erfindung werden die erfindungsgemäßen Verbindungen in Kombination mit Heparin oder einem low molecular weight (LMW)-Heparin-Derivat verabreicht.

Bei einer bevorzugten Ausführungsform der Erfindung werden die erfindungsgemäßen Verbindungen in Kombination mit einem Vitamin K-Antagonisten, wie beispielhaft und vorzugsweise Coumarin, verabreicht.

Unter den Blutdruck senkenden Mitteln werden vorzugsweise Verbindungen aus der Gruppe der Calcium-Antagonisten, Angiotensin AII-Antagonisten, ACE-Hemmer, Endothelin-Antagonisten, Renin-Inhibitoren, alpha-Rezeptoren-Blocker, beta-Rezeptoren-Blocker, Mineralocorticoid-Rezeptor-Antagonisten sowie der Diuretika verstanden.

Bei einer bevorzugten Ausführungsform der Erfindung werden die erfindungsgemäßen Verbindungen in Kombination mit einem Calcium-Antagonisten, wie beispielhaft und vorzugsweise Nifedipin, Amlodipin, Verapamil oder Diltiazem, verabreicht.

Bei einer bevorzugten Ausführungsform der Erfindung werden die erfindungsgemäßen Verbindungen in Kombination mit einem alpha-1-Rezeptoren-Blocker, wie beispielhaft und vorzugsweise Prazosin, verabreicht.

Bei einer bevorzugten Ausuhrungsform der Erfindung werden die erfindungsgemäßen Verbindungen in Kombination mit einem beta-Rezeptoren-Blocker, wie beispielhaft und vorzugsweise Propranolol, Atenolol, Timolol, Pindolol, Alprenolol, Oxprenolol, Penbutolol, Bupranolol, Metipranolol, Nadolol, Mepindolol, Carazalol, Sotalol, Metoprolol, Betaxolol, Celiprolol, Bisoprolol, Carteolol, Esmolol, Labetalol, Carvedilol, Adaprolol, Landiolol, Nebivolol, Epanolol oder Bucindolol, verabreicht.

Bei einer bevorzugten Ausführungsform der Erfindung werden die erfindungsgemäßen Verbindungen in Kombination mit einem Angiotensin AII-Antagonisten, wie beispielhaft und vorzugsweise Losartan, Candesartan, Valsartan, Telmisartan oder Embursatan, verabreicht.

Bei einer bevorzugten Ausführungsform der Erfindung werden die erfindungsgemäßen Verbindungen in Kombination mit einem ACE-Hemmer, wie beispielhaft und vorzugsweise Enalapril, Captopril, Lisinopril, Ramipril, Delapril, Fosinopril, Quinopril, Perindopril oder Trandopril, verabreicht.

Bei einer bevorzugten Ausführungsform der Erfindung werden die erfindungsgemäßen Verbindungen in Kombination mit einem Endothelin-Antagon isten, wie beispielhaft und vorzugsweise Bosentan, Darusentan, Ambrisentan oder Sitaxsentan, verabreicht.

Bei einer bevorzugten Ausführungsform der Erfindung werden die erfindungsgemäßen Verbindungen in Kombination mit einem Renin-Inhibitor, wie beispielhaft und vorzugsweise Aliskiren, SPP-600 oder SPP-800, verabreicht.

Bei einer bevorzugten Ausführungsform der Erfindung werden die erfindungsgemäßen Verbindungen in Kombination mit einem Mineralocorticoid-Rezeptor-Antagonisten, wie beispielhaft und vorzugsweise Spironolacton oder Eplerenon, verabreicht.

Bei einer bevorzugten Ausführungsform der Erfindung werden die erfindungsgemäßen Verbindungen in Kombination mit einem Diuretikum, wie beispielhaft und vorzugsweise Furosemid, Bumetanid, Torsemid, Bendroflumethiazid, Chlorthiazid, Hydrochlorthiazid, Hydroflumethiazid, Methyclothiazid, Polythiazid, Trichlormethiazid, Chlorthalidon, Indapamid, Metolazon, Quinethazon, Acetazolamid, Dichlorphenamid, Methazolamid, Glycerin, Isosorbid, Mannitol, Amilorid oder Triamteren, verabreicht.

Unter den Fettstoffwechsel verändernden Mitteln werden vorzugsweise Verbindungen aus der Gruppe der CETP-Inhibitoren, Thyroidrezeptor-Agonisten, Cholesterinsynthese-Inhibitoren wie HMG-CoA-Reduktase- oder Squalensynthese-Inhibitoren, der ACAT-Inhibitoren, MTP-Inhibitoren, PPAR-alpha-, PPAR-gamma- und/oder PPAR-delta-Agonisten, Cholesterin-Absorptionshemmer, polymeren Gallensäureadsorber, Gallensäure-Reabsorptionshemmer, Lipase-Inhibitoren sowie der Lipoprotein(a)-Antagonisten verstanden.

Bei einer bevorzugten Ausführungsform der Erfindung werden die erfindungsgemäßen Verbindungen in Kombination mit einem CETP-Inhibitor, wie beispielhaft und vorzugsweise Torcetrapib (CP-529 414), JJT-705 oder CETP-vaccine (Avant), verabreicht.

Bei einer bevorzugten Ausführungsform der Erfindung werden die erfindungsgemäßen Verbindungen in Kombination mit einem Thyroidrezeptor-Agonisten, wie beispielhaft und vorzugsweise D-Thyroxin, 3,5,3'-Triiodothyronin (T3), CGS 23425 oder Axitirome (CGS 26214), verabreicht.

Bei einer bevorzugten Ausführungsform der Erfindung werden die erfindungsgemäßen Verbindungen in Kombination mit einem HMG-CoA-Reduktase-Inhibitor aus der Klasse der Statine, wie beispielhaft und vorzugsweise Lovastatin, Simvastatin, Pravastatin, Fluvastatin, Atorvastatin, Rosuvastatin oder Pitavastatin, verabreicht.

Bei einer bevorzugten Ausführungsform der Erfindung werden die erfindungsgemäßen Verbindungen in Kombination mit einem Squalensynthese-Inhibitor, wie beispielhaft und vorzugsweise BMS-188494 oder TAK-475, verabreicht.

Bei einer bevorzugten Ausführungsform der Erfindung werden die erfindungsgemäßen Verbindungen in Kombination mit einem ACAT-Inhibitor, wie beispielhaft und vorzugsweise Avasimibe, Melinamide, Pactimibe, Eflucimibe oder SMP-797, verabreicht.

Bei einer bevorzugten Ausführungsform der Erfindung werden die erfindungsgemäßen Verbindungen in Kombination mit einem MTP-Inhibitor, wie beispielhaft und vorzugsweise Implitapide, BMS-201038, R-103757 oder JTT-130, verabreicht.

Bei einer bevorzugten Ausführungsform der Erfindung werden die erfindungsgemäßen Verbindungen in Kombination mit einem PPAR-gamma-Agonisten, wie beispielhaft und vorzugsweise Pioglitazone oder Rosiglitazone, verabreicht.

Bei einer bevorzugten Ausführungsform der Erfindung werden die erfindungsgemäßen Verbindungen in Kombination mit einem PPAR-delta-Agonisten, wie beispielhaft und vorzugsweise GW 501516 oder BAY 68-5042, verabreicht.

Bei einer bevorzugten Ausführungsform der Erfindung werden die erfindungsgemäßen Verbindungen in Kombination mit einem Cholesterin-Absorptionshemmer, wie beispielhaft und vorzugsweise Ezetimibe, Tiqueside oder Pamaqueside, verabreicht.

Bei einer bevorzugten Ausführungsform der Erfindung werden die erfindungsgemäßen Verbindungen in Kombination mit einem Lipase-Inhibitor, wie beispielhaft und vorzugsweise Orlistat, verabreicht.

Bei einer bevorzugten Ausführungsform der Erfindung werden die erfindungsgemäßen Verbindungen in Kombination mit einem polymeren Gallensäureadsorber, wie beispielhaft und vorzugsweise Cholestyramin, Colestipol, Colesolvam, CholestaGel oder Colestimid, verabreicht.

Bei einer bevorzugten Ausführungsform der Erfindung werden die erfindungsgemäßen Verbindungen in Kombination mit einem Gallensäure-Reabsorptionshemmer, wie beispielhaft und vorzugsweise ASBT (= IBAT)-Inhibitoren wie z.B. AZD-7806, S-8921, AK-105, BARI-1741, SC-435 oder SC-635, verabreicht.

Bei einer bevorzugten Ausführungsform der Erfindung werden die erfindungsgemäßen Verbindungen in Kombination mit einem Lipoprotein(a)-Antagonisten, wie beispielhaft und vorzugsweise Gemcabene calcium (CI-1027) oder Nicotinsäure, verabreicht.

Weiterer Gegenstand der vorliegenden Erfindung sind Arzneimittel, die mindestens eine erfindungsgemäße Verbindung, üblicherweise zusammen mit einem oder mehreren inerten, nichttoxischen, pharmazeutisch geeigneten Hilfsstoffen enthalten, sowie deren Verwendung zu den zuvor genannten Zwecken.

Die erfindungsgemäßen Verbindungen können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, otisch oder als Implantat bzw. Stent.

Für diese Applikationswege können die erfindungsgemäßen Verbindungen in geeigneten Applikationsformen verabreicht werden.

Für die orale Applikation eignen sich nach dem Stand der Technik funktionierende, die erfindungsgemäßen Verbindungen schnell und/oder modifiziert abgebende Applikationsformen, die die erfindungsgemäßen Verbindungen in kristalliner und/oder amorphisierter und/oder gelöster Form enthalten, wie z.B. Tabletten (nicht-überzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer), Nasentropfen, -lösungen oder -sprays, lingual, sublingual oder buccal zu applizierende Tabletten, Filme/Oblaten oder Kapseln, Suppositorien, Ohren- oder Augenpräparationen, Vaginalkapseln, wäßrige Suspensionen (Lotionen, Schüttelmixturen), lipophile Suspensionen, Salben, Cremes, transdermale therapeutische Systeme (z.B. Pflaster), Milch, Pasten, Schäume, Streupuder, Implantate oder Stents.

Bevorzugt sind die orale oder parenterale Applikation, insbesondere die orale und die intravenöse Applikation.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Lactose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und/oder Geruchskorrigentien.

Im Allgemeinen hat es sich als vorteilhaft erwiesen, bei parenteraler Applikation Mengen von etwa 0.001 bis 1 mg/kg, vorzugsweise etwa 0.01 bis 0.5 mg/kg Körpergewicht zur Erzielung wirksamer Ergebnisse zu verabreichen. Bei oraler Applikation beträgt die Dosierung etwa 0.01 bis 100 mg/kg, vorzugsweise etwa 0.01 bis 20 mg/kg und ganz besonders bevorzugt 0.1 bis 10 mg/kg Körpergewicht.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die nachfolgenden Ausführungsbeispiele erläutern die Erfindung. Die Erfindung ist nicht auf die Beispiele beschränkt.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

### A. Beispiele

### Abkürzungen und Akronyme:

- abs.: absolut
- Ac: Acetyl
- AIBN: 2,2'-Azobis-(2-methylpropionitril)
- aq.: wässrig, wässrige Lösung
- ATP: Adenosin-5'-triphosphat
- Bn: Benzyl
- Brij^{®}: Polyethylenglycoldodecylether
- BSA: bovines Serumalbumin
- Bsp.: Beispiel
- Bu: Butyl
- c: Konzentration
- ca.: *circa,* ungefähr
- cat.: katalytisch
- CI: chemische Ionisation (bei MS)
- d: Tag(e)
- DAST: Diethylaminoschwefeltrifluorid
- DC: Dünnschichtchromatographie
- DCI: direkte chemische Ionisation (bei MS)
- DDQ: 2,3-Dichlor-5,6-dicyano-1,4-benzochinon
- de: Diastereomerenüberschuss
- DMF: Dimethylformamid
- DMSO: Dimethylsulfoxid
- d. Th.: der Theorie (bei Ausbeute)
- DTT: Dithiothreitol
- EDC: *N'*-(3-Dimethylaminopropyl)-*N*-ethylcarbodiimid-Hydrochlorid
- ee: Enantiomerenüberschuss
- EI: Elektronenstoß-Ionisation (bei MS)
- ent: enantiomerenrein, Enantiomer
- eq.: Äquivalent(e)
- ESI: Elektrospray-Ionisation (bei MS)
- Et: Ethyl
- GC: Gaschromatographie
- ges.: gesättigt
- GTP: Guanosin-5'-triphosphat
- h: Stunde(n)
- HATU: *O*-(7-Azabenzotriazol-1-yl)-*N,N,N',N'*-tetramethyluronium-Hexafluorophosphat
- HOBt: 1 -Hydroxy-*1H*-benzotriazol-Hydrat
- HPLC Hochdruck-,: Hochleistungsflüssigchromatographie
- iPr: Isopropyl
- konz.: konzentriert
- LC-MS: Flüssigchromatographie-gekoppelte Massenspektroskopie
- LDA: Lithiumdiisopropylamid
- LiHMDS: Lithiumhexamethyldisilazid [Lithium-bis(trimethylsilyl)amid]
- Me: Methyl
- min: Minute(n)
- MS: Massenspektroskopie
- NBS: *N*-Bromsuccinimid
- NMP: *N*-Methylpyrrolidin-2-on
- NMR: Kernresonanzspektroskopie
- *p*: para
- Pd/C: Palladium auf Aktivkohle
- Ph: Phenyl
- PMB: *p*-Methoxybenzyl
- Pr: Propyl
- rac: racemisch, Racemat
- R_{f}: Retentionsindex (bei DC)
- RP: reverse phase (Umkehrphase, bei HPLC)
- RT: Raumtemperatur
- Rₜ: Retentionszeit (bei HPLC oder GC)
- s.o.: siehe oben
- tBu: *tert*.-Butyl
- TEA: Triethanolamin
- TFA: Trifluoressigsäure
- THF: Tetrahydrofuran
- UV: Ultraviolett-Spektroskopie
- v/v: Volumen zu Volumen-Verhältnis (einer Lösung)
- zus.: zusammen

### GC-MS- und LC-MS-Methoden:

### Methode 1 (GC-MS):

Instrument: Micromass GCT, GC 6890; Säule: Restek RTX-35, 15 m x 200 µm x 0.33 µm; konstanter Fluss mit Helium: 0.88 ml/min; Ofen: 705°C; Inlet: 250°C; Gradient: 70°C, 30°C/min → 310°C (3 min halten).

### Methode 2 (LC-MS):

Gerätetyp MS: Waters Micromass Quattro Micro; Gerätetyp HPLC: Agilent 1100 Serie; Säule: Thermo Hypersil GOLD 3 µ 20 mm x 4 mm; Eluent A: 1 1 Wasser + 0.5 ml 50%-ige Ameisensäure, Eluent B: 11 Acetonitril + 0.5 ml 50%-ige Ameisensäure; Gradient: 0.0 min 100% A → 3.0 min 10% A → 4.0 min 10% A → 4.01 min 100% A (Fluss 2.5 ml/min) → 5.00 min 100% A; Ofen: 50°C; Fluss: 2 ml/min; UV-Detektion: 210 nm.

### Methode 3 (LC-MS):

Gerätetyp MS: Micromass ZQ; Gerätetyp HPLC: HP 1100 Series; UV DAD; Säule: Phenomenex Gemini 3 µ 30 mm x 3.00 mm; Eluent A: 1 1 Wasser + 0.5 ml 50%-ige Ameisensäure, Eluent B: 11 Acetonitril + 0.5 ml 50%-ige Ameisensäure; Gradient: 0.0 min 90% A → 2.5 min 30% A → 3.0 min 5% A → 4.5 min 5% A; Fluss: 0.0 min 1 ml/min → 2.5 min/3.0 min/4.5 min 2 ml/min; Ofen: 50°C; UV-Detektion: 210 nm.

### Methode 4 (LC-MS):

Instrument: Micromass Quattro Premier mit Waters UPLC Acquity; Säule: Thermo Hypersil GOLD 1.9 µ 50 mm x 1 mm; Eluent A: 11 Wasser + 0.5 ml 50%-ige Ameisensäure, Eluent B: 11 Acetonitril + 0.5 ml 50%-ige Ameisensäure; Gradient: 0.0 min 90% A → 0.1 min 90% A → 1.5 min 10% A → 2.2 min 10% A; Fluss: 0.33 ml/min; Ofen:50°C; UV-Detektion: 210 nm.

### Methode 5 (LC-MS):

Instrument: Waters Acquity SQD UPLC System; Säule: Waters Acquity UPLC HSS T3 1.8 µ, 50 mm x 1 mm; Eluent A: 1 1 Wasser + 0.25 ml 99%-ige Ameisensäure, Eluent B: 1 1 Acetonitril + 0.25 ml 99%-ige Ameisensäure; Gradient: 0.0 min 90% A → 1.2 min 5% A → 2.0 min 5% A; Fluss: 0.40 ml/min; Ofen: 50°C; UV-Detektion: 210-400 nm.

### Methode 6 (GC-MS):

Instrument: Thermo DFS, Trace GC Ultra; Säule: Restek RTX-35, 15 m x 200 µm x 0.33 µm; konstanter Fluss mit Helium: 1.20 ml/min; Ofen: 60°C; Inlet: 220°C; Gradient: 60°C, 30°C/min → 300°C (3.33 min halten).

### Methode 7 (LC-MS):

Instrument: Waters Acquity SQD UPLC System; Säule: Waters Acquity UPLC HSS T3 1.8 µ, 30 mm x 2 mm; Eluent A: 11 Wasser + 0.25 ml 99%-ige Ameisensäure, Eluent B: 11 Acetonitril + 0.25 ml 99%-ige Ameisensäure; Gradient: 0.0 min 90% A → 1.2 min 5% A → 2.0 min 5% A; Fluss: 0.60 ml/min; Ofen: 50°C; UV-Detektion: 208-400 mn.

### Ausgangsverbindungen und Intermediate:

### Beispiel 1A

### tert.-Butyl-1-(3-brombenzyl)cyclopropancarboxylat

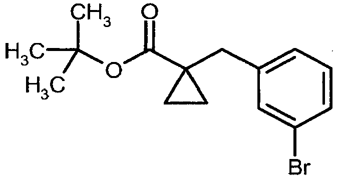

14.8 ml (105.48 mmol) Diisopropylamin wurden unter Argon in 66 ml trockenem THF vorgelegt und auf -40°C abgekühlt. Es wurden langsam 42.2 ml (105.48 mmol) n-Butyllithium-Lösung (2.5 M in Hexan) zugetropft und das Gemisch 30 min nachgerührt. Die Reaktionslösung wurde danach auf -78°C gekühlt und mit einer Lösung von 10.0 g (70.32 mmol) Cyclopropancarbonsäure-*tert*.-butylester in 17 ml THF versetzt. Nach 4 h Rühren bei -78°C wurde eine Lösung von 19.34 g (77.36 mmol) 3-Brombenzylbromid in 17 ml THF zugegeben. Das Reaktionsgemisch wurde langsam über Nacht auf RT erwärmt, bevor vorsichtig mit wässriger Ammoniumchlorid-Lösung versetzt wurde und dreimal mit Ethylacetat extrahiert wurde. Die vereinigten organischen Phasen wurden mit gesättigter Natriumchlorid-Lösung gewaschen, über Magnesiumsulfat getrocknet und im Vakuum eingeengt. Das Rohprodukt wurde durch Chromatographie an 750 g Silicagel gereinigt (Laufmittel Cyclohexan/Dichlormethan 50:1, dann 5:1). Es wurden so 13.3 g (60.7% d. Th.) der Titelverbindung erhalten.

GC-MS (Methode 1): Rₜ = 5.94 min; m/z = 256 (M-C4H₈)⁺.

¹H-NMR (400 MHz, DMSO-*d*₆): δ [ppm] = 7.46 (s, 1H), 7.38 (m, 1H), 7.25 (m, 2H), 2.82 (s, 2H), 1.28 (s, 9H), 1.08 (q, 2H), 0.87 (q, 2H).

### Beispiel 2A

### tert.-Butyl-1-(3-brom-4-fluorbenzyl)cyclopropancarboxylat

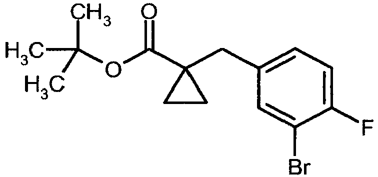

199.5 ml (1.42 mol) Diisopropylamin wurden unter Argon in 1300 ml trockenem THF vorgelegt und auf -50°C abgekühlt. Es wurden langsam 569.1 ml (1.42 mol) n-Butyllithium-Lösung (2.5 M in Hexan) zugetropft. Das resultierende Gemisch wurde auf 0°C erwärmt und dann auf -70°C abgekühlt. Die Reaktionslösung wurde mit einer Lösung von 161.9 g (1.14 mol) Cyclopropancarbonsäure-*tert*.-butylester in 380 ml THF versetzt, wobei die Temperatur unterhalb von -60°C gehalten wurde. Nach 4 h Rühren bei -78°C wurde eine Lösung von 262 g (0.95 mol) 2-Brom-4-(brom-methyl)-1-fluorbenzol in 480 ml THF zugegeben, wobei die Temperatur wiederum unterhalb von -60°C gehalten wurde. Das Reaktionsgemisch wurde dann langsam über Nacht auf RT erwärmt, bevor vorsichtig mit 1.5 Liter gesättigter wässriger Ammoniumchlorid-Lösung und 3.0 Liter Ethylacetat versetzt wurde. Nach Phasentrennung wurde die wässrige Phase mit Ethylacetat extrahiert. Die vereinigten organischen Phasen wurden mit gesättigter Natriumchlorid-Lösung gewaschen, über Magnesiumsulfat getrocknet und im Vakuum eingeengt. Das Rohprodukt wurde durch Chromatographie an 3 kg Silicagel gereinigt (Laufmittel Cyclohexan/Dichlormethan 9:1, dann 5:1). Es wurden so 189.9 g (50.4% d. Th.) der Titelverbindung erhalten.

¹H-NMR (400 MHz, DMSO-*d*₆): δ [ppm] = 0.86-0.92 (m, 2H), 1.06-1.12 (m, 2H), 1.30 (s, 9H), 2.81 (s, 2H), 7.27-7.33 (m, 2H), 7.55-7.60 (m, 1H).

### Beispiel 3A

### tert.-Butyl-1-[3-(benzylamino)benzyl]cyclopropancarboxylat

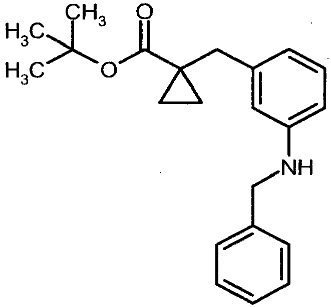

Unter Argon und trockenen Bedingungen wurden 13.3 g (42.73 mmol) *tert*.-Butyl-1-(3-brombenzyl)cyclopropancarboxylat, 5.6 ml (51.28 mmol) Benzylamin, 1.96 g (2.14 mmol) Tris(dibenzylidenaceton)dipalladium, 4.93 g (51.28 mmol) Natrium-*tert*.-butylat sowie 1.06 g (1.71 mmol) (+/-)-2,2'-Bis(diphenylphosphino)-1,1'-binaphthyl in 50 ml Toluol suspendiert. Die Reaktionsmischung wurde anschließend für 1.5 h bei 110°C gerührt. Das Gemisch wurde dann über Kieselgur abgesaugt, der Rückstand mit Toluol nachgewaschen und das Filtrat eingeengt. Der Filtrat-Rückstand wurde in Ethylacetat aufgenommen und zweimal mit wässriger Ammoniumchlorid-Lösung gewaschen. Die organische Phase wurde über Magnesiumsulfat getrocknet und im Vakuum eingeengt. Das Rohprodukt wurde durch Chromatographie an Silicagel gereinigt (Laufmittel Cyclohexan/Ethylacetat 20:1). Es wurden so 6.98 g (48.4% d. Th.) der Titelverbindung erhalten.

LC-MS (Methode 2): Rₜ = 2.75 min; m/z = 338 (M+H)⁺.

¹H-NMR (400 MHz, DMSO-d₆): δ [ppm] = 7.35-7.26 (m, 4H), 7.20 (t, 1H), 6.91 (t, 1H), 6.45 (s, 1H), 6.38 (m, 2H), 6.12 (t, 1H), 4.23 (d, 2H), 2.69 (s, 2H), 1.28 (s, 9H), 0.99 (q, 2H), 0.69 (q, 2H).

### Beispiel 4A

### tert.-Butyl-1-[3-(benzylamino)-4-fluorbenzyl]cyclopropancarboxylat

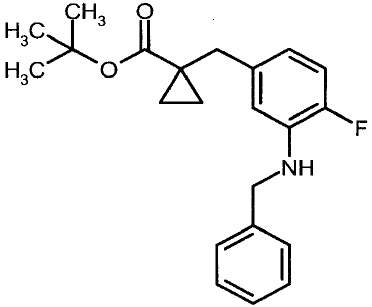

Unter Argon und trockenen Bedingungen wurden 174.0 g (528.5 mmol) *tert*.-Butyl-1-(3-brom-4fluorbenzyl)cyclopropancarboxylat, 69.2 ml (634.2 mmol) Benzylamin, 4.84 g (5.29 mmol) Tris-(dibenzylidenacetou)dipalladium, 60.95 g (634.2 mmol) Natrium-*tert*.-butylat sowie 3.29 g (5.29 mmol) (+/-)-2,2'-Bis(diphenylphosphino)-1,1'-binaphthyl in 1218 ml Toluol suspendiert. Die Reaktionsmischung wurde anschließend für 2.0 h bei 11.0°C gerührt. Nach Abkühlen wurde das Reaktionsgemisch mit 2.1 Liter Ethylacetat und 1.7 Liter halbgesättigter wässriger Ammoniumchlorid-Lösung versetzt. Nach Phasentrennung wurde die organische Phase mit gesättigter Ammoniumchlorid-Lösung und gesättigter Natriumchlorid-Lösung gewaschen, über Magnesiumsulfat getrocknet und im Vakuum eingeengt. Das Rohprodukt wurde durch Chromatographie an 3.7 kg Silicagel gereinigt (Laufmittel Petrolether/Ethylacetat 20:1). Es wurden so 145.0 g (68.7% d. Th.) der Titelverbindung erhalten.

¹H-NMR (400 MHz, DMSO-*d*₆): δ [ppm] = 0.51-0.66 (m, 2H), 0.86-0.99 (m, 2H), 1.25 (m, 9H), 2.65 (s, 2H), 4.30 (d, 2H), 6.07 (t, 1H), 6.29-6.54 (m, 2H), 6.88 (dd, 1H), 7.15-7.25 (m, 1H), 7.25-7.42 (m, 4H).

### Beispiel 5A

### tert.-Butyl-1-(3-aminobenzyl)cyclopropancarboxylat

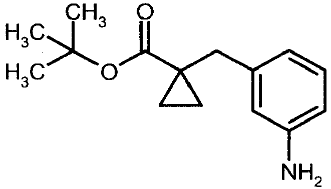

6.98 g (22.43 mmol) *tert*.-Butyl-1.-[3-(benzylamino)benzyl]cyclopropancarboxylat wurden in 50 ml Ethanol und 50 ml THF, gelöst und mit 0.48 g (0.45 mmol) Palladium (10% auf Kohle) versetzt. Bei RT wurde 2 h lang unter einer Wasserstoffatmosphäre bei Normaldruck gerührt. Das Reaktionsgemisch wurde danach über Kieselgur abgesaugt, der Rückstand mit THF nachgewaschen und das Filtrat eingeengt. Das Rohprodukt wurde durch Chromatographie an Silicagel gereinigt (Laufmittel Cyclohexan/Ethylacetat 10:1). Es wurden 3.66 g (65.9% d. Th.) der Titelverbindung erhalten.

LC-MS (Methode 3): Rₜ = 1.84 min; m/z = 192 (M+H)⁺.

¹H-NMR (400 MHz, DMSO-*d*₆): δ [ppm] = 6.88 (t, 1H), 6.42 (s, 1H), 6.37 (dd, 2H), 4.89 (d, 2H), 2.69 (s, 2H), 1.31 (s, 9H), 1.03 (q, 2H), 0.75 (q, 2H).

### Beispiel 6A

### tert.-Butyl-1-(3-amino-4-fluorbenzyl)cyclopropancarboxylat

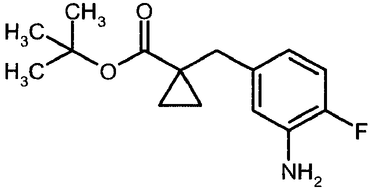

145.0 g (407.9 mmol) *tert*.-Butyl-1-[3-(benzylamino)-4-fluorbenzyl]cyclopropancarboxylat wurden in 1450 ml Ethanol gelöst und mit 9.67 g Palladiumhydroxid (20% auf Kohle) versetzt. Die Suspension wurde bei RT 18 h lang unter einer Wasserstoffatmosphäre bei Normaldruck gerührt. Das Reaktionsgemisch wurde danach über Kieselgur abgesaugt und das Filtrat eingeengt. Nach Trocknen im Hochvakuum wurde der Rückstand mit 500 ml Pentan versetzt, worauf das Produkt als Feststoff ausfiel. Die Suspension wurde 1 h im Eisbad gerührt. Der Feststoff wurde dann abgesaugt, zweimal mit wenig Pentan gewaschen und im Hochvakuum getrocknet. Es wurden so 88.5 g (73.6% d. Th.) der Titelverbindung erhalten.

¹H-NMR (400 MHz, DMSO-*d*₆): δ [ppm] = 0.70-0.80 (m, 2H), 1.00-1.10 (m, 2H), 1.30 (s, 9H), 2.68 (s, 2H), 4.98 (s, 2H), 6.28-6.45 (m, 1H), 6.63 (dd, 1H), 6.84 (dd, 1H).

### Allgemeine Vorschrift 1: Herstellung von Benzylalkoholen aus Benzoesäuren

Eine 0.5 M Lösung der betreffenden Benzoesäure in Toluol wurde bei RT mit 1.3 eq. Triethylamin und anschließend mit 1.2 eq. Chlorameisensäuremethylester versetzt. Das Gemisch wurde über Nacht bei RT gerührt. Die entstandene Suspension wurde danach über Celite filtriert und der Rückstand mit Toluol nachgewaschen. Das Filtrat wurde eingeengt, der Filtrat-Rückstand in THF (1.5 ml/mmol) gelöst und dann zu einer auf -78°C gekühlten Suspension von 1.2 eq. Lithiumaluminiumhydrid in THF (1 ml/mmol) getropft. Nach 1.5 h bei -78°C wurde die Reaktionsmischung auf RT erwärmt und über Nacht weitergerührt. Die entstandene Suspension wurde in 5%-ige Natronlauge (5 ml/mmol) gegossen, das Gemisch über Celite filtriert und der Rückstand mit Ethylacetat nachgewaschen. Das Filtrat wurde mehrfach mit Ethylacetat extrahiert. Die vereinigten organischen Phasen wurden mit gesättigter Kochsalz-Lösung gewaschen, über Magnesiumsulfat getrocknet und im Vakuum eingeengt.

Nach dieser Vorschrift wurden die beiden folgenden Verbindungen aus den entsprechenden Benzoesäuren hergestellt:

| **Beispiel** | **Name / Struktur** | **Analytische Daten** |
|---|---|---|
| **7A** | (3-Brom-5-fluorphenyl)methanol | **LC-MS (Methode 4):** |
| | 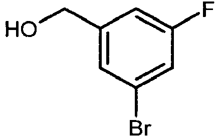 | **Rₜ = 0.96 min; m/z = 187 [M-H₂O]⁺.** |
| **8A** | **(3-Brom-2-fluorphenyl)methanol** | **LC-MS (Methode 5):** |
| | 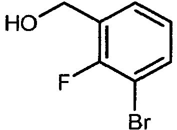 | **Rₜ = 0.81 min; m/z = 187 [M-H₂O]⁺.** |
| | | **¹H-NMR (400 MHz, DMSO-d₆): 8 [ppm] = 7.54-7.66 (m, 1H), 7.47 (t, 1H), 7.16 (t, 1H), 5.39 (t, 1H), 4.56 (d, 2H).** |

### Allgemeine Vorschrift 2: Herstellung von Benzylbromiden aus Benzylalkoholen

*Methode 2A:* Der betreffende Benzylalkohol wurde in DMF (2 ml/mmol) vorgelegt und mit 2 eq. Tetrabromkohlenstoff versetzt. Anschließend wurden portionsweise innerhalb von 30 min 2 eq. Triphenylphosphin zugegeben und die Mischung über Nacht bei RT gerührt. Das Reaktionsgemisch wurde dann in Wasser gegossen und mit *tert*.-Butylmethylether extrahiert. Die organischen Phasen wurden über Magnesiumsulfat getrocknet und eingeengt. Das Rohprodukt wurde anschließend durch Flash-Chromatographie an Kieselgel gereinigt (Laufmittel Cyclohexan).

*Methode 2B:* Der betreffende Benzylalkohol wurde in Dichlormethan (2 ml/mmol) vorgelegt, mit 1.2 eq. Triphenylphosphindibromid versetzt und über Nacht bei RT gerührt. Anschließend wurde die Reaktionsmischung mit Wasser gewaschen, und die organische Phase wurde über Magnesiumsulfat getrocknet und eingeengt. Das Rohprodukt wurde durch Flash-Chromatographie an Kieselgel gereinigt (Laufmittel Cyclohexan).

Nach diesen Methoden wurden die folgenden Verbindungen hergestellt:

| **Beispiel** | **Name / Struktur** | **Methode** | **Analytische Daten** |
|---|---|---|---|
| **9A** | 1-Brom-3-(brommethyl)-5-fluorbenzol | 2A | **LC-MS (Methode 5):** |
| | 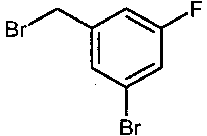 | | **Rₜ = 1.17 min; m/z = 289 [M+NH₄]⁺.** |
| **10A** | 1-Brom-3-(brommethyl)-2-fluorbenzol | 2B | **GC-MS (Methode 1):** |
| | 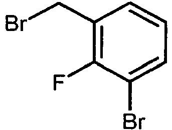 | | **Rₜ = 4.32 min; m/z = 268 [M]⁺.** |
| | | | **¹H-NMR (400 MHz, DMSO-*d*₆): δ [ppm] = 7.66-7.78 (m, 1H), 7.49-7.63 (m, 1H), 7.18 (t, 1H), 4.73 (s, 2H).** |
| **11A** | **2-Brom-4-(brommethyl)-1-methylbenzol** | **2B** | **GC-MS (Methode 1):** |
| | | | **Rₜ = 4.93 min; m/z = 263 [M]⁺.** |
| | 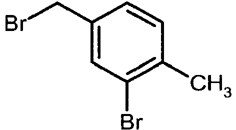 | | |
| | **[aus (3-Brom-4-methylphenyl)methanol (CAS Reg.-Nr. 68120-35-4)]** | | |

### Allgemeine Vorschrift 3: Alkylierung von Ester-Enolaten mit Benzylbromiden

Eine 0.3 M Lösung von Diisopropylamin in THF wurde unter Argon auf -40°C abgekühlt und mit 1 eq. *n*-Butyllithium (als Lösung in Hexan) versetzt. Nach 30 min wurde die Lösung auf -78°C abgekühlt und mit 0.8 eq. einer Lösung des betreffenden Carbonsäureesters in THF (0.7 M) versetzt. Die Reaktionsmischung wurde 4 h bei -78°C gerührt und anschließend mit 0.75 eq. des betreffenden Benzylbromids in THF (0.6 M) versetzt. Das Reaktionsgemisch wurde über Nacht unter Erwärmung auf RT gerührt. Danach wurde gesättigte wässrige Ammoniumchlorid-Lösung zugesetzt. Das Gemisch wurde mit Ethylacetat extrahiert, und die organische Phase wurde über Magnesiumsulfat getrocknet und eingeengt. Das so erhaltene Rohprodukt wurde durch Flash-Chromatographie an Kieselgel gereinigt (typisches Laufmittel-Gemisch: Cyclohexan/Ethylacetat 15:1 → 10:1).

Nach der Allgemeinen Vorschrift 3 wurden die folgenden Verbindungen hergestellt:

| **Beispiel** | **Name / Struktur** | **Analytische Daten** |
|---|---|---|
| **12A** | *tert*.-Butyl-1-(3-brom-5-fluorbenzyl)-cyclopropancarboxylat | **GC-MS (Methode 1):** |
| | | **Rₜ = 5.64 min; m/z = 272/274 [M-C₄H₉+].** |
| | 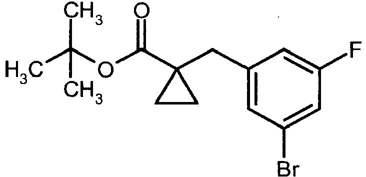 | **¹H-NMR (400 MHz, DMSO-*d*₆): δ [ppm] = 7.37 (dd, 1H), 7.33 (s, 1H), 7.13 (d, 1H), 2.84 (s, 2H), 1.25-1.32 (m, 9H), 1.06-1.14 (m, 2H), 0.89-0.93 (m, 2H).** |
| **13A** | *tert*.-Butyl-1-(3-brom-2-fluorbenzyl)-cyclopropancarboxylat | **¹H-NMR (400 MHz, DMSO-*d*₆): δ [ppm] = 7.50-7.59 (m, 1H), 7.39 (t, 1H), 7.11 (t, 1H), 2.91 (s, 2H), 1.22-1.29 (m,** 9H), **1.10-1.16 (m, 2H), 0.84-0.91 (m, 2H).** |
| | 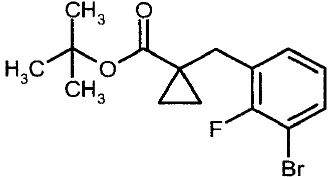 | |
| **14A** | ***tert*.-Butyl-.1-(3-brom-4-methylbenzyl)-cyclopropancarboxylat** | **LC-MS (Methode 2):** |
| | | **Rₜ = 3.02 min; m/z = 325/327 [M+H]⁺.** |
| | 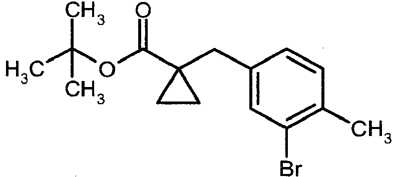 | |

### Allgemeine Vorschrift 4: Buchwald-Hartwig-Reaktion von Phenylbromiden zu N-Benzylphenylaminen

Unter Argonatmosphäre wurden 1.2 eq. Natrium-*tert*.-butylat in Toluol (1.5 ml/mmol) suspendiert, mit 1 eq. des betreffenden Phenylbromids, 1.2 eq. Benzylamin, 0.05 eq. Tris(dibenzylidenaceton)-dipalladium sowie 0.04 eq. *rac*-2,2'-Bis(diphenylphosphino)-1,1'-binaphthyl versetzt und das Gemisch anschließend für 2 h auf 110°C erhitzt. Nach Abkühlen auf RT wurde der Ansatz mit gesättigter wässriger Ammoniumchlorid-Lösung und Ethylacetat versetzt und über Celite filtriert. Die organische Phase wurde je einmal mit gesättigter Ammoniumchlorid-Lösung und gesättigter Natriumchlorid-Lösung gewaschen, dann über Magnesiumsulfat getrocknet und eingeengt. Das so erhaltene Rohprodukt wurde durch Flash-Chromatographie an Kieselgel gereinigt (typisches Laufmittel-Gemisch: Cyclohexan/Ethylacetat 50:1).

Nach der Allgemeinen Vorschrift 4 wurden die folgenden Verbindungen hergestellt:

| **Beispiel** | **Name / Struktur** | **Analytische Daten** |
|---|---|---|
| **15A** | *tert*.-Butyl-1-[3-(benzylamino)-5-fluorbenzyl]-cyclopropancarboxylat | **LC-MS (Methode 4): Rₜ = 1.60 min; m/z = 356 [M+H]⁺.** |
| | 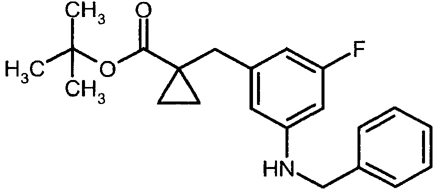 | **¹H-NMR (400 MHz, DMSO-*d*₆): δ [ppm] = 7.28-7.37 (m, 4H), 7.16-7.28 (m, 1H), 6.50 (t, 1H), 6.31 (s, 1H), 6.04-6.20 (m, 2H), 4.23 (d, 2H), 2.68 (s, 2H), 1.22-1.32 (m, 9H), 0.97-1.06 (m, 2H), 0.67-0.79 (m, 2H).** |
| **16A** | *tert*.-Butyl-1-[3-(benzylamino)-4-fluorbenzyl]-cyclopropancarboxylat | **LC-MS (Methode 4): Rₜ = 1.63 min; m/z = 356 [M+H]⁺.** |
| | 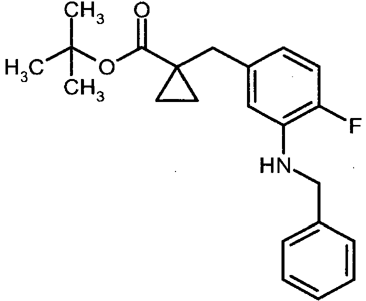 | **¹H-NMR (400 MHz, DMSO-*d*₆): δ [ppm] = 7.27-7.39 (m, 4H), 7.22 (d, 1H), 6.88 (dd, 1H), 6.43 (dd, 1H), 6.31-6.38 (m, 1H), 6.02-6.13 (m, 1H), 4.30 (d, 2H), 2.64 (s, 2H), 1.25 (s, 9H), 0.89-0.96 (m, 2H), 0.55-0.63 (m, 2H).** |
| **17A** | *tert*.-Butyl-1-[3-(benzylamino)-2-fluorbenzyl]-cyclopropancarboxylat | **LC-MS (Methode 4): Rₜ = 1.66 min; m/z = 356 [M+H]⁺.** |
| | 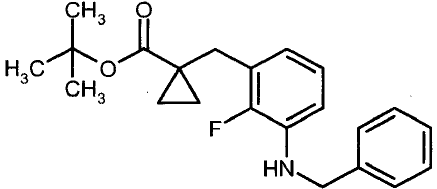 | **¹H-NMR (400 MHz, DMSO-*d*₆): δ [ppm] = 7.25-7.41 (m, 4H), 7.13-7.25 (m, 1H), 6.75 (t, 1H), 6.48 (t, 1H), 6.39 (t, 1H), 6.02-6.16 (m, 1H), 4.32 (d, 2H), 2.85 (s, 2H), 1.29 (s, 9H), 1.03-1.12 (m, 2H), 0.70-0.83 (m, 2H).** |

| **Beispiel** | **Name / Struktur** | **Analytische Daten** |
|---|---|---|
| **18A** | ***tert*.-Butyl-1-[3-(benzylamino)-4-methylbenzyl]cyclopropancarboxylat** | **LC-MS (Methode 5): Rₜ = 1.42 min; m/z = 352 [M+H]⁺.** |
| | 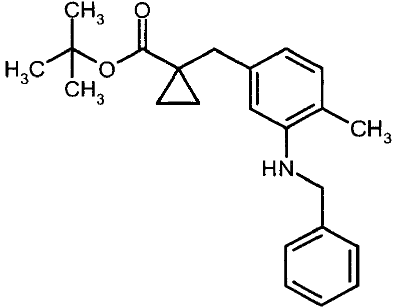 | |

### Allgemeine Vorschrift 5: Hydrierung von N-Benzylphenylaminen zu Phenylaminen

Das betreffende *N*-Benzylphenylamin wurde in einem 1:1-Gemisch aus Ethanol und THF (5 ml/ mmol) gelöst, mit 10% Palladium auf Aktivkohle (35 mg/mmol) versetzt und das Gemisch bei RT und 1 bar Wasserstoffatmosphäre über Nacht gerührt. Anschließend wurde das Reaktionsgemisch über Celite filtriert, der Rückstand mit Ethanol nachgewaschen und das Filtrat eingeengt. Das so erhaltene Rohprodukt wurde durch Flash-Chromatographie an Kieselgel gereinigt (typisches Laufmittel-Gemisch: Cyclohexan/Ethylacetat 3:1).

Nach der Allgemeinen Vorschrift 5 wurden die folgenden Verbindungen hergestellt:

| **Beispiel** | **Name / Struktur** | **Analytische Daten** |
|---|---|---|
| **19A** | *tert.*-Butyl-1-(3-amino-5-fluorbenzyl)-cyclopropancarboxylat | **LC-MS (Methode 5): Rₜ = 1.14 min; m/z = 266 [M+H]⁺.** |
| | 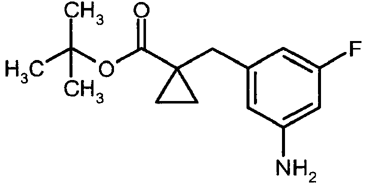 | **¹H-NMR (400 MHz, DMSO-*d*₆): δ [ppm] = 6.25 (s, 1H), 6.11 (d, 1H), 6.14 (d, 1H), 5.28 (s, 2H), 2.68 (s, 2H), 1.30 (s, zu, 1.01-1.11 (m, 2H), 0.71-0.85 (m, 2H).** |
| **20A** | *tert*.-Butyl-t-(3-amino-2-fluorbenzyl)-cyclopropancarboxylat | **LC-MS (Methode 5): Rₜ = 1.14 min; m/z = 266 [M+H]⁺.** |
| | 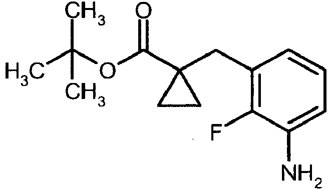 | **¹H-NMR (400 MHz, DMSO-*d₆*): δ [ppm] = 6.68-6.87 (m, 1H), 6.51-6.66 (m, 1H), 6.39-6.51 (m, 1H), 4.98 (s, 2H), 2.83 (s, 2H), 1.25-1.38 (m, 9H), 1.01-1.13 (m, 2H), 0.68-0.84 (m, 2H).** |
| **21A** | ***tert*.-Butyl-1-(3-amino-4-methylbenzyl)-cyclopropancarboxylat** | **GC-MS (Methode 1): Rₜ = 6.45 min; m/z = 261 [M]⁺.** |
| | 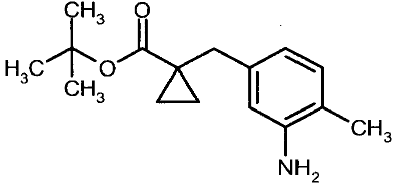 | |

### Beispiel 22A und Beispiel 23A

### tert.-Butyl-1-(3-amino-2-chlorbenzyl)cyclopropancarboxylat und tert.-Butyl-1-(5-amino-2-chlorbenzyl)cyclopropancarboxylat

1.0 g (4.40 mmol) *tert*.-Butyl-1-(3-aminobenzyl)cyclopropancarboxylat wurden in 10.0 ml Acetonitril vorgelegt und bei 0°C portionsweise mit 540 mg (4.40 mmol) *N*-Chlorsuccinimid versetzt. Nach 30 min wurde die Mischung auf RT erwärmt und über Nacht bei dieser Temperatur weitergerührt. Nach Einengen im Vakuum wurde der Rückstand in Dichlormethan aufgenommen und mit gesättigter Natriumhydrogencarbonat-Lösung und gesättigter Natriumchlorid-Lösung gewaschen. Die organische Phase wurde über Natriumsulfat getrocknet und im Vakuum eingeengt. Aus dem so erhaltenen Rohprodukt wurden durch Chromatographie an Kieselgel (Laufmittel-Gradient Cyclohexan/Ethylacetat 10:1 → 6:1) 217 mg einer Mischfraktion (ca. 1:1), bestehend aus *tert*.-Butyl-1-(3-amino-2-chlorbenzyl)cyclopropancarboxylat und *tert*.-Butyl-1-(3-amino-4-chlorbenzyl)cyclopropancarboxylat, sowie 995.5 mg *tert*.-Butyl-1-(5-amino-2-chlorbenzyl)cyclopropancarboxylat, geringfügig verunreinigt durch das Ausgangsmaterial *tert*.-Butyl-1-(3-aminobenzyl)cyclopropancarboxylat, isoliert. Die Mischfraktion (217 mg) wurde durch präparative RP-HPLC weiter aufgetrennt [Säule: Sunfire C18 5 µm, 250 mm x 20 mm; Injektionsvolumen: 0.50 ml; Temperatur: 25°C; Eluent: 55% Acetonitril / 40% Wasser / 5% (Wasser + 1% TFA); Fluss: 35 ml/min; Detektion: 210 nm]. Auf diese Weise konnten 85 mg (7.5% d. Th.) *tert*.-Butyl-1-(3-amino-2-chlorbenzyl)cyclopropancarboxylat (*Beispiel 22A*) isoliert werden. Ausgehend von 995.5 mg des leicht verunreinigten Materials wurden durch präparative RP-HPLC (Eluent: Acetonitril/Wasser-Gradient) 677.6 mg (59.5% d. Th.) an reinem *tert*.-Butyl-1-(5-amino-2-chlorbenzyl)cyclopropancarboxylat (*Beispiel 23A*) isoliert.

### Beispiel 22A

### tert.-Butyl-1-(3-amino-2-chlorbenzyl)cyclopropancarboxylat

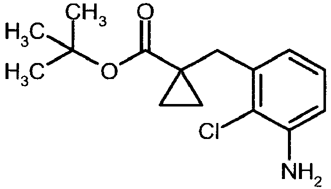

LC-MS (Methode 4): Rₜ = 1.38 min; m/z = 282 (M+H)⁺.

¹H-NMR (400 MHz, DMSO-*d*₆): δ [ppm] = 0.67-0.77 (m, 2H), 1.06-1.15 (m, 2H), 1.28 (s, 9H), 2.94 (s, 2H), 6.57 (d, 1H), 6.61-6.72 (m, 1H), 6.95 (t, 1H).

### Beispiel 23A

### tert.-Butyl-1-(5-amino-2-chlorbenzyl)cyclopropancarboxylat

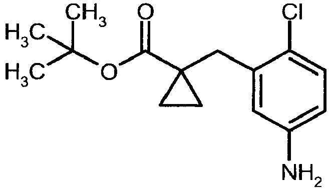

LC-MS (Methode 5): Rₜ = 1.17 min; m/z = 282 (M+H)⁺.

¹H-NMR (400 MHz, DMSO-*d*₆): δ [ppm] = 0.63-0.79 (m, 2H), 1.03-1.16 (m, 2H), 1.29 (s, 9H), 2.86 (s, 2H), 5.14 (s, 2H), 6.39 (dd, 1H), 6.58 (d, 1H), 6.98 (d, 1H).

### Beispiele 24A - 26A

### tert.-Butyl-1-(3-amino-4-chlor-2-fluorbenzyl)cyclopropancarboxylat, tert.-Butyl-1-(3-amino-6-chlor-2-fluorbenzyl)cyclopropancarboxylat und tert.-Butyl-1-(3-amino-4,6-dichlor-2-fluorbenzyl)cyclopropancarboxylat

129 mg (0.486 mmol) *tert*.-Butyl-1-(3-amino-2-fluorbenzyl)cyclopropancarboxylat wurden in 1.3 ml Acetonitril gelöst und bei RT portionsweise mit 71.4 mg (0.535 mmol) *N*-Chlorsuccinimid versetzt. Die Reaktionsmischung wurde auf 50°C erwärmt und 1.5 h gerührt. Nach Abkühlen wurde das Lösungsmittel im Vakuuum entfernt. Der Rückstand wurde in Dichlormethan aufgenommen und mit gesättigter Natriumhydrogencarbonat-Lösung und gesättigter Natriumchlorid-Lösung gewaschen. Die organische Phase wurde über Natriumsulfat getrocknet und im Vakuum eingeengt. Das so erhaltene Rohprodukt wurde durch präparative RP-HPLC (Eluent: Acetonitril / Wasser) teilweise in seine Komponenten aufgetrennt. Es wurden 37 mg (25% d. Th.) einer Mischung (ca. 1.5:1) aus *tert*.-Butyl-1-(3-amino-4-chlor-2-fluorbenzyl)cyclopropancarboxylat und *tert*.-Butyl-1-(3-amino-6-chlor-2-fluorbenzyl)cyclopropancarboxylat (*Beispiel 24A* bzw. *Beispiel 25A*) sowie 34 mg (21% d. Th.) *tert*.-Butyl-1-(3-amino-4,6-dichlor-2-fluorbenzyl)cyclopropancarboxylat (*Beispiel 26A*) erhalten.

### Beispiel 24A und Beispiel 25A

### tert.-Butyl-1-(3-amino-4-chlor-2-fluorbenzyl)cyclopropancarboxylat und tert.-Butyl-1-(3-amino-6-chlor-2-fluorbenzyl)cyclopropancarboxylat (ca. 1.5:1-Gemisch)

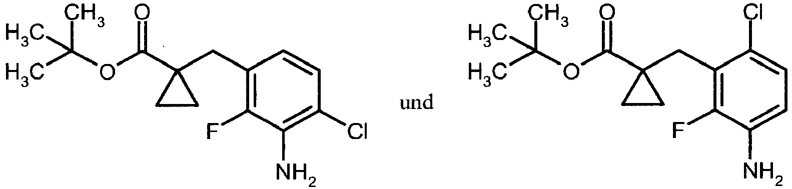

LC-MS (Methode 4): Rₜ = 1.43 min; m/z = 282 (M+H)⁺ und Rₜ = 1.45 min; m/z = 282 (M+H)⁺ (Verhältnis ca. 1:1.5).

### tert.-Butyl-1-(3-amino-4-chlor-2-fluorbenzyl)cyclopropancarboxylat (Beispiel 24A):

¹H-NMR (400 MHz, DMSO-*d*₆): δ [ppm] = 0.74-0.82 (m, 2H), 1.05-1.11 (m, 2H), 1.29 (s, 9H), 2.82 (s, 2H), 5.25 (s, 2H), 6.53 (t, 1H), 6.99 (dd, 1H).

### tert.-Butyl-1-(3-amino-6-chlor-2-fluorbenzyl)cyclopropancarboxylat (Beispiel 25A):

¹H-NMR (400 MHz, DMSO-*d*₆): δ [ppm] = 0.36-0.46 (m, 2H), 0.95-1.01 (m, 2H), 1.38 (s, 9H), 3.19 (d, 2H), 5.25 (s, 2H), 6.65 (t, 1H), 6.89-6.95 (m, 1H).

### Beispiel 26A

### tert.-Butyl-1-(3-amino-4,6-dichlor-2-fluorbenzyl)cyclopropancarboxylat

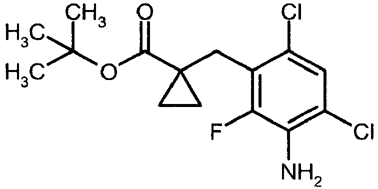

LC-MS (Methode 4): Rₜ = 1.57 min; m/z = 278/280.

¹H-NMR (400 MHz, DMSO-*d*₆): δ [ppm] = 0.41-0.49 (m, 2H), 0.96-1.06 (m, 2H), 1.37 (s, 9H), 3.17 (d, 2H), 5.53 (s, 2H), 7.22 (d, 1H).

### Beispiel 27A

### tert.-Butyl-1-(5-amino-2-chlor-4-fluorbenzyl)cyclopropancarboxylat

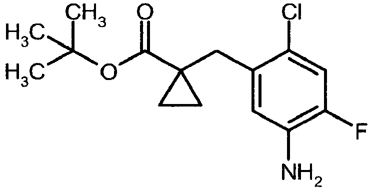

2.0 g (7.54 mmol) *tert*.-Butyl-1-(3-amino-4-fluorbenzyl)cyclopropancarboxylat wurden in 20.0 ml Acetonitril gelöst und bei RT in mehreren Portionen mit 1.06 g (7.92 mmol) *N*-Chlorsuccinimid versetzt. Die Reaktionsmischung wurde 80 min bei RT und anschließend 8 h bei 45°C gerührt. Nach Abkühlen wurde das Acetonitril im Vakuum entfernt. Der Rückstand wurde in Dichlormethan aufgenommen und mit gesättigter Natriumhydrogencarbonat-Lösung und gesättigter Natriumchlorid-Lösung gewaschen. Die organische Phase wurde über Natriumsulfat getrocknet und im Vakuum eingeengt. Aus dem so erhaltenen Rohprodukt wurden durch Chromatographie an Silicagel (Laufmittel Cyclohexan/Ethylacetat 30:1) 1.41 g (62.3% d. Th.) der Titelverbindung isoliert.

LC-MS (Methode 5): Rₜ = 1.23 min; m/z = 244.

¹H-NMR (400 MHz, DMSO-*d*₆): δ [ppm] = 0.72-0.79 (m, 2H), 1.09-1.15 (m, 2H), 1.29 (s, 9H), 2.84 (s, 2H), 5.24 (s, 2H), 6.80 (d, 1H), 7.09 (d, 1H).

### Beispiel 28A

### 2-Brom-4-(brommethyl)-1-chlorbenzol

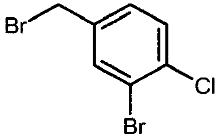

### Stufe 1:

199.0 g (0.845 mol) 3-Brom-4-chlorbenzoesäure wurden in 2.5 Liter THF gelöst, auf -10°C abgekühlt und bei dieser Temperatur mit 1.69 Liter (1.69 mol) einer 1 M Boran-Lösung in THF versetzt. Die Reaktionsmischung wurde über Nacht auf RT erwärmt und dann mit gesättigter wässriger Ammoniumchlorid-Lösung versetzt. Nach Zugabe von Wasser wurde zweimal mit Ethylacetat extrahiert. Die vereinigten organischen Phasen wurden über Magnesiumsulfat getrocknet und im Vakuum eingeengt. Erhalten wurden 206 g (3-Brom-4-chlorphenyl)methanol als Rohprodukt, das ohne weitere Reinigung umgesetzt wurde.

### Stufe 2:

260 g (Rohprodukt aus mehreren Chargen, ca. 1.05 mol) (3-Brom-4-chlorphenyl)methanol wurden in 2.86 Liter Dichlormethan gelöst, auf -5°C gekühlt und langsam mit 127.1 g (44.6 ml, 459.6 mmol) Phosphortribromid versetzt. Nach Ende der Zugabe wurde noch 1 h bei -5°C nachgerührt und die Mischung dann mit Dichlormethan und Wasser verdünnt. Die organische Phase wurde abgetrennt, über Magnesiumsulfat getrocknet und im Vakuum eingeengt. Erhalten wurden als Rohprodukt 280.5 g (ca. 84% d. Th.) 2-Brom-4-(brommethyl)-1-chlorbenzol.

GC-MS (Methode 1): Rₜ = 5.36 min; m/z = 281/283/285 (M+H)⁺.

¹H-NMR (400 MHz, DMSO-*d*₆): δ [ppm] = 4.71 (s, 2H), 7.49 (dd, 1H), 7.63 (d, 1 H), 7.89 (d, 1H).

### Beispiel 29A

### tert.-Butyl-1-(3-brom-4-chlorbenzyl)cyclopropancarboxylat

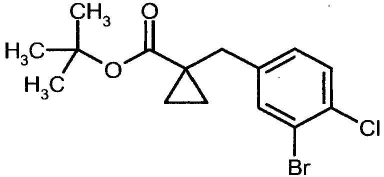

140.2 ml (1.0 mol) Diisopropylamin wurden unter Argon in 1200 ml trockenem THF gelöst und auf -30°C abgekühlt. Es wurden 400 ml (1.0 mol) *n*-Butyllithium-Lösung (2.5 M in Hexan) zugetropft. Das resultierende Gemisch wurde auf 0°C erwärmt und dann auf -70°C abgekühlt. Die Reaktionslösung wurde mit einer Lösung von 94.8 g (0.667 mol) Cyclopropancarbonsäure-*tert*.-butylester in 750 ml THF versetzt, wobei die Temperatur unterhalb von -60°C gehalten wurde. Nach 4 h Rühren bei -60°C wurde eine Lösung von 208.6 g (0.733 mol) 2-Brom-4-(brommethyl)-1-chlorbenzol in 550 ml THF zugegeben, wobei die Temperatur wiederum unterhalb von -60°C gehalten wurde. Das Reaktionsgemisch wurde langsam über Nacht auf RT erwärmt, bevor vorsichtig mit gesättigter wässriger Ammoniumchlorid-Lösung versetzt wurde. Nach Phasentrennung wurde die wässrige Phase mit Ethylacetat extrahiert. Die vereinigten organischen Phasen wurden über Magnesiumsulfat getrocknet und im Vakuum eingeengt. Das Rohprodukt wurde durch Chromatographie an Silicagel gereinigt (Laufmittel Cyclohexan/Dichlormethan 4:1). Es wurden 95.5 g (41.4% d. Th.) der Titelverbindung erhalten.

GC-MS (Methode 1): Rₜ = 6.54 min; m/z = 288/290 (M-C₄H₈)⁺.

LC-MS (Methode 4): Rₜ = 1.65 min; m/z = 288/290 (M-C₄H₈)⁺.

### Beispiel 30A

### tert.-Butyl-1-[3-(benzylamino)-4-chlorbenzyl]cyclopropancarboxylat

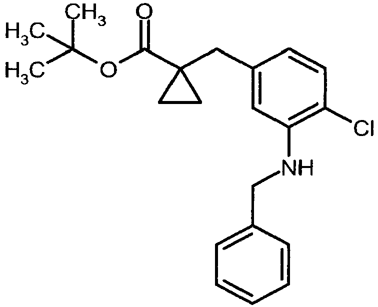

Unter Argon wurden zu 633 ml trockenem Toluol nacheinander 95.0 g (274.8 mmol) *tert*.-Butyl-1-(3-brom-4-chlorbenzyl)cyclopropancarboxylat, 36.0 ml (330.0 mmol) Benzylamin, 12.58 g (13.7 mmol) Tris(dibenzylidenaceton)dipalladium, 31.69 g (329.8 mmol) Natrium-*tert*.-butylat sowie 6.85 g (5.29 mmol) (+/-)-2,2'-Bis(diphenylphosphino)-1,1'-binaphthyl gegeben. Die Reaktionsmischung wurde 3.0 h bei 110°C und anschließend über Nacht bei RT gerührt. Das Reaktionsgemisch wurde dann über Kieselgur abgesaugt und der Rückstand gründlich mit Toluol und Ethylacetat gewaschen. Die vereinigten Filtrate wurden im Vakuum eingeengt. Das Rohprodukt wurde durch Chromatographie an Silicagel gereinigt (Laufmittel Petrolether/Ethylacetat 10:1). Es wurden 50.0 g der Titelverbindung erhalten (48.9% d. Th.).

LC-MS (Methode 5): Rₜ = 1.48 min; m/z = 372 (M+H)⁺.

### Beispiel 31A

### tert.-Butyl-1-(3-amino-4-chlorbenzyl)cyclopropancarboxylat

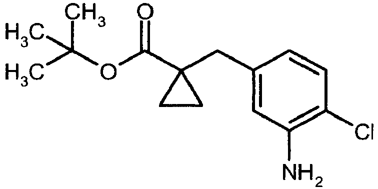

50.0 g (134.4 mmol) *tert*.-Butyl-1-[3-(benzylamino)-4-chlorbenzyl]cyclopropancarboxylat wurden in 1.5 Liter Ethylacetat gelöst und mit 1.43 g (1.34 mmol) Palladium (10% auf Kohle) versetzt. Die Reaktionsmischung wurde bei RT über Nacht unter einer Wasserstoffatmosphäre bei Normaldruck gerührt. Das Reaktionsgemisch wurde danach über Kieselgur abgesaugt und das Filtrat eingeengt. Das Rohprodukt wurde durch Chromatographie an Silicagel gereinigt (Laufmittel Petrolether/ Ethylacetat 10:1). Das erhaltene Produkt wurde in einem Methanol/Wasser-Gemisch (70:30) verrührt und der Feststo ff isoliert. Es wurden 24.3 g (64.1% d. Th.) der Zielverbindung erhalten.

LC-MS (Methode 5): Rₜ = 1.22 min; m/z = 282 (M+H)⁺.

¹H-NMR (400 MHz, DMSO-*d*₆): δ [ppm] = 0.74-0.82 (m, 2H), 1.02-1.09 (m, 2H), 1.30 (s, 9H), 2.69 (s, 2H), 5.21 (br. s, 2H), 6.42 (dd, 1H), 6.67 (d, 1H), 7.05 (d, 1H).

### Beispiel 32A

### 5-(4-Chlor-3-nitrobenzyl)-2,2-dimethyl-1,3-dioxan-4,6-dion

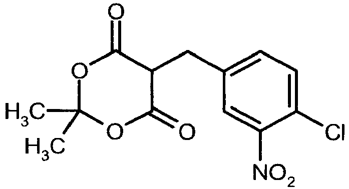

10.0 g (49.6 mmol) 4-Chlor-3-nitrobenzoesäure wurden mit 200 ml Dichlormethan, 7.87 g (54.6 mmol) Meldrumsäure und 9.70 g (79.4 mmol) 4-*N,N*-Dimethylaminopyridin versetzt. Die Reaktionsmischung wurde auf 0°C gekühlt und tropfenweise mit einer Lösung von 8.9 ml (57.1 mmol) *N,N'*-Diisopropylcarbodiimid in 100 ml Dichlormethan versetzt. Die Reaktionsmischung wurde über Nacht bei 0°C gerührt und der ausgefallene Niederschlag dann über Celite abfiltriert. Das Filtrat wurde dreimal mit gesättigter Kaliumhydrogensulfat-Lösung und einmal mit gesättigter Natriumchlorid-Lösung gewaschen, über Magnesiumsulfat getrocknet und im Vakuum auf ein Volumen von ca. 200 ml eingeengt. Nach Abkühlen auf 0°C wurden zunächst 33.5 ml Essigsäure hinzugefügt, dann portionsweise 4.69 g (124 mmol) Natriumborhydrid. Die Reaktionsmischung wurde über Nacht bei 0°C gerührt, bevor 300 ml Wasser hinzugefügt wurden. Nach 10 min kräftigem Rühren wurde die abgetrennte organische Phase mit 300 ml Wasser und 200 ml gesättigter Natriumchlorid-Lösung gewaschen, über Magnesiumsulfat getrocknet und im Vakuum eingeengt. Der Rückstand wurde mit Cyclohexan/Ethylacetat (5:1) verrührt, und der ausgefallene Feststoff wurde abgesaugt und isoliert. Die Mutterlauge wurde im Vakuum eingeengt, der Rückstand erneut mit Cyclohexan/Ethylacetat (5:1) verrührt und der ausgefallene Feststoff wiederum isoliert. Dieser Vorgang wurde noch einmal wiederholt. Alle Feststoff-Chargen wurden vereinigt und im Hochvakuum getrocknet. Erhalten wurden insgesamt 11.15 g des Zielprodukts (ca. 70% d. Th., noch verunreinigt durch Reste von 1,3-Diisopropylharnstoff).

¹H-NMR (400 MHz, DMSO-d₆): δ [ppm] = 1.71 (s, 3H), 1.80 (s, 3H), 3.51 (d, 2H), 3.80 (t, 1H), 7.46 (d, 1H), 7.56 (dd, 1H), 7.90 (d, 1H).

### Beispiel 33A

### tert.-Butyl-2-(4-chlor-3-nitrobenzyl)acrylat

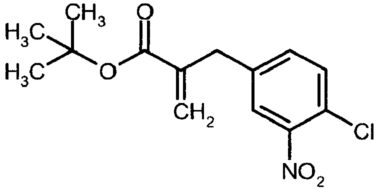

11.1 g 5-(4-Chlor-3-nitrobenzyl)-2,2-dimethyl-1,3-dioxan-4,6-dion (verunreinigt durch Reste von 1,3-Diisopropylharnstoff) wurden in 40 ml THF und 40 ml (31 g) *tert*.-Butanol gelöst und mit 16.4 g (88.5 mmol) *N*,*N*-Dimethylmethyleniminiumiodid versetzt. Die resultierende Suspension wurde über Nacht bei 70°C gerührt und dann nach Abkühlen auf Wasser gegeben. Die wässrige Phase wurde dreimal mit Ethylacetat extrahiert. Die vereinigten organischen Phasen wurden mit gesättigter Natriumchlorid-Lösung gewaschen, über Magnesiumsulfat getrocknet und im Vakuum eingeengt. Der Rückstand wurde durch Chromatographie an Silicagel gereinigt (Laufmittel Cyclohexan/Ethylacetat 100:1 → 50:1). Es wurden 8.22 g des Zielprodukts erhalten (ca. 78% d. Th.).

GC-MS (Methode 1): Rₜ = 6.44 min; m/z = 224 (M-C₃H₅O₂)⁺.

¹H-NMR (500 MHz, DMSO-d₆): δ [ppm] = 1.37 (s, 9H), 3.67 (s, 2H), 5.71 (s, 1H), 6.13 (s, 1H), 7.53 (dd, 1H), 7.72 (d, 1H), 7.90 (d, 1H).

### Beispiel 34A

### (+/-)-tert.-Butyl-1-(4-chlor-3-nitrobenzyl)-2,2-difluorcyclopropancarboxylat

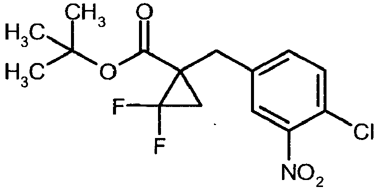

Eine Lösung von 8.22 g (27.6 mmol) *tert*.-Butyl-2-(4-chlor-3-nitrobenzyl)acrylat in 79.5 ml Diethylenglykoldimethylether wurde auf 140°C erhitzt und mit 4.2 g (27.6 mmol) Natriumchlor-(difluor)acetat versetzt. Die Reaktionsmischung wurde anschließend auf 160°C erhitzt und 1 h bei dieser Temperatur gerührt, bevor weitere 4.2 g Natriumchlor(difluor)acetat hinzugefügt wurden. Nach einer weiteren Stunde bei 160°C wurden erneut 4.2 g Natriumchlor(difluor)acetat zugesetzt und die Mischung nochmals 2 h gerührt. Nach Abkühlen auf RT wurde das Gemisch dann auf Eiswasser gegeben. Die wässrige Phase wurde dreimal mit Ethylacetat extrahiert. Die vereinigten organischen Phasen wurden mit gesättigter Natriumchlorid-Lösung gewaschen, über Magnesiumsulfat getrocknet und im Vakuum eingeengt. Verbliebener Diethylenglykoldimethylether wurde im Hochvakuum entfernt. Aus dem erhaltenen Rohprodukt wurde das Zielprodukt durch Chromatographie an Silicagel isoliert (Laufmittel Cyclohexan/Dichlormethan 20:1 → 3:1). Es wurden auf diese Weise 1.50 g der Titelverbindung erhalten (9.6% d. Th.).

LC-MS (Methode 5): Rₜ = 1.27 min; m/z = 365 (M+H₂O)⁺.

¹H-NMR (400 MHz, DMSO-d₆): δ [ppm] = 1.30 (s, 9H), 2.12-2.25 (m, 2H), 2.85 (d, 1H), 3.42 (d, 1H), 7.63 (dd, 1H), 7.77 (d, 1H), 7.98 (d, 1H).

### Beispiel 35A

### (+/-)-tert.-Butyl-1-(3-amino-4-chlorbenzyl)-2,2-difluorcyclopropancarboxylat

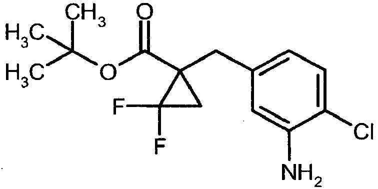

1.60 g (4.60 mmol) (+/-)-*tert*.-Butyl-1-(4-chlor-3-nitrobenzyl)-2,2-difluorcyclopropancarboxylat wurden in 20 ml Dioxan gelöst und bei RT mit 4.36 g (23.0 mmol) Zinn(II)chlorid und einigen Tropfen 1 N Salzsäure versetzt. Die Reaktionsmischung wurde 1 h auf 70°C erhitzt. Nach Abkühlen auf RT wurde dann mit 10 ml 10%-iger wässriger Kaliumfluorid-Lösung versetzt. Es wurde dreimal mit Ethylacetat extrahiert, und die vereinigten organischen Phasen wurden über Magnesiumsulfat getrocknet und im Vakuum eingeengt. Der Rückstand wurde durch Chromatographie an Silicagel gereinigt (Laufmittel Cyclohexan/Ethylacetat 50:1 → 10:1). Es wurden 1.16 g des Zielprodukts erhalten (79.3% d. Th.).

LC-MS (Methode 4): Rₜ = 1.54 min; m/z = 318 (M+H)⁺.

¹H-NMR (400 MHz, DMSO-d₆): δ [ppm] = 1.32 (s, 9H), 1.79-1.98 (m, 1H), 2.01-2.22 (m, 1H), 2.53 (d, 1H, verdeckt), 3.19 (d, 1H), 5.19-5.34 (m, 2H), 6.19-6.43 (m, 1H), 6.66 (d, 1H), 7.09 (d, 1H).

### Beispiel 36A

### tert.-Butyl-cyclobutancarboxylat

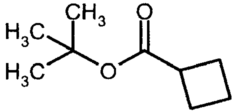

5.2 g (52.3 mmol) Cyclobutancarbonsäure in 100 ml THF wurden unter Argon mit 99 µl (0.78 mmol) Bortrifluorid-Diethylether-Komplex versetzt. Anschließend wurden portionsweise 13.7 g (62.75 mmol) *tert*.-Butyl-2,2,2-trichlorethanimidoat hinzugefügt und die Mischung dann bei Raumtemperatur über Nacht gerührt. Das Reaktionsgemisch wurde danach mit 5 g Natriumhydrogencarbonat versetzt und 15 min nachgerührt. Nach Filtration wurde die Lösung im Vakuum bis zur Trockene eingeengt. Das Rohprodukt wurde durch Chromatographie an Silicagel gereinigt (Laufmittel Cyclohexan/Essigsäureethylester 4:1). Es wurden 5.2 g (64% d. Th.) der Titelverbindung als gelbliches Öl erhalten.

GC-MS (Methode 1): Rₜ = 2.08 min; m/z = 101 (M-C₄H₇)⁺.

### Beispiel 37A

### tert.-Butyl-cyclopentylacetat

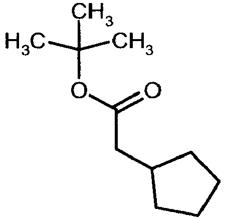

136 ml einer 1 M Lösung von Kalium-*tert*.-butylat in THF (136 mmol) wurden auf 0°C gekühlt und tropfenweise mit 21.0 g (143.2 mmol) Cyclopentylessigsäurechlorid versetzt. Nach Ende der Zugabe wurde die Suspension auf RT erwärmt und über Nacht gerührt, bevor sie auf gesättigte wässrige Ammoniumchlorid-Lösung gegeben wurde. Die Mischung wurde dreimal mit Diethylether extrahiert. Die vereinigten organischen Phasen wurden über Magnesiumsulfat getrocknet und im Vakuum eingeengt. Aus dem Rückstand wurde durch Kugelrohrdestillation (1.6 mbar 160-180°C) das gewünschte Produkt isoliert. Es wurden 17.58 g der Zielverbindung erhalten (66.6% d. Th.).

¹H-NMR (400 MHz, DMSO-d₆): δ [ppm] = 1.02-1.20 (m, 2H), 1.39 (s, 9H), 1.42-1.63 (m, 4H), 1.66-1.81 (m, 2H), 1.98-2.14 (m, 1H), 2.15-2.23 (m, 2H).

### Beispiel 38A

### 4-Cyanophenylessigsäuremethylester

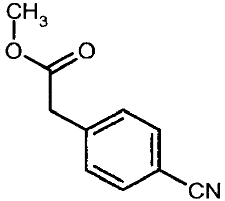

Eine Lösung von 10 g (43.7 mmol) 4-.Bromphenylessigsäuremethylester in 44 ml NMP wurde mit 5.1 g (56.7 mmol) Kupfer(I)cyanid versetzt und in einem Mikrowellengerät für 60 min auf 200°C erhitzt. Das Reaktionsgemisch wurde anschließend durch Flash-Chromatographie an Kieselgel aufgereinigt (Laufmittel Cyclohexan/Ethylacetat 5:1). Es wurden 4.65 g (61% d. Th.) der Titelverbindung erhalten.

GC-MS (Methode 1): Rₜ = 5.13 min; m/z = 175 (M)⁺.

¹H-NMR (400 MHz, DMSO-d₆): δ [ppm] = 3.24-3.35 (m, 3H), 3.83 (s, 2H), 7.49 (d, 2H), 7.80 (d, 2H).

### Beispiel 39A

### Methyl-[4-(trifluormethyl)phenyl]acetat

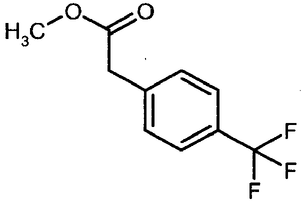

6.0 g (29.4 mmol) [4-(Trifluormethyl)phenyl]essigsäure wurden in 67.1 ml Toluol und 46.2 ml Methanol gelöst und unter Kühlung tropfenweise mit 26.5 ml (52.9 mmol) einer 2 M Lösung von Trimethylsilyldiazomethan in Diethylether versetzt. Nach Ende der Zugabe wurde die Kühlung entfernt und die Mischung 1 h bei RT nachgerührt, bevor überschüssiges Trimethylsilyldiazomethan durch Zugabe von 2.0 ml Essigsäure zerstört wurde. Die Reaktionsmischung wurde im Vakuum eingeengt und das Rohprodukt durch Chromatographie an Silicagel gereinigt (Laufmittel Cyclohexan/Ethylacetat 80:1). Erhalten wurden 4.33 g der Zielverbindung (67.6% d. Th.).

GC-MS (Methode 1): Rₜ = 3.23 min; m/z = 218 (M)⁺.

¹H-NMR (400 MHz, DMSO-*d*₆): δ [ppm] = 3.63 (s, 3H), 3.83 (s, 2H), 7.51 (d, 2H), 7.69 (d, 2H).

Auf analoge Weise wurden die beiden folgenden Verbindungen erhalten:

### Beispiel 40A

### Methyl-[3-fluor-4-(trifluormethyl)phenyl]acetat

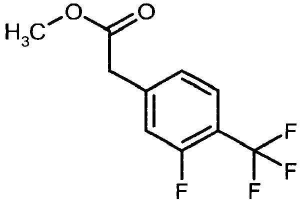

Ausgehend von 1.80 g (8.10 mmol) [3-Fluor-4-(trifluormethyl)phenyl]essigsäure wurden 1.58 g der Zielverbindung erhalten (82.6% d. Th.).

GC-MS (Methode 1): Rₜ = 3.31 min; m/z = 236 (M)⁺.

¹H-NMR (400 MHz, DMSO-*d*₆): δ [ppm] = 3.65 (s, 3H), 3.87 (s, 2H), 7.34 (d, 1H), 7.46 (d, 1H), 7.75 (t, 1H).

### Beispiel 41A

### Methyl-[4-(trifluormethoxy)phenyl]acetat

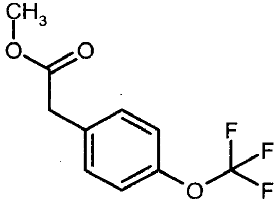

Ausgehend von 1.80 g (8.18 mmol) [4-(Trifluormethoxy)phenyl]essigsäure wurden 1.09 g der Zielverbindung erhalten (56.7% d. Th.).

GC-MS (Methode 1): Rₜ = 3.21 min; m/z = 234 (M)⁺.

¹H-NMR (400 MHz, DMSO-*d*₆): δ [ppm] = 3.62 (s, 3H), 3.75 (s, 2H), 7.26-7.35 (m, 2H), 7.35-7.46 (m, 2H).

### Beispiel 42A

### 1-Brom-4-(2-brom-1-fluorethyl)benzol

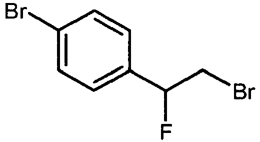

5.0 g (27.31 mmol) 4-Bromstyrol wurden in 40 ml Dichlormethan gelöst, auf 0°C gekühlt und mit 13.21 g (81.94 mmol) Triethylamin-Trihydrofluorid versetzt. Anschließend wurden in drei Portionen 5.83 g (32.78 mmol) *N*-Bromsuccinimid zugegeben. Die Mischung wurde über Nacht bei RT gerührt. Nach Verdünnen mit Dichlormethan wurde die Reaktionsmischung auf Eiswasser gegeben. Die organische Phase wurde nacheinander mit 1 N Salzsäure, Wasser und gesättigter Natriumhydrogencarbonat-Lösung gewaschen, über Magnesiumsulfat getrocknet und im Vakuum eingeengt. Der Rückstand wurde durch Chromatographie an Silicagel gereinigt (Laufmittel Pentan). Es wurden 4.14 g (53.8% d. Th.) der Titelverbindung isoliert.

GC-MS (Methode 1): Rₜ = 4.94 min; m/z = 277/281/283 (M+H)⁺

¹H-NMR (400 MHz, DMSO-*d*₆): δ [ppm] = 3.75-4.04 (m, 2H), 5.84 (dt, 1H), 7.31-7.51 (m, 2H), 7.55-7.78 (m, 2H).

### Beispiel 43A

### 1-Brom-4-(1-fluorvinyl)benzol

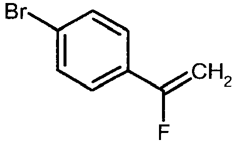

Zu einer auf 0°C gekühlten Lösung von 1.0 g (3.55 mmol) 1-Brom-4-(2-brom-1-fluorethyl)benzol in 10 ml Pentan wurden 796 mg (7.09 mmol) Kalium-*tert*.-butylat in mehreren Portionen gegeben. Die resultierende Suspension wurde 30 min bei 0°C und dann 1 h bei RT gerührt. Der Feststoff wurde abfiltriert, und das Filtrat wurde mit gesättigter Ammoniumchlorid-Lösung gewaschen, über Magnesiumsulfat getrocknet und vorsichtig im Vakuum eingeengt. Es wurden 0.61 g (85.6% d. Th.) der Titelverbindung erhalten.

GC-MS (Methode 1): Rₜ = 3.14 min; m/z = 200/202 (M+H)⁺

¹H-NMR (400 MHz, DMSO-*d*₆): δ [ppm] = 5.10 (dd, 1H), 5.47 (dd, 1H), 7.48-7.61 (m, 2H), 7.62-7.72 (m, 2H).

### Beispiel 44A

### tert.-Butyl-cyclopentyl(4-methylphenyl)acetat

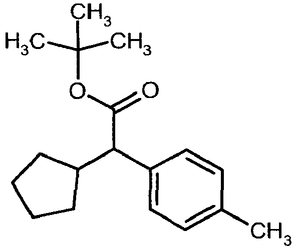

Unter Argon wurden 19.58 g (174.5 mmol) Kalium-*tert*.-butylat in 250 ml DMF vorgelegt, auf 0°C abgekühlt und langsam mit 30 g (145.4 mmol) *tert*.-Butyl-(4-methylphenyl)acetat, gelöst in 50 ml DMF, versetzt. Die Mischung wurde anschließend 30 min bei 0°C gerührt. Danach wurden 18.95 ml (174.5 mmol) Cyclopentylbromid langsam zugetropft und das Gemisch 2 h bei 0°C nachgerührt. Die Reaktionslösung wurde dann mit 200 ml Wasser und 200 ml Diethylether versetzt. Die wässrige Phase wurde zweimal mit Diethylether extrahiert, und die vereinigten organischen Phasen wurden über Magnesiumsulfat getrocknet. Nach Filtration wurde das Lösungsmittel im Vakuum entfernt. Es wurden 36.15 g (132.7 mmol, 91% d. Th.) eines farblosen Feststoffs isoliert.

¹H-NMR (400 MHz, DMSO-d₆, δ/ppm): 7.19 (2H, d), 7.11 (2H, d), 3.12 (1H, d), 2.45-2.29 (1H, m), 2.27 (3H, s), 1.89-1.71 (1H, m), 1.67-1.45 (3H, m), 1.44-1.15 (3H, m), 1.36 (9H, s), 1.02-0.84 (1H, m).

MS (DCI): m/z = 292 (M+NH₄)⁺

GC-MS (Methode 1): Rₜ = 5.89 min; m/z = 218 (M+H-C₄H₉)⁺.

### Beispiel 45A

### tert.-Butyl-[4-(brommethyl)phenyl](cyclopentyl)acetat

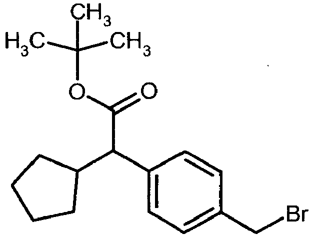

10 g (36.44 mmol) *tert*.-Butyl-cyclopentyl(4-methylphenyl)acetat, 6.811 g (38.26 mmol) *N*-Bromsuccinimid und 299 mg (1.82 mmol) 2,2'-Azobis-(2-methylpropannitril) in 10 ml Tetrachlormethan wurden zwei Stunden unter Rückfluss gerührt. Nach vollständiger Umsetzung wurde das entstandene Succinimid abfiltriert und der Filter-Rückstand mit Dichlormethan nachgewaschen. Die vereinigten Filtrate wurden im Vakuum eingeengt. Das Rohprodukt wurde über Kieselgel chromatographisch gereinigt (Laufmittel Cyclohexan/Essigsäureethylester 50:1). Es wurden 9.9 g (28.04 mmol, 77% d. Th.) eines gelblichen Feststoffs isoliert.

¹H-NMR (400 MHz, DMSO-d₆, δ/ppm): 7.39 (2H, d), 7.30 (2H, d), 4.68 (2H, s), 3.21 (1H, d), 2.45-2.31 (1H, m), 1.89-1.74 (1H, m), 1.69-1.45 (3H, m), 1.44-1.16 (3H, m), 1.35 (9H, s), 1.02-0.88 (1H, m).

MS (DCI): m/z = 370/372 (M+NH₄)⁺.

### Beispiel 46A

### (+/-)-(4-Bromphenyl)(cyclopentyl)essigsäureethylester

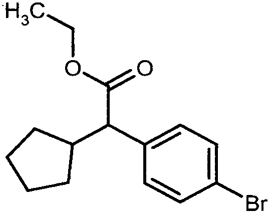

Unter Argonatmosphäre wurden 5.54 g (49.4 mmol) Kalium-*tert*.-butylat in 100 ml DMF gelöst und auf 0°C gekühlt. Anschließend wurden 10 g (41.1 mmol) 4-Bromphenylessigsäureethylester, gelöst in 20 ml DMF, zugegeben. Das Reaktionsgemisch wurde 30 min bei 0°C nachgerührt und anschließend tropfenweise mit 5.29 ml (49.4 mmol) Cyclopentylbromid versetzt. Das Gemisch wurde 1 h bei 0-5°C gerührt und anschließend auf Wasser (1 Liter) gegeben und mit Ethylacetat extrahiert. Die organische Phase wurde mit gesättigter Kochsalz-Lösung gewaschen, über Magnesiumsulfat getrocknet und eingeengt. Es wurden 12.41 g (97% d. Th.) der Titelverbindung in Form eines gelblichen Öls erhalten.

LC-MS (Methode 5): Rₜ = 1.44 min; m/z = 311/313 (M+H)⁺.

¹H-NMR (400 MHz, DMSO-d₆): δ [ppm] = 0.89-1.04 (m, 1H), 1.05-1.69 (m, 9H), 1.72-1.86 (m, 1H), 2.34-2.48 (m, 1H), 3.37 (d, 1H), 3.92-4.17 (m, 2H), 7.24-7.37 (m, 2H), 7.44-7.57 (m, 2H).

### Beispiel 47A

### (+/-)-(4-Cyanophenyl)(cyclopentyl)essigsäuremethylester

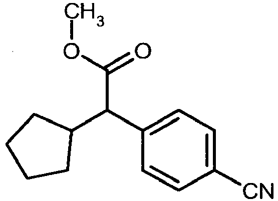

Unter Argonatmosphäre wurden 2.56 g (22.8 mmol) Kalium-*tert*.-butylat in 20 ml DMF gelöst, auf 0°C gekühlt und tropfenweise mit 2 g (11.4 mmol) 4-Cyanophenylessigsäuremethylester versetzt. Nach beendeter Zugabe wurden 1.47 ml (13.7 mmol) Cyclopentylbromid langsam zugetropft. Das Reaktionsgemisch wurde 2 h bei 0°C und anschließend über Nacht bei RT gerührt. Danach wurde das Reaktionsgemisch mit Wasser versetzt und 15 min gerührt, dann mit Ethylacetat versetzt und weitere 15 min gerührt. Die organische Phase wurde abgetrennt und die wässrige Phase mehrfach mit Ethylacetat extrahiert. Die vereinigten organischen Phasen wurden über Magnesiumsulfat getrocknet und eingeengt. Der Rückstand wurde flash-chromatographisch über Kieselgel gereinigt (Laufmittel Cyclohexan/Ethylacetat 5:1). Es wurden 1.29 g (46% d. Th.) der Titelverbindung erhalten.

LC-MS (Methode 5): Rₜ = 1.19 min; m/z = 244 (M+H)⁺.

### Beispiel 48A

### (+/-)-(4-Nitrophenyl)(cyclopentyl)essigsäureethylester

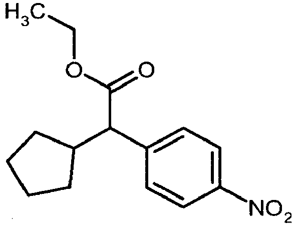

Unter Argon wurden 644 mg (5.7 mmol) Kalium-*tert*.-butylat in 10 ml DMF gelöst und auf 0°C gekühlt. Anschließend wurden 1000 mg (4.8 mmol) 4-Nitrophenylessigsäureethylester, gelöst in 2 ml DMF, zugegeben. Das Reaktionsgemisch wurde 30 min bei 0°C gerührt und dann tropfenweise mit 855 mg (5.7 mmol) Cyclopentylbromid versetzt. Das Reaktionsgemisch wurde anschließend für 1.5 h auf 70°C erhitzt, dann abgekühlt, in Wasser gegossen und mit Ethylacetat extrahiert. Die organische Phase wurde über Natriumsulfat getrocknet und eingeengt. Das erhaltene Rohprodukt wurde flash-chromatographisch über Kieselgel gereinigt (Laufmittel Isohexan/Ethylacetat 10:1). Es wurden 716 mg (51% d. Th.) der Titelverbindung erhalten.

LC-MS (Methode 5): Rₜ = 1.32 min; m/z = 278 (M+H)⁺.

¹H-NMR (400 MHz, DMSO-d₆): δ [ppm] = 0.91-1.05 (m, 1H), 1.14 (t, 3H), 1.19-1.70 (m, 6H), 1.74-1.89 (m, 1H), 3.62 (d, 1H), 3.96-4.18 (m, 2H), 7.65 (d, 2H), 8.20 (d, 2H).

### Beispiel 49A

### tert.-Butyl-[4-(acetoxymethyl)phenyl](cyclopentyl)acetat

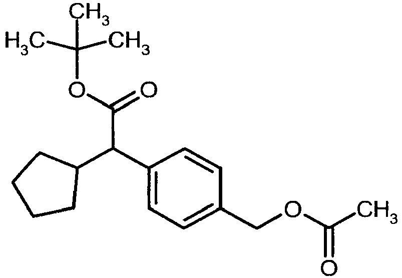

16.3 g (84.9 mmol) Cäsiumacetat wurden in 80 ml DMF vorgelegt und bei RT mit 20.0 g (ca. 75% Reinheit, ca. 42.5 mmol) *tert*.-Butyl-[4-(brommethyl)phenyl](cyclopentyl)acetat versetzt. Das Gemisch wurde 1.5 h bei 50°C kräftig gerührt und dann nach Abkühlen auf 100 ml Ethylacetat gegeben. Die organische Phase wurde nacheinander mit 80 ml Wasser und 80 ml gesättigter Natriumchlorid-Lösung gewaschen, über Magnesiumsulfat getrocknet und im Vakuum eingeengt. Nach Chromatographie an Silicagel (Laufmittel Cyclohexan/Ethylacetat 40:1 → 10:1) wurden 11.72 g der Zielverbindung erhalten (76.4% d. Th.).

LC-MS (Methode 5): Rₜ = 1.42 min; m/z = 350 (M+H₂O)⁺.

¹H-NMR (400 MHz, DMSO-*d*₆): δ [ppm] = 0.82-1.02 (m, 1H), 1.15-1.31 (m, 2H), 1.35 (s, 9H), 1.38-1.47 (m, 1H), 1.47-1.69 (m, 3H), 1.76-1.88 (m, 1H), 2.06 (s, 3H), 2.32-2.45 (m, 1H), 3.21 (d, 1H), 5.04 (s, 2H), 7.22-7.38 (m, 4H).

### Beispiel 50A

### Methyl-(4-chlorphenyl)(cyclopentyl)acetat

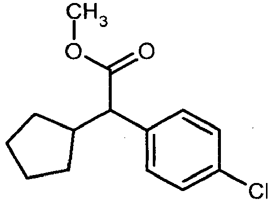

Eine Suspension von 3.65 g (32.5 mmol) Kalium-*tert*.-butylat in 65 ml abs. DMF wurde auf 0°C gekühlt und tropfenweise mit einer Lösung von 5.0 g (27.08 mmol) Methyl-4-chlorphenylacetat in ca. 2 ml abs. DMF versetzt. Die Mischung wurde 30 min bei 0°C gerührt, bevor 4.84 g (32.5 mmol) Cyclopentylbromid langsam zugetropft wurden. Die Reaktionsmischung wurde 1 h bei 0°C gerührt und dann auf Wasser gegeben und mit Ethylacetat extrahiert. Die organische Phase wurde über Natriumsulfat getrocknet, im Vakuum eingeengt und der Rückstand im Hochvakuum getrocknet. Das Rohprodukt wurde durch Chromatographie an Silicagel gereinigt (Laufmittel Cyclohexan/ Ethylacetat 100:1). Es wurden 6.28 g der Zielverbindung erhalten (91.8% d. Th.).

GC-MS (Methode 1): Rₜ = 6.07 min; m/z = 193 (M-C₂H₃O₂)⁺.

¹H-NMR (400 MHz, DMSO-*d*₆): δ [ppm] = 0.96-1.04 (m, 1H), 1.08-1.37 (m, 2H), 1.37-1.48 (m, 1H), 1.49-1.70 (m, 3H), 1.79 (dtd, 1H), 2.33-2.50 (m, 1H), 3.42 (d, 1H), 3.58 (s, 3H), 7.29-7.46 (m, 4H).

Auf analoge Weise wurden die folgenden Verbindungen hergestellt:

### Beispiel 51A

### Methyl-cyclopentyl[4-(trifluormethyl)phenyl]acetat

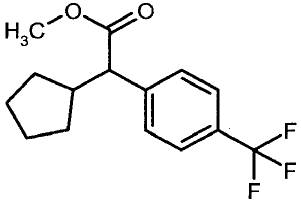

Ausgehend von 4.3 g (19.7 mmol) Methyl-[4-(trifluonnethyl)phenyl]acetat und 3.53 g (23.7 mmol) Cyclopentylbromid wurden 4.98 g der Zielverbindung erhalten (88.2% d. Th.).

LC-MS (Methode 4): Rₜ = 1.57 min; m/z = 287 (M+H)⁺.

¹H-NMR (400 MHz, DMSO-d₆): δ [ppm] = 0.90-1.04 (m, 1H), 1.16-1.33 (m, 2H), 1.37-1.49 (m, 1H), 1.49-1.70 (m, 3H), 1.76-1.88 (m, 1H), 2.41.-2.49 (m, 1H), 3.56 (d, 1H), 3.60 (s, 3H), 7.53-7.62 (m, 2H), 7.66-7.74 (m, 2H).

### Beispiel 52A

### Methyl-cyclopentyl(3,4-dichlorphenyl)acetat

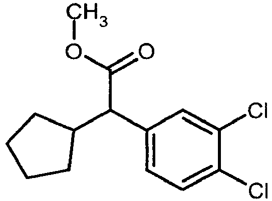

Ausgehend von 1.5 g (6.85 mmol) 3,4-Dichlorphenylessigsäuremethylester und 1.22 g (8.22 mmol) Cyclopentylbromid wurden 0.70 g der Zielverbindung erhalten (35.6% d. Th.).

MS (DCI): m/z = 304 (M+NH₄)⁺.

¹H-NMR (400 MHz, DMSO-*d*₆): δ [ppm] = 0.86-1.08 (m, 1H), 1.12-1.26 (m, 1H), 1.26-1.36 (m, 1H), 1.38-1.49 (m, 1H), 1.49-1.68 (m, 3H), 1.73-1.83 (m, 1H), 2.36-2.47 (m, 1H), 3.50 (d, 1H), 3.60 (s, 3H), 7.32-7.41 (m, 1H), 7.48-7.54 (m, 1H), 7.57-7.63 (m, 1H).

### Beispiel 53A

### Methyl-(4-chlor-2-fluorphenyl)(cyclopentyl)acetat

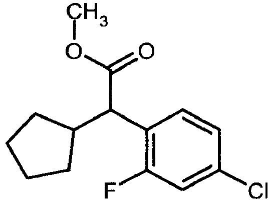

Ausgehend von 6.5 g (32.1 mmol) Methyl-(4-chlor-2-fluorphenyl)acetat und 5.74 g (38.5 mmol) Cyclopentylbromid wurden 7.55 g der Zielverbindung erhalten (86.9% d. Th.).

LC-MS (Methode 5): Rₜ = 1.07 min; m/z = 271 (M+H)⁺.

¹H-NMR (400 MHz, DMSO-*d*₆): δ [ppm] = 0.88-1.01 (m, 1H), 1.20-1.30 (m, 1H), 1.34-1.64 (m, 5H), 1.79-1.91 (m, 1H), 2.41-2.48 (m, 1H), 3.60 (s, 3H), 3.69 (d, 1H), 7.30 (dd, 1H), 7.43 (dd, 1H), 7.48 (t, 1H).

### Beispiel 54A

### Ethyl-cyclopentyl(2,4-dichlorphenyl)acetat

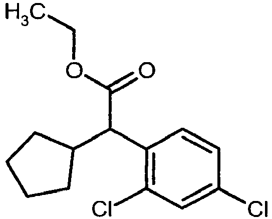

Ausgehend von 1.5 g (6.43 mmol) Ethyl-(2,4-dichlorphenyl)acetat und 1.15 g (7.72 mmol) Cyclopentylbromid wurden 1.60 g der Zielverbindung erhalten (82.8% d. Th.).

LC-MS (Methode 5): Rₜ = 1.52 min; m/z = 191.

¹H-NMR (400 MHz, DMSO-d₆): δ [ppm] = 0.86-1.01 (m, 1H), 1.13 (t, 3H), 1.25-1.40 (m, 2H), 1.40-1.49 (m, 1H), 1.49-1.70 (m, 3H), 1.79-1.91 (m, 1H), 2.45-2.53 (m, 1H), 3.87 (d, 1H), 3.99-4.13 (m, 2H), 7.45 (dd, 1H), 7.54 (d, 1H), 7.63 (d, 1H).

### Beispiel 55A

### Methyl-cyclopentyl[3-fluor-4-(trifluormethyl)phenyl]acetat

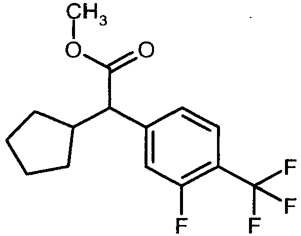

Ausgehend von 1.50 g (6.35 mmol) Methyl-[3-fluor-4-(trifluormethyl)phenyl]acetat und 1.14 g (7.62 mmol) Cyclopentylbromid wurden 1.78 g der Zielverbindung erhalten (92.1 % d. Th.).

¹H-NMR (400 MHz, DMSO-*d*₆): δ [ppm] = 0.93-1.06 (m, 1H), 1.19-1.35 (m, 2H), 1.38-1.67 (m, 4H), 1.75-1.86 (m, 1H), 2.41-2.49 (m, 1H), 3.61 (s, 3H), 3.62 (d, 1H), 7.41 (d, 1H), 7.52 (d, 1H), 7.76 (t, 1H).

### Beispiel 56A

### Methyl-cyclopentyl[4-(trifluormethoxy)phenyl]acetat

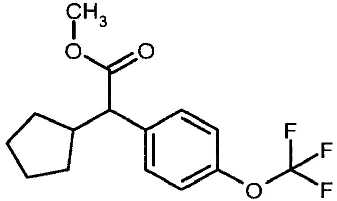

Ausgehend von 1.0 g (4.27 mmol) Methyl-[4-(trifluormethoxy)phenyl]acetat und 0.76 g (5.12 mmol) Cyclopentylbromid wurden 1.09 g der Zielverbindung erhalten (71.4% d. Th.).

MS (DCI): m/z = 320 (M+NH₄)⁺.

¹H-NMR (400 MHz, DMSO-*d*₆): δ [ppm] = 0.93-1.02 (m, 1H), 1.15-1.33 (m, 2H), 1.35-1.48 (m, 1H), 1.48-1.68 (m, 3H), 1.73-1.86 (m, 1H), 2.40-2.49 (m, 1H), 3.48 (d, 1H), 3.59 (s, 3H), 7.28-7.36 (m, 2H), 7.42-7.51 (m, 2H).

### Beispiel 57A

### (+/-)-Cyclopentyl(4-fluorphenyl)essigsäure-tert.-butylester

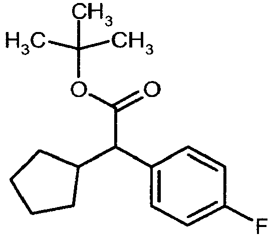

Unter Argon wurde zu 16.3 ml einer auf -10°C gekühlten 1 M Lösung von Lithiumhexamethyldisilazid in Toluol (16.3 mmol) eine Lösung von 2.0 g (10.85 mmol) *tert*.-Butylcyclopentylacetat in 5 ml abs. Toluol getropft. Nach 10 min bei -10°C wurde das Eisbad entfernt und die Mischung auf RT erwärmt. Zu dieser Reaktionsmischung wurde dann eine Mischung von 2.47 g (14.1 mmol) 1-Brom-4-fluorbenzol, 73.1 mg (0.326 mmol) Palladium(II)acetat und 269 mg (0.684 mmol) 2-Dicyclohexylphosphino-2'-(*N*,*N*-dimethylamino)-biphenyl in 10 ml abs. Toluol gegeben. Nach 1 h bei RT wurde die Reaktionsmischung über Nacht bei 80°C gerührt. Nach Abkühlen auf RT wurde von den ausgefallenen Salzen abgesaugt, der Rückstand mit Toluol nachgewaschen und das vereinigte Filtrat im Vakuum eingeengt. Das erhaltene Rohprodukt wurde über Kieselgel chromatographisch gereinigt (Laufmittel zunächst Cyclohexan, dann Cyclohexan/Ethylacetat 50:1). Es wurden 2.0 g der Titelverbindung isoliert (61.6% d. Th.).

¹H-NMR (400 MHz, DMSO-*d*₆): δ [ppm] = 0.89-1.02 (m, 1H), 1.19-1.30 (m, 2H), 1.35 (s, 9H), 1.46-1.66 (m, 4H), 1.76-1.87 (m, 1H), 2.31-2.42 (m, 1H), 3.23 (d, 1H), 7.09-7.19 (m, 2H), 7.31-7.41 (m, 2H).

### Beispiel 58A

### Ethyl-2-(4-ethylphenyl)-4,4,4-trifluor-3-methylbutanoat (racemisches Diastereomerengemisch)

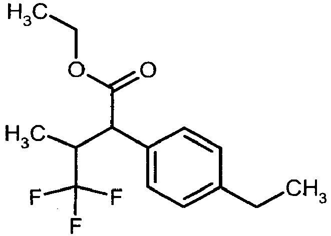

Unter Argon wurde zu 8.1 ml einer auf -10°C gekühlten 1 M Lösung von Lithiumhexamethyldisilazid in Toluol (8.1. mmol) eine Lösung von 1.29 g (7.02 mmol) (+/-)-Ethyl-4,4,4-trifluor-3-methylbutanoat in 2 ml abs. Toluol getropft. Nach 10 min bei -10°C wurde zu dieser Reaktionsmischung eine Mischung von 1.0 g (5.4 mmol) 1-Brom-4-ethylbenzol, 36.4 mg (0.16 mmol) Palladium(II)acetat und 134 mg (0.34 mmol) 2-Dicyclohexylphosphino-2'-(*N*,*N*-dimethylamino)-biphenyl in 5 ml abs. Toluol gegeben. Nach Ende der Zugabe wurde auf RT erwärmt und zunächst 1 h bei RT und dann 3 h bei 80°C weiter gerührt. Nach Abkühlen auf RT wurde von den ausgefallenen Salzen abgesaugt, der Rückstand mit Toluol nachgewaschen und das vereinigte Filtrat im Vakuum eingeengt. Es wurden 1.26 g Rohprodukt erhalten (80.9% d. Th.), das als solches weiter umgesetzt wurde.

GC-MS (Methode 1): Rₜ = 4.51 min und 4.53 min; jeweils m/z = 288 (M)⁺ (Diastereomerenverhältnis ca. 1:2.3).

### Beispiel 59A

### (1R,2S,5R)-5-Methyl-2-(propan-2-yl)cyclohexyl-(4-methylphenyl)acetat

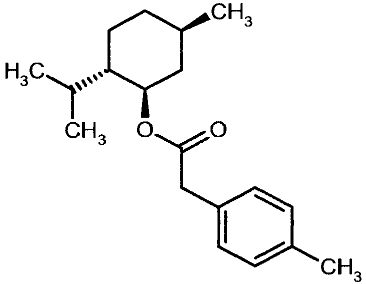

303.7 g (2022.46 mmol) (4-Methylphenyl)essigsäure und 301 g (1926.2 mmol) (1*R*,2*S*,5*R*)-5-Methyl-2-(propan-2-yl)cyclohexanol wurden in 933 ml Toluol vorgelegt, mit 2.5 ml (38.5 mmol) Methansulfonsäure versetzt und über Nacht unter Rückfluss an einem Wasserabscheider gerührt. Danach ließ man abkühlen und versetzte die Reaktionslösung mit einer Mischung aus 30 ml 45%-iger Natronlauge und 400 ml Wasser. Nach 30 min wurden die Phasen getrennt, und die organische Phase wurde über Natriumsulfat getrocknet, filtriert und am Rotationsverdampfer eingeengt. Es wurden 569.5 g des Zielprodukts erhalten (97% d. Th.).

¹H-NMR (500 MHz, DMSO-*d*₆): δ [ppm] = 7.12 (s, 4H), 4.56 (td, 1H), 3.57 (s, 2H), 2.50 (br. s, 1H), 2.27 (s, 3H), 1.84 (d, 1H), 1.77-1.70 (m, 1H), 1.66-1.57 (m, 2H), 1.48-1.37 (m, 1H), 1.32 (t, 1H), 1.10-0.89 (m, 2H), 0.86 (d, 3H), 0.81 (d, 3H), 0.65 (d, 3H).

### Beispiel 60A

### (1R,2S,5R)-2-Isopropyl-5-methylcyclohexyl-(2S)-cyclopentyl(4-methylphenyl)acetat

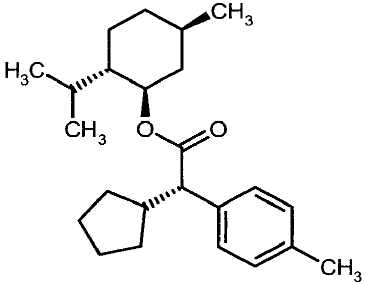

Unter Argon wurden 442.73 g (3945.5 mmol) Kalium-*tert*.-butylat in 1230 ml DMF bei -10°C vorgelegt und portionsweise mit 569 g (1972.7 mmol) (1*R*,2*S*,5*R*)-5-Methyl-2-(propan-2-yl)cyclohexyl-(4-methylphenyl)acetat versetzt. Anschließend wurden 352.81 g (2367.8 mmol) Cyclopentylbromid zugetropft, wobei die Temperatur zwischen -5°C und -10°C gehalten wurde. Nach 90 min Nachrühren bei -10°C wurde mit 1.6 Liter Wasser versetzt und 15 min bei RT gerührt. Es wurden 1.2 Liter Essigsäureethylester zugegeben, erneut 15 min gerührt und anschließend die Phasen getrennt. Die organische Phase wurde über Natriumsulfat getrocknet, filtriert und am Rotationsverdampfer eingeengt. Das Rohprodukt wurde in 2 Liter Methanol bei 50°C umkristallisiert. Es wurden 423.0 g des Zielprodukts erhalten (60% d. Th.).

¹H-NMR (400 MHz, DMSO-*d*₆): δ [ppm] = 7.19 (d, 2H), 7.11 (d, 2H), 4.55 (td, 1H), 3.26 (d, 1H), 2.27 (s, 3H), 1.83-1.73 (m, 2H), 1.68-1.24 (m, 11H), 1.23-1.13 (m, 1H), 1.04-0.94 (m, 2H), 0.88-0.77 (m, 8H), 0.66 (d, 3H).

### Beispiel 61A

### (1R,2S,5R)-2-Isopropyl-5-methylcyclohexyl-(2S)-[4-(brommethyl)phenyl](cyclopentyl)acetat

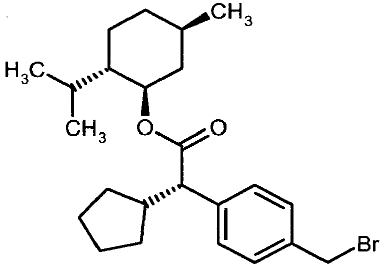

Die Titelverbindung kann gemäß US-Patent 5,714,494 durch Bromierung von (1*R*,2*S*,5*R*)-2-Isopropyl-5-methylcyclohexyl-(2*S*)-cyclopentyl(4-methylphenyl)acetat mit *N*-Bromsuccinimid (NBS) in Gegenwart von 2,2'-Azobis-(2-methylpropannitril) (AIBN) in siedendem Tetrachlorkohlenstoff hergestellt werden.

GC-MS (Methode 1): Rₜ = 9.15 min; keine Ionisation.

LC-MS (Methode 2): Rₜ = 3.54 min; keine Ionisation.

MS (DCI): m/z = 452/454 (M+NH₄)⁺.

### Beispiel 62A

### (-)-(1R,2S,5R)-2-Isopropyl-5-methylcyclohexyl-(2S)-cyclopentyl(4-ethylphenyl)acetat

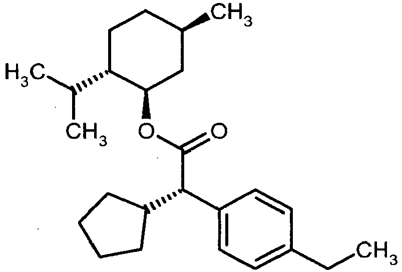

14.4 ml einer 1.6 M Lösung von Methyllithium in Diethylether (23.0 mmol) wurden auf 0°C gekühlt und mit 2.30 g (12.06 mmol) trockenem Kupfer(I)iodid versetzt. Die orange-gelbe Suspension wurde auf -78°C abgekühlt und tropfenweise mit einer Lösung von 5.0 g (11.48 mmol) (1*R*,2*S*,5*R*)-2-Isopropyl-5-methylcyclohexyl-(2*S*)-[4-(brommethyl)phenyl](cyclopentyl)acetat in 12.5 ml abs. THF versetzt. Nach Ende der Zugabe wurde die Reaktionsmischung langsam auf 0°C erwärmt und zunächst 3 h bei 0°C und dann 2 h bei RT gerührt. Danach wurden 200 ml 25%-ige wässrige Ammoniak-Lösung und 10 g Ammoniumacetat hinzugefügt. Die Mischung wurde 10 min kräftig gerührt und dann über Nacht ohne Rühren stehen gelassen. Nach Phasentrennung wurde die wässrige Phase zweimal mit Diethylether extrahiert. Die vereinigten organischen Phasen wurden mit gesättigter Ammoniumchlorid-Lösung gewaschen, über Magnesiumsulfat getrocknet und im Vakuum eingeengt. Der Rückstand wurde an Silicagel chromatographisch gereinigt (Laufmittel Cyclohexan/Dichlormethan 10:1 → 5:1). Es wurden 3.33 g der Zielverbindung erhalten (78.2% d. Th.).

MS (DCI): m/z = 388 (M+NH₄)⁺.

¹H-NMR (400 MHz, DMSO-*d*₆): δ [ppm] = 0.60-0.68 (m, 3H), 0.78-0.87 (m, 8H), 0.93-1.05 (m, 2H), 1.16 (t, 3H), 1.16-1.22 (m, 1H), 1.27-1.47 (m, 4H), 1.48-1.69 (m, 6H), 1.70-1.83 (m, 2H), 2.38-2.48 (m, 1H), 2.57 (q, 2H), 3.29 (d, 1H), 4.55 (td, 1H), 7.12-7.16 (m, 2H), 7.20-7.25 (m, 2H).

[α]_{D}²⁰ = -37.5°, c = 0.51, Chloroform.

### Beispiel 63A

### 1-Brom-4-(1,1-difluorethyl)benzol

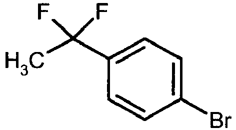

Unter Argon wurden bei Raumtemperatur 3.0 g (15.07 mmol) 1-(4-Bromphenyl)ethanon in 30 ml Dichlormethan vorgelegt und langsam mit 15.9 ml (120.57 mmol) [Ethyl(trifluor-λ⁴-sulfanyl)-amino]ethan (DAST) versetzt. Anschließend wurde die Reaktionslösung langsam auf 50°C erwärmt und über Nacht bei dieser Temperatur nachgerührt. Nach beendeter Umsetzung wurde die Reaktionslösung langsam auf Eiswasser gegossen. Anschließend wurde die organische Phase abgetrennt, und die wässrige Phase wurde noch dreimal mit Dichlormethan extrahiert. Die vereinigten organischen Phasen wurden über Magnesiumsulfat getrocknet. Nach Filtration wurde das Lösungsmittel im Vakuum entfernt. Das Rohprodukt wurde über Kieselgel chromatographisch gereinigt (Laufmittel Petrolether/Dichlormethan 4:1). Es wurden 2.56 g (11.58 mmol, 77% d. Th.) der Titelverbindung als gelbliche Flüssigkeit isoliert.

GC-MS (Methode 1): Rₜ = 2.84 min; m/z = 220/222 (M)⁺.

### Beispiel 64A

### (+)-Ethyl-(3R)-4,4,4-trifluor-3-methylbutanoat

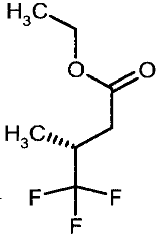

287 g (1.65 mol) (3*R*)-4,4,4-Trifluor-3-methylbutansäure [A. Gerlach und U. Schulz, Speciality Chemicals Magazine 24 (4), 37-38 (2004); CAS Acc.-Nr. 142:179196] in 580 ml Ethanol wurden bei Raumtemperatur langsam mit 133 ml (1.82 mol) Thionylchlorid versetzt. Die Reaktionslösung wurde anschließend auf 80°C erwärmt und 2 h bei dieser Temperatur gerührt. Danach wurde auf Raumtemperatur abgekühlt, langsam mit 250 ml Wasser versetzt und dreimal mit je 150 ml *tert.-*Butylmethylether extrahiert. Die vereinigten organischen Phasen wurden über Natriumsulfat getrocknet. Nach Filtration wurde das Lösungsmittel im Vakuum bei 30°C und einem Druck von 300 mbar entfernt. Das Rohprodukt wurde anschließend bei 100 mbar und einer Kopftemperatur von 65°C destilliert. Es wurden 225.8 g (113 mol, 74% d. Th.) der Titelverbindung als farblose Flüssigkeit isoliert.

¹H-NMR (400 MHz, DMSO-d₆, δ/ppm): 4.10 (2H, q), 2.88-2.72 (1H, m), 2.66-2.57 (1H, m), 2.46-2.36 (1H, m), 1.19 (3H, t), 1.11 (3H, d).

GC-MS (Methode 1): Rₜ = 1.19 min; m/z = 184 (M)⁺.

[α]_{D}²⁰ = +16.1°, c = 0.41, Methanol.

### Beispiel 65A

### Ethyl-4,4,4-trifluor-3-methyl-2-(4-methylphenyl)butanoat (Diastereomerengemisch)

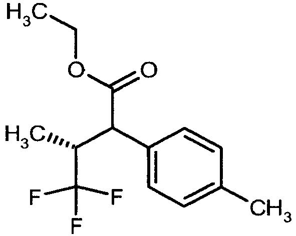

Unter Argon wurden 196.9 mg (0.88 mmol) Palladium(II)acetat und 724.8 mg (1.84 mmol) 2-Dicyclohexylphosphino-2'-(*N,N*-dimethylamino)-biphenyl in 50 ml wasserfreiem Toluol vorgelegt. Die Reaktionslösung wurde anschließend langsam mit 43.8 ml (43.8 mmol) einer 1 M Lösung von Lithiumhexamethyldisilazid in THF versetzt und 10 min bei RT gerührt. Nachfolgend wurde die Reaktionslösung auf -10°C abgekühlt, langsam mit 7 g (38.0 mmol) (+/-)-Ethyl-4,4,4-trifluor-3-methylbutanoat versetzt und 10 min bei -10°C nachgerührt. Danach wurden 5 g (29.2 mmol) 4-Bromtoluol, gelöst in 50 ml Toluol, zugetropft und die Reaktionslösung erst auf RT und dann auf 80°C erwärmt. Das Gemisch wurde 2 h bei dieser Temperatur gerührt, dann auf RT abgekühlt und über Nacht nachgerührt. Nach erfolgter Umsetzung (DC-Kontrolle; Laufmittel Cyclohexan/ Dichlormethan 2:1) wurde die Reaktionsmischung über Kieselgur filtriert, der Rückstand mehrfach mit Essigsäureethylester und Dichlormethan gewaschen und die vereinigten Filtrate im Vakuum eingeengt. Das erhaltene Rohprodukt wurde über Kieselgel chromatographisch gereinigt (Laufmittel Petrolether/Dichlormethan 4:1 → 3:1). Es wurden 3.91 g (14.3 mmol, 48.8% d. Th.) der Titelverbindung als farblose Flüssigkeit isoliert.

¹H-NMR (400 MHz, DMSO-d₆, δ/ppm): 7.26 (2H, d), 7.20-7.12 (2H, m), 4.17-3.95 (2H, m), 3.74 (0.25H, d), 3.66 (0.75H, d), 3.35-3.07 (1H, m), 2.29 (2.25H, s), 2.28 (0.75H, s), 1.17 (0.75H, d), 1.11 (3H, t), 0.76 (2.25H, d).

GC-MS (Methode 1): Rₜ = 4.20 min; m/z = 275 (M+H)⁺ (*Diastereomer 1*); Rₜ = 4.23 min; m/z = 275 (M+H)⁺ (*Diastereomer 2*).

### Beispiel 66A

### Ethyl-(3R)-2-(4-chlorphenyl)-4,4,4-trifluor-3-methylbutanoat (Diastereomerengemisch)

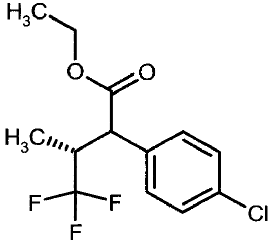

Herstellung von Lösung A: 163.9 ml einer 1 M Lösung von Lithiumhexamethyldisilazid in Toluol wurden unter Argon auf -10°C bis -20°C gekühlt (Kühlung mittels Aceton/Trockeneis) und langsam mit 20 g (108.6 mmol) (+)-Ethyl-(3*R*)-4,4,4-trifluor-3-methylbutanoat, gelöst in 150 ml Toluol, versetzt, wobei darauf geachtet wurde, dass eine Temperatur von -10°C nicht überschritten wurde. Die Lösung wurde anschließend 10 min bei maximal -10°C nachgerührt.

Herstellung von Lösung B: 27.03 g (141.2 mmol) 1-Brom-4-chlorbenzol wurden unter Argon bei RT in 100 ml Toluol gelöst und mit 731 mg (3.26 mmol) Palladium(II)acetat und 2.693 g (6.84 mmol) 2-Dicyclohexylphosphino-2'-(N,N-dimethylamino)-biphenyl versetzt. Die Lösung wurde 10 min bei RT nachgerührt.

Zunächst wurde das Kühlbad von Lösung A entfernt. Anschließend wurde zu der noch kalten Lösung A langsam die Lösung B hinzugetropft. Die nun vereinigten Lösungen wurden langsam auf RT erwärmt und 1 h bei dieser Temperatur gerührt. Anschließend wurde die Reaktionslösung auf 80°C (Innentemperatur) erwärmt und 3 h bei dieser Temperatur gerührt. Danach wurde die Reaktionslösung langsam auf RT abgekühlt und noch 12 h nachgerührt. Das Reaktionsgemisch wurde schließlich über Kieselgur filtriert, der Rückstand mehrfach mit Toluol nachgewaschen und die vereinigten Filtrate im Vakuum eingeengt. Das erhaltene Rohprodukt wurde über Kieselgel chromatographisch gereinigt (Laufmittel Cyclohexan/Dichlormethan 4:1). Es wurden 27.4 g (92.98 mmol, 86% d. Th.) der Titelverbindung als gelbes Öl in einem Diastereomerenverhältnis von 3:1 isoliert.

GC-MS (Methode 1): Rₜ = 4.45 min; m/z = 294 (M)⁺ (*Diastereomer 1*); Rₜ = 4.48 min; m/z = 294 (*M*)⁺ (*Diastereomer 2*)*.*

Analog zu Synthesebeispiel 65A und 66A wurden die folgenden Verbindungen erhalten:

| **Beispiel** | **Name / Struktur / Edukte** | **Analytische Daten** |
|---|---|---|
| **67A** | Ethyl-(3*R*)-4,4,4-trifluor-2-(4-isopropylphenyl)-3-methylbutanoat | GC-MS (Methode 1): Rₜ = 4.61 min; m/z = 302 (M)⁺ (*Diastereomer 1*); Rₜ = 4.64 min; m/z = 302 (M)⁺ *(Diastereomer 2*). |
| | 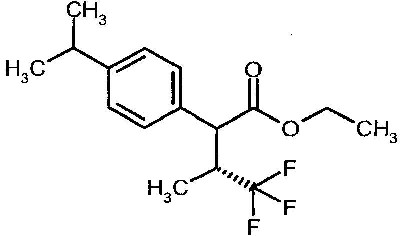 | |
| | aus 1-Brom-4-isopropylbenzol und Ethyl-(3R)-4,4,4-trifluor-3-melhylbutanoat | |
| **68A** | Ethyl-(3R)-2-(4-*tert*.-butylphenyl)-4,4,4-trifluor-3-methylbutanoat | GC-MS (Methode 1): Rₜ = 4.83 min; m/z = 317 (M+H)⁺ (*Diastereomer 1*); Rₜ = 4.85 min; m/z = 317 (M+H)⁺ *(Diastereomer 2*)*.* |
| | 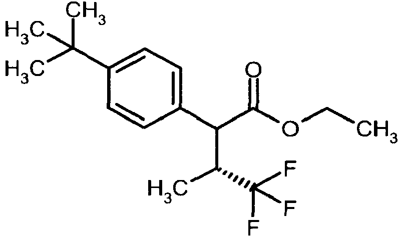 | |
| | | MS (DCI): m/z = 334 (M+NH₄)⁺. |
| | aus 1-Brom-4-*tert*.-butylbenzol und Ethyl-(3R)-4,4,4-trifluor-3-methylbutanoat | |
| **69A** | Ethyl-4,4,4-trifluor-3-methyl-2-[4-(trifluormethyl)-phenyl]butanoat | GC-MS (Methode 1): Rₜ = 3.43 min; m/z = 328 (M)⁺ (*Diastereomere 1*); Rₜ = 3.47 min; m/z = 328 (M)⁺ *(Diastereomer 2*). |
| | 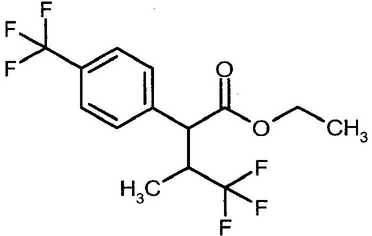 | |
| | aus 1-Brom-4-(trifluormethyl)benzol und Ethyl-4,4,4-trifluor-3-methylbutanoat | |
| **70A** | Ethyl-(3*R*)-4,4,4-trifluor-3-methyl-2-[4-(trifluormethyl)phenyl]butanoat | GC-MS (Methode 1): Rₜ = 3.38 min; m/z = 328 (M)⁺ *(Diastereomer 1*); Rₜ = 3.42 min; m/z = 328 (M)⁺ *(Diastereomer 2*). |
| | 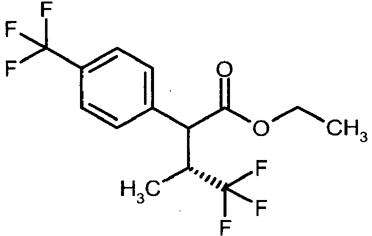 | |
| | aus 1-Brom-4-(trifluormethyl)benzol und Ethyl-(3*R*)-4,4,4-trifluor-3-methylbutanoat | |
| **71A** | Ethyl-(3*R*)-4,4,4-trifluor-3-methyl-2-[4-(1,1,1-trifluor-2-methylpropan-2-yl)phenyl]butanoat | GC-MS (Methode 1): Rₜ = 4.68 min; m/z = 370 (M)⁺. |
| | 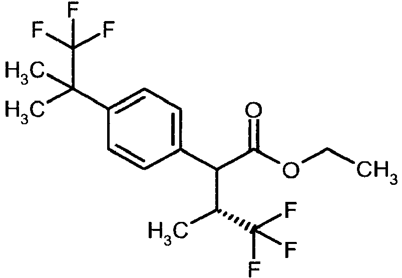 | |
| | aus 1-Brom-4-(1,1,1-trifluor-2-methylpropan-2-yl)-benzol und Ethyl-(3*R*)-4,4,4-trilluor-3-methylbutanoat | |
| **72A** | Ethyl-(3*R*)-2-[4-(1,1-difluorethyl)phenyl]-4,4,4-trifluor-3-methylbutanoat | GC-MS (Methode 1): Rₜ = 4.31 min; m/z = 304 (M-HF)⁺ (*Diastereomere 1*); Rₜ = 4.35 min; m/z = 304 (M-HF)⁺ (*Diastereomer 2).* |
| | 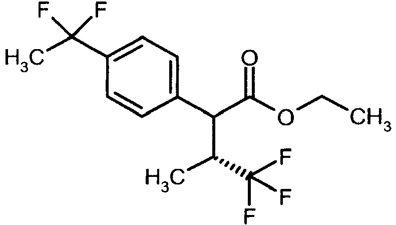 | |
| | | MS (DCI): m/z = 342 (M+NH₄)⁺. |
| | aus 1-Brom-4-(1,1-difluorethyl)benzol und Ethyl-(3*R*)-4,4,4-trifluor-3-methylbutanoat | |
| **73A** | Ethyl-(3*R*)-2-(4-cyclopropylphenyl)-4,4,4-trifluor-3-methylbutanoat | GC-MS (Methode 1): Rₜ = 5.19 min; m/z = 300 (M)⁺ *(Diastereomer 1*); Rₜ = 5.21 min; m/z = 300 (M)⁺ (*Diastereomer 2*). |
| | 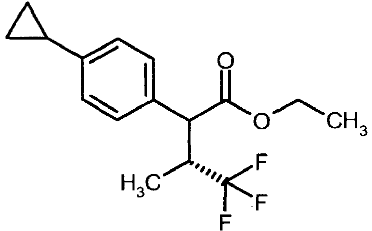 | |
| | aus 1-Brom-4-cyclopropylbenzol und Ethyl-(3*R*)-4,4,4-trifluor-3-methylbutanoat | |
| **74A** | Ethyt-(3*R*)-2-(4-chlor-3-methoxyphenyl)-4,4,4-trifluor-3-methylbutanoat | GC-MS (Methode 1): Rₜ = 5.34 min; m/z = 324/326 (M)⁺. |
| | 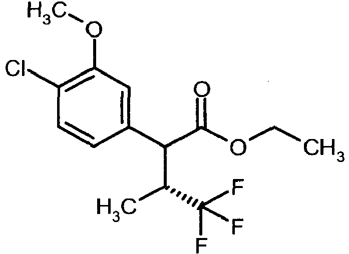 | |
| | aus 4-Brom-1-chlor-2-methoxybenzol und Ethyl-(3*R*)-4,4,4-trifluor-3-methylbutanoat | |
| **75A** | Ethyl-2-(4-chlorphenyl)-4,4,4-trifluor-3-methylbutanoat | GC-MS (Methode 1): Rₜ = 4.44 min; m/z = 294/296 (M)⁺ (*Diastereomer 1*); Rₜ = 4.48 min; m/z = 294/296 (M)⁺ (*Diastereomer 2*). |
| | 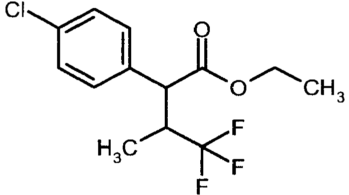 | |
| | aus 1-Brom-4-chlorbenzol und Ethyl-4,4,4-tritluor-3-methylbutanoat | |
| **76A** | Ethyl-4,4,4-trifluor-3-methyl-2-[4-(trifluormethoxy)phenyl]butanoat | GC-MS (Methode 1): Rₜ = 3.41 min; m/z = 344 (M)⁺ (*Diastereomer 1*); Rₜ = 3.44 min; m/z = 344 (M)⁺ (*Diastereomer 2*). |
| | 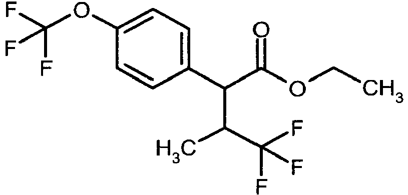 | |
| | aus 1-Brom-4-(trifluormethoxy)benzol und Ethyl-4,4,4-trifluor-3-methylbutanoat | |
| **77A** | Ethyl-2-(4-chlor-3-methylphenyl)-4,4,4-trifluor-3-methylbutanoat | GC-MS (Methode 1): Rₜ = 4.81 min; m/z = 308/310 (*M*)⁺ (*Diastereomer 1*); Rₜ = 4.84 min; m/z = 308/310 (*M*)⁺ (*Diastereomer 2*). |
| | 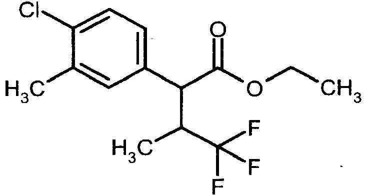 | |
| | aus 4-Brom-1-chlor-2-methylbenzol und Ethyl-4,4,4-trifluor-3-methylbutanoat | |

### Beispiel 78A

### (3R)-2-(4-Ethylphenyl)-4,4,4-trifluor-3-methylbutansäureethylester (Diastereomerengemisch)

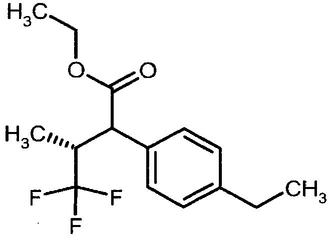

24.4 ml (24.4 mmol) einer 1 M Lösung von Lithiumhexamethyldisilazid in Toluol wurden auf -10°C abgekühlt und tropfenweise mit einer Lösung von 3.0 g (16.29 mmol) (+)-Ethyl-(3*R*)-4,4,4-trifluor-3-methylbutanoat in 15 ml abs. Toluol versetzt. Die Mischung wurde 10 min nachgerührt. Anschließend wurde bei -10°C eine zuvor hergestellte Lösung von 3.92 g (21.18 mmol) 1-Brom-4-ethylbenzol, 110 mg (0.49 mmol) Palladium(II)acetat und 404 mg (1.03 mmol) 2'-Dicyclohexylphosphino-2-(*N*,*N*-dimethylamino)biphenyl in 20 ml abs. Toluol zugetropft. Die resultierende Reaktionsmischung wurde zunächst 1 h bei RT, dann 3 h bei 80°C gerührt. Danach wurde die Mischung im Vakuum eingeengt und der Rückstand in Ethylacetat aufgenommen und auf Wasser gegeben. Die wässrige Phase wurde mit Ethylacetat rückextrahiert, und die vereinigten organischen Phasen wurden mit gesättigter Ammoniumchlorid-Lösung und gesättigter Natriumchlorid-Lösung gewaschen, über Magnesiumsulfat getrocknet und im Vakuum eingeengt. Aus dem Rückstand wurden nach Chromatographie an Silicagel (Laufmittel zunächst Cyclohexan, dann Gradient Cyclohexan/Ethylacetat 200:1 → 50:1) 3.051 g der Titelverbindung erhalten (64.9% d. Th., Diastereomerenverhältnis ca. 3:1).

LC-MS (Methode 4): Rₜ = 1.52 min; m/z = 289 (M+H)⁺ (*Neben-Diastereomer*); Rₜ = 1.54 min; m/z = 289 (M+H)⁺ (*Haupt-Diastereomer*).

¹H-NMR (400 MHz, DMSO-*d*₆): *Haupt-Diastereomer:* δ [ppm] = 0.76 (d, 3H), 1.13 (t, 3H), 1.17 (t, 3H), 2.55-2.63 (m, 2H), 3.21-3.31 (m, 1H), 3.67 (d, 1H), 3.95-4.16 (m, 2H), 7.15-7.23 (m, 2H), 7.25-7.31 (m, 2H).

Auf vergleichbare Weise wurden ausgehend von (+)-Ethyl-(3*R*)-4,4,4-trifluor-3-methylbutanoat und den entsprechenden Phenylbromiden die folgenden Verbindungen hergestellt:

### Beispiel 79A

### (3R)-4,4,4-Trifluor-2-(4-fluorphenyl)-3-methylbutansäureethylester (Diastereomerengemisch)

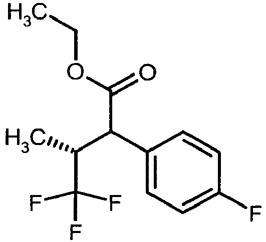

GC-MS (Methode 1): Rₜ = 3.63 min; m/z = 278 (*M*)⁺ *(Neben-Diastereomer*); Rₜ = 3.66 min; m/z = 278 (M)⁺ (*Haupt-Diastereomer*).

¹H-NMR (400 MHz, DMSO-*d*₆): *Haupt-Diastereomer:* δ [ppm] = 0.77 (d, 3H), 1.12 (t, 3H), 3.23-3.30 (m, 1H), 3.79 (d, 1H), 4.01-4.14 (m, 2H), 7.19-7.24 (m, 2H), 7.43-7.47 (m, 2H).

### Beispiel 80A

### (3R)-4,4,4-Trifluor-3-methyl-2-(4-vinylphenyl)butansäureethylester (Diastereomerengemisch)

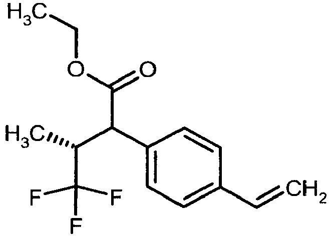

GC-MS (Methode 1): Rₜ = 4.64 min und 4.66 min; jeweils m/z = 286 (M)⁺.

¹H-NMR (400 MHz, DMSO-*d*₆): *Haupt-Diastereorner:* δ [ppm] = 0.79 (d, 3H), 1.12 (t, 3H), 3.22-3.32 (m, 1H), 3.73 (d, 1H), 3.99-4.17 (m, 2H), 5.28 (d, 1H), 5.84 (d, 1H), 6.72 (dd, 1H), 7.34-7.40 (m, 2H), 7.45-7.51 (m, 2H).

### Beispiel 81A

### (3R)-4,4,4-Trifluor-2-[4-(1-fluorvinyl)phenyl]-3-methylbutansäureethylester (Diastereomerengemisch)

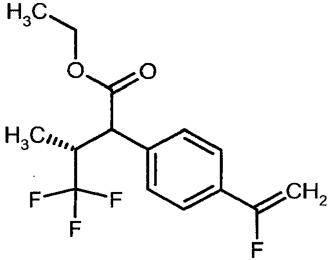

GC-MS (Methode 1): Rₜ = 4.60 min und 4.63 min; jeweils m/z = 304 (M)⁺.

LC-MS (Methode 5): Rₜ = 1.29 min und 1.30 min; jeweils m/z = 279.

¹H-NMR (400 MHz, DMSO-*d*₆): *Haupt-Diastereomer:* δ [ppm] = 0.79 (d, 3H), 1.12 (t, 3H), 3.34-3.38 (m, 1H), 3.81 (d, 1H), 3.99-4.17 (m, 2H), 4.97 (dd, 1H), 5.42 (dd, 1H), 7.46-7.49 (m, 2H), 7.63 (d, 2H).

### Beispiel 82A

### (3R)-2-(4-Chlor-3-fluorphenyl)-4,4,4-trifluor-3-methylbutansäureethylester (Diastereomerengemisch)

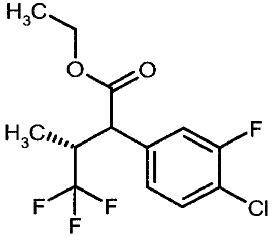

GC-MS (Methode 1): Rₜ = 4.33 min und 4.36 min; jeweils m/z = 312 (M)⁺.

¹H-NMR (400 MHz, DMSO-*d*₆): *Haupt-Diastereomer:* δ [ppm] = 0.80 (d, 3H), 1.08-1.19 (m, 3H), 3.34-3.41 (m, 1H), 3.88 (d, 1H), 4.01-4.18 (m, 2H), 7.28-7.34 (m, 1H), 7.51-7.64 (m, 2H).

### Beispiel 83A

### (3R)-2-(4-Chlor-2-fluorphenyl)-4,4,4-trifluor-3-methylbutansäureethylester (Diastereomerengemisch)

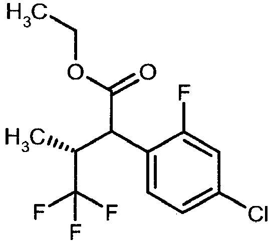

GC-MS (Methode 1): Rₜ = 4.21 min; m/z = 312 (M)⁺.

### Beispiel 84A

### Ethyl-2-[4-(brommethyl)phenyl]-4,4,4-trifluor-3-methylbutanoat

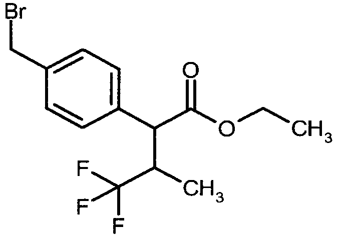

2.25 g (8.2 mmol) Ethyl-4,4,4-trifluor-3-methyl-2-(4-methylphenyl)butanoat, 1.53 g (8.6 mmol) *N-*Bromsuccinimid und 67 mg (0.41 mmol) 2,2'-Azobis-(2-methylpropannitril) in 36 ml Trichlormethan wurden über Nacht unter Rückfluss gerührt. Nach vollständiger Umsetzung wurde das Succinimid abfiltriert, der Filterrückstand mit Dichlormethan nachgewaschen und das Filtrat im Vakuum eingeengt. Das Rohprodukt wurde über Kieselgel chromatographisch gereinigt (Laufmittel Cyclohexan/Essigsäureethylester 40:1). Es wurden 2.667 g (7.5 mmol, 92% d. Th.) eines gelblichen Öls isoliert.

GC-MS (Methode 1): Rₜ = 5.72 min; m/z = 373 (M-Br)⁺ (*Diastereomere 1*); Rₜ = 5.74 min; m/z = 373 (M-Br)⁺ (*Diastereomer 2*).

### Beispiel 85A

### Ethyl-4,4,4-trifluor-3-methyl-2-[4-(2,2,2-trifluorethyl)phenyl]butanoat

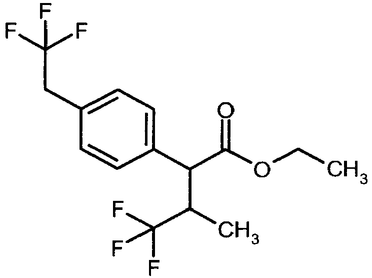

3.77 g (10.67 mmol) Ethyl-2-[4-(brommethyl)phenyl]-4,4,4-trifluor-3-methylbutanoat in 40 ml 1-Methylpyrrolidin-2-on wurden mit 529 mg (2.78 mmol) Kupfer(I)iodid und 4 g (20.82 mmol) Methyl-2,2-difluor-2-(fluorsulfonyl)acetat versetzt und über Nacht bei 80°C gerührt. Nach erfolgter Umsetzung wurde die Reaktionslösung langsam auf 100 ml Eiswasser gegossen. Das erhaltene Gemisch wurde anschließend dreimal mit Diethylether extrahiert. Die vereinigten organischen Phasen wurden über Magnesiumsulfat getrocknet. Nach Filtration wurde das Lösungsmittel im Vakuum entfernt. Das erhaltene Rohprodukt wurde über Kieselgel chromatographisch gereinigt (Laufmittel Cyclohexan/Dichlormethan 4:1). Es wurden 1.48 g (4.32 mmol, 41% d. Th.) der Titelverbindung als gelbliches Öl isoliert.

GC-MS (Methode 1): Rₜ = 4.06 min; m/z = 342 (M)⁺ (*Diastereomere 1*); Rₜ = 4.09 min; m/z = 342 (M)⁺ (*Diastereomere 2*).

MS (DCI): m/z = 360 (M+NH₄)⁺.

### Beispiel 86A

### Methyl-(4-chlorphenyl)(3-oxocyclopentyl)acetat

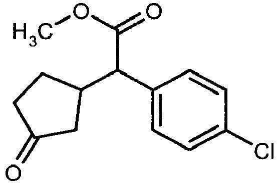

Unter Argon wurden 14.8 ml (105.6 mmol) Diisopropylamin in 150 ml THF vorgelegt, auf -30°C abgekühlt und langsam mit 42.3 ml (105.75 mmol) einer 2.5 M Lösung von *n*-Butyllithium in Hexan versetzt. Anschließend wurde die Reaktionslösung auf -20°C erwärmt, langsam mit 15 g (81.25 mmol) Methyl-(4-chlorphenyl)acetat, gelöst in 90 ml THF, versetzt und 2 h bei dieser Temperatur nachgerührt. Die Reaktionslösung wurde dann auf -78°C abgekühlt und langsam mit 7.2 ml (86.1 mmol) 2-Cyclopenten-1-on, gelöst in 60 ml THF, versetzt. Nach beendeter Zugabe wurde die Lösung 1 h bei dieser Temperatur nachgerührt. Nach DC-Kontrolle (Laufmittel Cyclohexan/Essigsäureethylester 9:1) wurde der Ansatz mit gesättigter wässriger Ammoniumchlorid-Lösung versetzt und in Essigsäureethylester aufgenommen. Die wässrige Phase wurde zweimal mit Essigsäureethylester extrahiert. Die vereinigten organischen Phasen wurden über Magnesiumsulfat getrocknet. Nach Filtration wurde das Lösungsmittel im Vakuum entfernt. Das Rohprodukt wurde über Kieselgel chromatographisch gereinigt (Laufmittel Cyclohexan/Essigsäureethylester 4:1). Es wurden 15.65 g (58.67 mmol, 72% d. Th.) der Titelverbindung als gelbliches Öl isoliert.

GC-MS (Methode 1): Rₜ = 7.02 min; m/z = 266 (M)⁺ (*Diastereomer 1*); Rₜ = 7.04 min; m/z = 266 (M)⁺ (*Diastereomer 2*).

MS (DCI): m/z = 284 (M+NH₄)⁺.

### Beispiel 87A

### Methyl-(4-chlorphenyl)(3,3-difluorcyclopentyl)acetat

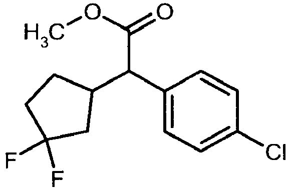

Unter Argon wurden 82.5 ml (82.14 mmol) einer 50%-igen Lösung von 1,1'-[(Trifluor-λ⁴-sulfanyl)imino]bis(2-methoxyethan) (Desoxofluor) in THF, verdünnt mit 200 ml Toluol, vorgelegt, auf 5°C abgekühlt und langsam mit 744 µl (5.87 mmol) einer 1 M Bortrifluorid-Diethylether-Komplex-Lösung versetzt. Die Mischung wurde 2 h bei 5°C nachgerührt. Anschließend wurde die Reaktionslösung langsam mit 15.65 g (58.67 mmol) Methyl-(4-chlorphenyl)(3-oxocyclopentyl)-acetat, gelöst in 200 ml Toluol, versetzt, dann auf 55°C erwärmt und 60 h bei dieser Temperatur nachgerührt. Die Reaktionsmischung wurde danach in ein auf 0°C gekühltes Gemisch bestehend aus 100 ml Toluol und 100 ml 2 M Natronlauge gegeben. Die organische Phase wurde abgetrennt, und die wässrige Phase wurde noch dreimal mit Essigsäureethylester extrahiert. Die vereinigten organischen Phasen wurden über Natriumsulfat getrocknet. Nach Filtration wurde das Lösungsmittel im Vakuum entfernt. Das Rohprodukt wurde über Kieselgel chromatographisch gereinigt (Laufmittel Cyclohexan/Essigsäureethylester 7:1). Es wurden 13.24 g (45.86 mmol, 78% d. Th.) der Titelverbindung als farbloses Öl isoliert.

MS (DCI): m/z = 306 (M+NH₄)⁺.

GC-MS (Methode 1): Rt = 5.83 min; m/z = 288 (M)⁺ *(Diastereomere 1*); Rₜ = 5.86 min; m/z = 288 (M)⁺ (*Diastereomer 2*).

### Beispiel 88A

### (+/-)-Ethyl-(2,2-difluorcyclopentyl)acetat

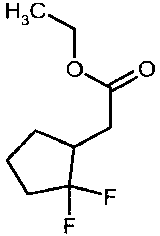

Zu einer Lösung von 52.8 ml (399.5 mmol) Diethylaminoschwefeltrifluorid (DAST) in 150 ml abs. Dichlormethan wurden bei RT 17.0 g (99.88 mmol) (+/-)-Ethyl-2-oxocyclopentylacetat getropft. Die Mischung wurde über Nacht unter Rückfluss erhitzt. Nach Abkühlen wurden weitere 13.2 ml (99.88 mmol) Diethylaminoschwefeltrifluorid (DAST) hinzugefügt, und die Mischung wurde erneut für 36 h unter Rückfluss gerührt. Nach Abkühlen wurde mit Dichlormethan verdünnt, vorsichtig mit gesättigter wässriger Natriumhydrogencarbonat-Lösung versetzt und dann kräftig gerührt. Die organische Phase wurde nacheinander mit gesättigter Natriumhydrogencarbonat-Lösung, zweimal mit 1 N Salzsäure und mit gesättigter Natriumchlorid-Lösung gewaschen, über Magnesiumsulfat getrocknet und im Vakuum eingeengt. Aus dem dunkel-braunen Rückstand wurde das Produkt durch Säulenchromatographie an Silicagel isoliert (Laufmittel Pentan/Dichlormethan 10:1 → 1:1). Es wurden 7.52 g (39% d. Th.) der Titelverbindung erhalten.

GC-MS (Methode 1): Rₜ = 2.88 min; m/z = 172.

¹H-NMR (400 MHz, DMSO-*d*₆): δ [ppm] = 1.18 (t, 3H), 1.33-1.48 (m, 1H), 1.61-1.77 (m, 2H), 1.92-2.20 (m, 3H), 2.24-2.38 (m, 1H), 2.43-2.60 (m, 2H), 4.07 (q, 2H).

### Beispiel 89A

### (+/-)-(4-Chlorphenyl)(2,2-difluorcyclopentyl)essigsäureethylester (Diastereomerengemisch)

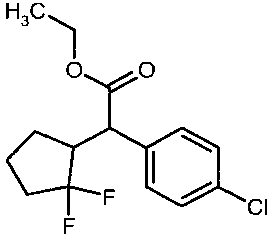

22.6 ml (22.6 mmol) einer 1 M Lösung von Lithiumhexamethyldisilazid in Toluol wurden auf -20°C abgekühlt und tropfenweise mit einer Lösung von 2.90 g (15.09 mmol) (+/-)-Ethyl-(2,2-difluorcyclopentyl)acetat in 20 ml abs. Toluol versetzt. Die Mischung wurde 10 min bei -20°C gerührt. Nach Entfernen der Kühlung wurde eine zuvor hergestellte Lösung von 3.75 g (19.61 mmol) 4-Bromchlorbenzol, 110 mg (0.49 mmol) Palladium(II)acetat und 374 mg (0.95 mmol) 2'-Dicyclohexylphosphino-2-(*N,N*-dimethylamino)biphenyl in 20 ml abs. Toluol zugetropft. Die resultierende Reaktionsmischung wurde zunächst 1 h bei RT und dann 2 h bei 90°C gerührt. Nach Abkühlen wurde die Reaktionsmischung auf Wasser gegeben. Die wässrige Phase wurde dreimal mit Ethylacetat extrahiert, und die vereinigten organischen Phasen wurden über Magnesiumsulfat getrocknet und im Vakuum eingeengt. Aus dem Rückstand wurde nach Chromatographie an Silicagel (Laufmittel Cyclohexan/Ethylacetat 50:1) 2.70 g der Titelverbindung erhalten (59.1% d. Th., Diastereomerenverhältnis ca. 1:4.3).

GC-MS (Methode 1): Rₜ = 6.09 min und 6.20 min.

¹H-NMR (400 MHz, DMSO-*d*₆): δ [ppm] = 1.01-1.27 (m, 3H), 1.37-1.50 (m, 1H), 1.51-1.75 (m, 3H), 1.94-2.23 (m, 3H), 2.84-3.07 (m, 1H), 3.55-3.79 (m, 1H), 3.93-4.20 (m, 2H), 7.29-7.53 (m, 4H).

### Beispiel 90A

### (+/-)-(4-Bromphenyl)(cyclopentyl)essigsäure

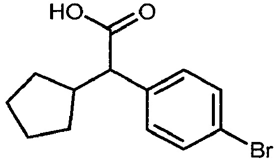

Zu einer Lösung von 30 g (96.4 mmol) (+/-)-(4-Bromphenyl)(cyclopentyl)essigsäureethylester in 655 ml Methanol wurden 386 ml (964 mmol) 10%-ige Natronlauge gegeben und die Mischung für 3 h unter Rückfluss erhitzt. Nach dem Abkühlen wurde die Lösung in 2 Liter Wasser eingerührt, durch Zugabe von verdünnter Salzsäure auf pH 1-2 gebracht und mit Ethylacetat extrahiert. Die organische Phase wurde über Magnesiumsulfat getrocknet und eingeengt. Es wurden 27.2 g (92% d. Th.) der Titelverbindung erhalten.

LC-MS (Methode 2): Rₜ = 2.34 min; m/z = 283/285 (M+H)⁺.

### Beispiel 91A

### (+/-)-(4-Cyanophenyl)(cyclopentyl)essigsäure

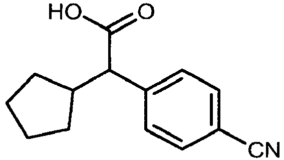

Eine Lösung von 192.5 mg (0.79 mmol) (+/-)-(4-Cyanophenyl)(cyclopentyl)essigsäuremethylester in 1.7 ml THF/Methanol (1:1) wurde mit 316.5 mg (7.9 mmol) Natriumhydroxid versetzt und 3 h bei RT gerührt. Das Reaktionsgemisch wurde danach in Wasser gegossen, mit 1 N Salzsäure neutralisiert und mit Ethylacetat extrahiert. Die organische Phase wurde über Magnesiumsulfat getrocknet und eingeengt. Es wurden 125.6 mg (69% d. Th.) der Titelverbindung erhalten.

LC-MS (Methode 2): Rₜ = 2.11 min; m/z = 230 (M+H)⁺.

### Beispiel 92A

### (+/-)-(4-Nitrophenyl)(cyclopentyl)essigsäure

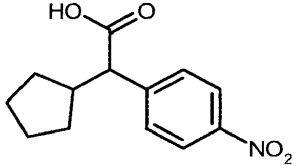

Eine Lösung von 715 mg (2.6 mmol) (+/-)-(4-Nitrophenyl)(cyclopentyl)essigsäureethylester in 6 ml THF/Methanol (1:1) wurde mit 1.03 g (25.8 mmol) Natriumhydroxid versetzt und über Nacht bei RT gerührt. Anschließend wurde das Reaktionsgemisch in Wasser gegossen, mit 1 N Salzsäure neutralisiert und mit Ethylacetat extrahiert. Die organische Phase wurde über Natriumsulfat getrocknet und eingeengt. Der Rückstand wurde in 80 ml Diethylether aufgenommen und mit 250 ml Petrolether versetzt. Der ausgefallene Feststoff wurde abgesaugt und mit Petrolether gewaschen. Das so erhaltene Produkt wurde durch präparative HPLC weiter gereinigt. Es wurden 89.5 mg (14% d. Th.) der Titelverbindung erhalten.

LC-MS (Methode 2): Rₜ = 2.21 min; m/z = 250 (M+H)⁺.

¹H-NMR (400 MHz, DMSO-d₆): δ [ppm] = 0.86-1.07 (m, 1H), 1.20-1.74 (m, 6H), 1.81-1.96 (m, 1H), 3.49 (d, 1H), 7.63 (d, 2H), 8.20 (d, 2H), 12.58 (br. s, 1H).

### Beispiel 93A

### (+)-(2S)-Cyclopentyl(4-methylphenyl)essigsäure

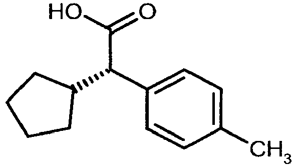

Eine Lösung von 1.0 g (2.8 mmol) (1*R*,2*S*,5*R*)-2-Isopropyl-5-methylcyclohexyl-(2*S*)-cyclopentyl-(4-methylphenyl)acetat in 5 ml 1,2-Dichlorethan wurde bei RT mit 1.53 ml (11.2 mmol) Iodtrimethylsilan versetzt und über Nacht gerührt. Anschließend wurde das Reaktionsgemisch mit 50 ml Ethylacetat verdünnt und mit Wasser gewaschen. Die organische Phase wurde über Magnesiumsulfat getrocknet und eingeengt. Das Rohprodukt wurde durch präparative HPLC gereinigt. Es wurden 539 mg (88% d. Th.) der Titelverbindung erhalten.

LC-MS (Methode 5): Rₜ = 1.13 min; m/z = 217 (M-H)⁻.

[α]_{D}²⁰ = +65.0°, c = 0.50, Chloroform.

### Beispiel 94A

### (+/-)-[4-(Acetoxymethyl)phenyl](cyclopentyl)essigsäure

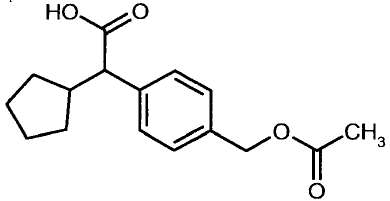

11.70 g (35.19 mmol) *tert*.-Butyl-[4-(acetoxymethyl)phenyl](cyclopentyl)acetat wurden in 108.5 ml Dichlormethan gelöst, auf 0°C gekühlt und mit 39.2 ml Trifluoressigsäure versetzt. Die Reaktionsmischung wurde zunächst 1.5 h bei 0°C, dann 1.5 h bei RT gerührt und anschließend im Vakuum eingeengt. Der Rückstand wurde in 50 ml Dichlormethan aufgenommen und die Lösung viermal mit je 30 ml Wasser gewaschen. Die organische Phase wurde über Magnesiumsulfat getrocknet und im Vakuum eingeengt und der Rückstand im Hochvakuum getrocknet. Es wurden 9.83 g der Zielverbindung als Rohprodukt erhalten (ca. 90% Reinheit, Ausbeute 91% d. Th.).

LC-MS (Methode 5): Rₜ = 1.02 min; m/z = 275 (M-H)⁻.

### Beispiel 95A

### (+/-)-(4-Chlorphenyl)(cyclopentyl)essigsäure

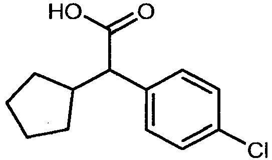

4.63 g (18.3 mmol) Methyl-(4-chlorphenyl)(cyclopentyl)acetat wurden in einem Gemisch aus 18.5 ml THF und 18.5 ml Methanol gelöst und bei RT mit 73.3 ml 10%-iger Natronlauge (183.2 mmol) versetzt. Das Reaktionsgemisch wurde über Nacht bei RT gerührt. Anschließend wurde das Gemisch durch Zugabe von 1 N Salzsäure angesäuert. Die wässrige Phase wurde mehrfach mit Ethylacetat extrahiert, und die vereinigten organischen Phasen wurden mit gesättigter Natriumchlorid-Lösung gewaschen, über Natriumsulfat getrocknet und im Vakuum eingeengt. Es wurden 4.31 g der Zielverbindung als Rohprodukt erhalten (Ausbeute 98.6% d. Th.).

LC-MS (Methode 2): Rₜ = 2.30 min; m/z = 193 (M-CO₂H)⁺.

¹H-NMR (400 MHz, DMSO-*d*₆): δ [ppm] = 0.87-1.03 (m, 1H), 1.17-1.33 (m, 2H), 1.35-1.47 (m, 1H), 1.47-1.69 (m, 3H), 1.77-1.90 (m, 1H), 2.33-2.47 (m, 1H), 3.27 (d, 1H), 7.30-7.42 (m, 4H), 12.36 (s, 1H).

Auf analoge Weise wurden die folgenden Carbonsäuren hergestellt:

| **Beispiel** | **Name / Struktur / Edukt** | **Analytische Daten** |
|---|---|---|
| **96A** | 2-(4-Ethylphenyl)-4,4,4-trifluor-3-methylbutansäure (*racemisches Diastereomerengemisch*, ca. 1:12) | GC-MS (Methode 1): Rₜ = 4.82 min; m/z = 260 (M)⁺. |
| | 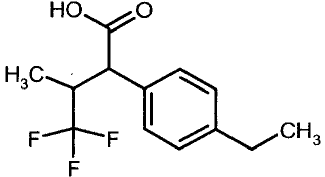 | ¹H-NMR (400 MHz, DMSO-*d₆*): *Haupt-Diastereorner:* δ [ppm] = 0.76 (d, 3H), 1.17 (t, 3H), 2.59 (q, 2H), 3.16-3.27 (m, 1H), 3.56 (d, 1H), 7.18-7.23 (m, 2H), 7.23-7.30 (m, 2H), 12.65 (br. s, 1H). |
| | aus Ethyl-2-(4-ethylphenyl)-4,4,4-trifluor-3-methylbutanoat (*racemisches Diastereomerengemisch*) | |
| **97A** | (+/-)-Cyclopentyl(3,4-dichlorphenyl)essigsäure | LC-MS (Methode 5): Rₜ = 1.22 min; m/z = 271/273 (M-H)⁻. |
| | 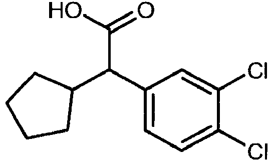 | ¹H-NMR (400 MHz, DMSO-*d*₆): δ [ppm] = 0.90-1.03 (m, 1H), 1.16-1.35 (m, 2H), 1.37-1.48 (m, 1H), 1.48-1.68 (m, 3H), 1.75-1.89 (m, 1H), 2.34-2.45 (m, 1H), 3.28-3.32 (m, 1H), 7.35 (dd, 1H), 7.54-7.64 (m, 2H), 12.52 (br. s, 1H). |
| | aus Methyl-cyclopentyl(3,4-dichlorphenyl)acetat | |

### Beispiel 98A

### (+/-)-Cyclopentyl[4-(trifluormethyl)phenyl]essigsäure

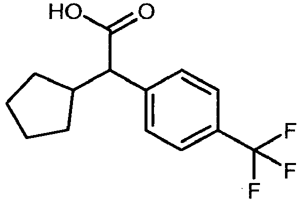

In einem Gemisch aus je 24.9 ml THF, Methanol und Wasser wurden 4.98 g (17.4 mmol) Methylcyclopentyl[4-(trifluormethyl)phenyl]acetat vorgelegt und bei 0°C mit 1.04 g (43.49 mmol) Lithiumhydroxid versetzt. Die Reaktionsmischung wurde auf RT erwärmt und 4 h bei dieser Temperatur gerührt. Anschließend wurde mit Wasser verdünnt und mit 1 N Salzsäure leicht angesäuert. Die wässrige Phase wurde dreimal mit Ethylacetat extrahiert. Die vereinigten organischen Phasen wurden über Magnesiumsulfat getrocknet und im Vakuum eingeengt. Es wurden 4.56 g der Zielverbindung als Rohprodukt erhalten (Ausbeute 96.3% d. Th.).

LC-MS (Methode 5): Rₜ = 1.18 min; m/z = 227 (M-CO₂H)⁻.

¹H-NMR (400 MHz, DMSO-*d*₆): δ [ppm] = 0.87-1.03 (m, 1H), 1.20-1.34 (m, 2H), 1.35-1.48 (m, 1H), 1.48-1.69 (m, 3H), 1.80-1.92 (m, 1H), 2.39-2.48 (m, 1H), 3.40 (d, 1H), 7.53-7.61 (m, 2H), 7.65-7.74 (m, 2H), 12.48 (br. s, 1H).

### Beispiel 99A

### (+/-)-Cyclopentyl(4-fluorphenyl)essigsäure

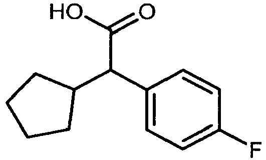

2.20 g (ca. 88%-ig, 6.96 mmol) (+/-)-Cyclopentyl(4-fluorphenyl)essigsäure-*tert*.-butylester wurden in 2.9 ml Dichlormethan gelöst und bei RT mit 10.7 ml Trifluoressigsäure versetzt. Die Mischung wurde 3 h bei RT gerührt und dann im Vakuum eingeengt. Der Rückstand wurde über Nacht im Hochvakuum getrocknet. Der erhaltene Feststoff wurde mit Acetonitril verrührt, anschließend abgesaugt und mit wenig Acetonitril gewaschen. Nach Trocknen im Hochvakuum wurden 720 mg einer ersten Feststoff-Charge erhalten. Das zuvor erhaltene Filtrat wurde eingeengt und der Rückstand mittels präparativer RP-HPLC (Eluent Acetonitril/Wasser) gereinigt. Erhalten wurden so weitere 529 mg des Zielprodukts (Ausbeute zusammen 80.8% d. Th.).

LC-MS (Methode 5): Rₜ = 1.08 min; m/z = 221 (M-H)⁻.

¹H-NMR (400 MHz, DMSO-*d*₆): δ [ppm] = 0.85-1.03 (m, 1H), 1.16-1.33 (m, 2H), 1.35-1.47 (m, 1H), 1.48-1.69 (m, 3H), 1.75-1.90 (m, 1H), 2.35-2.47 (m, 1H), 3.26 (d, 1H), 7.09-7.19 (m, 2H), 7.31-7.41 (m, 2H), 12.30 (s, 1H).

### Beispiel 100A

### (+/-)-(4-Chlor-2-fluorphenyl)(cyclopentyl)essigsäure

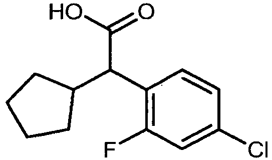

7.55 g (27.9 mmol) Methyl-(4-chlor-2-fluorphenyl)(cyclopentyl)acetat wurden in je 32 ml THF, Methanol und Wasser gelöst und unter Eiskühlung mit 11.15 g (287.9 mmol) Natriumhydroxid versetzt. Die Reaktionsmischung wurde über Nacht bei RT gerührt, dann mit Wasser verdünnt und mit 1 N Salzsäure auf pH 2 eingestellt. Die wässrige Phase wurde dreimal mit Ethylacetat extrahiert. Die vereinigten organischen Phasen wurden über Natriumsulfat getrocknet und im Vakuum eingeengt. Der verbliebene Feststoff wurde mit Wasser verrührt, abgesaugt und gründlich im Vakuum getrocknet. Es wurden 6.96 g der Zielverbindung erhalten (97.2% d. Th.).

LC-MS (Methode 5): Rₜ = 1.18 min; m/z = 211 (M-CO₂H)⁻.

¹H-NMR (400 MHz, DMSO-*d*₆): δ [ppm] = 0.86-1.00 (m, 1H), 1.25-1.47 (m, 3H), 1.49-1.65 (m, 3H), 1.80-1.94 (m, 1H), 2.39-2.48 (m, 1H), 3.56 (d, 1H), 7.29 (dd, 1H), 7.41 (dd, 1H), 7.48 (t, 1H), 12.52 (br. s, 1H).

Auf analoge Weise wurden die folgenden Carbonsäuren hergestellt:

| **Beispiel** | **Name / Struktur / Edukt** | **Analytische Daten** |
|---|---|---|
| **101A** | (+/-)-Cyclopentyl(2,4-dichlorphenyl)essigsäure | LC-MS (Methode 5): Rₜ = 1.22 min; m/z = 271 (M-H)⁻. |
| | 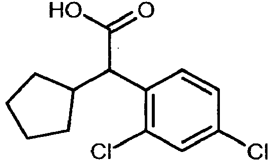 | ¹H-NMR (400 MHz, DMSO-*d*₆): δ [ppm] = 0.88-0.98 (m, 1H), 1.27-1.36 (m, 2H), 1.39-1.47 (m, 1H), 1.48-1.69 (m, 3H), 1.83-1.93 (m, 1H), 2.42-2.48 (m, 1H), 3.78 (d, 1H), 7.42-7.46 (m, 1H), 7.52-7.56 (m, 1H), 7.60-7.64 (m, 1H), 12.59 (br. s, 1H). |
| | aus Ethyl-cyclopentyl(2,4-dichlorphenyl)acetat | |
| **102A** | (+/-)-Cyclopentyl[3-fluor-4-(trifluoromethyl)-phenyl]essigsäure | LC-MS (Methode 5): Rₜ = 1.17 min; m/z = 245 (M-CO₂H)⁻. |
| | 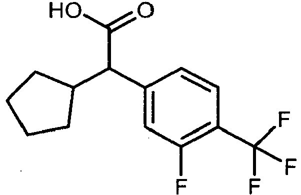 | ¹H-NMR (400 MHz, DMSO-*d₆*): δ [ppm] = 0.89-1.06 (m, 1H), 1.19-1.36 (m, 2H), 1.37-1.49 (m, 1H), 1.49-1.69 (m, 3H), 1.76-1.92 (m, 1H), 2.38-2.48 (m, 1H), 3.45 (d, 1H), 7.40 (d, 1H), 7.50 (d, 1H), 7.75 (t, 1H), 12.62 (br. s, 1H). |
| | aus Methyl-cyclopentyl[3-fluor-4-(trifluormethyl)phenyl]acetat | |
| **103A** | (+/-)-Cyclopentyl[4-(trifluormethoxy)phenyl]-essigsäure | LC-MS (Methode 5): Rt = 1.18 min; m/z = 287 (M-H)⁻. |
| | 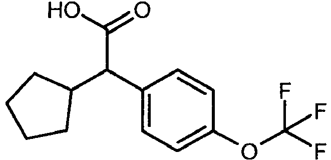 | ¹H-NMR (400 MHz, DMSO-*d₆*): δ [ppm] = 0.88-1.02 (m, 1H), 1.20-1.33 (m, 2H), 1.37-1.48(m, 1H), 1.48-1.69 (m, 3H), 1.78-1.90 (m, 1H), 2.35-2.47 (m, 1H), 3.32 (d, 1H), 7.27-7.35 (m, 2H), 7.42-7.50 (m, 2H), 12.40 (br. s, 1H). |
| | aus Methyl-cyclopentyl[4-(trifluormethoxy)-phenyl]acetat | |

### Beispiel 104A

### (+)-(2S)-Cyclopentyl(4-ethylphenyl)essigsäure

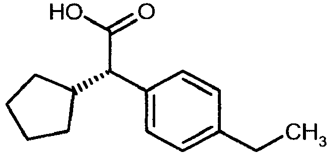

515 mg (1.39 mmol) (-)-(1*R*,2*S*,5*R*)-2-Isopropyl-5-methylcyclohexyl-(2*S*)-cyclopentyl(4-ethylphenyl)acetat wurden in 17 ml Trifluoressigsäure über Nacht bei RT gerührt. Die Reaktionsmischung wurde danach im Vakuum eingeengt und der Rückstand mit Dichlormethan aufgenommen. Die Lösung wurde mit Wasser gewaschen, über Natriumsulfat getrocknet und im Vakuum eingeengt. Das erhaltene Rohprodukt wurde durch Chromatographie an Silicagel gereinigt (Laufmittel Cyclohexan/Ethylacetat 10:1 → 2:1). Es wurden so 286 mg der Zielverbindung erhalten (88.5% d. Th.).

LC-MS (Methode 5): Rₜ = 1.17 min; m/z = 231 (M-H)⁻.

¹H-NMR (400 MHz, DMSO-*d*₆): δ [ppm] = 0.89-1.01 (m, 1H), 1.16 (m, 3H), 1.20-1.33 (m, 2H), 1.36-1.46 (m, 1H), 1.48-1.67 (m, 3H), 1.78-1.88 (m, 1H), 2.37-2.47 (m, 1H), 2.57 (q, 2H), 3.18 (d, 1H), 7.12-7.17 (m, 2H), 7.19-7.25 (m, 2H), 12.17 (br. s, 1H).

[α]_{D}²⁰ = +50.4°, c = 0.455, Chloroform.

### Beispiel 105A

### (+)-(2S,3R)-2-(4-Chlorphenyl)-4,4,4-trifluor-3-methylbutansäure

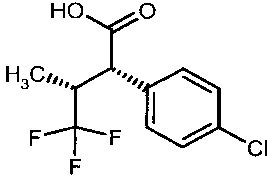

5.086 g (17.26 mmol) Ethyl-(3*R*)-2-(4-chlorphenyl)-4,4,4-trifluor-3-methylbutanoat wurden in 68 ml Dioxan gelöst und mit 34 ml 1 N Natronlauge versetzt. Der Ansatz wurde 2 h bei 50°C gerührt. Das Reaktionsgemisch wurde dann mit 1 N Salzsäure auf pH 1 angesäuert und mehrmals mit Dichlormethan extrahiert. Die vereinigten organischen Phasen wurden mit gesättigter Natrium-chlorid-Lösung gewaschen, über Natriumsulfat getrocknet und im Vakuum eingeengt. Es wurden 3.9 g (14.63 mmol, 85% d. Th., 83% de) der Zielverbindung erhalten.

¹H-NMR (400 MHz, DMSO-d₆, δ/ppm): 12.95-12.73 (1H, br. s), 7.49-7.34 (4H, m), 3.68 (1H, d), 3.31-3.18 (1H, m), 1.20 (0.25H, d), 0.78 (2.75H, d).

GC-MS (Methode 1): Rt = 4.85 min; m/z = 266 (M)⁺.

[α]_{D}²⁰ = +57.2°, c = 0.41, Methanol.

Auf analoge Weise wurden die in der folgenden Tabelle aufgeführten Verbindungen hergestellt:

| **Beispiel** | **Name / Struktur / Edukt** | **Analytische Daten** |
|---|---|---|
| **106A** | 4,4,4-Trifluor-3-methyl-2-(4-methylphenyl)-butansäure | GC-MS (Methode 1): |
| | | Rt = 4.48 min; m/z = 246 (M)⁺. |
| | 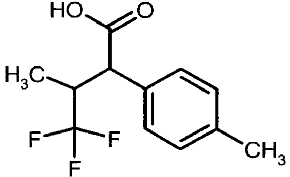 | |
| | aus Ethyl-4,4,4-trifluor-3-methyl-2-(4-methylphenyl)butanoat | |
| **107A** | (2*S*,3*R*)-4,4,4-Trifluor-3-methyl-2-[4-(trifluormethyl)phenyl]butansäure | GC-MS (Methode 1): |
| | | Rₜ = 3.85 min; m/z = 300 (M)⁺. |
| | 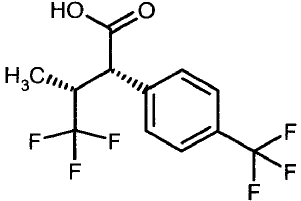 | |
| | aus Ethyl-(2*S*,3*R*)-4,4,4-trifluor-3-methyl-2-[4-(trifluormethyl)phenyl]butanoat | |
| **108A** | 4,4,4-Trifluor-3-methyl-2-[4-(trifluormethyl)-phenyl]butansäure | GC-MS (Methode 1): |
| | | Rₜ = 3.85 min; m/z = 300 (M)⁺. |
| | 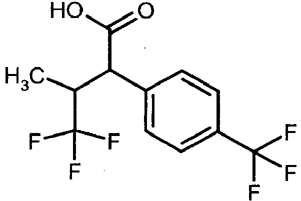 | |
| | aus Ethyl-4,4,4-trifluor-3-methyl-2-[4-(trifluormethyl)phenyl]butanoat | |
| **109A** | (2*S*,3*R*)-4,4,4-Trifluor-2-(4-isopropylphenyl)-3-methylbutansäure | ¹H-NMR (400 MHz, DMSO-d₆, δ/ppm): 12.56 (1H, br. s), 7.25 (4H, q), 3.56 (1H, d), 3.28-3.16 (1H, m), 2.94-2.81 (1H, m), 1.19 (6H, d), 0.75 (3H, d). |
| | 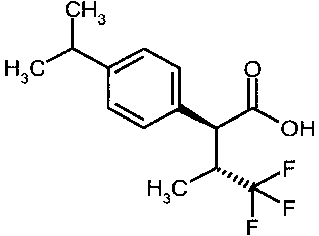 | |
| | | GC-MS (Methode 1): |
| | | Rₜ = 4.93 min; m/z = 274 (M)⁺. |
| | aus Ethyl-(3*R*)-4,4,4-trifluor-2-(4-isopropylphenyl)-3-methylbutanoat | |
| **110A** | (2*S*,3*R*)-2-(4-*tert*.-Butylphenyl)-4,4,4-trifluor-3-methylbutansäure | GC-MS (Methode 1): |
| | | Rₜ = 5.15 min; m/z = 288 (M)⁺. |
| | 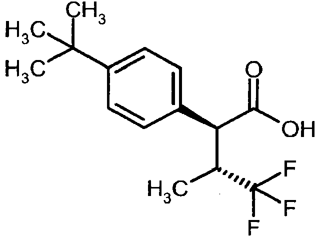 | |
| | aus Ethyl-(2*S*,3*R*)-2-(4-*tert*.-butylphenyl)-4,4,4-trifluor-3-methylbutanoat | |
| **111A** | (2*S*,3*R*)-4,4,4-Trifluor-3-methyl-2-[4-(1,1,1-trifluor-2-methylpropan-2-yl)phenyl]butansäure | ¹H-NMR (400 MHz, DMSO-d₆, δ/ppm): 12.90-12.40 (1H, br. s), 7.53 (2H, d), 7.40 (2H, d), 3.69 (0.11H, d), 3.64 (0.89H, d), 3.30-3.20 (1H, m), 1.55 (6H, s), 1.21 (0.33H, d), 0.76 (2.67H, d). |
| | 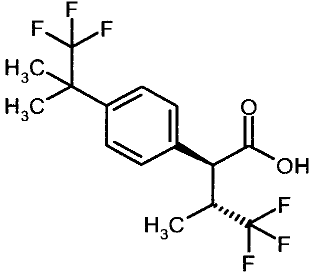 | |
| | | LC-MS (Methode 5): |
| | | Rₜ = 1.19 min; m/z = 341 (M-H)⁻. |
| | aus Ethyl-(2*S*,3*R*)-4,4,4-trifluor-3-methyl-2-[4-(1,1,1-trifluor-2-methylpropan-2-yl)phenyl]-butanoat | |
| **112A** | 4,4,4-Trifluor-3-methyl-2-[4-(2,2,2-trifluorethyl)-phenyl]butansäure | ¹H-NMR (400 MHz, DMSO-d₆, δ/ppm): 12.95-12.59 (1H, br. s), 7.37 (4H, q), 3.70-3.57 (3H, m), 3.30-3.18 (1H, m), 0.76 (3H, d). |
| | 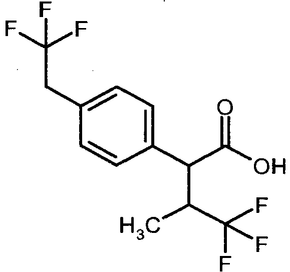 | |
| | | GC-MS (Methode 1): |
| | | Rₜ = 4.45 min; m/z = 315 (M+H)⁺. |
| | aus Ethyl-4,4,4-trifluor-3-methyl-2-[4-(2,2,2-trifluorethyl)phenyl]butanoat | |
| **113A** | (4-Chlorphenyl)(3,3-difluorcyclopentyl)-essigsäure | ¹H-NMR (400 MHz, DMSO-d₆, δ/ppm): 12.59 (1H, br. s), 7.38 (4H, q), 3.51 (0.5H, d), 3.48 (0.5H, d), 2.77-2.60 (1H, m), 2.42-2.27 (0.5H, m), 2.26-1.20 (5.5H, m). |
| | 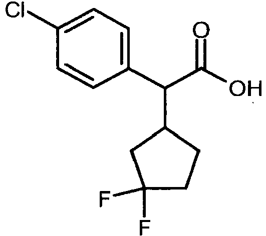 | |
| | | GC-MS (Methode 1): |
| | | Rt = 6.33 min; m/z = 274 (M)⁺ *(Diastereomer 1*); |
| | aus Methyl-(4-chlorphenyl)(3,3-difluorcyclopentyl)acetat | Rₜ = 6.38 min; m/z = 274 (M)⁺ (*Diastereomer 2).* |
| **114A** | (2*S*,3*R*)-2-[4-(1,1-Difluorethyl)phenyl]-4,4,4-trifluor-3-methylbutansäure | LC-MS (Methode 2): Rₜ = 2.34 min; m/z = 295 (M-H)-. |
| | 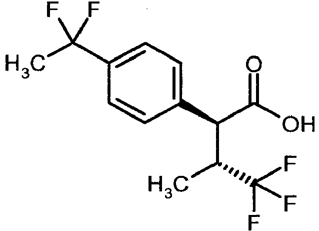 | |
| | aus Ethyl-(3*R*)-2-[4-(1,1-difluorethyl)phenyl]-4,4,4-trifluor-3-methylbutanoat | |
| **115A** | (2*S*,3*R*)-2-(4-Cyclopropylphenyl)-4,4,4-trifluor-3-methylbutansäure | ¹H-NMR (400 MHz, DMSO-d₆, δ/ppm): 12.78-12.52 (1H, br. s), 7.22 (2H, d), 7.05 (2H, d), 3.54 (1H, d), 3.27-3.12 (1H, m), 1.94-1.82 (1H, m), 0.97-0.89 (2H, m), 0.75 (3H, d), 0.69-0.62 (2H, m). |
| | 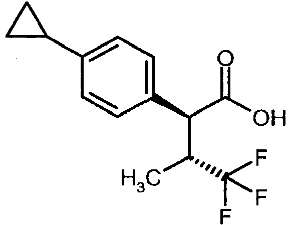 | |
| | | GC-MS (Methode 1): |
| | | Rₜ = 5.51 min; m/z = 273 (M+H)⁺. |
| | aus Ethyl-(3*R*)-2-(4-cyclopropylphenyl)-4,4,4-trifluor-3-methylbutanoat | |
| **116A** | (2*S*,3*R*)-2-(4-Chlor-3-methoxyphenyl)-4,4,4-trifluor-3-methylbutansäure | ¹H-NMR (400 MHz, DMSO-d₆, δ/ppm): 12.91-12.71. (1H, br. s), 7.41 (1H, d), 7.18 (1H, d), 6.98 (1H, dd), 3.86 (3H, s), 3.66 (1H, d), 3.40-3.19 (1H, m), 0.79 (3H, d). |
| | 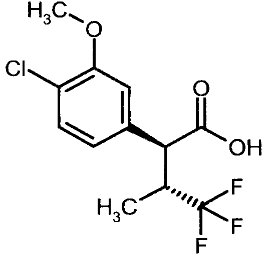 | |
| | | LC-MS (Methode 2): Rₜ = 2.20 min; m/z = 295/297 (M-H)⁻. |
| | aus Ethyl-(3*R*)-2-(4-chlor-3-methoxyphenyl)-4,4,4-trifluor-3-methylbutanoat | |
| **117A** | 2-(4-Chlorphenyl)-4,4,4-trifluor-3-methylbutansäure | LC-MS (Methode 5): |
| | | Rₜ = 1.04 min; m/z = 265/267 |
| | 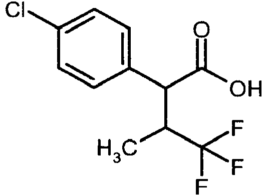 | (M-H)⁻ (*Diastereomere 1*); |
| | | Rₜ = 1.06 min; m/z = 265/267 |
| | | (M-H)⁻ (*Diastereomer 2).* |
| | aus Ethyl-2-(4-chlorphenyl)-4,4,4-trifluor-3-methylbutanoat | |
| **118A** | 4,4,4-Trifluor-3-methyl-2-[4-(trifluormethoxy)-phenyl]butansäure | GC-MS (Methode 1): |
| | | Rₜ = 3.85 min; m/z = 316 (M)⁺. |
| | 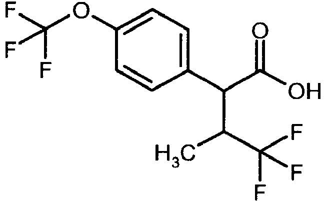 | |
| | aus Ethyl-4,4,4-trifluor-3-methyl-2-[4-(trifluormethoxy)phenyl]butanoat | |
| **119A** | 2-(4-Chlor-3-methylphenyl)-4,4,4-trifluor-3-methylbutansäure | GC-MS (Methode 1): |
| | | Rₜ = 5.20 min; m/z = 280/282 (M)⁺ |
| | 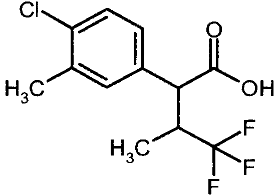 | *(Diastereomer 1*); |
| | | Rt = 5.23 min; m/z = 280/282 (M)⁺ |
| | | (*Diastereomere 2*). |
| | aus Ethyl-2-(4-chlor-3-methylphenyl)-4,4,4-trifluor-3-methylbutanoat | |

### Beispiel 120A

### (3R)-2-(4-Ethylphenyl)-4,4,4-trifluor-3-methylbutansäure (Diastereomerengemisch)

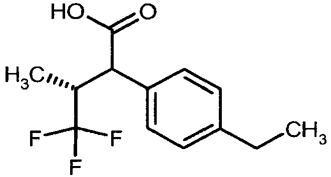

3.0 g (3*R*)-2-(4-Ethylphenyl)-4,4,4-trifluor-3-methylbutansäureethylester (ca. 88% Reinheit, ca. 9.16 mmol; Diastereomerengemisch) wurden in einem Gemisch aus je 12.4 ml Methanol, THF und Wasser gelöst und portionsweise mit 5.49 g (137.35 mmol) Natriumhydroxid versetzt. Die Reaktionsmischung wurde 9 h bei 40°C gerührt. Nach Abkühlen wurden die flüchtigen Lösungsmittel im Vakuum weitgehend entfernt und der Rückstand mit Wasser verdünnt. Durch Zugabe von Salzsäure wurde angesäuert, und die wässrige Phase wurde dreimal mit Ethylacetat extrahiert. Die vereinigten organischen Phasen wurden über Natriumsulfat getrocknet, im Vakuum eingeengt und der Rückstand im Hochvakuum getrocknet. Erhalten wurden 2.61 g der Titelverbindung als Rohprodukt, welches nicht weiter aufgereinigt wurde (Diastereomerenverhältnis ca. 9:1).

LC-MS (Methode 5): Rₜ = 1.08 min; m/z = 259 (M-H)⁻ (*Neben-Diastereomer*); Rₜ = 1.11 min; m/z = 259 (M-H)⁻ (*Haupt-Diastereomer*).

¹H-NMR (400 MHz, DMSO-*d₆*): *Haupt-Diastereomer:* δ [ppm] = 0.76 (d, 3H), 1.17 (t, 3H), 2.54-2.66 (m, 4H), 3.10-3.29 (m, 1H), 3.56 (d, 1H), 7.14-7.22 (m, 2H), 7.22-7.32 (m, 2H), 12.58 (br. s, 1H).

Auf vergleichbare Weise (Reaktionstemperatur: RT bis +40°C; Reaktionszeit: 9-12 h) wurden aus den entsprechenden Estern die folgenden Carbonsäuren hergestellt:

### Beispiel 121A

### (3R)-4,4,4-Trifluor-2-(4-fluorphenyl)-3-methylbutansäure (Diastereomerengemisch)

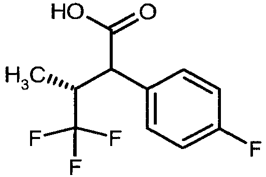

Diastereomerenverhältnis ca. 9:1.

¹H-NMR (400 MHz, DMSO-*d₆*): *Haupt-Diastereomer:* δ [ppm] = 0.77 (d, 3H), 3.18-3.30 (m, 1H), 3.67 (d, 1H), 7.17-7.24 (m, 2H), 7.39-7.47 (m, 2H), 12.78 (br. s, 1H).

### Beispiel 122A

### (3R)-4,4,4-Trifluor-3-methyl-2-(4-vinylphenyl)butansäure (Diastereomerengemisch)

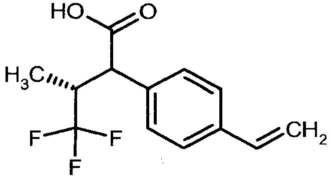

Diastereomerenverhältnis ca. 10:1.

LC-MS (Methode 5): Rₜ = 1.04 min; m/z = 257 (M-H)⁻ (*Neben-Diastereomer*); Rₜ = 1.06 min; m/z = 257 (M-H)⁻ (*Haupt-Diastereomer*).

¹H-NMR (400 MHz, DMSO-*d*₆): *Haupt-Diastereomer:* δ [ppm] = 0.78 (d, 3H), 3.18-3.31 (m, 1H), 3.62 (d, 1H), 5.28 (d, 1H), 5.84 (d, 1H), 6.73 (dd, 1H), 7.31-7.39 (m, 2H), 7.40-7.54 (m, 2H), 12.74 (br. s, 1H).

### Beispiel 123A

### (3R)-4,4,4-Trifluor-2-[4-(1-fluorvinyl)phenyl]-3-methylbutansäure (Diastereomerengemisch)

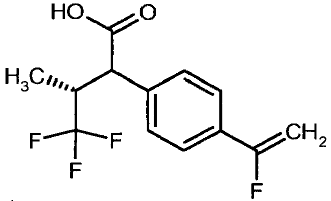

Diastereomerenverlältnis ca. 9:1.

GC-MS (Methode 1): Rₜ = 4.97 min; m/z = 276 (M)⁺.

¹H-NMR (400 MHz, DMSO-*d*₆): *Haupt-Diastereomer:* δ [ppm] = 0.78 (d, 3H), 3.16-3.29 (m, 1H), 3.70 (d, 1H), 4.96 (dd, 1H), 5.34 (d, 1H), 5.47 (d, 1H), 7.39-7.51 (m, 2H), 7.58-7.69 (m, 2H), 12.83 (br. s, 1H).

### Beispiel 124A

### (4-Chlorphenyl)(2,2-difluorcyclopentyl)essigsäure (Diastereomerengemisch)

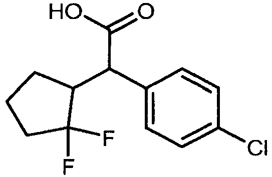

2.70 g (8.92 mmol) (4-Chlorphenyl)(2,2-difluorcyclopentyl)essigsäureethylester (Diastereomerengemisch) wurden in 10 ml Methanol, 10 ml THF und 5 ml Wasser gelöst und bei RT mit 7.13 g (89.18 mmol) 50%-iger Natronlauge versetzt. Die Reaktionsmischung wurde über Nacht bei RT gerührt. Danach wurde mit Wasser verdünnt und mit Salzsäure sauer gestellt. Die wässrige Phase wurde dreimal mit Ethylacetat extrahiert, und die vereinigten organischen Phasen wurden über Magnesiumsulfat getrocknet, im Vakuum eingeengt und der Rückstand im Hochvakuum getrocknet. Erhalten wurden 2.39 g der Zielverbindung (97.6% d. Th., Diastereomerenverhältnis ca. 1:1).

LC-MS (Methode 5): Rₜ = 1.05 min und 1.07 min; jeweils m/z = 273 (M-H)⁻.

Auf vergleichbare Weise wurden aus den entsprechenden Estern die folgenden Carbonsäuren hergestellt:

### Beispiel 125A

### (3R)-2-(4-Chlor-3-fluorphenyl)-4,4,4-trifluor-3-methylbutansäure (Diastereomerengemisch)

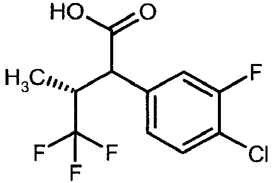

Diastereomerenverhältnis ca. 1:1.

GC-MS (Methode 1): Rₜ = 4.79 min; m/z = 284 (M)⁺.

¹H-NMR (400 MHz, DMSO-*d*₆): *beide Diastereomer:* δ [ppm] = 0.80/1.19 (je d, 3H), 3.18-3.29 (m, 1 H), 3.74/3.77 (je dd, 1 H), 7.28 (d, 1H), 7.43-7.65 (m, 2H), 12.91/13.24 (je br. s, 1H).

### Beispiel 126A

### (3R)-2-(4-Chlor-2-fluorphenyl)-4,4,4-trifluor-3-methylbutansäure (Diastereomerengemisch)

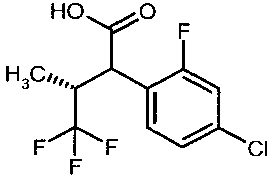

LC-MS (Methode 4): Rₜ = 1.25 min; m/z = 283 (M-H)⁻.

¹H-NMR (400 MHz, DMSO-*d₆*): *Haupt-Diastereomer:* δ [ppm] = 0.87 (d, 3H), 3.27-3.37 (m, 1H), 4.02 (d, 1H), 7.35 (dd, 1H), 7.45-7.52 (m, 2H), 13.02 (br. s, 1H).

### Beispiel 127A

### 1-(3-{[(2S)-2-Cyclopentyl-2-(4-methylphenyl)acetyl]amino}benzyl)cyclopropancarbonsäure-tert.-butylester

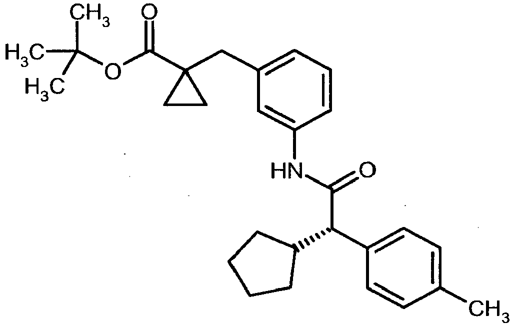

Zu einer Lösung von 200 mg (916 µmol) (+)-(2*S*)-Cyclopentyl(4-methylphenyl)essigsäure in 1 ml DMF wurden bei RT 453 mg (1191 µmol) HATU und 479 µl (2750 µmol) *N,N*-Diisopropylethylamin gegeben. Das Gemisch wurde 30 min gerührt. Anschließend wurden 249 mg (1008 µmol) *tert.*-Butyl-1-(3-aminobenzyl)cyclopropancarboxylat hinzugefügt. Nach Rühren über Nacht wurde das Reaktionsgemisch direkt über präparative HPLC in seine Komponenten aufgetrennt. Es wurden so 328 mg (80% d. Th.) der Titelverbindung erhalten.

LC-MS (Methode 5): Rₜ = 1.49 min; m/z = 448 (M+H)⁺.

¹H-NMR (400 MHz, DMSO-d₆): δ [ppm] = 0.74-0.85 (m, 2H), 0.89-1.01 (m, 1H), 1.01-1.10 (m, 2H), 1.14-1.69 (m, 15H), 1.70-1.87 (m, 1H), 2.25 (s, 3H), 2.78 (s, 2H), 6.89 (d, 1H), 7.04-7.20 (m, 3H), 7.25-7.32 (m, 2H), 7.36 (d, 1H), 7.50 (s, 1H), 9.91 (s, 1H).

### Beispiel 128A

### (+/-)-1-[3-({[4-(Acetoxymethyl)phenyl](cyclopentyl)acetyl}amino)benzyl]cyclopropancarbonsäure-tert. -butylester

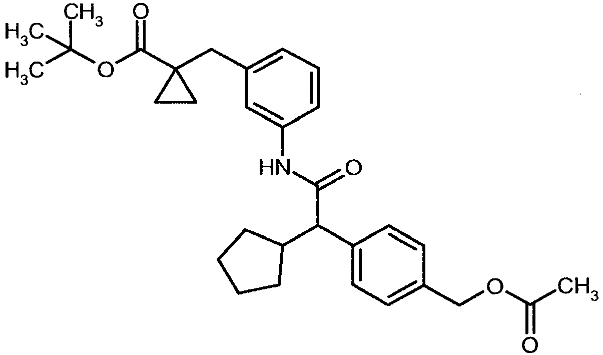

9.50 g (34.4 mmol) (+/-)-[4-(Acetoxymethyl)phenyl](cyclopentyl)essigsäure wurden in 67.5 ml DMF gelöst und mit 5.58 g (41.25 mmol) 1-Hydroxy-1*H*-benzotriazol-Hydrat (HOBt) versetzt. Die Mischung wurde auf 0°C gekühlt und mit 15.0 ml (85.95 mmol) *N,N-*Diisopropylethylamin und 10.63 g (42.97 mmol) *tert*.-Butyl-1-(3-aminobenzyl)cyclopropancarboxylat, gelöst in wenig DMF, versetzt. Die Reaktionsmischung wurde anschließend in mehreren Portionen mit 14.38 g (37.82 mmol) HATU versetzt, langsam auf RT erwärmt und dann über Nacht gerührt. Danach wurde das Gemisch auf gesättigte wässrige Natriumcarbonat-Lösung gegeben. Nach Phasentrennung wurde die wässrige Phase mehrfach mit Ethylacetat extrahiert, und die vereinigten organischen Phasen wurden mit gesättigter Natriumchlorid-Lösung gewaschen, über Natriumsulfat getrocknet und im Vakuum eingeengt. Das Rohprodukt wurde durch Chromatographie an Silicagel gereinigt (Laufmittel Dichlormethan/Ethylacetat 50:1 → 20:1). Es wurden so 12.86 g der Zielverbindung erhalten (69.4% d. Th.).

LC-MS (Methode 5): Rₜ = 1.40 min; m/z = 450 (M-C₄H₇)⁺.

¹H-NMR (400 MHz, DMSO-d₆): δ [ppm] = 0.77-0.83 (m, 2H), 0.91-1.02 (m, 1H), 1.03-1.08 (m, 2H), 1.21-1.29 (m, 1H), 1.26 (s, 9H), 1.32-1.40 (m, 1H), 1.41-1.70 (m, 4H), 1.72-1.84 (m, 1H), 2.04 (s, 3H), 2.55-2.65 (m, 1H), 2.78 (s, 2H), 3.39 (d, 1H), 5.02 (s, 2H), 6.89 (d, 1H), 7.15 (t, 1H), 7.27-7.33 (m, 2H), 7.35 (d, 1H), 7.38-7.43 (m, 2H), 7.50 (s, 1H), 9.96 (s, 1H).

### Beispiel 129A

### (+/-)-1-[3-({Cyclopentyl[4-(hydroxymethyl)phenyl]acetyl} amino)benzyl]cyclopropancarbonsäure-tert. -butylester

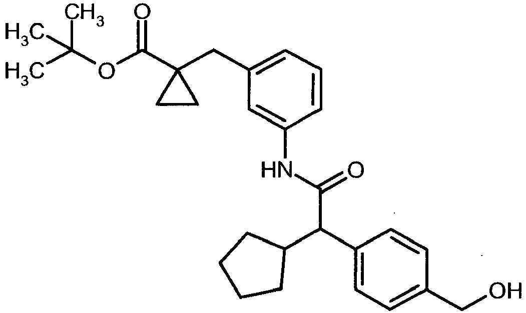

12.50 g (24.72 mmol) (+/-)-1-[3-({[4-(Acetoxymethyl)phenyl](cyclopentyl)acetyl}amino)benzyl]-cyclopropancarbonsäure-tert.-butylester wurden in 228 ml einer 2 M Lösung von Ammoniak in Methanol gelöst und 2 h bei 30°C, dann 2 h bei 40°C und zuletzt über Nacht bei RT gerührt. Die Lösung wurde anschließend im Vakuum eingeengt und der Rückstand durch Chromatographie an Silicagel gereinigt (Laufmittel Cyclohexan/Ethylacetat 10:1 → 2:1). Es wurden 11.88 g (96.5% d. Th.) der Zielverbindung erhalten.

LC-MS (Methode 4): Rₜ = 1.47 min; m/z = 462 (M-H)⁻.

¹H-NMR (400 MHz, DMSO-d₆): δ [ppm] = 0.77-0.83 (m, 2H), 0.92-1.03 (m, 1H), 1.03-1.09 (m, 2H), 1.21-1.28 (m, 1H), 1.27 (s, 9H), 1.30-1.40 (m, 1H), 1.41-1.69 (m, 4H), 1.73-1.83 (m, 1H), 2.54-2.65 (m, 1H), 2.78 (s, 2H), 3.36 (d, 1H), 4.44 (d, 2H), 5.10 (t, 1H), 6.89 (d, 1H), 7.15 (t, 1H), 7.24 (d, 2H), 7.35 (d, 3H), 7.50 (s, 1H), 9.93 (s, 1H).

### Beispiel 130A

### (+/-)-1-(3-{[Cyclopentyl(4-formylphenyl)acetyl]amino}benzyl)cyclopropancarbonsäure-tert.-butylester

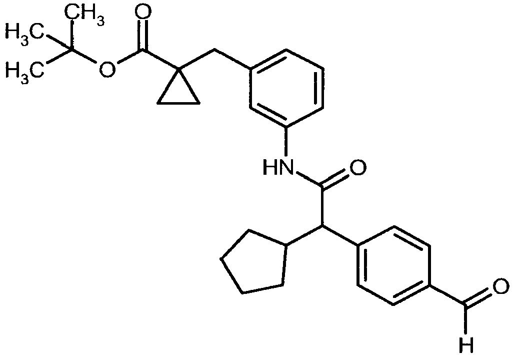

Eine Lösung von 4.0 g (8.63 mmol) (+/-)-1-[3-({Cyclopentyl[4-(hydroxymethyl)phenyl]acetyl}-amino)benzyl]cyclopropancarbonsäure-*tert*.-butylester in 20 ml Dichlormethan wurde auf 0°C gekühlt und mit 4.39 g (10.35 mmol) Dess-Martin-Reagenz [1,1,1-Triacetoxy-1,1-dihydro-1,2-benziodoxol-3(1*H*)-on] versetzt. Nach Ende der Zugabe wurde die Kühlung entfernt und die Reaktionsmischung 3 h bei RT nachgerührt. Dann wurde mit Dichlormethan verdünnt und die Lösung nacheinander mit Wasser, gesättigter Natriumcarbonat-Lösung und gesättigter Natriumchlorid-Lösung gewaschen. Die organische Phase wurde mit Magnesiumsulfat getrocknet und im Vakuum eingeengt. Das Rohprodukt wurde durch Chromatographie an Silicagel gereinigt (Laufmittel Cyclohexan/Ethylacetat 50:1 → 3:1). Es wurden 2.27 g der Zielverbindung erhalten (53.2% d. Th.).

LC-MS (Methode 2): Rₜ = 2.86 min; m/z = 460 (M-H)⁻.

¹H-NMR (400 MHz, DMSO-d₆): δ [ppm] = 0.78-0.84 (m, 2H), 0.92-1.02 (m, 1H), 1.03-1.08 (m, 2H), 1.26 (s, 9H), 1.27-1.39 (m, 2H), 1.42-1.51 (m, 1H), 1.51-1.70 (m, 3H), 1.76-1.86 (m, 1H), 2.58-2.68 (m, 1H), 2.78 (s, 2H), 3.53 (d, 1H), 6.91 (d, 1H), 7.16 (t, 1H), 7.36 (d, 1H), 7.51 (s, 1H), 7.60-7.68 (m, 2H), 7.82-7.90 (m, 2H), 9.97 (s, 1H), 10.06 (s, 1H).

### Beispiel 131A

### (+/-)-1-(3-{[Cyclopentyl(4-vinylphenyl)acetyl]amino}benzyl)cyclopropancarbonsäure-tert.-butylester

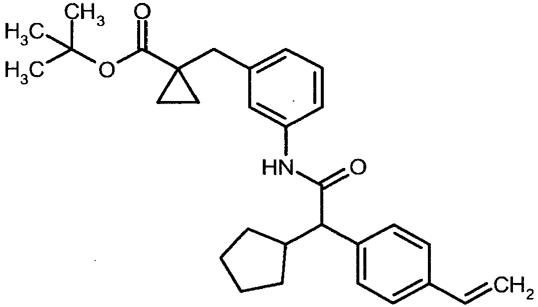

39.0 mg (0.98 mmol, 60% in Mineralöl) Natriumhydrid wurden in 3.0 ml abs. THF suspendiert, auf 0°C abgekühlt und mit 301.8 mg (0.84 mmol) Methyltriphenylphosphoniumbromid versetzt. Die Mischung wurde 30 min bei 0°C gerührt, bevor 300 mg (0.65 mmol) (+/-)-1-(3-{[Cyclopentyl(4-formylphenyl)acetyl]amino}benzyl)cyclopropancarbonsäure-*tert*.-butylester zugegeben wurden. Die Reaktionsmischung wurde über Nacht bei RT gerührt und dann im Vakuum eingeengt. Der Rückstand wurde in Acetonitril aufgenommen, filtriert und das erhaltene Filtrat durch präparative RP-HPLC gereinigt (Acetonitril/Wasser-Eluent). Es wurden 219.3 mg (73.4% d. Th.) der Zielverbindung erhalten.

LC-MS (Methode 5): Rₜ = 1.52 min; m/z = 460 (M+H)⁺.

¹H-NMR (400 MHz, DMSO-d₆): δ [ppm] = 0.80 (d, 2H), 0.92-1.02 (m, 1H), 1.06 (d, 2H), 1.22-1.29 (m, 1H), 1.26 (s, 9H), 1.32-1.49 (m, 2H), 1.49-1.71 (m, 3H), 1.74-1.83 (m, 1H), 2.56-2.67 (m, 1H), 2.78 (s, 2H), 3.38 (d, 1H), 5.22 (d, 1H), 5.78 (d, 1H), 6.70 (dd, 1H), 6.89 (d, 1H), 7.15 (t, 1H), 7.29-7.46 (m, 5H), 7.50 (s, 1H), 9.96 (s, 1H).

### Beispiel 132A

### (+/-)-1-[3-({Cyclopentyl[4-(2,2-difluorvinyl)phenyl]acetyl}amino)benzyl]cyclopropancarbonsäure-tert.butylester

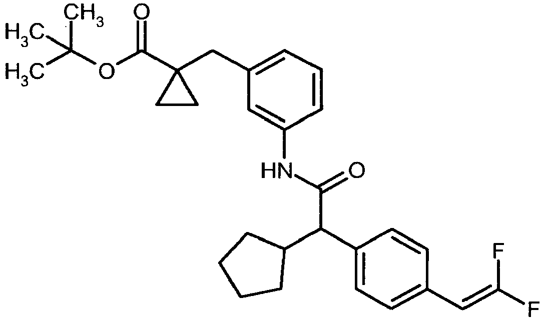

Zu einer Mischung von 800.0 mg (1.73 mmol) (+/-)-1-(3-{[Cyclopentyl(4-formylphenyl)acetyl]-amino}benzyl)cyclopropancarbonsäure-*tert*.-butylester und 681.9 mg (2.60 mmol) Triphenylphosphin wurden bei 60°C 475.6 mg (3.12 mmol) Natriumchlor(difluor)acetat gegeben. Die Mischung wurde auf 110°C erhitzt und 30 min bei dieser Temperatur gerührt. Nach dem Abkühlen wurde das Gemisch auf Wasser gegossen. Es wurde mit Ethylacetat versetzt, und die organische Phase wurde abgetrennt, mit gesättigter Natriumchlorid-Lösung gewaschen, mit Magnesiumsulfat getrocknet und im Vakuum eingeengt. Das Rohprodukt wurde durch präparative RP-HPLC gereinigt (Acetonitril/Wasser-Eluent). Es wurden 319.2 mg (37.2% d. Th.) der Zielverbindung erhalten.

LC-MS (Methode 5): Rₜ = 1.53 min; m/z = 494 (M-H)⁻.

¹H-NMR (400 MHz, DMSO-d₆): δ [ppm] = 0.76-0.85 (m, 2H), 0.92-1.03 (m, 1H), 1.03-1.09 (m, 2H), 1.19-1.26 (m, 1H), 1.26 (s, 9H), 1.32-1.41 (m, 1H), 1.41-1.69 (m, 4H), 1.74-1.84 (m, 1H), 2.57-2.64 (m, 1H), 2.78 (s, 2H), 3.38 (d, 1H), 6.90 (d, 1H), 7.15 (t, 1H), 7.28-7.33 (m, 2H), 7.36 (d, 1H), 7.38-7.45 (m, 2H), 7.50 (s, 1H), 9.97 (s, 1H).

### Beispiel 133A

### (+/-)-1-[3-({Cyclopentyl[4-(2,2-difluorethyl)phenyl]acetyl}amino)benzyl]cyclopropancarbonsäure-tert.butylester

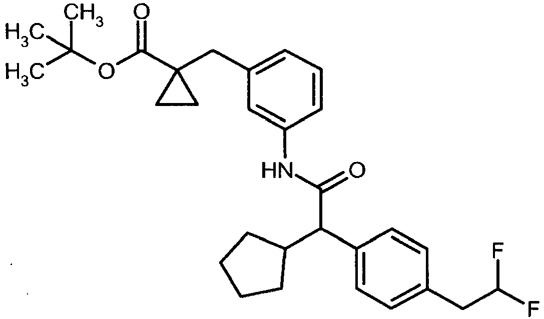

Zu einer Lösung von 200.0 mg (0.404 mmol) (+/-)-1-[3-({Cyclopentyl[4-(2,2-difluorvinyl)phenyl]-acetyl}amino)benzyl]cyclopropancarbonsäure-*tert*.butylester in 5 ml Ethanol wurden 100 mg Pd/C (10%) gegeben. Die Mischung wurde über Nacht bei Normaldruck unter einer Wasserstoffatmosphäre kräftig gerührt. Danach wurde der Ansatz über Celite abfiltriert und das Filtrat im Vakuum eingeengt. Das Rohprodukt wurde durch präparative RP-HPLC gereinigt (Acetonitril/ Wasser-Eluent). Es wurden 151.1 mg (75.2% d. Th.) der Zielverbindung erhalten.

LC-MS (Methode 5): Rₜ = 1.43 min; m/z = 498 (M+H)⁺.

¹H-NMR (400 MHz, DMSO-d₆): δ [ppm] = 0.77-0.83 (m, 2H), 0.91-1.02 (m, 1H), 1.02-1.08 (m, 2H), 1.23-1.29 (m, 1H), 1.26 (s, 9H), 1.30-1.39 (m, 1H), 1.40-1.70 (m, 4H), 1.74-1.84 (m, 1H), 2.55-2.64 (m, 1H), 2.78 (s, 2H), 3.12 (td, 2H), 3.38 (d, 1H), 6.20 (dt, 1H), 6.89 (d, 1H), 7.15 (t, 1H), 7.23 (d, 2H), 7.32-7.41 (m, 3H), 7.51 (s, 1H), 9.95 (s, 1H).

### Beispiel 134A

### (+/-)-1-[3-({Cyclopentyl[4-(trifluormethyl)phenyl]acetyl}amino)benzyl]cyclopropancarbonsäure-tert.-butylester

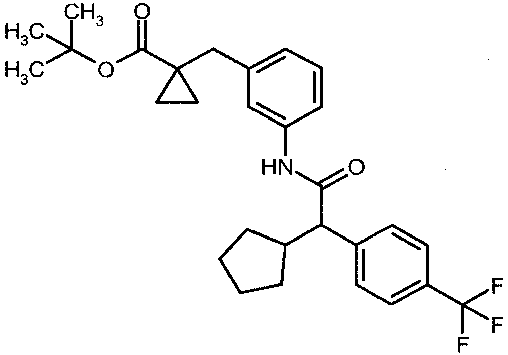

3.0 g (11.02 mmol) (+/-)-Cyclopentyl[4-(trifluormethyl)phenyl]essigsäure wurden in 16.0 ml DMF gelöst und mit 1.79 g (13.22 mmol) 1-Hydroxy-1*H*-benzotriazol-Hydrat versetzt. Die Mischung wurde auf 0°C gekühlt und mit 3.8 ml (22.04 mmol) *N,N*-Diisopropylethylamin und 3.41 g (13.77 mol) *tert*.-Butyl-1-(3-aminobenzyl)cyclopropancarboxylat, gelöst in wenig DMF, versetzt. Die Reaktionsmischung wurde danach in mehreren Portionen mit 5.03 g (13.22 mmol) HATU versetzt, langsam auf RT erwärmt und anschließend 3 h bei RT gerührt. Der Ansatz wurde dann auf Wasser gegeben. Nach Phasentrennung wurde die wässrige Phase dreimal mit Ethylacetat extrahiert, und die vereinigten organischen Phasen wurden mit gesättigter Natriumchlorid-Lösung gewaschen, über Magnesiumsulfat getrocknet und im Vakuum eingeengt. Das Rohprodukt wurde durch Chromatographie an Silicagel gereinigt (Laufmittel Cyclohexan/Ethylacetat 20:1 → 10:1). Es wurden 4.89 g der Zielverbindung erhalten (88.5% d. Th.).

LC-MS (Methode 5): Rₜ = 1.49 min; m/z = 502 (M+H)⁺.

¹H-NMR (400 MHz, DMSO-d₆): δ [ppm] = 0.77-0.83 (m, 2H), 0.92-1.00 (m, 1H), 1.03-1.08 (m, 2H), 1.25 (s, 9H), 1.26-1.40 (m, 2H), 1.43-1.50 (m, 1H), 1.50-1.72 (m, 3H), 1.75-1.86 (m, 1H), 2.56-2.72 (m, 1H), 2.78 (s, 2H), 3.52 (d, 1H), 6.91 (d, 1H), 7.17 (t, 1H), 7.33-7.39 (m, 1H), 7.50 (s, 1H), 7.61-7.66 (m, 2H), 7.66-7.73 (m, 2H), 10.06 (s, 1H).

### Beispiel 135A

### (+/-)-1-(3-{[(4-Chlorphenyl)(cyclopentyl)acetyl]amino}benzyl)cyclopropancarbonsäure-tert.-butylester

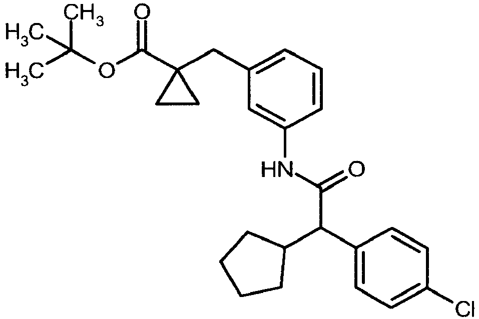

3.50 g (14.66 mmol) (+/-)-Cyclopentyl(4-chlorphenyl)essigsäure wurden in 28.8 ml DMF gelöst und mit 2.38 g (17.6 mmol) 1-Hydroxy-1*H*-benzotriazol-Hydrat versetzt. Die Mischung wurde auf 0°C gekühlt und mit 10.2 ml (58.65 mmol) *N,N*-Diisopropylethylamin und 4.98 g (91% Reinheit, 18.33 mmol) *tert*.-Butyl-1-(3-aminobenzyl)cyclopropancarboxylat, gelöst in wenig DMF, versetzt. Die Reaktionsmischung wurde danach in mehreren Portionen mit 6.13 g (16.13 mmol) HATU versetzt, 30 min bei 0°C gerührt, dann langsam auf RT erwärmt und 3 h bei dieser Temperatur nachgerührt. Anschließend wurde der Ansatz auf gesättigte wässrige Natriumcarbonat-Lösung gegeben. Nach Phasentrennung wurde die wässrige Phase mehrfach mit Ethylacetat extrahiert, und die vereinigten organischen Phasen wurden mit gesättigter Natriumchlorid-Lösung gewaschen, über Natriumsulfat getrocknet und im Vakuum eingeengt. Das Rohprodukt wurde durch Chromatographie an Silicagel gereinigt (Laufmittel Dichlormethan/Ethylacetat 10:1 → 8:1). Es wurden so 6.63 g der Zielverbindung erhalten (96.6% d. Th.).

LC-MS (Methode 4): Rₜ = 1.73 min; m/z = 412 (M-C₄H₈)⁺.

¹H-NMR (400 MHz, DMSO-d₆): δ [ppm] = 0.77-0.83 (m, 2H), 0.91-1.01 (m, 1H), 1.01-1.08 (m, 2H), 1.21-1.29 (m, 1H), 1.26 (s, 9H), 1.32-1.40 (m, 1H), 1.40-1.70 (m, 4H), 1.70-1.84 (m, 1H), 2.55-2.60 (m, 1H), 2.78 (s, 2H), 3.40 (d, 1H), 6.87-6.93 (m, 1H), 7.16 (t, 1H), 7.30-7.46 (m, 5H), 7.50 (s, 1H), 9.99 (s, 1H).

### Allgemeine Vorschrift 6: HATU-vermittelte Amidkupplung von substituierten Phenylessigsäure-Derivaten mit Anilinen

Zu einer Lösung des betreffenden Phenylessigsäure-Derivats (ca. 0.8 bis 2.0 eq., 0.15 bis 1.5 mol/l) und eines Anilins (ca. 0.8 bis 2.0 eq., 0.15 bis 1.5 mol/l) in einem Gemisch aus DMF und Pyridin (Mischungsverhältnis ca. 3:1 bis 1.5:1) wurde bei 0°C oder RT HATU (1.0 bis 2.0 eq.) gegeben. Alternativ kann statt Pyridin auch *N,N*-Diisopropylethylamin (2.0 bis 5.0 eq.), optional in Gegenwart von HOBt (1.0 bis 2.0 eq.), verwendet werden. Die resultierende Mischung wurde für 4 h bis 48 h bei Temperaturen von RT bis 60°C gerührt. Gegebenenfalls wurde nach 24 h ein weiterer Anteil an dem Anilin oder an der Phenylessigsäure zusammen mit HATU hinzugefügt. Nach Ende der Reaktion wurde das Rohprodukt nach Entfernen des Lösungsmittels im Vakuum durch präparative RP-HPLC (Eluent: Acetonitril/Wasser-Gradient) oder alternativ, nach wässriger Aufarbeitung der Reaktionsmischung, durch Chromatographie an Silicagel (Eluent: Cyclohexan/ Ethylacetat- oder Dichlormethan/Methanol-Gemische) gereinigt. Auch die Kombination beider Reinigungsmethoden kann verwendet werden, um das Zielprodukt in reiner Form zu erhalten.

Die folgenden Beispiele wurden gemäß der Allgemeinen Vorschrift 6 hergestellt:

| **Beispiel** | **Name / Struktur / Edukte** | **Analytische Daten** |
|---|---|---|
| **136A** | (+/-)-1-(3-{[Cyclopentyl(4-bromphenyl)acetyl]-amino}-4-fluorbenzyl)cyclopropancarbonsäure-*tert*.-butylester | LC-MS (Methode 5): |
| | | Rₜ = 1.56 min; m/z = 547/549 (M+NH₄)⁺. |
| | 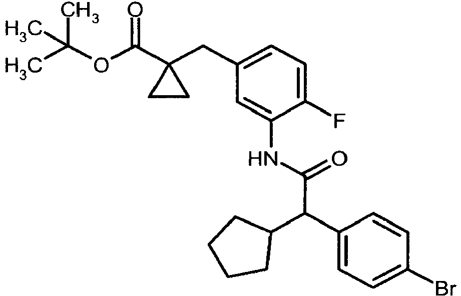 | ¹H-NMR (400 MHz, DMSO-*d*₆): δ [ppm]= 0.80 (q, 2H), 0.89-1.00 (m, 1H), 1.01-1.06 (m, 2H), 1.22-1.72 (m, 15H), 1.72-1.88 (m, 1H), 2.77 (s, 2H), 3.60 (s, 1H), 6.90-7.03 (m, 1H), 7.11 (dd, 1H), 7.37 (d, 2H), 7.51 (d, 2H), 7.68 (dd, 1H), 9.81 (s, 1H). |
| **137A** | (+/-)-1-(3- {[Cyclopentyl(3,4-dichlorphenyl)acetyl]-amino}benzyl)cyclopropancarbonsäure-*tert*.-butylester | LC-MS (Methode 5): |
| | | Rₜ = 1.55 min; m/z = 446 (M-C₄H₈)⁺. |
| | 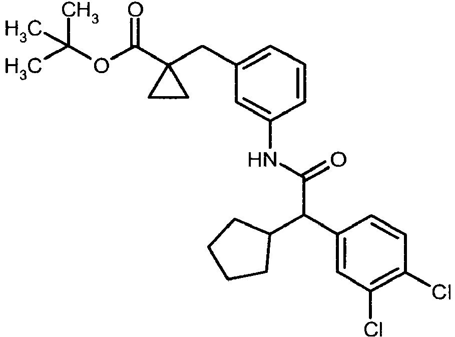 | ¹H-NMR (400 MHz, DMSO-*d*₆): δ [ppm] = 0.79-0.84 (m, 2H), 0.94-1.02 (m, 1H), 1.02-1.08 (m, 2H), 1.21-1.30 (m, 1H), 1.26 (s, 9H), 1.31-1.43 (m, 1H), 1.43-1.68 (m, 4H), 1.70-1.84 (m, 1H), 2.50-2.60 (m, 1H), 2.78 (s, 2H), 3.42 (d, 1H), 6.92 (d, 1H), 7.17 (t, 1H), 7.29-7.42 (m, 2H), 7.50 (s, 1H), 7.57-7.65 (m, 2H), 10.04 (s, 1H). |
| | aus *tert*.-Butyl-1-(3-aminobenzyl)cyclopropancarboxylat und (+/-)-Cyclopentyl(3,4-dichlorphenyl)essigsäure | |
| **138A** | (+/-)-1-(3-{[(4-Chlor-2-fluorphenyl)(cyclopentyl)-acetyl]amino}benzyl)cyclopropancarbonsäure-*tert*.-butylester | LC-MS (Methode 5): Rt = 1.52 min; m/z = 486 (M+H)⁺. |
| | 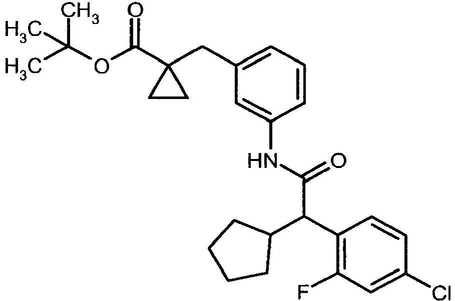 | ¹H-NMR (400 MHz, DMSO-*d*₆): δ [ppm] = 0.78-0.84 (m, 2H), 0.95-1.02 (m, 1H), 1.03-1.09 (m, 2H), 1.26 (s, 9H), 1.30-1.60 (m, 5H), 1.61-1.79 (m, 2H), 2.48-2.57 (m, 1H), 2.78 (s, 2H), 3.77 (d, 1H), 6.92 (d, 1H), 7.17 (t, 1H), 7.29 (dd, 1H), 7.34-7.43 (m, 2H), 7.52 (s, 1H), 7.70 (t, 1H), 10.12 (s, 1H). |
| | aus *tert*.-Butyl-1-(3-aminobenzyl)cyclopropancarboxylat und (+/-)-(4-Chlor-2-fluorphenyl)-(cyclopentyl)essigsäure | |
| **139A** | (+/-)-1-(3-{[Cyclopentyl(2,4-dichlorphenyl)-acetyl]amino}benzyl)cyclopropancarbonsäure-*tert*.-butylester | LC-MS (Methode 5): |
| | | Rₜ = 1.56 min, m/z = 446 (M-C₄H₈)⁺. |
| | 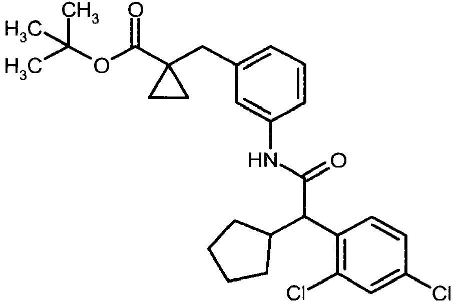 | ¹H-NMR (400 MHz, DMSO-*d*₆): δ [ppm] = 0.77-0.83 (m, 2H), 0.95-1.02 (m, 1H), 1.04-1.10 (m, 2H), 1.26 (s, 9H), 1.38-1.63 (m, 5H), 1.64-1.79 (m, 2H), 2.51-2.57 (m, 1H, verdeckt), 2.79 (s, 2H), 3.96 (d, 1H), 6.92 (d, 1H), 7.17 (t, 1H), 7.40 (d, 1H), 7.44 (dd, 1H), 7.51 (s, 1H), 7.58 (d, 1H), 7.78 (d, 1H), 10.09 (s, 1H). |
| | aus *tert*.-Butyl-1-(3-aminobenzyl)cyclopropancarboxylat und (+/-)-Cyclopentyl(2,4-dichlorphenyl)-essigsäure | |
| **140A** | (+-/-)-1-[3-({Cyclopentyl[3-fluor-4-(trifluormethyl)-phenyl]acetyl}amino)benzyl]cyclopropancarbonsäure-*tert*.-butylester | LC-MS (Methode 5): Rₜ = 1.51 min; m/z = 520 (M+H)⁺. |
| | | ¹H-NMR (400 MHz, DMSO-*d*₆): δ [ppm] = 0.78-0.84 (m, 2H), 0.94-1.02 (m, 1H), 1.02-1.08 (m, 2H), 1.25 (s, 9H), 1.26-1.34 (m, 1H), 1.34-1.43 (m, 1H), 1.44-1.70 (m, 4H), 1.75-1.83 (m, 1H), 2.55-2.65 (m, 1H), 2.78 (s, 2H), 3.54 (d, 1H), 6.92 (d, 1H), 7.17 (t, 1H), 7.35 (d, 1H), 7.43 (d, 1H), 7.48-7.56 (m, 2H), 7.75 (t, 1H), 10.10 (s, 1H). |
| | 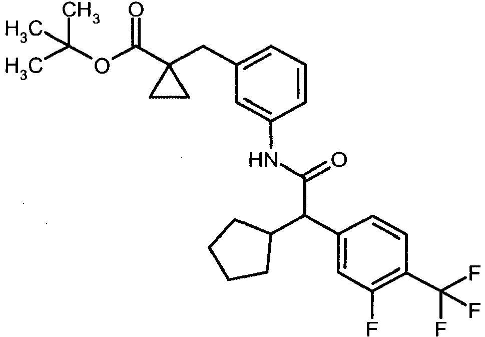 | |
| | aus *tert*.-Butyl-1-(3-aminobenzyl)cyclopropancarboxylat und (+/-)-Cyclopentyl[3-fluor-4-(trifluormethyl)phenyl]essigsäure | |
| **141A** | (+/-)-1-[3-({Cyclopentyl[4-(trifluormethoxy)phenyl]-acetyl}amino)benzyl]cyclopropancarbonsäure-*tert*.-butylester | LC-MS (Methode 5): Rₜ = 1.51 min; m/z = 518 (M+H)⁺. |
| | 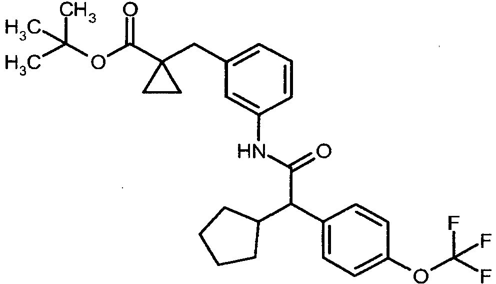 | ¹H-NMR (400 MHz, DMSO-*d*₆): δ [ppm] = 0.77-0.84 (m, 2H), 0.92-1.01 (m, 1H), 1.03-1.08 (m, 2H), 1.26 (s, 9H), 1.26-1.41 (m, 2H), 1.42-1.71 (m, 4H), 1.72-1.84 (m, 1H), 2.52-2.62 (m, 1H), 2.78 (s, 2H), 3.45 (d, 1H), 6.91 (d, 1H), 7.16 (t, 1H), 7.27-7.39 (m, 3H), 7.47-7.55 (m, 3H), 10.02 (s, 1H). |
| | aus *tert*.-Butyl-1-(3-aminobenzyl)cyclopropancarboxylat und (+/-)-Cyclopentyl[4-(trifluormethoxy)-phenyl]essigsäure | |
| **142A** | (+/-)-1-(3-{[(4-Chlorphenyl)(cyclopentyl)acetyl]-amino}-2-fluorbenzyl)cyclopropancarbonsäure-*tert*.-butylester | LC-MS (Methode 4): Rₜ = 1.77 min; m/z = 485 (M-H)-. |
| | 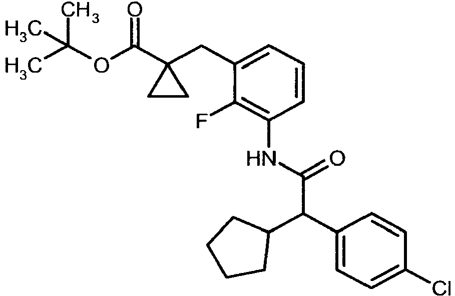 | ¹H-NMR (400 MHz, DMSO-d₆): δ [ppm] = 0.77-0.86 (m, 2H), 0.89-1.02 (m, 1H), 1.07-1.14 (m, 2H), 1.18-1.71 (m, 15H), 1.73-1.85 (m, 1H), 2.80-2.94 (m, 2H), 3.63 (d, 1H), 7.00-7.14 (m, 2H), 7.33-7.48 (m, 4H), 7.56-7.74 (m, 1H), 9.81 (s, 1H). |
| | aus *tert*.-Butyl-1-(3-amino-2-fluorbenzyl)-cyclopropancarboxylat und (+/-)-Cyclopentyl-(4-chlorphenyl)essigsäure | |
| **143A** | (+/-)-1-(3-{[Cyclopentyl(4-cyanophenyl)acetyl]-amino}-4-fluorbenzyl)cyclopropancarbonsäure-*tert*.-butylester | LC-MS (Methode 2): Rₜ = 2.90 min; m/z = 475 (M-H)⁻. |
| | 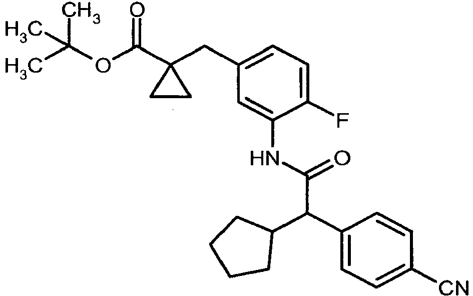 | ¹H-NMR (400 MHz, DMSO-d₆): δ [ppm] = 0.76-0.84 (m, 2H), 0.88-1.00 (m, 1H), 1.03 (q, 2H), 1.19-1.73 (m, 15H), 1.75-1.86 (m, 1H), 2.76 (s, 2H), 3.72 (d, 1H), 6.94-7.04 (m, 1H), 7.12(dd, 1H), 7.61 (d, 2H), 7.68 (dd, 1H), 7.80 (d, 2H), 9.89 (s, 1H). |
| | aus *tert*.-Butyl-1-(3-amino-4-fluorbenzyl)-cyclopropancarboxylat und (+/-)-(4-Cyanophenyl)-(cyclopentyl)essigsäure | |
| **144A** | (+/-)-1-(3-{[Cyclopentyl(4-nitrophenyl)acetyl]-amino}-4-fluorbenzyl)cyclopropancarbonsäure-*tert*.-butylester | LC-MS (Methode 5): Rt = 1.47 min; m/z = 495 (M-H)⁻. |
| | 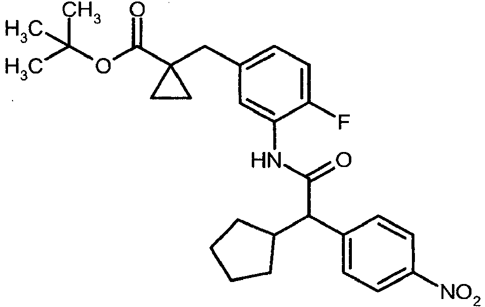 | ¹H-NMR (400 MHz, DMSO-*d₆*): δ [ppm] = 0.80 (q, 2H), 0.96 (dq, 1H), 1.01-1.06 (m, 2H), 1.16-1.76 (m, 15H), 1.76-1.92 (m, 1H), 2.77 (s, 2H), 3.80 (d, 1H), 6.92-7.05 (m, 1H), 7.12 (dd, 1H), 7.62-7.82 (m, 3H), 8.21 (d, 2H), 9.94 (s, 1H). |
| | aus *tert*.-Butyl-1-(3-amino-4-fluorbenzyl)-cyclopropancarboxylat und (+/-)-Cyclopentyl-(4-nitrophenyl)essigsäure | |
| **145A** | (+)-1-(3-{[(2*S*)-2-Cyclopentyl-2-(4-ethylphenyl)-acetyl]amino}benzyl)cyclopropancarbonsäure-*tert*.-butylester | LC-MS (Methode 5): Rₜ = 1.54 min; m/z = 462 (M+H)⁺. |
| | 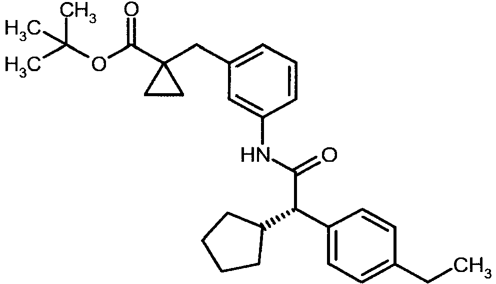 | ¹H-NMR (400 MHz, DMSO-*d₆*): δ [ppm] = 0.77-0.83 (m, 2H), 0.92-1.02 (m, 1H), 1.02-1.08 (m, 2H), 1.15 (t, 3H), 1.21-1.27 (m, 1H), 1.26 (s, 9H), 1.30-1.40 (m, 1H), 1.41-1.69 (m, 4H), 1.70-1.83 (m, 1 H), 2.53-2.61 (m, 3H), 2.78 (s, 2H), 3.34 (d, 1H), 6.89 (d, 1 H), 7.10-7.19 (m, 3H), 7.31 (d, 2H), 7.36 (d, 1H), 7.51 (s, 1H), 9.92 (s, 1H). |
| | aus *tert*.-Butyl-1-(3-aminobenzyl)cyclopropancarboxylat und (2*S*)-Cyclopentyl(4-ethylphenyl)-essigsäure | |
| | | [α]_{D}²⁰ = +42.9°, c = 0.600, Chloroform. |
| **146A** | (+)-1-(3-{[(2*S*)-2-Cyclopentyl-2-(4-ethylphenyl)-acetyl]amino}-5-fluorbenzyl)cyclopropancarbonsäure-*tert.*-butylester | LC-MS (Methode 2): Rₜ = 3.23 min; m/z = 478 (M-H)⁻. |
| | 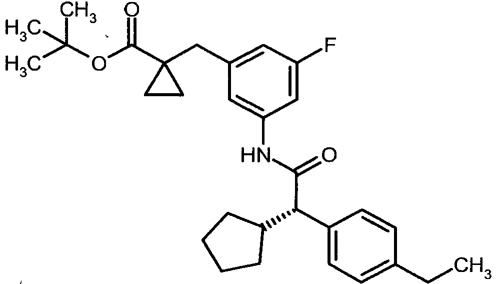 | ¹H-NMR (400 MHz, DMSO-*d₆*): δ [ppm] = 0.81-0.89 (m, 2H), 0.91-1.03 (m, 1H), 1.09 (q, 2H), 1.15 (t, 3H), 1.18-1.23 (m, 1H), 1.25(s, 9H), 1.30-1.40 (m, 1H), 1.41-1.68 (m, 4H), 1.70-1.84 (m, 1H), 2.51-2.62 (m, 3H), 2.78 (s, 2H), 3.32 (d, 1H), 6.71 (d, |
| | aus *tert*.-Butyl-1-(3-amino-5-fluorbenzyl)-cyclopropancarboxylat und (2*S*)-Cyclopentyl-(4-ethylphenyl)essigsäure | 1H), 7.09-7.18 (m, 2H), 7.23 (s, 1H), 7.26-7.33 (m, 2H), 7.39 (d, 1H), 10.14 (s, 1H). |
| | | [α]_{D}²⁰ = +34.1°, c = 0.500, Chloroform. |
| **147A** | (+)-1-(3-{[(2*S*)-2-Cyclopentyl-2-(4-ethylphenyl)-acetyl]amino}-4-fluorbenzyl)cyclopropancarbonsäure-*tert*.-butylester | LC-MS (Methode 5): Rₜ = 1.54 min; m/z = 480 (M+H)⁺. |
| | 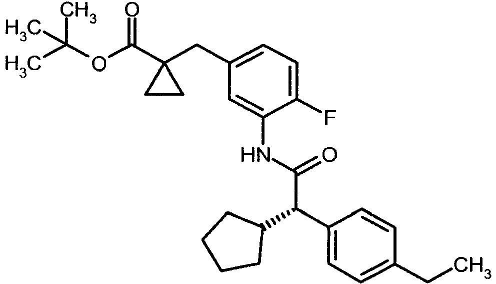 | ¹H-NMR (400 MHz, DMSO-*d₆):* δ [ppm] = 0.80 (q, 2H), 0.9.1-0.99 (m, 1H), 1.00-1.05 (m, 2H), 1.16 (t, 3H), 1.26 (s, 9H), 1.26-1.40 (m, 2H), 1.40-1.48 (m, 1H), 1.48-1.71 (m, 3H), 1.71-1.84 (m, 1H), 2.55-2.62 (m, 2H), 2.76 (s, 2H), 3.55 (d, 1H), 6.91-7.03 (m, |
| | aus *tert*.-Butyl-1-(3-amino-4-fluorbenzyl)-cyclopropancarboxylat und (2*S*)-Cyclopentyl(4-ethylphenyl)essigsäure | 1H), 7.04-7.11 (m, 1H), 7.14 (d, 2H), 7.32 (d, 2H), 7.70 (dd, 1H), 9.72 (s, 1H). |
| | | [α]_{D}²⁰ = +66.6°, c = 0.575, Chloroform. |
| **148A** | (+/-)-1-(3-{[2-(4-Ethylphenyl)-4,4,4-trifluor-3-methyl-butanoyl]amino}benzyl)cyclopropancarbonsäure-*tert*.-butylester | LC-MS (Methode 5): |
| | | Rₜ = 1.49 min; m/z = 434 (M-C₄H₇)⁺. |
| | 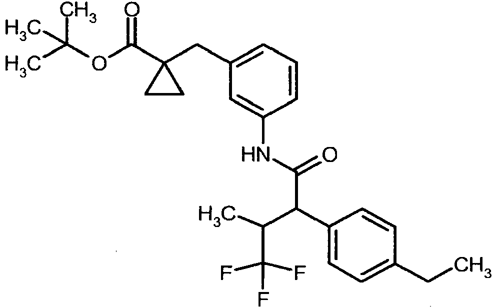 | ¹H-NMR (400 MHz, DMSO-*d₆*): δ [ppm] = 0.78 (d, 3H), 0.79-0.83 (m,2H), 1.02-1.08 (m, 2H), 1.15 (t, 3H), 1.25 (s, 9H), 2.57 (q, 2H), 2.77 (s, 2H), 3.34-3.41 (m, 1H), 3.78 (d, 1H), 6.90 (d, 1H), 7.11-7.16 (m, 1H), 7.16-7.22 (m, 2H), 7.32 (d, 3H), 7.47 (s, 1H), 10.10 (s, 1H). |
| | aus *tert*.-Butyl-1-(3-aminobenzyl)cyclopropan-carboxylat und 2-(4-Ethylphenyl)-4,4,4-trifluor-3-methylbutansäure | |
| **149A** | (+/-)-1-(3-{[2-(4-Ethylphenyl)-4,4,4-trifluor-3-methylbutanoyl]amino}-4-fluorbenzyl)cyclopropan-carbonsäure-*tert*. -butylester | LC-MS (Methode 5): Rₜ = 1.50 min; m/z = 506 (M-H)⁻. |
| | 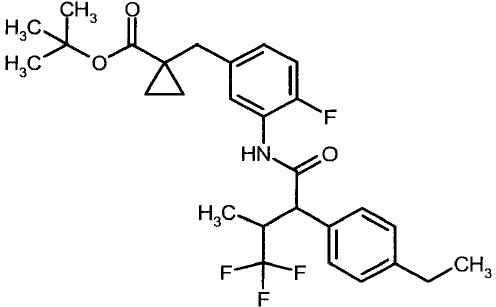 | ¹H-NMR (400 MHz, DMSO-*d₆*): δ [ppm] = 0.77 (d, 3H), 0.78-0.83 (m, 2H), 0.99-1.06 (m, 2H), 1.16 (s, 3H), 1.25 (s, 9H), 2.58 (q, 2H), 2.76 (d, 2H), 3.32-3.38 (m, 1H), 4.03 (d, 1H), 6.91-7.01 (m, 1H), 7.10 (dd, 1H), 7.17-7.22 (m, 2H), 7.29-7.35 (m, 2H), 7.73 (dd, 1H), 9.93 (s, 1H). |
| | aus *tert*.-Butyl-1-(3-amino-4-fluorbenzyl)-cyclopropancarboxylat und 2-(4-Ethylphenyl)-4,4,4-trifluor-3-methylbutansäure | |
| **150A** | (+/-)-1-(3- {[(4-Chlorphenyl)(cyclopentyl)acetyl]-amino}-4-fluorbenzyl)cyclopropancarbonsäure-*tert*.-butylester | LC-MS (Methode 5): |
| | | Rₜ = 1.51 min; m/z = 484/485 (M-H)⁻. |
| | 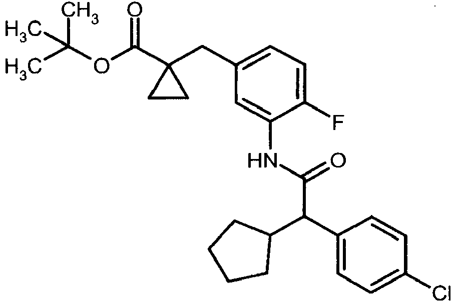 | ¹H-NMR (400 MHz, DMSO-*d₆*): δ [ppm] = 0.80 (q, 2H), 0.91-1.00 (m, 1H), 1.03 (q, 2H), 1.26 (s, 9H), 1.27-1.40 (m, 2H), 1.40-1.70 (m, 4H), 1.72-1.85 (m, 1H), 2.53-2.59 (m, 1H), 2.77 (s, 2H), 3.61 (d, 1H), 6.93-7.03 (m, 1H), 7.11 (dd, 1H), 7.34-7.40 (m, 2H), 7.40-7.47 (m, 2H), 7.69 (dd, 1H), 9.81 (s, 1H). |
| | aus *tert*.-Butyl-1-(3-amino-4-fluorbenzyl)-cyclopropancarboxylat und (+/-)-Cyclopentyl-(4-chlorphenyl)essigsäure | |
| **151A** | (+/-)-1-(3-{[Cyclopentyl(4-fluorphenyl)acetyl]-amino}-4-fluorbenzyl)cyclopropancarbonsäure-*tert*.-butylester | LC-MS (Methode 5): Rₜ = 1.49 min; m/z = 468 (M-H)⁻. |
| | 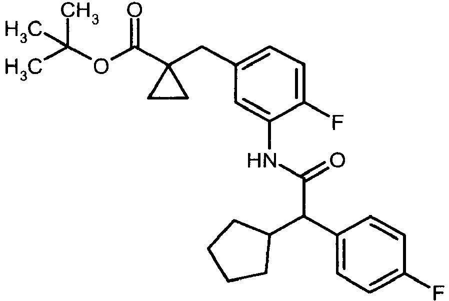 | ¹H-NMR (400 MHz, DMSO-*d₆*): δ [ppm] = 0.74-0.83 (m, 2H), 0.88-1.00 (m, 1H), 1.00-1.06 (m, 2H), 1.26 (s, 9H), 1.28-1.39 (m, 2H), 1.42-1.71 (m, 4H), 1.71-1.86 (m, 1H), 2.50-2.58 (m, 1H, verdeckt), 2.77 (s, 2H), 3.60 (d, 1H), 6.90-7.04 (m, 1H), 7.05-7.21 (m, 3H), 7.44 (dd, 2H), 7.70 (dd, 1H), 9.78 (s, 1H). |
| | aus *tert*.-Butyl-1-(3-amino-4-fluorbenzyl)-cyclopropancarboxylat und (+/-)-Cyclopentyl-(4-fluorphenyl)essigsäure | |
| **152A** | (+/-)-1-(3-{[(4-Chlor-2-fluorphenyl)(cyclopentyl)-acetyl]amino}-4-fluorbenzyl)cyclopropancarbonsäure-*tert*.butylester | LC-MS (Methode 5): Rₜ = 1.57 min; m/z = 502 (M-H)⁻. |
| | 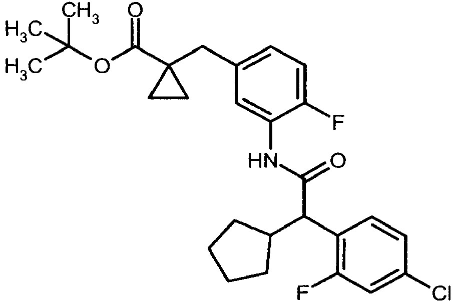 | ¹H-NMR (400 MHz, DMSO-*d₆*): δ [ppm] = 0.77-0.84 (m, 2H), 0.93-1.00 (m, 1H), 1.00-1.07 (m, 2H), 1.27 (s, 9H), 1.34-1.62 (m, 5H), 1.62-1.73 (m, 1H), 1.73-1.84 (m, 1H), 2.43-2.53 (m, 1H), 2.78 (s, 2H), 3.94 (d, 1H), 6.95-7.06 (m, 1H), 7.12 (dd, 1H), 7.29 (dd, 1H), 7.40 (dd, 1H), 7.59 (dd, 1H), 7.68 (t, 1H), 9.94 (s, 1H). |
| | aus *tert*.-Butyl-1-(3-amino-4-fluorbenzyl)-cyclopropancarboxylat und (+/-)-(4-Chlor-2-fluorphenyl)(cyclopentyl)essigsäure | |
| **153A** | (+)-1-(4-Fluor-3-{[(2*S*,3*R*)-4,4,4-trifluor-2-(4-fluorphenyl)-3-methylbutanoyl]amino}benzyl)-cyclopropancarbonsäure-*tert.*-butylester | LC-MS (Methode 5): Rₜ = 1.43 min; m/z = 496 (M-H)⁻. |
| | 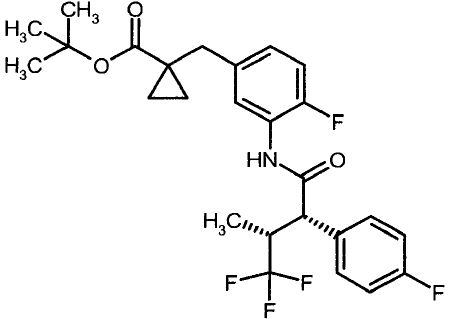 | ¹H-NMR (400 MHz, DMSO-*d₆*): δ [ppm] = 0.78 (d, 3H), 0.79-0.83 (m, 2H), 1.03 (q, 2H), 1.25 (s, 9H), 2.76 (d, 2H), 3.33-3.41 (m, 1H), 4.09 (d, 1H), 6.93-7.02 (m, 1H), 7.11 (dd, 1H), 7.20 (t, 2H), 7.47 (dd, 2H), 7.72 (dd, 1H), 9.99 (s, 1H). |
| | aus *tert*.-Butyl-1-(3-amino-4-fluorbenzyl)cyclo-propancarboxylat und (3*R*)-4,4,4-Trifluor-2-(4-fluorphenyl)-3-methylbutansäure (*Diastereomerengemisch*) | [α]_{D}²⁰ = +109.2°, c = 0.545, Chloroform. |
| **154A** | (+)-1-(3- {[(2*S*,3*R*)-2-(4-Ethylphenyl)-4,4,4-trifluor-3-methylbutanoyl]amino}-4-fluorbenzyl)-cyclopropancarbonsäure-*tert.*-butylester | LC-MS (Methode 5): Rₜ = 1.51 min; m/z = 506 (M-H)⁻. |
| | 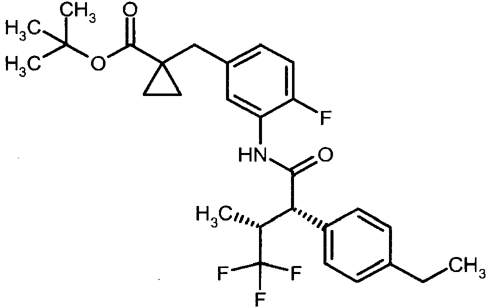 | ¹H-NMR (400 MHz, DMSO-*d₆*): δ [ppm] = 0.77 (d, 3H), 0.78-0.82 (m, 2H), 0.99-1.06 (m, 2H), 1.16 (t, 3H), 1.25 (s, 9H), 2.58 (q, 2H), 2.72-2.83 (m, 2H), 3.33-3.40 (m, 1H), 4.00-4.05 (m, 1H), 6.94-7.01 (m, 1H), 7.10 (dd, 1H), 7.16-7.23 (m, 2H), 7.31-7.36 (m, 2H), 7.73 (dd, 1H), 9.94 (s, 1H). |
| | aus *tert*.-Butyl-1-(3-amino-4-fluorbenzyl)cyclo-propancarboxylat und (3*R*)-2-(4-Ethylphenyl)-4,4,4-trifluor-3-methylbutansäure (*Diastereomerengemisch*) | |
| | | [α]_{D}²⁰ = +73.3°, c = 0.525, Chloroform. |
| **155A** | (+)-1-(3-{[(2*S*,3*R*)-2-(4-Ethylphenyl)-4,4,4-trifluor-3-methylbutanoyl]amino}-2-fluorbenzyl)-cyclopropancarbonsäure-*tert.*-butylester | LC-MS (Methode 5): Rₜ = 1.48 min; m/z = 506 (M-H)⁻. |
| | 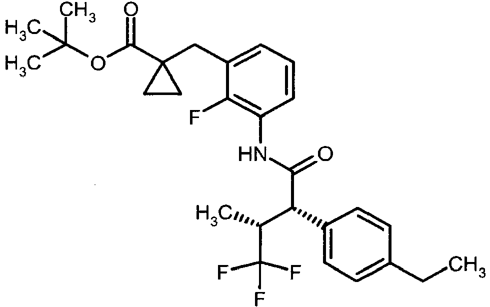 | ¹H-NMR (400 MHz, DMSO-*d₆*): δ [ppm] = 0.77 (d, 3H), 0.79-0.83 (m, 2H), 1.06-1.11 (m, 2H), 1.17 (t, 3H), 1.24 (s, 9H), 2.58 (q, 2H), 2.78-2.93 (m, 2H), 3.33-3.39 (m, 1H), 4.05 (d, 1H), 6.98-7.11 (m, 2H), 7.16-7.23 (m, 2H), 7.28-7.36 (m, 2H), 7.64 (td, 1H), 9.94 (s, 1H). |
| | aus *tert*.-Butyl-1-(3-amino-2-fluorbenzyl)cyclo-propancarboxylat und (3*R*)-2-(4-Ethylphenyl)-4,4,4-trifluor-3-methylbutansäure (*Diastereomerengemisch*) | [α]_{D}²⁰ = +34.2°, c = 0.505, Chloroform. |
| **156A** | (+)-1-(4-Fluor-3-{[(2*S*,3*R*)-4,4,4-trifluor-3-methyl-2-(4-vinylphenyl)butanoyl]amino}benzyl)cyclo-propancarbonsäure-*tert*.-butylester | LC-MS (Methode 5): Rₜ = 1.48 min; m/z = 504 (M-H)⁻. |
| | 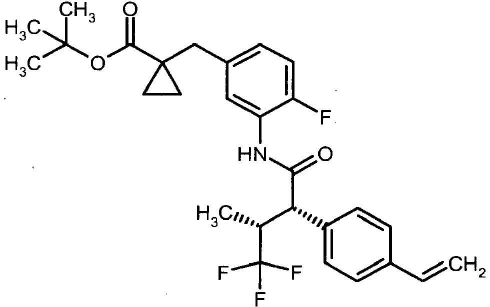 | ¹H-NMR (400 MHz, DMSO-*d₆*): δ [ppm] = 0.75-0.82 (m, 5H), 0.99-1.06 (m, 2H), 1.25 (s, 9H), 2.76 (s, 2H), 3.34-3.46 (m, 1H), 4.04-4.11 (m, 1H), 5.26 (d, 1H), 5.83 (d, 1H), 6.72 (dd, 1H), 6.98 (td, 1H), 7.11 (dd, 1H), 7.36-7.43 (m, 2H), 7.44-7.50 (m, 2H), 7.63-7.76 (m, 1H), 9.97 (s, 1H). |
| | aus *tert*.-Butyl-1-(3-amino-4-fluorbenzyl)cyclo-propancarboxylat und (3*R*)-4,4,4-Trifluor-3-methyl-2-(4-vinylphenyl)butansäure (*Diastereomerengemisch*) | |
| | | [α]_{D}²⁰ = +103°, c = 0.260, Chloroform. |
| **157A** | 1-[4-Fluor-3-({(2*S*,3*R*)-4,4,4-trifluor-2-[4-(1-fluor-vinyl)phenyl]-3-methylbutanoyl}amino)benzyl]-cyclopropancarbonsäure-*tert*.-butylester | LC-MS (Methode 5): Rₜ = 1.47 min; m/z = 522 (M-H)⁻. |
| | 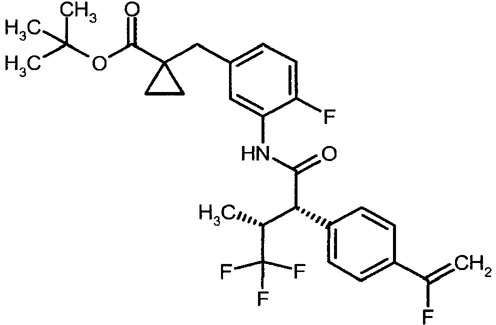 | ¹H-NMR (400 MHz, DMSO-*d₆*): δ [ppm] = 0.76-0.83 (m, 5H), 0.99-1.05 (m, 2H), 1.24 (s, 9H), 2.76 (d, 2H), 3.40 (dd, 1H), 4.12 (d, 1H), 4.95 (dd, 1H), 5.38 (dd, 1H), 6.98-7.03 (m, 1H), 7.11 (dd, 1H), 7.44-7.53 (m, 2H), 7.60-7.66 (m, 2H), 7.70 (dd, 1H), 10.02 (s, 1H). |
| | aus *tert*.-Butyl-1-(3-amino-4-fluorbenzyl)cyclopropancarboxylat und (3*R*)-4,4,4-Trifluor-2-[4-(1-fluorvinyl)phenyl]-3-methylbutansäure (*Diastereomerengemisch*) | |
| **158A** | 1-(3-{[(2*S*,3*R*)-2-(4-Chlorphenyl)-4,4,4-trifluor-3-methylbutanoyl]amino}-2-fluorbenzyl)cyclo-propancarbonsäure-*tert*.-butylester | LC-MS (Methode 5): Rₜ = 1.46 min; m/z = 512 (M-H)⁻. |
| | 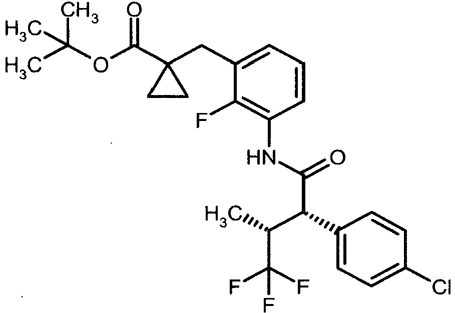 | ¹H-NMR (400 MHz, DMSO-*d₆*): δ [ppm] = 0.71-0.87 (m, 5H), 1.04-1.14 (m, 2H), 1.24 (s, 9H), 2.77-2.95 (m, 2H), 4.12 (d, 1H), 6.97-7.15 (m, 2H), 7.40-7.51 (m, 4H), 7.62 (t, 1H), 10.01 (s, 1H). |
| | aus *tert*.-Butyl-1-(3-amino-2-fluorbenzyl)cyclo-propancarboxylat und (+)-(2*S*,3*R*)-2-(4-Chlorphenyl)-4,4,4-trifluor-3-methylbutansäure | |
| **159A** | 1-(2-Chlor-5-{[(2*S*,3*R*)-2-(4-chlorphenyl)-4,4,4-trifluor-3-methylbutanoyl]amino}benzyl)-cyclopropancarbonsäure-*tert*.-butylester | LC-MS (Methode 5): Rₜ = 1.52 min; m/z = 474 (M-C₄H₈)⁺. |
| | 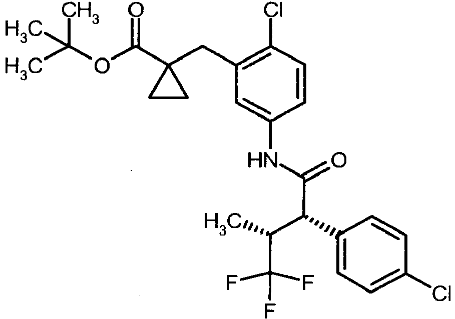 | ¹H-NMR (400 MHz, DMSO-*d₆*): δ [ppm] = 0.79 (d, 3H), 0.81-0.85 (m, 2H), 1.15-1.18 (m, 2H), 1.20 (s, 9H), 2.90 (s, 2H), 3.34-3.44 (m, 1H), 3.83 (d, 1H), 7.30 (d, 1H), 7.38-7.46 (m, 5H), 7.63 (d, 1H), 10.31 (s, 1H). |
| | aus *tert*.-Butyl-1-(5-amino-2-chlorbenzyl)cyclopropancarboxylat und (+)-(2*S*,3*R*)-2-(4-Chlorphenyl)-4,4,4-trifluor-3-methylbutansäure | |
| **160A** | (+)-1-(2-Chlor-3- {[(2*S*,3*R*)-2-(4-chlorphenyl)-4,4,4-trifluor-3-methylbutanoyl]amino}benzyl)-cyclopropancarbonsäure-*tert*.-butylester | LC-MS (Methode 5): Rₜ = 1.50 min; m/z = 474 (M-C₄H₈)⁺. |
| | 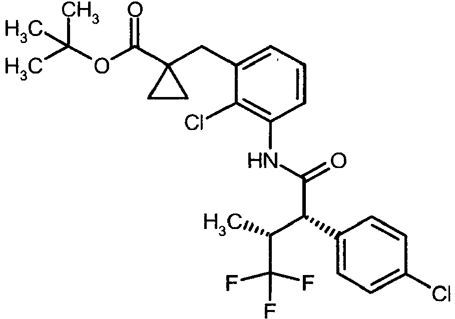 | ¹H-NMR (400 MHz, DMSO-*d₆*): δ [ppm] = 0.74-0.78 (m, 2H), 0.80 (d, 3H), 1.11-1.18 (m, 2H), 1.24 (s, 9H), 2.89-3.04 (m, 2H), 3.34-3.45 (m, 1H), 4.08-4.17 (m, 1H), 7.14-7.27 (m, 2H), 7.30-7.39 (m, 1H), 7.41-7.53 (m, 4H), 9.83 (s, 1H). |
| | aus *tert*.-Butyl-1-(3-amino-2-chlorbenzyl)cyclo-propancarboxylat und (+)-(2*S*,3*R*)-2-(4-Chlorphenyl)-4,4,4-trifluor-3-methylbutansäure | |
| **161A** | (+)-1-(4-Chlor-3-{[(2*S*,3*R*)-2-(4-chlor-3-fluor-phenyl)-4,4,4-trifluor-3-methylbutanoyl]amino}-benzyl)cyclopropancarbonsäure-*tert*.-butylester | LC-MS (Methode 4): Rₜ = 1.72 min; m/z = 446 (M-H)⁻. |
| | 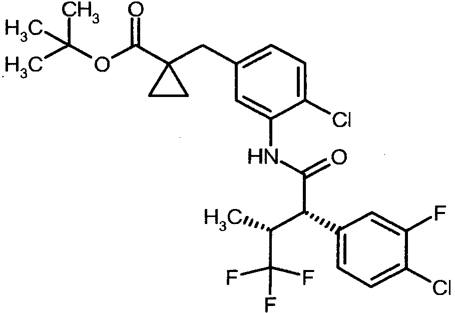 | ¹H-NMR (400 MHz, DMSO-*d₆*): δ [ppm] = 0.77-0.85 (m, 5H), 1.03 (q, 2H), 1.23 (s, 9H), 2.72-2.85 (m, 2H), 3.42 (dd, 1H), 4.12 (d, 1H), 7.05 (dd, 1H), 7.32 (dd, 1H), 7.36 (d, 1H), 7.40-7.45 (m, 1H), 7.50 (dd, 1H), 7.62 (t, 1H), 9.87 (s, 1H). |
| | aus *tert*.-Butyl-1-(3-amino-4-chlorbenzyl)-cyclopropancarboxylat und (3*R*)-2-(4-Chlor-3-fluor-phenyl)-4,4,4-trifluor-3-methylbutansäure | [α]_{D}²⁰ = +73°, c = 0.290, Chloroform. |
| **162A** | (+)-1-(4-Chlor-3-{[(2*S*,3*R*)-2-(4-chlor-2-fluorphenyl)-4,4,4-trifluor-3-methylbutanoyl]amino}benzyl)-cyclopropancarbonsäure-*tert*.-butylester | LC-MS (Methode 5): Rₜ = 1.56 min; m/z = 546/548 (M-H)⁻. |
| | 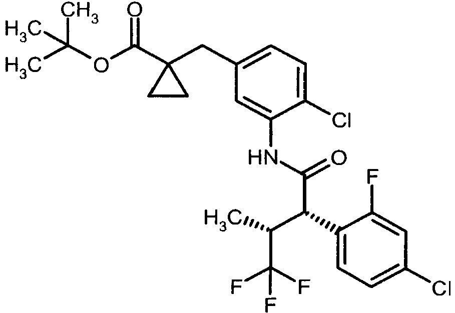 | ¹H-NMR (400 MHz, DMSO-*d₆*): δ [ppm] = 0.81-0.83 (m, 2H), 0.85 (d, 3H), 1.01-1.07 (m, 2H), 1.27 (s, 9H), 2.76-2.85 (m, 2H), 3.34-3.43 (m, 1H), 4.31-4.39 (m, 1H), 7.04-7.13 (m, 1H), 7.32-7.39 (m, 3H), 7.51 (dd, 1H), 7.62 (t, 1H), 10.02 (s, 1H). |
| | aus *tert*.-Butyl-1-(3-amino-4-chlorbenzyl)-cyclopropancarboxylat und (3*R*)-2-(4-Chlor-2-fluor-phenyl)-4,4,4-trifluor-3-methylbutansäure | [α]_{D}²⁰ = +28.0°, c = 0.250, Chloroform. |
| **163A** | *tert*.-Butyl-1-(3-{[(2*S*,3*R*)-2-(4-chlorphenyl)-4,4,4-trifluor-3-methylbutanoyl]amino}-4-metlaylbenzyl)-cyclopropancarboxylat | LC-MS (Methode 4): Rₜ = 1.63 min; m/z = 508/510 (M-H)⁻. |
| | 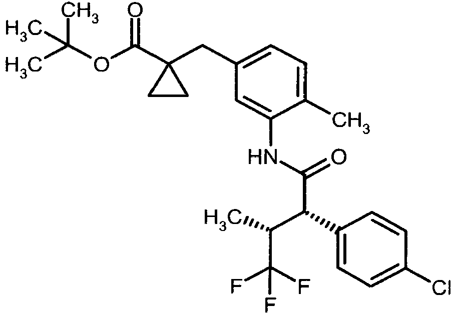 | ¹H-NMR (400 MHz, DMSO-*d₆*): δ [ppm] = 0.73-0.78 (m, 2H), 0.80 (d, 3H), 0.98-1.03 (m, 2H), 1.26 (s, 9H), 1.96 (s, 3H), 2.69-2.81 (m, 2H), 3.32-3.43 (m, 1H, teilweise verdeckt), 3.94 (d, 1H), 6.93 (dd, 1H), 7.02-7.11 (m, 2H), 7.38-7.51 (m, 4H), 9.59 (s, 1H). |
| | aus *tert*.-Butyl-1-(3-amino-4-methylbenzyl)cyclo-propancarboxylat und (2*S*,3*R*)-2-(4-Chlorphenyl)-4,4,4-trifluor-3-methylbutansäure | |
| **164A** | *tert.*-Butyl-1-(3-{[(4-chlorphenyl)(3,3-difluorcyclo-pentyl)acetyl]amino}-4-fluorbenzyl)cyclopropancarboxylat *(Diastereomerengemisch)* | LC-MS (Methode 5): Rₜ = 1.44 min; m/z = 520/522 (M-H)⁻. |
| | 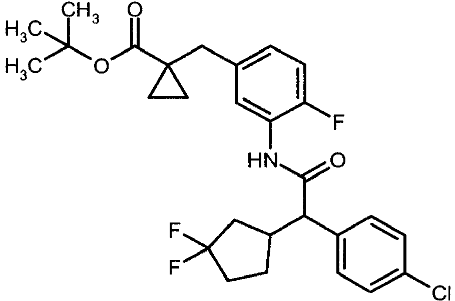 | |
| | aus *tert*.-Butyl-1-(3-amino-4-fluorbenzyl)cyclo-propancarboxylat und (4-Chlorphenyl)-(3,3-difluorcyclopentyl)essigsäure | |
| **165A** | *tert*.-Butyl-1-[3-({4,4,4-trifluor-3-methyl-2-[4-(tri-fluormethyl)phenyl]butanoyl}amino)benzyl]-cyclopropancarboxylat | LC-MS (Methode 5): Rₜ = 1.46 min; m/z = 528 (M-H)⁻. |
| | 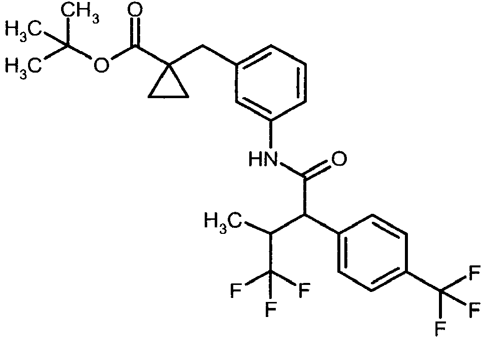 | |
| | aus *tert*.-Butyl-1-(3-aminobenzyl)cyclopropan-carboxylat und 4,4,4-Trifluor-3-methyl-2-[4-(trifluormethyl)phenyl]butansäure | |
| **166A** | *tert.*-Butyl-1-(4-fluor-3-{[(2*S*,3*R*)-4,4,4-trifluor-2-(4-isopropylphenyl)-3-methylbutanoyl]amino}-benzyl)cyclopropancarboxylat | LC-MS (Methode 5): Rₜ = 1.55 min; m/z = 520 (M-H)⁻. |
| | 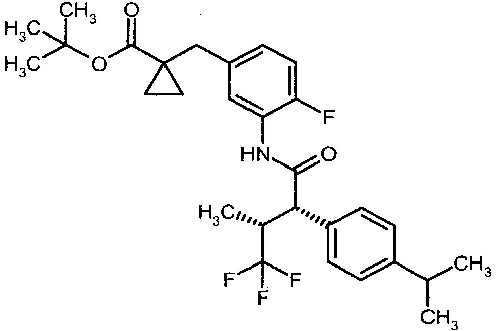 | ¹H-NMR (400 MHz, DMSO-*d₆*): δ [ppm] = 0.72-0.86 (m, 5H), 0.99-1.07 (m, 2H), 1.18 (d, 6H), 1.25 (s, 9H), 2.69-2.82 (m, 2H), 2.82-2.93 (m, 1H), 3.24-3.42 (m, 1H, teilweise verdeckt), 4.03 (d, 1H), 6.93-7.01 (m, 1H), 7.06-7.14 (m, 1H), 7.22 (d, 2H), 7.34 (d, 2H), 7.75 (dd, 1H), 9.93 (s, 1H). |
| | aus *tert*.-Butyl-1-(3-amino-4-fluorbenzyl)-cyclopropancarboxylat und (2*S*,3*R*)-4,4,4-Trifluor-2-(4-isopropylphenyl)-3-methylbutansäure | |
| **167A** | *tert.* -Butyl-1-[3-({(2*S*,3*R*)-2-[4-(1,1-difluorethyl)-phenyl]-4,4,4-trifluor-3-methylbutanoyl}amino)-4-fluorbenzyl]cyclopropancarboxylat | LC-MS (Methode 5): Rₜ = 1.41 min; m/z = 542 (M-H)⁻. |
| | 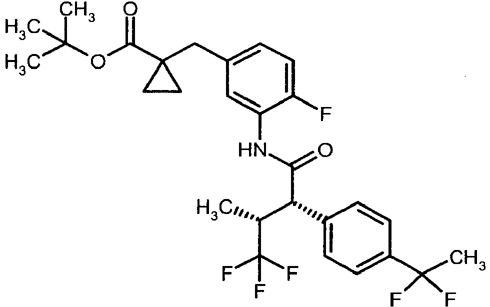 | ¹H-NMR (400 MHz, DMSO-*d₆*): δ [ppm] = 0.74-0.84 (m, 5H), 1.00-1.05 (m, 2H), 1.24 (s, 9H), 1.95 (t, 3H), 2.70-2.82 (m, 2H), 3.34-3.48 (m, 1H), 4.15 (d, 1H), 6.95-7.02 (m, 1H), 7.07-7.15 (m, 1H), 7.52-7.60 (m, 4H), 7.71 (dd, 1H), 10.02 (s, 1H). |
| | aus *tert*.-Butyl-1-(3-amino-4-fluorbenzyl)cyclo-propancarboxylat und (2*S*,3*R*)-2-[4-(1,1-Difluorethyl)-phenyl]-4,4,4-trifluor-3-methylbutansäure | |
| **168A** | *tert*.-Butyl-1-(3-{[(2S,3R)-2-(4-*tert*.-butylphenyl)-4,4,4-trifluor-3-methylbutanoyl]amino}-4-fluorbenzyl)cyclopropancarboxylat | LC-MS (Methode 5): Rₜ = 1.52 min; m/z = 534 (M-H)⁻. |
| | 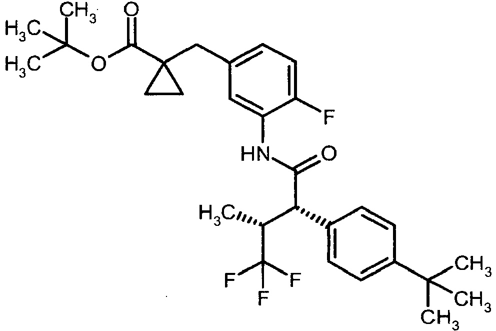 | ¹H-NMR (400 MHz, DMSO-*d₆*): δ [ppm] = 0.73-0.83 (m, 5H), 0.99-1.07 (m, 2H), 1.24 (s, 9H), 1.26 (s, 9H), 2.70-2.83 (m, 2H), 3.27-3.42 (m, 1H, teilweise verdeckt), 4.04 (d, 1H), 6.93-7.01 (m, 1H), 7.06-7.15 (m, 1H), 7.31-7.41 (m, 4H), 7.75 (dd, 1H), 9.93 (s, 1H). |
| | aus *tert*.-Butyl-1-(3-amino-4-fluorbenzyl)cyclo-propancarboxylat und (2*S*,3*R*)-2-(4-*tert*.-Butyl-phenyl)-4,4,4-trifluor-3-methylbutansäure | |
| **169A** | *tert*.-Butyl-1-(3-{[(2*S*,3*R*)-2-(4-cyclopropylphenyl)-4,4,4-trifluor-3-methylbutanoyl]amino}-4-fluorbenzyl)cyclopropancarboxylat | LC-MS (Methode 5): Rₜ = 1.50 min; m/z = 518 (M-H)⁻. |
| | 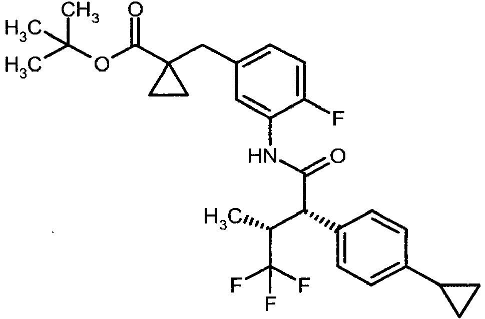 | ¹H-NMR (400 MHz, DMSO-*d₆*): δ [ppm] = 0.60-0.68 (m, 2H), 0.73-0.83 (m, 5H), 0.89-0.96 (m, 2H), 0.99-1.07 (m, 2H), 1.25 (s, 9H), 1.82-1.93 (m, 1H), 2.69-2.83 (m, 2H), 3.23-3.39 (m, 1H, teilweise verdeckt), 4.01 (d, 1H), 6.92-7.00 (m, 1H), 7.01-7.14 (m, 3H), 7.29 (d, 2H), 7.71 (dd, 1H), 9.90 (s, 1H). |
| | aus *tert*.-Butyl-1-(3-amino-4-fluorbenzyl)cyclo-propancarboxylat und (2*S*,3*R*)-2-(4-Cyclopropyl-phenyl)-4,4,4-trifluor-3-methylbutansäure | |
| **170A** | *tert*.-Butyl-1-(3-{[(2*S*,3*R*)-2-(4-chlor-3-methoxy-phenyl)-4,4,4-trifluor-3-methylbutanoyl]amino}-4-fluorbenzyl)cyclopropancarboxylat | LC-MS (Methode 5): Rₜ = 1.44 min; m/z = 542/544 (M-H)⁻. |
| | 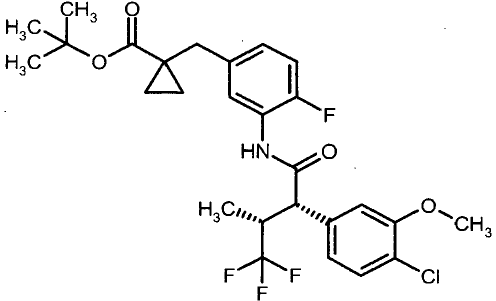 | |
| | aus *tert*.-Butyl-1-(3-amino-4-fluorbenzyl)cyclo-propancarboxylat und (2*S*,3*R*)-2-(4-Chlor-3-methoxyphenyl)-4,4,4-trifluor-3-methylbutansäure | |
| **171A** | *tert*.-Butyl-1-(3-{[2-(4-chlorphenyl)-4,4,4-trifluor-3-methylbutanoyl]amino}benzyl)cyclopropancarboxylat | LC-MS (Methode 5): Rₜ = 1.47 min; m/z = 494 (M-H)⁻. |
| | 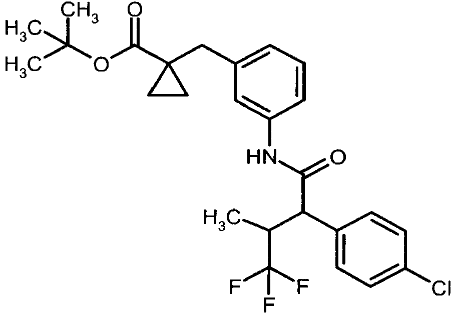 | |
| | aus *tert*.-Butyl-1-(3-aminobenzyl)cyclopropancarboxylat und 2-(4-Chlorphenyl)-4,4,4-trifluor-3-methylbutansäure | |
| **172A** | *tert*.-Butyl-1-(3-{[4,4,4-trifluor-3-methyl-2-(4-methyl-phenyl)butanoyl]amino}benzyl)cyclopropan-carboxylat | LC-MS (Methode 5): Rₜ = 1.45 min; m/z = 474 (M-H)⁻. |
| | 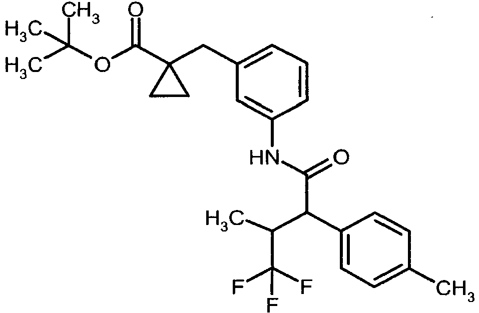 | |
| | aus *tert*.-Butyl-1-(3-aminobenzyl)cyclopropan-carboxylat und 4,4,4-Trifluor-3-methyl-2-(4-methylphenyl)butansäure | |
| **173A** | *tert*.-Butyl-1-[3-({4,4,4-trifluor-3-methyl-2-[4-(trifluormethoxy)phenyl]butanoyl}amino)-benzyl]cyclopropancarboxylat | LC-MS (Methode 5): Rₜ = 1.48 min; m/z = 544 (M-H)⁻. |
| | 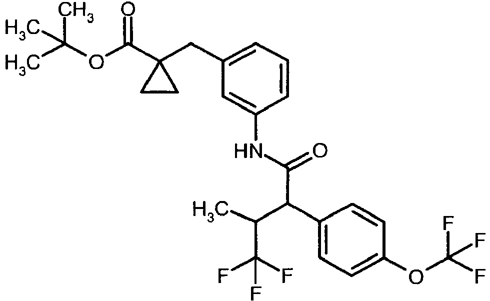 | |
| | aus *tert*.-Butyl-1-(3-aminobenzyl)cyclopropan-carboxylat und 4,4,4-Trifluor-3-methyl-2-[4-(trifluormethoxy)phenyl]butansäure | |
| **174A** | *tert*.-Butyl-1-(3-{[2-(4-chlor-3-methylphenyl)-4,4,4-trifluor-3-methylbutanoyl]amino}benzyl)cyclo-propancarboxylat | LC-MS (Methode 5): Rₜ = 1.48 min; m/z = 508/510 (M-H)⁻. |
| | 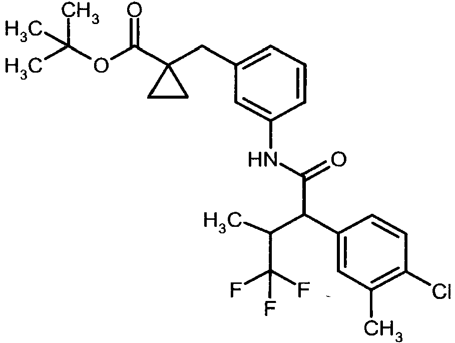 | |
| | aus *tert*.-Butyl-1-(3-aminobenzyl)cyclopropancarboxylat und 2-(4-Chlor-3-methylphenyl)-4,4,4-trifluor-3-methylbutansäure | |

### Beispiel 175A

### 1-(3-{[(2S,3R)-2-(4-Chlorphenyl)-4,4,4-trifluor-3-methylbutanoyl]amino}-4-fluorbenzyl)-cyclopropancarbonsäure-tert.-butylester

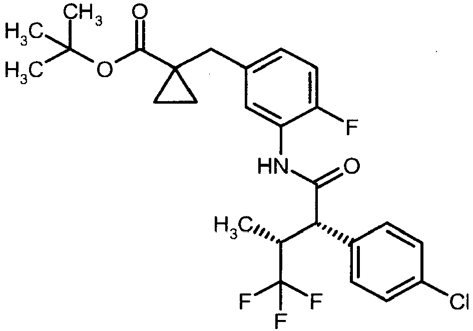

Entsprechend der Allgemeinen Vorschrift 6 wurden aus 1.2 g (4.5 mmol) (2*RS*,3*R*)-2-(4-Chlor-phenyl)-4,4,4-trifluor-3-methylbutansäure und 1.3 g (4.95 mmol) 1-(3-Amino-4-fluorbenzyl)cyclo-propancarbonsäure-*tert*.-butylester nach flash-chromatographischer Reinigung des Rohprodukts (Kieselgel; Laufmittel Cyclohexan/Ethylacetat 20:1) 1.6 g (69% d. Th.) der Titelverbindung erhalten.

LC-MS (Methode 5): Rₜ = 1.48 min; m/z = 512 (M-H)⁻.

¹H-NMR (400 MHz, DMSO-d₆): δ [ppm] = 0.71-0.92 (m, 5H), 0.99-1.07 (m, 2H), 1.25 (s, 9H), 2.68-2.87 (m, 2H), 4.09 (d, 1H), 6.91-7.05 (m, 1H), 7.11 (dd, 1H), 7.35-7.54 (m, 4H), 7.70 (dd, 1H), 10.0 (s, 1H).

### Beispiel 176A

### 1-(3-{[(4-Chlorphenyl)(2,2-difluorcyclopentyl)acetyl]amino}-4-fluorbenzyl)cyclopropan-carbonsäure-tert.-butylester (racemisches Diastereomerengemisch)

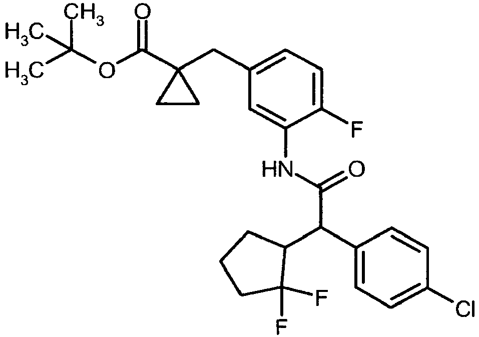

Zu einer Mischung von 4.0 ml DMF und 1.0 ml Pyridin wurden 490 mg (1.78 mmol) (4-Chlorphenyl)(2,2-difluorcyclopentyl)essigsäure (als Diastereomerengemisch) gegeben. Die resultierende Lösung wurde bei RT mit 568.0 mg (2.14 mmol) *tert*.-Butyl-1-(3-amino-4-fluorbenzyl)cyclopropancarboxylat und 881.7 mg (2.34 mmol) HATU versetzt und über Nacht bei RT gerührt. Die Reaktionsmischung wurde dann auf gesättigte wässrige Ammoniumchlorid-Lösung gegeben und dreimal mit Ethylacetat extrahiert. Die vereinigten organischen Phasen wurden über Magnesiumsulfat getrocknet und im Vakuum eingeengt. Das Rohprodukt wurde durch Chromatographie an Silicagel gereinigt (Laufmittel Cyclohexan/Ethylacetat 20:1). Eine weitere Reinigung des so gewonnenen Produkts erfolgte durch präparative RP-HPLC (Acetonitril/Wasser-Eluent). Es wurden 774.0 mg (83.1% d. Th.) der Zielverbindung erhalten.

LC-MS (Methode 5): Rₜ = 1.42 min; m/z = 466 (M-C₄H₈)⁺.

¹H-NMR (400 MHz, DMSO-d₆): *beide Diastereomere:* δ [ppm] = 0.76-0.83 (m, 2H), 0.99-1.06 (m, 2H), 1.08-1.21 (m, 1H), 1.21-1.29 (m, 9H), 1.46-1.67 (m, 2H), 1.68-1.76 (m, 1H), 1.95-2.24 (m, 2H), 2.70-2.83 (m, 2H), 3.18 (m, 1H), 4.01/4.07 (d, 1H), 6.92-7.04 (m, 1H), 7.11 (ddd, 1H), 7.32-7.42 (m, 2H), 7.43-7.51 (m, 2H), 7.63-7.74 (m, 1H), 9.85 (s, 1H).

### Beispiel 177A

### (+/-)-1-[3-(2-{[4-(2-Cyanovinyl)phenyl]-2-cyclopentylacetyl}amino)-4-fluorbenzyl]cyclopropancarbonsäure-tert.-butylester

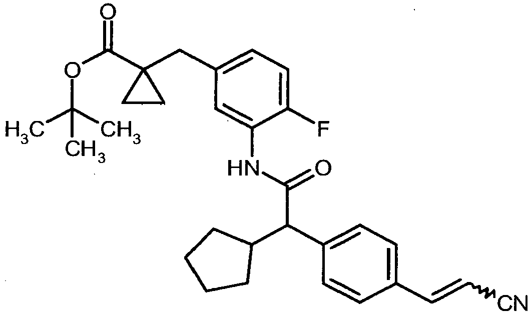

Zu einer Lösung von 627 mg (1.18 mmol) (+/-)-1-(3-{[2-(4-Bromphenyl)-2-cyclopentylacetyl]-amino}-4-fluorbenzyl)cyclopropancarbonsäure-*tert*.-butylester in 12.5 ml DMF wurden 86 µl (1.3 mmol) Acrylnitril, 8 mg (35 µmol) Palladiumacetat, 32 mg (106 µmol) Tri-(o-tolyl)phosphin und 254 µl (1.8 mmol) Triethylamin gegeben. Das Reaktionsgemisch wurde in der Mikrowelle für 1 h auf 150°C erhitzt. Anschließend wurden nochmals die gleichen Mengen an Acrylnitril, Palladiumacetat, Tri-(o-tolyl)phosphin und Triethylamin zugegeben und das Gemisch erneut für 1 h bei 150°C in der Mikrowelle gerührt. Das Reaktionsgemisch wurde anschließend ohne weitere Aufarbeitung direkt über präparative HPLC in seine Komponenten aufgetrennt. Es wurden 497 mg (79% d. Th.) der Titelverbindung erhalten.

LC-MS (Methode 2): Rₜ = 2.93 min; m/z = 503 (M+H)⁺.

### Beispiel 178A

### 1-[3-(2S)-(2-{[4-(E-2-Cyanovinyl)phenyl]-2-cyclopentylacetyl}amino)-4-fluorbenzyl]cyclopropancarbonsäure-tert.-butylester

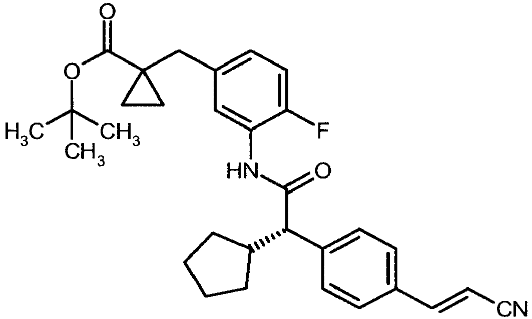

Aus 200 mg (+/-)-1-[3-(2-{[4-(2-Cyanovinyl)phenyl]-2-cyclopentylacetyl}amino)-4-fluorbenzyl]-cyclopropancarbonsäure-*tert*.-butylester wurden durch präparative HPLC an chiraler Phase [Säule: Daicel Chiralpak AS-H, 5 µm, 250 mm x 20 mm; Injektionsvolumen: 0.25 ml; Temperatur: 38°C; Eluent: 80% Isohexan / 20% (Ethanol + 0.2% TFA + 1% Wasser); Fluss: 15 ml/min; Detektion: 220 nm] 89 mg des reinen 2S-Enantiomers erhalten.

Rₜ = 4.64 min [Säule: Daicel Chiralpak AS-H, 5 µm, 250 mm x 4.6 mm; Elutionsmittel: Isohexan / (Isopropanol + 0.2% TFA + 1% Wasser) 70:30 (v/v); Fluss: 1 ml/min; UV-Detektion: 220 nm; Temperatur: 40°C].

¹H-NMR (400 MHz, DMSO-d₆): δ [ppm] = 0.76-0.86 (m, 2H), 0.90-1.00 (m, 1H), 1.00-1.07 (m, 2H), 1.21-1.72 (m, 15H), 1.71-1.88 (m, 1H), 2.76 (s, 2H), 3.59-3.69 (m, 1H), 6.44 (s, 1H), 6.92-7.05 (m, 1H), 7.11 (dd, 1H), 7.48 (d, 2H), 7.58-7.63 (m, 2H), 7.64-7.71 (m, 1H), 9.81 (s, 1H).

### Beispiel 179A

### (2S,3R)-2-(4-Chlorphenyl)-4,4,4-trifluor-3-methylbutanoylchlorid

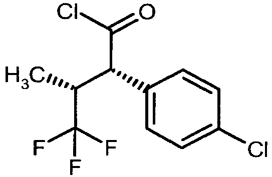

19.5 g (73.13 mmol) (2*S*,3*R*)-2-(4-Chlorphenyl)-4,4,4-trifluor-3-methylbutansäure wurden in 860 ml Dichlormethan gelöst und mit 0.5 ml DMF versetzt. Anschließend wurden bei -5°C bis -10°C (Eis/Aceton-Kühlbad) 73 ml (146.26 mmol) eine 2 M Lösung von Oxalylchlorid in Dichlormethan langsam zugetropft und die Mischung 1 h bei dieser Temperatur nachgerührt. Nach vollständiger Umsetzung wurde die Reaktionslösung im Vakuum eingedampft und der erhaltene Rückstand in 200 ml Dichlormethan aufgenommen und anschließend wieder bis zur Trockene eingeengt. Es wurden 20.1 g (70.5 mmol, 96% d. Th.) der Titelverbindung als farbloses Öl erhalten. Das so erhaltene Produkt wurde ohne weitere Aufreinigung und ohne weitere spektroskopische Charakterisierung in Folgereaktionen eingesetzt.

### Beispiel 180A

### (4-Chlorphenyl)(3,3-difluorcyclopentyl)acetylchlorid (Diastereomerengemisch)

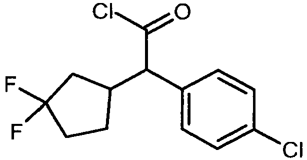

470 mg (1.71 mmol) (4-Chlorphenyl)(3,3-difluorcyclopentyl)essigsäure wurden in 8 ml Dichlormethan gelöst und mit einem Tropfen DMF versetzt. Anschließend wurden bei 0°C 1.7 ml (3.42 mmol) Oxalsäuredichlorid langsam zugetropft und die Mischung 1 h bei dieser Temperatur nachgerührt. Nach vollständiger Umsetzung wurde die Reaktionslösung im Vakuum eingedampft und der erhaltene Rückstand in 50 ml Dichlormethan aufgenommen und anschließend wieder bis zur Trockene eingeengt. Es wurden 500 mg (99% d. Th.) der Titelverbindung als farbloses Öl erhalten. Das so erhaltene Produkt wurde ohne weitere Aufreinigung und ohne weitere spektroskopische Charakterisierung in Folgereaktionen eingesetzt.

### Beispiel 181A

### (2S,3R)-4,4,4-Trifluor-3-methyl-2-[4-(trifluormethyl)phenyl]butanoylchlorid

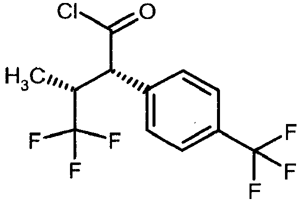

Die Titelverbindung wurde auf analoge Weise ausgehend von (2*S*,3*R*)-4,4,4-Trifluor-3-methyl-2-[4-(trifluormethyl)phenyl]butansäure hergestellt.

### Allgemeine Vorschrift 7: Amidkupplung von Anilinen mit in situ generierten Säurechloriden

Eine Lösung (0.1 bis 1.5 mol/l) des betreffenden Anilins (0.8 bis 2.0 eq.) und *N,N*-Diisopropylethylamin (1.0 bis 3.0 eq.) in abs. THF oder abs. Dichlormethan wurde unter Argon auf -10°C bis 0°C gekühlt und tropfenweise mit einer konzentrierten Lösung des frisch hergestellten Säurechlorids (0.8 bis 2.0 eq.) in abs. THF oder abs. Dichlormethan versetzt. Nach Ende der Zugabe wurde langsam auf RT erwärmt und entweder direkt aufgearbeitet oder noch 2 h bis 12 h bei RT nachgerührt, bevor aufgearbeitet wurde. Nach Verdünnen der Reaktionsmischung mit Ethylacetat oder Dichlormethan wurde die organische Phase nacheinander mit 1 N Salzsäure und gesättigter Natriumchlorid-Lösung gewaschen, über Magnesium- oder Natriumsulfat getrocknet und im Vakuum eingeengt. Das Rohprodukt wurde durch präparative RP-HPLC (Eluent: Acetonitril/Wasser-Gradient), durch Chromatographie an Silicagel (Eluent: Cyclohexan/Ethylacetat- oder Dichlormethan/Methanol-Gemische) oder durch Verrühren in organischen Lösungsmitteln wie z.B. Diisopropylether gereinigt. Auch eine Kombination dieser Reinigungsmethoden kann verwendet werden, um das Zielprodukt in reiner Form zu isolieren.

Die folgenden Verbindungen wurden gemäß der Allgemeinen Vorschrift 7 hergestellt:

| **Beispiel** | **Name / Struktur / Edukte** | **Analytische Daten** |
|---|---|---|
| **182A** | 1-(4,6-Dichlor-3-{[(2*S*,3*R*)-2-(4-chlorphenyl)-4,4,4-trifluor-3-methylbutanoyl]amino}-2-fluorbenzyl)-cyclopropancarbonsäure-*tert*.-butylester | LC-MS (Methode 5): Rₜ = 1.57 min; m/z = 580/582 (M-H)⁻. |
| | 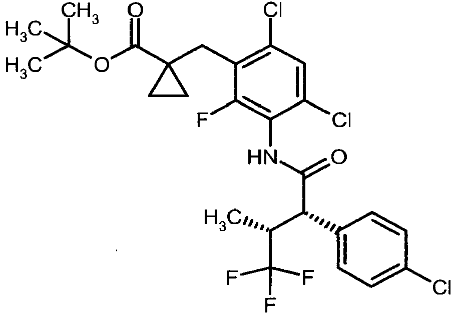 | |
| | aus (2*S*,3*R*)-2-(4-Chlorphenyl)-4,4,4-trifluor-3-methylbutanoylchlorid und *tert*.-Butyl-1-(3-amino-4,6-dichlor-2-fluorbenzyl)cyclopropancarboxylat | |
| **183A** | (+)-1-(2-Chlor-5-{[(2*S*,3*R*)-2-(4-chlorphenyl)-4,4,4-trifluor-3-methylbutanoyl]amino}-4-fluorbenzyl)-cyclopropancarbonsäure-*tert*.-butylester | LC-MS (Methode 4): |
| | | Rₜ = 1.76 min; m/z = 546/548 (M-H)⁻. |
| | 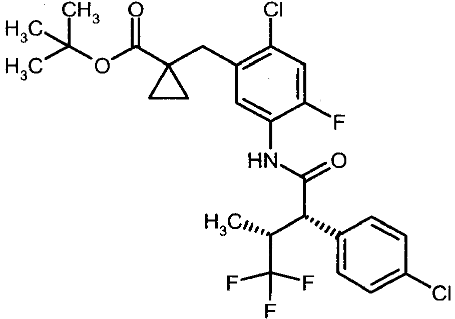 | ¹H-NMR (400 MHz, DMSO-d₆): δ [ppm] = 0.75-0.82 (m, 5H), 1.14 (q, 2H), 1.21 (s, 9H), 2.81-2.96 (m, 2H), 3.34-3.44 (m, 1H), 4.05-4.1.2 (m, 1H), 7.35-7.47 (m, 5H), 7.89-7.96 (m, 1 H), 10.13 (s, 1 H). [α]_{D}²⁰ = +122.1°, c = 0.370, Chloroform. |
| | aus (2*S*,3*R*)-2-(4-Clolorphenyl)-4,4,4-trifluor-3-methylbutanoylchlorid und *tert*.-Butyl-1-(5-amino-2-chlor-4-fluorbenzyl)cyclopropancarboxylat | |
| **184A** | *tert*.-Butyl-1-(4-chlor-3-{[(4-chlorphenyl)-(3,3-difluorcyclopeutyl)acetyl]amino}benzyl)-cyclopropancarboxylat (*Diastereomerengemisch*) | LC-MS (Methode 4): |
| | | Rₜ = 1.67 min; m/z = 536/538 (M-H)⁻. |
| | 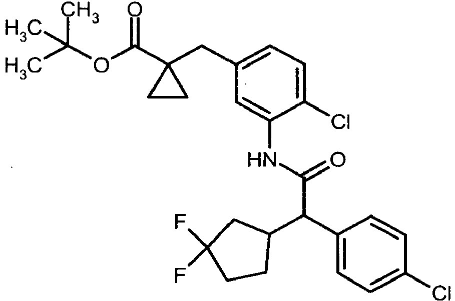 | |
| | aus (4-Chlorphenyl)(3,3-difluorcyclopentyl)-acetylchlorid und *tert*.-Butyl-1-(3-amino-4-chlorbenzyl)cyclopropancarboxylat | |
| **185A** | *tert*.-Butyl-1-[4-chlor-3-({(2*S*,3*R*)-4,4,4-trifluor-3-methyl-2-[4-(trifluormethyl)phenyl]butanoyl}-amino)benzyl]cyclopropancarboxylat | LC-MS (Methode 4): Rₜ = 1.68 min; m/z = 562 (M-H)⁻. |
| | 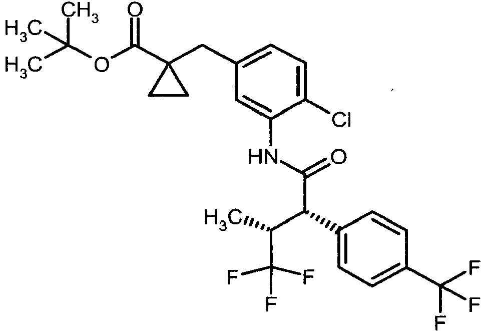 | ¹H-NMR (400 MHz, DMSO-d₆): δ [ppm] = 0.76-0.84 (m, 5H), 0.99-1.07 (m, 2H), 1.22 (s, 9H), 2.70-2.85 (q, 2H), 3.36-3.52 (m, 1H), 4.22 (d, 1H), 7.05 (dd, 1H), 7.35 (d, 1H), 7.53 (d, 1H), 7.69 (d, 2H), 7.76 (d, 2H), 9.89 (s, 1H). |
| | aus (2*S*,3*R*)-4,4,4-Trifluor-3-methyl-2-[4-(trifluormethyl)phenyl]butanoylchlorid und *tert*.-Butyl-1-(3-amino-4-chlorbenzyl)cyclopropancarboxylat | |

### Beispiel 186A und Beispiel 187A

### 1-(4-Chlor-3-{[(2S,3R)-2-(4-chlorphenyl)-4,4,4-trifluor-3-methylbutanoyl]amino}-2-fluorbenzyl)-cyclopropancarbonsäure-tert.-butylester und 1-(6-Chlor-3-{[(2S,3R)-2-(4-chlorphenyl)-4,4,4-trifluor-3-methylbutanoyl]amino}-2-fluorbenzyl)-cyclopropancarbonsäure-tert.-butylester

33.0 mg eines Gemisches aus *tert*.-Butyl-1-(3-amino-4-chlor-2-fluorbenzyl)cyclopropancarboxylat und *tert*.-Butyl-1-(3-amino-6-chlor-2-fluorbenzyl)cyclopropancarboxylat (Beispiel 24A/25A, Verhältnis ca. 1.5:1) wurden in 0.16 ml abs. THF gelöst, auf -10°C gekühlt und tropfenweise mit einer Lösung von 38 mg (0.132 mmol) (2*S*,3*R*)-2-(4-Chlorphenyl)-4,4,4-trifluor-3-methylbutanoyl-chlorid in ca. 0.1 ml abs. THF versetzt. Nach Ende der Zugabe wurde die Mischung langsam auf RT erwärmt und nach 2 h mit Wasser versetzt. Es wurde dreimal mit Ethylacetat extrahiert, und die vereinigten organischen Phasen wurden mit 1 N Salzsäure und gesättigter Natriumchlorid-Lösung gewaschen, über Magnesiumsulfat getrocknet und im Vakuum eingeengt. Das erhaltene Produktgemisch wurde durch präparative RP-HPLC (Acetonitril/Wasser-Eluent) aufgetrennt. Es wurden so 23.2 mg (38% d. Th.) 1-(4-Chlor-3-{[(2*S*,3*R*)-2-(4-chlorphenyl)-4,4,4-trifluor-3-methyl-butanoyl]amino}-2-fluorbenzyl)cyclopropancarbonsäure-*tert*.-butylester (*Beispiel 186A*) sowie 18.7 mg (31% d. Th.) 1-(6-Chlor-3-{[(2*S*,3*R*)-2-(4-chlorphenyl)-4,4,4-trifluor-3-methylbutanoyl]-amino}-2-fluorbenzyl)cyclopropancarbonsäure-*tert*.-butylester (*Beispiel 187A*) erhalten.

### Beispiel 186A

### 1-(4-Chlor-3-{[(2S,3R)-2-(4-chlorphenyl)-4,4,4-trifluor-3-methylbutanoyl]amino}-2-fluorbenzyl)-cyclopropancarbonsäure-tert.-butylester

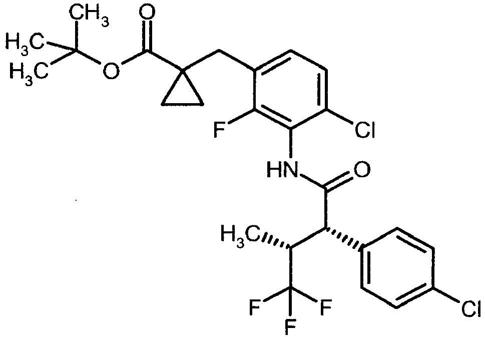

LC-MS (Methode 4): Rₜ = 1.65 min; m/z = 546/548 (M-H)⁻.

¹H-NMR (400 MHz, DMSO-*d₆*): δ [ppm] = 0.79 (d, 5H), 1.06-1.11 (m, 2H), 1.26 (s, 9H), 2.83 (s, 2H), 3.33-3.40 (m, 1H), 3.95 (d, 1H), 7.26 (s, 2H), 7.40-7.47 (m, 4H), 10.02 (s, 1H).

### Beispiel 187A

### 1-(6-Chlor-3-{[(2S,3R)-2-(4-chlorphenyl)-4,4,4-trifluor-3-methylbutanoyl]amino}-2-fluorbenzyl)-cyclopropancarbonsäure-tert.-butylester

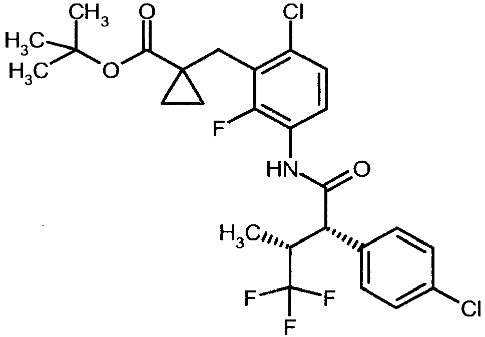

LC-MS (Methode 4): Rₜ = 1.82 min; m/z = 546/548 (M-H)⁻.

¹H-NMR (400 MHz, DMSO-*d*₆): δ [ppm] = 0.45-0.53 (m, 2H), 0.78 (d, 3H), 0.97-1.06 (m, 2H), 1.32 (s, 9H), 3.16-3.24 (m, 2H), 3.34-3.44 (m, 1H), 4.11 (d, 1H), 7.24 (d, 1H), 7.45 (s, 4H), 7.70 (t, 1H), 10.17 (s, 1H).

### Beispiel 188A

### 1-(4-Chlor-3-{[(2S,3R)-2-(4-chlorphenyl)-4,4,4-trifluor-3-methylbutanoyl]amino}benzyl)-2,2-difluorcyclopropancarbonsäure-tert.-butylester (Diastereomerengemisch)

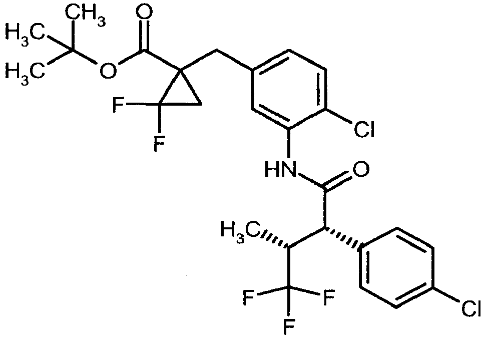

1.15 g (3.62 mmol) (+/-)-*tert*.-Butyl-1-(3-amino-4-chlorbenzyl)-2,2-difluorcyclopropancarboxylat wurden in 5 ml DMF gelöst und bei RT mit 1.49 g (5.43 mmol) (+)-(2*S*,3*R*)-2-(4-Chlorphenyl)-4,4,4-trifluor-3-methylbutansäure, 2.5 ml Pyridin und 1.79 g (4.71 mmol) HATU versetzt. Die Reaktionsmischung wurde anschließend über Nacht bei 40°C gerührt. Nach dem Abkühlen wurde das Gemisch mit Ethylacetat verdünnt, und die Lösung wurde mit 1 N Salzsäure und gesättigter Natriumchlorid-Lösung gewaschen, über Magnesiumsulfat getrocknet und im Vakuum eingeengt. Der Rückstand wurde durch Chromatographie an Silicagel gereinigt (Laufmittel Cyclohexan/ Ethylacetat 30:1). Es wurden 1.82 g der Titelverbindung erhalten (88.8% d. Th.).

LC-MS (Methode 4): Rₜ = 1.86 min; m/z = 564 (M-H)⁻.

¹H-NMR (400 MHz, DMSO-*d₆*): *beide Diastereomere:* δ [ppm] = 0.80 (d, 3H), 1.20/1.27 (je s, zus. 9H), 1.91-2.02 (m, 1H), 2.03-2.16 (m, 1H), 2.64-2.71 (m, 1H), 3.26-3.32 (m, 1H, verdeckt), 3.35-3.41 (m, 1H), 4.10/4.12 (je d, zus. 1H), 7.09 (dt, 1H), 7.34-7.52 (m, 6H), 9.84/9.86 (je d, zus. 1H).

### Beispiel 189A

### tert.-Butyl-1-(4-chlor-3-{[(2S,3R)-2-(4-chlorphenyl)-4,4,4-trifluor-3-methylbutanoyl]amino}-benzyl)cyclopropancarboxylat

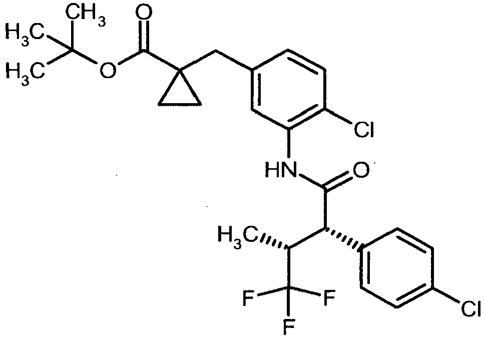

368 mg (1.38 mmol) (2*S*,3*R*)-2-(4-Chlorphenyl)-4,4,4-trifluor-3-methylbutansäure wurden in 15 ml Dichlormethan gelöst, mit 246 mg (1.84 mmol) 1-Chlor-*N,N*,2-trimethylprop-1-en-1-amin versetzt und anschließend 30 min bei Raumtemperatur gerührt. Danach wurde die Reaktionsmischung mit 279 µl (3.45 mmol) Pyridin sowie 324 mg (1.15 mmol) *tert*.-Butyl-1-(3-amino-4-chlorbenzyl)-cyclopropancarboxylat versetzt und 1 h bei Raumtemperatur nachgerührt. Das Reaktionsgemisch wurde dann im Vakuum eingeengt und das erhaltene Rohprodukt durch präparative RP-HPLC gereinigt (Acetonitril/Wasser-Eluent). Es wurden 540 mg der Zielverbindung erhalten (88% d. Th.).

LC-MS (Methode 5): Rₜ = 1.52 min; m/z = 528/530 (M-H)⁻.

¹H-NMR (400 MHz, DMSO-*d₆*): δ [ppm] = 0.76-0.83 (m, 5H), 1.01-1.06 (m, 2H), 1.24 (s, 9H), 2.72-2.85 (m, 2H), 3.32-3.43 (m, 1H, teilweise verdeckt durch H₂O-Signal), 4.10 (d, 1H), 7.05 (d, 1H), 7.35 (d, 1H), 7.41-7.50 (m, 5H), 9.83 (s, 1H).

Auf analoge Weise wurden die in der folgenden Tabelle aufgeführten Verbindungen hergestellt:

| **Beispiel** | **Name / Struktur / Edukte** | **Analytische Daten** |
|---|---|---|
| **190A** | *tert*.-Butyl-1-(4-chlor-3- {[(2*S*,3*R*)-2-(4-chlor-3-methoxyphenyl)-4,4,4-trifluor-3-methylbutanoyl]-amino}benzyl)cyclopropancarboxylat | LC-MS (Methode 4): Rₜ = 1.68 min; m/z = 558/560 (M-H)⁻. |
| | 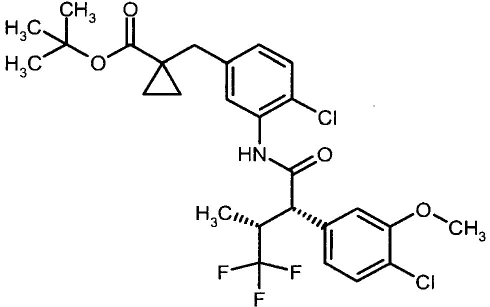 | ¹H-NMR (400 MHz, DMSO-d₆): δ [ppm] = 0.78-0.87 (m, 5H), 1.00-1.08 (m, 2H), 1.23 (s, 9H), 2.78 (q, 2H), 3.36-3.50 (m, 1H), 3.86 (s, 3H), 4.07 (d, 1H), 6.99-7.07 (m, 2H), 7.23 (d, 1H), 7.35 (d, 1H), 7.41 (d, 1H), 7.44 (d, 1H), 9.81 (s, 1H). |
| | aus *tert*.-Butyl-1-(3-amino-4-chlorbenzyl)cyclo-propancarboxylat und (2*S*,3*R*)-2-(4-Chlor-3-methoxy-phenyl)-4,4,4-trifluor-3-methylbutansäure | |
| **191A** | *tert*.-Butyl-1-[4-chlor-3-({(2*S*,3*R*)-4,4,4-trifluor-3-methyl-2-[4-(1,1,1-trifluor-2-methylpropan-2-yl)-phenyl]butanoyl}amino)benzyl]cyclopropancarboxylat | LC-MS (Methode 5): Rₜ = 1.61 min; m/z = 604/606 (M-H)⁻. |
| | 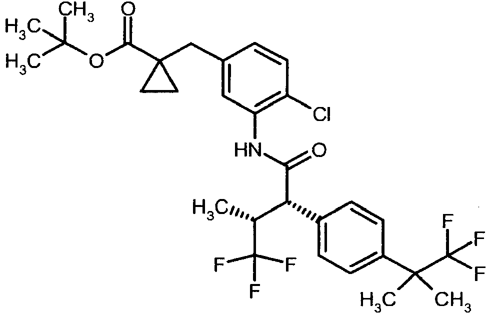 | |
| | aus *tert*.-Butyl-1-(3-amino-4-chlorbenzyl)cyclo-propancarboxylat und (2*S*,3*R*)-4,4,4-Trifluor-3-methyl-2-[4-(1,1,1-trifluor-2-methylpropan-2-yl)-phenyl]butansäure | |

| **Beispiel** | **Name / Struktur / Edukte** | **Analytische Daten** |
|---|---|---|
| **192A** | *tert*.-Butyl-1-[4-chlor-3-({4,4,4-trifluor-3-methyl-2-[4-(2,2,2-trifluorethyl)phenyl]butanoyl}amino)-benzyl]cyclopropancarboxylat | LC-MS (Methode 5): Rₜ = 1.50 min; m/z = 576/578 (M-H)⁻. |
| | 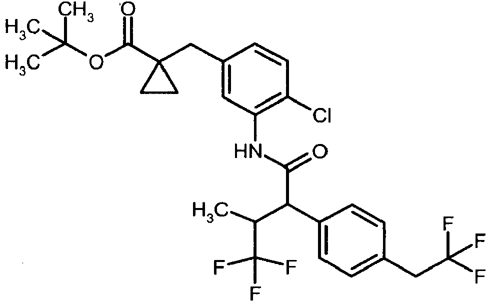 | |
| | aus *tert*.-Butyl-1-(3-amino-4-chlorbenzyl)cyclopropancarboxylat und 4,4,4-Trifluor-3-methyl-2-[4-(2,2,2-trifluorethyl)phenyl]butansäure | |

### Beispiel 193A

### (+/-)-tert.-Butyl-1-[(3-brom-4-chlorphenyl)(hydroxy)methyl]cyclopropancarboxylat

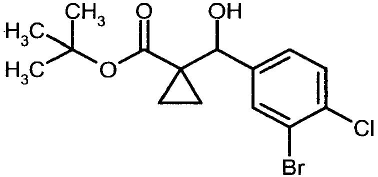

Zu einer auf -20°C bis -30°C abgekühlten Lösung von 14.8 ml (105.5 mmol) Diisopropylamin in 60 ml abs. THF wurden tropfenweise 42.2 ml (105.5 mmol) einer 2.5 M *n*-Butyllithium-Lösung in Hexan getropft. Nach Ende der Zugabe wurde 30 min bei -20°C bis -30°C nachgerührt. Anschließend wurde auf -78°C abgekühlt und bei dieser Temperatur tropfenweise eine Lösung von 12.0 g (84.4 mmol) Cyclopropancarbonsäure-*tert*.-butylester in 60 ml abs. THF hinzugefügt. Nach 4 h bei -78°C wurde dann eine Lösung von 15.4 g (70.3 mmol) 3-Brom-4-chlorbenzaldehyd in 60 ml abs. THF hinzugefügt. Die Reaktionsmischung wurde über Nacht langsam auf RT erwärmt, bevor mit gesättigter wässriger Ammoniumchlorid-Lösung versetzt und mit Ethylacetat extrahiert wurde. Die organische Phase wurde über Magnesiumsulfat getrocknet und im Vakuum eingeengt. Das Rohprodukt wurde durch Chromatographie an Silicagel gereinigt (Laufmittel Cyclohexan/Ethylacetat 40:1 → 10:1). Es wurden 16.2 g der Zielverbindung erhalten (52.5% d. Th.).

LC-MS (Methode 4): Rₜ = 1.47 min; m/z = 286/288.

¹H-NMR (400 MHz, DMSO-*d₆*): δ [ppm] = 0.84-0.99 (m, 2H), 1.02-1.17 (m, 2H), 1.24 (s, 9H), 4.88 (d, 1H), 5.55 (d, 1H), 7.39 (dd, 1 H), 7.57 (d, 1H), 7.71 (d, 1H).

### Beispiel 194A

### (+/-)-tert.-Butyl-1-[(3-brom-4-chlorphenyl)(methoxy)methyl]cyclopropancarboxylat

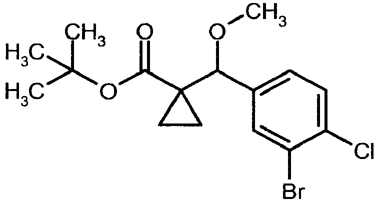

16.0 g (44.2 mmol) (+/-)-*tert*.-Butyl-1-[(3-brom-4-chlorphenyl)(hydroxy)methyl]cyclopropancarboxylat wurden in 80 ml DMF gelöst und bei RT mit 4.1 ml (66.6 mmol) Methyliodid versetzt. Die Reaktionsmischung wurde auf+10°C gekühlt und in mehreren Portionen mit 1.95 g (60% in Mineralöl, 48.7 mmol) Natriumhydrid versetzt. Zehn Minuten nach Ende der Zugabe wurde die Mischung auf RT erwärmt und weitere 1.5 h bei RT nachgerührt. Das Reaktionsgemisch wurde dann auf Wasser gegeben und dreimal mit Ethylacetat extrahiert. Die vereinigten organischen Phasen wurden im Vakuum eingeengt und der Rückstand durch Chromatographie an Silicagel gereinigt (Laufmittel Cyclohexan/Ethylacetat 50:1). Es wurden 15.3 g der Zielverbindung erhalten (92.2% d. Th.).

GC-MS (Methode 1): Rₜ = 6.5 min.

¹H-NMR (400 MHz, DMSO-*d₆*): δ [ppm] = 0.76-0.82 (m, 1H), 0.89-1.08 (m, 3H), 1.30 (s, 9H), 3.16 (s, 3H), 4.69 (s, 1H), 7.36 (dd, 1H), 7.62 (d, 1H), 7.71 (d, 1H).

### Beispiel 195A

### (+/-)-tert.-Butyl-1-{[3-(benzylamino)-4-chlorphenyl](methoxy)methyl}cyclopropancarboxylat

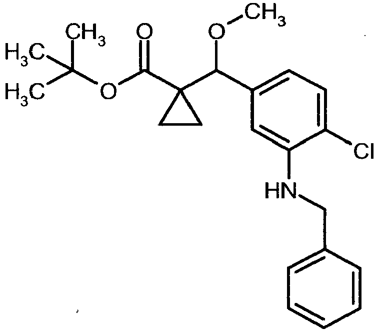

In einem trockenen, mit Argon gespülten Reaktionskolben wurden 4.60 g (47.9 mmol) Natrium-*tert.*-butylat in 100 ml abs. Toluol suspendiert und mit 5.2 ml (47.9 mmol) Benzylamin, 0.99 g (1.6 mmol) *rac*-2,2'-Bis(diphenylphosphino)-1,1'-binaphthyl, 1.83 g (2.0 mmol) Tris(dibenzyliden-aceton)dipalladium sowie 15.0 g (39.9 mmol) (+/-)-*tert*.-Butyl-1-[(3-brom-4-chlorphenyl)-(methoxy)methyl]cyclopropancarboxylat versetzt. Die Reaktionsmischung wurde 3 h auf 110°C erhitzt. Nach dem Abkühlen wurde die Mischung mit Ethylacetat und gesättigter wässriger Ammoniumchlorid-Lösung versetzt und über Celite filtriert. Die organische Phase wurde mit gesättigter Ammoniumchlorid-Lösung und gesättigter Natriumchlorid-Lösung gewaschen, über Magnesiumsulfat getrocknet und im Vakuum eingeengt. Das Rohprodukt wurde durch Chromatographie an Silicagel gereinigt (Laufmittel Cyclohexan/Ethylacetat 40:1 → 20:1). Es wurden 12.0 g der Zielverbindung erhalten (74.8% d. Th.).

LC-MS (Methode 5): Rₜ = 1.48 min; m/z = 402 (M+H)⁺.

¹H-NMR (400 MHz, DMSO-*d₆*): δ [ppm] = 0.22 (ddd, 1H), 0.56 (ddd, 1H), 0.68 (ddd, 1H), 0.78 (ddd, 1H), 1.30 (s, 9H), 2.98 (s, 3H), 4.40 (dd, 2H), 4.65 (s, 1H), 6.17 (t, 1H), 6.38 (d, 1H), 6.43 (dd, 1H), 7.18-7.22 (m, 2H), 7.28-7.33 (m, 4H).

### Beispiel 196A

### (+/-)-tert.-Butyl-1-[(3-amino-4-chlorphenyl)(methoxy)methyl]cyclopropancarboxylat

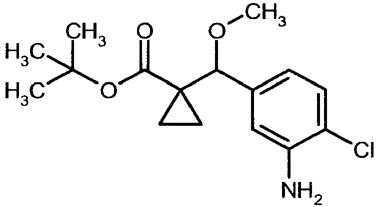

6.20 g (15.4 mmol) (+/-)-*tert*.-Butyl-1-{[3-(benzylamino)-4-chlorphenyl](methoxy)methyl}cyclopropancarboxylat wurden in 300 ml Ethylacetat gelöst, mit Argon inertisiert und mit 350 mg Palladium (10% auf Kohle) versetzt. Die Reaktionsmischung wurde bei RT insgesamt 24 h unter einer Wasserstoffatmosphäre bei Normaldruck gerührt. Die Reaktionsmischung wurde dann über Celite filtriert, der Rückstand gut mit Ethylacetat nachgewaschen und das vereinigte Filtrat im Vakuum eingeengt. Das Rohprodukt wurde durch Chromatographie an Silicagel gereinigt (Laufmittel Cyclohexan/Ethylacetat 30:1 → 10:1). Es wurden 3.36 g der Zielverbindung erhalten (69.9% d. Th.).

LC-MS (Methode 5): Rₜ = 1.22 min; m/z = 312 (M+H)⁺.

¹H-NMR (400 MHz, DMSO-d₆): δ [ppm] = 0.47-0.56 (m, 1H), 0.81-0.95 (m, 3H), 1.34 (s, 9H), 3.13 (s, 3H), 4.69 (s, 1H), 5.21-5.39 (m, 2H), 6.44 (dd, 1H), 6.72 (d, 1H), 7.13 (d, 1H).

### Beispiel 197A

### tert.-Butyl-1-[(4-chlor-3-{[(2S,3R)-2-(4-chlorphenyl)-4,4,4-trifluor-3-methylbutanoyl]amino}-phenyl)(methoxy)methyl]cyclopropancarboxylat (Diastereomerengemisch)

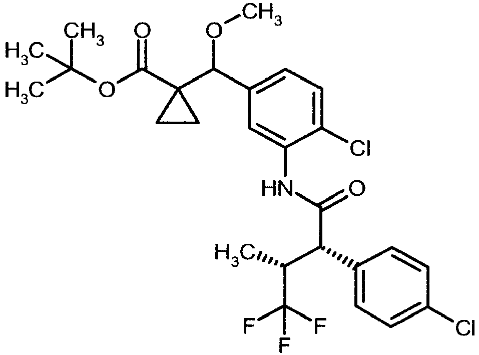

200 mg (0.641 mmol) (+/-)-*tert*.-Butyl-1-[(3-amino-4-chlorphenyl)(methoxy)methyl]cyclopropancarboxylat und 188 mg (0.706 mmol) (+)-(2*S*,3*R*)-2-(4-Chlorphenyl)-4,4,4-trifluor-3-methylbutansäure wurden in 2.3 ml DMF und 0.7 ml Pyridin gelöst und bei RT mit 293 mg (0.770 mmol) HATU versetzt. Die Reaktionsmischung wurde über Nacht bei RT gerührt. Anschließend wurden weitere 0.6 eq. (+)-(2*S*,3*R*)-2-(4-Chlorphenyl)-4,4,4-trifluor-3-methylbutansäure hinzugefügt und die Reaktionsmischung 14 h bei 45°C gerührt. Nach dem Abkühlen wurde das Gemisch mit Ethylacetat verdünnt, und die Lösung wurde mit 1 N Salzsäure und gesättigter Natriumchlorid-Lösung gewaschen, über Magnesiumsulfat getrocknet und im Vakuum eingeengt. Der Rückstand wurde durch Chromatographie an Silicagel gereinigt (Laufmittel Cyclohexan/Ethylacetat 30:1). Es wurden 168 mg der Zielverbindung erhalten (39.9% d. Th.).

LC-MS (Methode 5): Rₜ = 1.53 min; m/z = 558/560 (M-H)⁻.

¹H-NMR (400 MHz, DMSO-*d₆*): *beide Diastereomere:* δ [ppm] = 0.51-0.62 (m, 1H), 0.80 (d, 3H), 0.84-0.96 (m, 3H), 1.26/1.30 (je s, zus. 9H), 3.13 (s, 3H), 3.35-3.44 (d, 1H), 4.13/4.14 (je d, zus. 1H), 4.74 (s, 1H), 7.10 (dd, 1H), 7.39-7.53 (m, 6H), 9.91/9.92 (je s, zus. 1H).

### Beispiel 198A

### tert.-Butyl-1-[(3-brom-4-chlorphenyl)(hydroxy)methyl]cyclobutancarboxylat

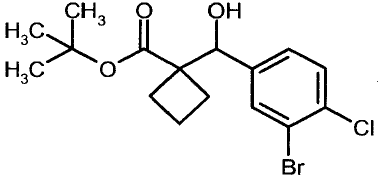

Zu einer auf -20°C bis -30°C abgekühlten Lösung von 7.6 ml (54.12 mmol) Diisopropylamin in 30 ml abs. THF wurden tropfenweise 21.65 ml (54.12 mmol) einer 2.5 M *n*-Butyllithium-Lösung in Hexan getropft. Nach Ende der Zugabe wurde 30 min bei -20°C bis -30°C nachgerührt. Anschließend wurde auf -78°C abgekühlt und bei dieser Temperatur tropfenweise eine Lösung von 6.2 g (39.7 mmol) *tert*.-Butylcyclobutancarboxylat in 10 ml abs. THF hinzugefügt. Nach 4 h bei -78°C wurde dann eine Lösung von 7.9 g (36.1 mmol) 3-Brom-4-chlorbenzaldehyd in 10 ml abs. THF hinzugefügt. Die Reaktionsmischung wurde über Nacht langsam auf RT erwärmt, bevor mit gesättigter wässriger Ammoniumchlorid-Lösung versetzt und mit Ethylacetat extrahiert wurde. Die organische Phase wurde über Magnesiumsulfat getrocknet und im Vakuum eingeengt. Das Rohprodukt wurde durch Chromatographie an Silicagel gereinigt (Laufmittel Cyclohexan/Ethylacetat 50:1 → 10:1). Es wurden 7.77 g der Zielverbindung erhalten (56% d. Th.).

¹H-NMR (400 MHz, DMSO-*d₆*): δ [ppm] = 1.33 (s, 9H), 1.55-1.77 (m, 2H), 1.98-2.11 (m, 2H), 2.21-2.35 (m, 1H), 2.35-2.47 (m, 1H), 4.70 (d, 1H), 5.89 (d, 1H), 7.31 (dd, 1H), 7.53-7.60 (m, 2H).

### Beispiel 199A

### tert.-Butyl-1-[(3-brom-4-chlorphenyl)(trideuteromethoxy)methyl]cyclopropancarboxylat

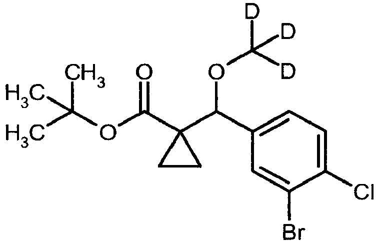

3 g (8.3 mmol) *tert*.-Butyl-1-[(3-brom-4-chlorphenyl)(hydroxy)methyl]cyclopropancarboxylat wurden in 10 ml DMF gelöst und bei RT mit 774 µl (12.44 mmol) Trideuteromethyliodid versetzt. Die Reaktionsmischung wurde auf +10°C gekühlt und in mehreren Portionen mit 398 mg (60% in Mineralöl, 9.95 mmol) Natriumhydrid versetzt. Zehn Minuten nach Ende der Zugabe wurde die Mischung auf RT erwärmt und weitere 1.5 h bei RT gerührt. Die Reaktionsmischung wurde dann auf Wasser gegeben und dreimal mit Ethylacetat extrahiert. Die vereinigten organischen Phasen wurden im Vakuum eingeengt und der Rückstand durch Chromatographie an Silicagel gereinigt (Laufmittel Cyclohexan/Ethylacetat 50:1). Es wurden 2.74 g der Zielverbindung erhalten (87% d. Th.).

¹H-NMR (400 MHz, DMSO-*d₆*): δ [ppm] = 0.75-0.83 (m, 1H), 0.88-1.07 (m, 3H), 1.30 (s, 9H), 4.69 (s, 1H), 7.36 (dd, 1H), 7.62 (d, 1H), 7.71 (d, 1H).

Auf analoge Weise wurden die in der folgenden Tabelle aufgeführten Verbindungen hergestellt:

| **Beispiel** | **Name / Struktur / Edukte** | **Analytische Daten** |
|---|---|---|
| **200A** | *tert*.-Butyl-1-[(3-brom-4-chlorphenyl)-(ethoxy)methyl]cyclopropancarboxylat | ¹H-NMR (400 MHz, DMSO-d₆): δ [ppm] = 0.71-0.79 (m, 1H), 0.89-1.04 (m, 3H), 1.07 (t, 3H), 1.31 (s, 9H), 3.25-3.43 (m, 2H, teilweise verdeckt durch H₂O-Signal), 4.81 (s, 1H), 7.36 (dd, 1H), 7.61 (d, 1H), 7.70 (d, 1H). |
| | 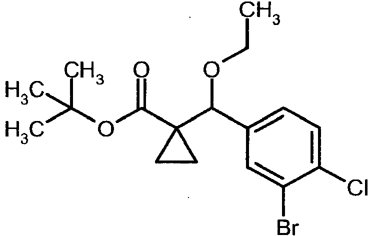 | |
| | aus *tert*.-Butyl-1-[(3-brom-4-chlorphenyl)-(hydroxy)methyl]cyclopropancarboxylat und Iodethan | |
| **201A** | *tert.*-Butyl-1-[(3-brom-4-chlorphenyl)(trideuteromethoxy)methyl]cyclobutancarboxylat | ¹H-NMR (400 MHz, DMSO-d₆): δ [ppm] = 1.34 (s, 9H), 1.47-1.59 (m, 1H), 1.62-1.75 (m, 1H), 2.05-2.15 (m, 2H), 2.20-2.35 (m, 2H), 4.35 (s, 1H), 7.32 (dd, 1H), 7.61 (d, 1H), 7.63 (d, 1H). |
| | 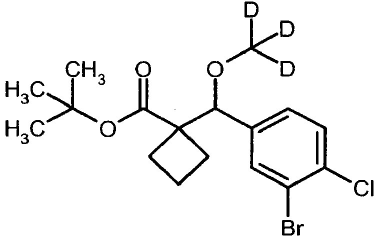 | |
| | aus *tert.*-Butyl-1-[(3-brom-4-chlorphenyl)-(hydroxy)methyl]cyclobutancarboxylat und Trideuteromethyliodid | |
| **202A** | *tert.*-Butyl-1-[(3-brom-4-chlorphenyl)-(methoxy)methyl]cyclobutancarboxylat | ¹H-NMR (400 MHz, DMSO-d₆): δ [ppm] = 1.37 (s, 9H), 1.47-1.59 (m, 1H), 1.61-1.75 (m, 1H), 2.05-2.15 (m, 2H), 2.21-2.36 (m, 2H), 3.19 (s, 3H), 4.35 (s, 1H), 7.32 (dd, 1H), 7.61 (d, 1H), 7.63 (d, 1H). |
| | 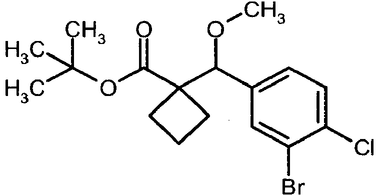 | |
| | aus *tert*.-Butyl-1-[(3-brom-4-chlorphenyl)-(hydroxy)methyl]cyclobutancarboxylat und Iodmethan | |
| **203A** | *tert*.-Butyl-1-[(3-brom-4-chlorphenyl)-(ethoxy)methyl]cyclobutancarboxylat | ¹H-NMR (400 MHz, DMSO-d₆): δ [ppm] = 1.11 (t, 3H), 1.35 (s, 9H), 1.44-1.54 (m, 1H), 1.61-1.73 (m, 1H), 2.04-2.15 (m, 2H), 2.17-2.31 (m, 2H), 3.27-3.37 (q, 2H, verdeckt durch H₂O-Signal), 4.47 (s, 1H), 7.34 (dd, 1H), 7.59-7.64 (m, 2H). |
| | 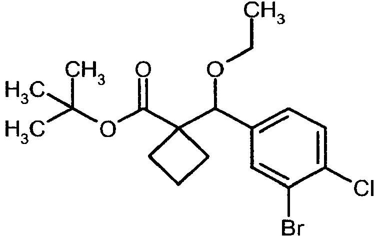 | |
| | aus *tert*.-Butyl-1-[(3-brom-4-chlorphenyl)-(hydroxy)methyl]cyclobutancarboxylat und Iodethan | |

Analog zu Synthesebeispiel 30A wurden die folgenden Verbindungen erhalten:

| **Beispiel** | **Name / Struktur / Edukte** | **Analytische Daten** |
|---|---|---|
| **204A** | *tert.*-Butyl-1-{[3-(benzylamino)-4-chlorphenyl]-(trideuteromethoxy)methyl}cyclopropancarboxylat | LC-MS (Methode 4): Rₜ = 1.71 min; m/z = 405 (M+H)⁺. |
| | 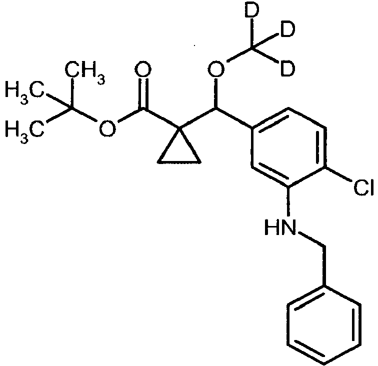 | ¹H-NMR (400 MHz, DMSO-d₆): δ [ppm] = 0.19-0.27 (m, 1H), 0.53-0.60 (m, 1H), 0.64-0.72 (m, 1H), 0.74-0.81 (m, 1H), 1.30 (s, 9H), 4.40 (d, 2H), 4.65 (s, 1H), 6.17 (t, 1H), 6.39 (s, 1H), 6.43 (d, 1H), 7.17-7.23 (m, 2H), 7.30 (d, 4H). |
| | aus *tert*.-Butyl-1-[(3-brom-4-chlorphenyl)-(trideuteromethoxy)methyl]cyclopropancarboxylat und Benzylamin | |
| **205A** | *tert*.-Butyl-1-{[3-(benzylamino)-4-chlorphenyl]-(ethoxy)methyl}cyclopropancarboxylat | LC-MS (Methode 5): Rₜ = 1.59 min; m/z = 416 (M+H)⁺. |
| | 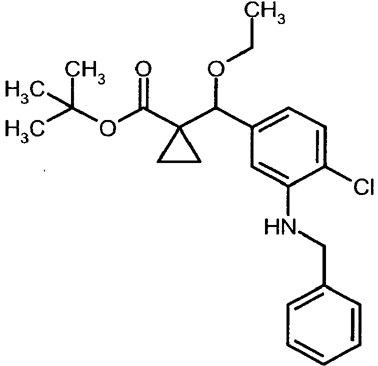 | ¹H-NMR (400 MHz, DMSO-*d₆*): δ [ppm] = 0.16-0.26 (m, 1H), 0.50-0.61 (m, 1H), 0.62-0.71 (m, 1H), 0.74-0.84 (m, 1H), 0.91 (t, 3H), 1.31 (s, 9H), 3.01-3.11 (m, 1H), 3.12-3.24 (m, 1H), 4.40 (d, 2H), 4.76 (s, 1H), 6.18 (t, 1H), 6.35-6.45 (m, 2H), 7.14-7.24 (m, 2H), 7.25-7.35 (m, 4H). |
| | aus *tert.*-Butyl-1-[(3-brom-4-chlorphenyl)-(ethoxy)methyl]cyclopropancarboxylat und Benzylamin | |
| **206A** | *tert*.-Butyl-1-{[3-(benzylamino)-4-chlorphenyl]-(trideuteromethoxy)methyl}cyclobutancarboxylat | LC-MS (Methode 5): Rₜ = 1.59 min; m/z = 419 (M+H)⁺. |
| | 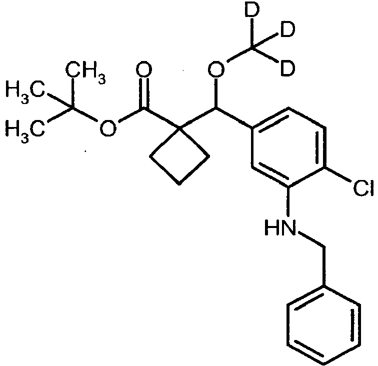 | ¹H-NMR (400 MHz, DMSO-*d₆*): δ [ppm] = 1.10-1.25 (m, 1H), 1.31 (s, 9H), 1.43-1.59 (m, 1H), 1.78-1.94 (m, 2H), 2.00 (t, 2H), 4.10 (s, 1H), 4.41 (d, 2H), 6.17 (t, 1H), 6.41-6.47 (m, 2H), 7.15-7.23 (m, 2H), 7.24-7.35 (m, 4H). |
| | aus *tert*.-Butyl-1-[(3-brom-4-chlorphenyl)-(trideuteromethoxy)methyl]cyclobutancarboxylat und Benzylamin | |
| **207A** | *tert*.-Butyl-1-{[3-(benzylamino)-4-chlorphenyl]-(methoxy)methyl}cyclobutancarboxylat | LC-MS (Methode 4): Rₜ = 1.78 min; m/z = 416 (M+H)⁺. |
| | 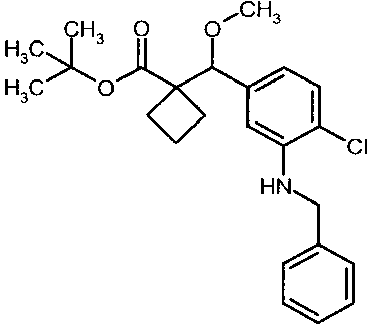 | |
| | aus *tert*.-Butyl-1-[(3-brom-4-chlorphenyl)-(methoxy)methyl]cyclobutancarboxylat und Benzylamin | |
| **208A** | *tert*.-Butyl-1-{[3-(benzylamino)-4-chlorphenyl]-(ethoxy)methyl}cyclobutancarboxylat | LC-MS (Methode 5): Rₜ = 1.67 min; m/z = 430 (M+H)⁺. |
| | 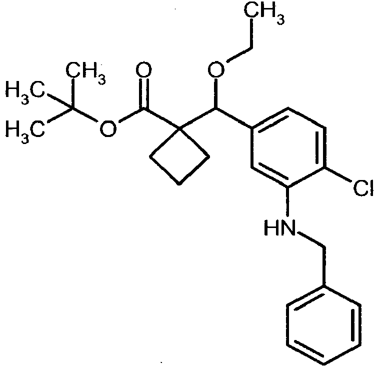 | |
| | aus *tert*.-Butyl-1-[(3-brom-4-chlorphenyl)-(ethoxy)methyl]cyclobutancarboxylat und Benzylamin | |

Analog zu Synthesebeispiel 196A wurden die folgenden Verbindungen erhalten:

| **Beispiel** | **Name / Struktur / Edukt** | **Analytische Daten** |
|---|---|---|
| **209A** | *tert.-*Butyl-1-[(3-amino-4-chlorphenyl)-(trideuteromethoxy)methyl]cyclopropan-carboxylat | LC-MS (Methode 5): Rₜ = 1.18 min; m/z = 315 (M+H)⁺. |
| | 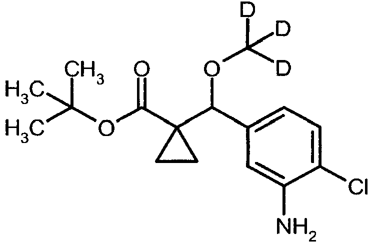 | ¹H-NMR (400 MHz, DMSO-d₆): δ [ppm] = 0.50-0.56 (m, 1H), 0.82-0.95 (m, 3H), 1.34 (s, 9H), 4.69 (s, 1H), 5.35 (s, 2H), 6.44 (dd, 1H), 6.72 (d, 1H), 7.13 (d, 1H). |
| | aus *tert.* -Butyl-1-{[3-(benzylamino)-4-chlor-phenyl](trideuteromethoxy)methyl} cyclopropancarboxylat | |
| **210A** | *tert.*-Butyl-1-[(3-amino-4-chlorphenyl)-(ethoxy)methyl]cyclopropancarboxylat | LC-MS (Methode 5): Rₜ = 1.27 min; m/z = 326 (M+H)⁺. |
| | 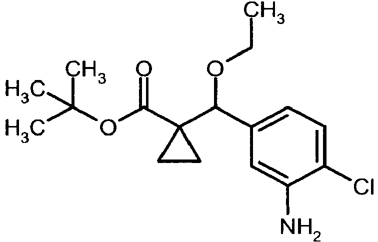 | ¹H-NMR (400 MHz, DMSO-d₆): δ [ppm] = 0.46-0.54 (m, 1H), 0.79-0.95 (m, 3H), 1.07 (t, 3H), 1.35 (s, 9H), 3.25-3.38 (m, 2H, teilweise verdeckt durch H₂O-Signal), 4.81 (s, 1H), 5.32 (s, 2H), 6.44 (dd, 1H), 6.72 (d, 1H), 7.12 (d, 1H). |
| | aus *tert*.-Butyl-1-{[3-(benzylamino)-4-chlor-phenyl](ethoxy)methyl}cyclopropancarboxylat | |

| **Beispiel** | **Name / Struktur / Edukt** | **Analytische Daten** |
|---|---|---|
| **211A** | *tert.*-Butyl-1-[(3-amino-4-chlorphenyl)-(trideuteromethoxy)methyl]cyclobutan-carboxylat | LC-MS (Methode 5): Rₜ = 1.29 min; m/z = 329 (M+H)⁺. |
| | 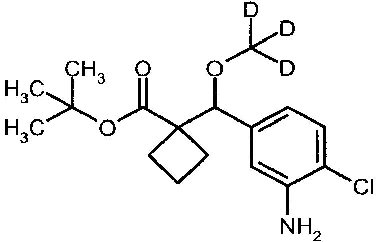 | ¹H-NMR (400 MHz, DMSO-*d₆*): δ [ppm] = 1.35 (s, 9H), 1.39-1.50 (m, 1H), 1.57-1.71 (m, 1H), 2.02-2.15 (m, 2H), 2.16-2.34 (m, 2H), 4.14 (s, 1H), 5.34 (s, 2H), 6.44 (dd, 1H), 6.73 (d, 1H), 7.13 (d, 1H). |
| | aus *tert*.-Butyl-1-{[3-(benzylamino)-4-chlor-phenyl](trideuteromethoxy)methyl}cyclobutan-carboxylat | |
| **212A** | *tert*.-Butyl-1-[(3-amino-4-chlorphenyl)-(methoxy)methyl]cyclobutancarboxylat | LC-MS (Methode 5): Rₜ = 1.30 min; m/z = 326 (M+H)⁺. |
| | 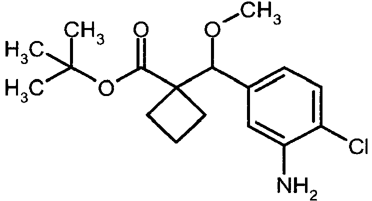 | ¹H-NMR (400 MHz, DMSO-d₆): δ [ppm] = 1.35 (s, 9H), 1.38-1.50 (m, 1H), 1.56-1.71 (m, 1H), 2.02-2.14 (m, 2H), 2.16-2.35 (m, 2H), 3.14 (s, 3H), 4.14 (s, 1H), 5.34 (s, 2H), 6.44 (dd, 1 H), 6.73 (d, 1H), 7.13 (d, 1H). |
| | aus *tert*.-Butyl-1-{[3-(benzylamino)-4-chlorphenyl](methoxy)methyl}cyclobutancarboxylat | |

| **Beispiel** | **Name / Struktur / Edukt** | **Analytische Daten** |
|---|---|---|
| **213A** | *tert.*-Butyl-1-[(3-amino-4-chlorphenyl)-(ethoxy)methyl]cyclobutancarboxylat | LC-MS (Methode 5): Rₜ = 1.37 min; m/z = 340 (M+H)⁺. |
| | 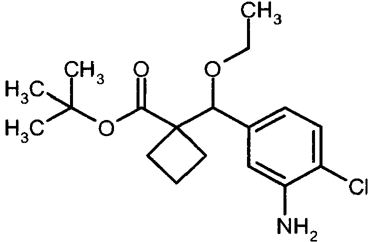 | ¹H-NMR (400 MHz, DMSO-d₆): δ [ppm] = 1.09 (t, 3H), 1.31-1.44 (m, 1H), 1.36 (s, 9H), 1.54-1.68 (m, 1H), 1.98-2.29 (m, 4H), 3.20-3.38 (m, 2H, teilweise verdeckt durch H₂O-Signal), 4.27 (s, 1H), 5.33 (s, 2H), 6.46 (dd, 1H), 6.75 (d, 1H), 7.13 (d, 1H). |
| | aus *tert*.-Butyl-1-{[3-(benzylamino)-4-chlorphenyl](ethoxy)methyl}cyclobutancarboxylat | |

Analog zu Synthesebeispiel 29A wurde die folgende Verbindung erhalten:

| **Beispiel** | **Name / Struktur / Edukte** | **Analytische Daten** |
|---|---|---|
| **214A** | *tert*.-Butyl-1-[1-(4-chlorphenyl)propyl]-cyclopropancarboxylat | GC-MS (Methode 6): Rₜ = 5.90 min; m/z = 312/314 (M+NH₄)⁺. |
| | 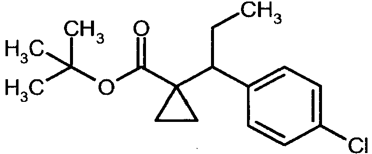 | GC-MS (Methode 1): Rₜ = 5.61 min; m/z = 238/240 (M-C₄H₈)⁺. |
| | aus *tert*.-Butylcyclopropancarboxylat und 1-(-Brompropyl)-4-chlorbenzol [CAS Reg.-No. 2940-56-9] | |

### Beispiel 215A

### 1-[1-(4-Chlor-3-nitrophenyl)propyl]cyclopropancarbonsäure

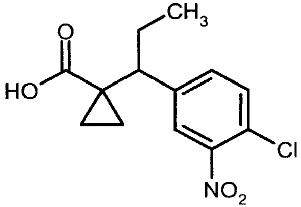

262 mg (0.89 mmol) *tert*.-Butyl-1-[1-(4-chlorphenyl)propyl]cyclopropancarboxylat wurden in 8 ml Dichlormethan gelöst, auf 0°C gekühlt und portionsweise mit 284 mg (2.14 mmol) Nitrosyltetrafluoroborat versetzt. Anschließend wurde die Reaktionlösung vier Stunden bei einer Temperatur zwischen -10°C und 0°C gerührt. Die Reaktionsmischung wurde danach auf Wasser gegeben und dreimal mit Dichlormethan extrahiert. Die vereinigten organischen Phasen wurden über Magnesiumsulfat getrocknet und im Vakuum eingeengt. Es wurden 262 mg des Zielprodukts erhalten, welches ohne weitere Aufreinigung in der Folgestufe eingesetzt wurde.

LC-MS (Methode 5): Rₜ = 1.01 min; m/z = 282/284 (M-H)⁻.

### Beispiel 216A

### Methyl-1-[1-(4-chlor-3-nitrophenyl)propyl]cyclopropancarboxylat

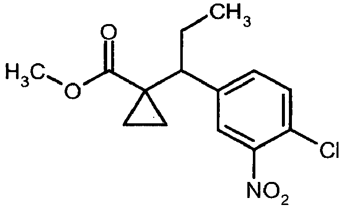

262 mg (ca. 0.93 mmol) 1-[1-(4-Chlor-3-nitrophenyl)propyl]cyclopropancarbonsäure wurden in 5 ml Methanol gelöst, auf 0°C gekühlt und tropfenweise mit 0.14 ml (1.85 mmol) Thionylchlorid versetzt. Anschließend wurde die Reaktionlösung langsam auf Raumtemperatur erwärmt und über Nacht bei dieser Temperatur gerührt. Die Reaktionsmischung wurde danach bis zur Trockene eingeengt und der Rückstand in 10 ml Dichlormethan aufgenommen. Die Lösung wurde mit Wasser gewaschen, über Magnesiumsulfat getrocknet und im Vakuum eingeengt. Es wurden 224 mg der Zielverbindung erhalten (81% d. Th.).

MS (DCI): m/z = 315 (M+NH₄)⁺.

### Beispiel 217A

### Methyl-1-[1-(3-amino-4-chlorphenyl)propyl]cyclopropancarboxylat

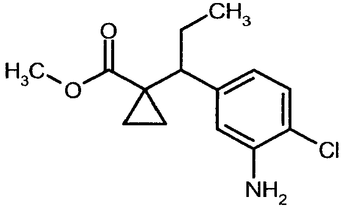

224 mg (0.76 mmol) Methyl-1-[1-(4-chlor-3-nitrophenyl)propyl]cyclopropancarboxylat wurden in 48 ml Ethylacetat gelöst, mit Argon inertisiert und mit 40 mg Palladium (10% auf Kohle) versetzt. Die Reaktionsmischung wurde bei RT insgesamt 24 h unter einer Wasserstoffatmosphäre bei Normaldruck gerührt. Das Gemisch wurde danach über Celite filtriert, der Rückstand gut mit Ethylacetat nachgewaschen und das vereinigte Filtrat im Vakuum eingeengt. Es wurden 195 mg der Zielverbindung erhalten (96% d. Th.).

LC-MS (Methode 5): Rₜ = 1.15 min; m/z = 268 (M+H)⁺.

¹H-NMR (400 MHz, DMSO-d₆): δ [ppm] = 0.51-0.59 (m, 1H), 0.74 (t, 3H), 0.77-0.84 (m, 1H), 0.94-1.01 (m, 1H), 1.02-1.09 (m, 1H), 1.64-1.81 (m, 2H), 2.68-2.75 (m, 1H), 3.55 (s, 3H), 5.04-5.39 (m, 2H), 6.41 (dd, 1H), 6.68 (d, 1H), 7.06 (d, 1H).

### Beispiel 218A

### tert.-Butyl-1-(3-amino-4-cyclopropylbenzyl)cyclopropancarboxylat

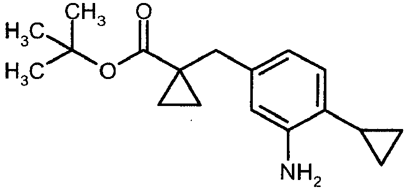

200 mg (0.71 mmol) *tert*.-Butyl-1-(3-amino-4-chlorbenzyl)cyclopropancarboxylat wurden in 6 ml Dioxan gelöst, mit Argon inertisiert und mit 122 mg (1.42 mmol) Cyclopropylboronsäure, 256 mg (1.21 mmol) Kaliumphosphat, 5 mg (0.02 mmol) Tricyclohexylphosphin sowie 6.5 mg (0.007 mmol) Tris(dibenzylidenaceton)dipalladium versetzt. Die Reaktionsmischung wurde dann in einer Mikrowellenapparatur (Biotage) 1 h bei einer Zieltemperatur von 150°C gerührt. Nach DC-Reaktionskontrolle (Laufmittel Cyclohexan/Ethylacetat 4:1) wurden weitere 10 mg Tricyclohexylphosphin und 13 mg Tris(dibenzylidenaceton)dipalladium zudosiert und die Reaktionsmischung erneut für 2 h bei 150°C gerührt. Anschließend wurde das Gemisch über Kieselgur filtriert, der Rückstand mit Dioxan nachgewaschen und das vereinigte Filtrat bis zur Trockene eingeengt. Der erhaltene Rückstand wurde über Kieselgel chromatographisch gereinigt (Laufmittel Cyclohexan/Ethylacetat 4:1). Es wurden 120 mg (59% d. Th.) der Zielverbindung isoliert.

LC-MS (Methode 5): Rₜ = 1.14 min; m/z = 288 (M+H)⁺.

### Beispiel 219A

### tert.-Butyl-1-{(4-chlor-3-{[(2S,3R)-2-(4-chlorphenyl)-4,4,4-trifluor-3-methylbutanoyl]amino}-phenyl)(trideuteromethoxy)methyl}cyclopropancarboxylat (Diastereomerengemisch)

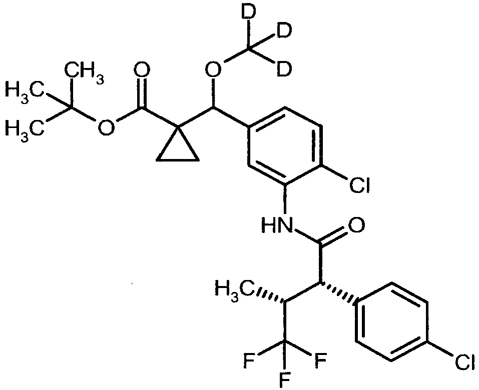

661 mg (2.1 mmol) *tert*.-Butyl-1-[(3-amino-4-chlorphenyl)(trideuteromethoxy)methyl]cyclopro-pancarboxylat und 560 mg (2.1 mmol) (2*S*,3*R*)-2-(4-Chlorphenyl)-4,4,4-trifluor-3-methylbutansäure wurden in 4 ml DMF und 2 ml Pyridin gelöst und bei RT mit 1.038 g (2.73 mmol) HATU versetzt. Die Reaktionsmischung wurde über Nacht bei RT gerührt. Danach wurde das Gemisch mit Ethylacetat verdünnt, und die Lösung wurde mit 1 N Salzsäure und gesättigter Natriumchlorid-Lösung gewaschen, über Magnesiumsulfat getrocknet und im Vakuum eingeengt. Der Rückstand wurde durch Chromatographie an Silicagel gereinigt (Laufmittel Cyclohexan → Cyclohexan/ Ethylacetat 100:1 → Cyclohexan/Ethylacetat 50:1). Es wurden 912 mg (77% d. Th.) der Titelverbindung erhalten.

LC-MS (Methode 5): Rₜ = 1.55 min; m/z = 561/563 (M-H)⁻.

¹H-NMR (400 MHz, DMSO-*d₆*): δ [ppm] = 0.53-0.62 (m, 1H), 0.80 (d, 3H), 0.84-0.96 (m, 3H), 1.26 (s, 4.5H), 1.30 (s, 4.5H), 3.29-3.46 (m, 1H, teilweise verdeckt durch H₂O-Signal), 4.12 (d, 0.5H), 4.15 (d, 0.5H), 4.74 (s, 1H), 7.07-7.13 (m, 1H), 7.40-7.54 (m, 6H), 9.91 (d, 1H).

Analog zu Synthesebeispiel 189A oder 197A wurden die folgenden Verbindungen erhalten:

| **Beispiel** | **Name / Struktur / Edukte** | **Analytische Daten** |
|---|---|---|
| **220A** | *tert*.-Butyl-1-[(4-chlor-3-{[(2*S*,3*R*)-2-(4-chlor-phenyl)-4,4,4-trifluor-3-methylbutanoyl]amino}-phenyl)(ethoxy)methyl]cyclopropancarboxylat *(Diastereomerengemisch)* | LC-MS (Methode 5): Rₜ = 1.62 min; m/z = 572/574 (M-H)⁻. |
| | | ¹H-NMR (400 MHz, DMSO-*d₆*): δ [ppm] = 0.49-0.59 (m, 1H), 0.76-0.96 (m, 6H), 1.01-1.09 (m, 3H), 1.28 (s, 4.5H), 1.31 (s, 4.5H), 3.20-3.46 (m, 3H, teilweise verdeckt durch H₂O-Signal), 4.11 (d, 0.5H), 4.14 (d, 0.5H), 4.86 (d, 1H), 7.10 (dd, 1H), 7.39-7.53 (m, 6H), 9.91 (s, 1H). |
| | 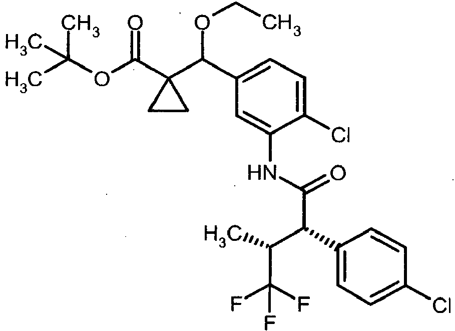 | |
| | aus *tert.* -Butyl-1-[(3-amino-4-chlorphenyl)-(ethoxy)methyl]cyclopropancarboxylat und (2S,3R)-2-(4-Chlorphenyl)-4,4,4-trifluor-3-methylbutansäure | |
| **221A** | *tert.-*Butyl-1-{(4-chlor-3-{[(*2S*,3*R*)-2-(4-chlorphenyl)-4,4,4-trifluor-3-methylbutanoyl]amino}-phenyl)(trideuteromethoxy)methyl}cyclobutan-carboxylat (*Diastereomerengemisch*) | LC-MS (Methode 4): Rₜ = 1.81 min; m/z = 575/577 (M-H)⁻. |
| | | ¹H-NMR (400 MHz, DMSO-*d₆*): δ [ppm] = 0.80 (d, 3H), 1.21 (s, 4.5H), 1.29 (s, 4.5H), 1.36-1.51 (m, 1H), 1.57-1.70 (m, 1H), 1.98-2.12 (m, 2H), 2.13-2.34 (m, 2H), 3.28-3.45 (m, 1H, teilweise verdeckt durch H₂O-Signal), 4.15 (dd, 1H), 4.26 (d, 1H), 7.08 (dd, 1H), 7.41-7.57 (m, 6H), 9.88 (d, 1 H). |
| | 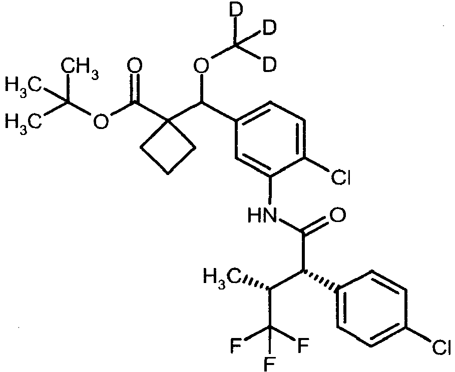 | |
| | aus *tert*.-Butyl-1-[(3-amino-4-chlorphenyl)-(trideuteromethoxy)methyl]cyclobutancarboxylat und (2*S*,3*R*)-2-(4-Chlorphenyl)-4,4,4-trifluor-3-methylbutansäure | |
| **222A** | *tert*.-Butyl-1-[(4-chlor-3-{[(2*S*,3*R*)-2-(4-chlor-phenyl)-4,4,4-trifluor-3-methylbutanoyl]amino}-phenyl)(methoxy)methyl]cyclobutancarboxylat (*Diastereomerengemisch*) | LC-MS (Methode 5): Rₜ = 1.62 min; m/z = 572/574 (M-H)⁻. |
| | | ¹H-NMR (400 MHz, DMSO-*d₆*): δ [ppm] = 0.80 (d, 3H), 1.22 (s, 4.5H), 1.30 (s, 4.5H), 1.36-1.51 (m, 1H), 1.57-1.70 (m, 1H), 1.98-2.13 (m, 2H), 2.14-2.35 (m, 2H), 3.15 (d, 3H), 3.29-3.46 (m, 1H, teilweise verdeckt durch H₂O-Signal), 4.15 (dd, 1H), 4.26 (d, 1H), 7.08 (dd, 1H), 7.41-7.57 (m, 6H), 9.88 (d, 1H). |
| | 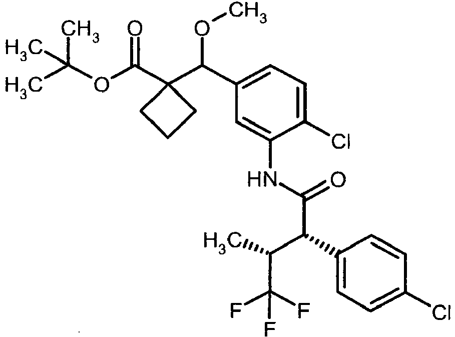 | |
| | aus *tert*.-Butyl-1-[(3-amino-4-chlorphenyl)-(methoxy)methyl]cyclobutancarboxylat und (2*S*,3*R*)-2-(4-Chlorphenyl)-4,4,4-trifluor-3-methylbutansäure | |
| **223A** | *tert*.-Butyl-1-[(4-chlor-3-{[(2*S*,3*R*)-2-(4-chlor-phenyl)-4,4,4-trifluor-3-methylbutanoyl]amino}-phenyl)(ethoxy)methyl]cyclobutancarboxylat (*Diastereomerengemisch*) | LC-MS (Methode 4): Rₜ = 1.87 min; m/z = 586/588 (M-H)-. |
| | | ¹H-NMR (400 MHz, DMSO-*d₆*): δ [ppm] = 0.80 (d, 3H), 1.03-1.12 (m, 3H), 1.24 (s, 4.5H), 1.32 (s, 4.5H), 1.34-1.46 (m, 1H), 1.55-1.69 (m, 1H), 1.96-2.30 (m, 4H), 3.23-3.46 (m, 3H, teilweise verdeckt durch H₂O-Signal), 4.14 (dd, 1H), 4.38 (d, 1H), 7.11 (dd, 1H), 7.40-7.59 (m, 6H), 9.88 (d, 1H). |
| | 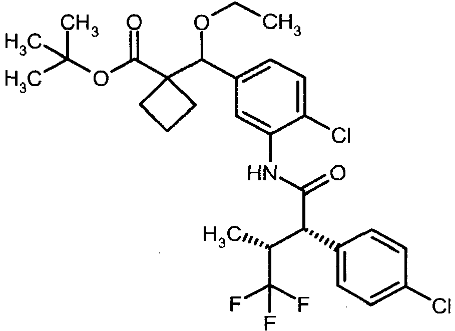 | |
| | aus *tert*.-Butyl-1-[(3-amino-4-chlorphenyl)-(ethoxy)methyl]cyclobutancarboxylat und (2*S*,3*R*)-2-(4-Chlorphenyl)-4,4,4-trifluor-3-methylbutansäure | |
| **224A** | Methyl-1-[1-(4-chlor-3-{[(2*S*,3*R*)-2-(4-chlorphenyl)-4,4,4-trifluor-3-methylbutanoyl]amino}phenyl)-propyl]cyclopropancarboxylat (*Diastereomerengemisch*) | LC-MS (Methode 5): Rₜ = 1.46 min; m/z = 514/516 (M-H)⁻. |
| | 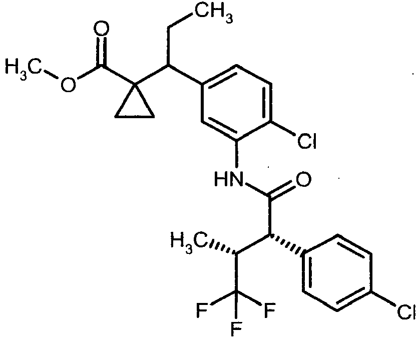 | |
| | aus Methyl-1-[1-(3-amino-4-chlorphenyl)propyl]-cyclopropancarboxylat und (2S,3R)-2-(4-Chlor-phenyl)-4,4,4-trifluor-3-methylbutansäure | |
| **225A** | *tert*.-Butyl-1-(3-{[(2*S*,3*R*)-2-(4-chlorphenyl)-4,4,4-trifluor-3-methylbutanoyl]amino}-4-cyclopropyl-benzyl)cyclopropancarboxylat | LC-MS (Methode 4): Rt = 1.77 min; m/z = 534/536 (M-H)⁻. |
| | | ¹H-NMR (400 MHz, DMSO-*d₆*): δ [ppm] = 0.29-0.37 (m, 1H), 0.41-0.49 (m, 1H), 0.57-0.70 (m, 2H), 0.73-0.78 (m, 2H), 0.80 (d, 3H), 0.97-1.03 (m, 2H), 1.26 (s, 9H), 1.64-1.74 (m, 1H), 2.68-2.80 (m, 2H), 3.29-3.45 (m, 1H, teilweise verdeckt durch H₂O-Signal), 4.01 (d, 1H), 6.80 (d, 1H), 6.93 (d, 1H), 7.14 (s, 1H), 7.42-7.50 (m, 4H), 9.68 (s, 1H). |
| | 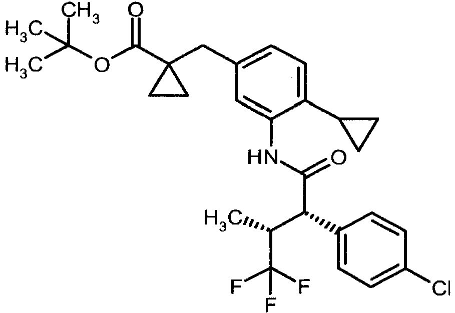 | |
| | aus *tert*.-Butyl-1-(3-amino-4-cyclopropylbenzyl)-cyclopropancarboxylat und (2*S*,3*R*)-2-(4-Chlor-phenyl)-4,4,4-trifluor-3-methylbulansäure | |

### Beispiel 226A

### tert.-Butyl-1-[(4-chlor-3-nitrophenyl)(hydroxy)methyl]cyclopropancarboxylat

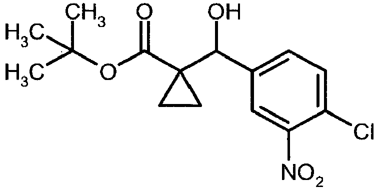

Zu einer auf -20°C bis -30°C abgekühlten Lösung von 3.4 ml (24.3 mmol) Diisopropylamin in 20 ml abs. THF wurden tropfenweise 9.7 ml (24.3 mmol) einer 2.5 M *n*-Butyllithium-Lösung in Hexan gegeben. Nach Ende der Zugabe wurde 30 min bei -20°C bis -30°C nachgerührt. Anschließend wurde auf -78°C abgekühlt und bei dieser Temperatur tropfenweise eine Lösung von 2.53 g (17.8 mmol) Cyclopropancarbonsäure-*tert*.-butylester in 15 ml abs. THF hinzugefügt. Nach 4 h bei -78°C wurde dann eine Lösung von 3 g (16.2 mmol) 4-Chlor-3-nitrobenzaldehyd in 15 ml abs. THF hinzugefügt. Die Reaktionsmischung wurde über Nacht langsam auf RT erwärmt, danach drei Tage bei RT stehen gelassen und dann mit gesättigter wässriger Ammoniumchlorid-Lösung versetzt. Das Gemisch wurde mit Ethylacetat extrahiert, und die organische Phase wurde über Magnesiumsulfat getrocknet und im Vakuum eingeengt. Das Rohprodukt wurde durch Chromatographie an Silicagel gereinigt (Laufmittel Cyclohexan/Ethylacetat 50:1 → 20:1). Es wurden 3.21 g der Zielverbindung erhalten (60.6% d. Th.).

LC-MS (Methode 2): Rₜ = 2.42 min; m/z = 328 (M+H)⁺.

¹H-NMR (400 MHz, DMSO-*d₆*): δ [ppm] = 0.91-1.01 (m, 2H), 1.05-1.12 (m, 1H), 1.14-1.21 (m, 1 H), 1.23 (s, 9H), 4.91 (d, 1H), 5.74 (d, 1H), 7.67-7.76 (m, 2H), 8.00 (d, 1 H).

Analog zu Synthesebeispiel 226A wurde die folgende Verbindung erhalten:

| **Beispiel** | **Name / Struktur / Edukte** | **Analytische Daten** |
|---|---|---|
| **227A** | *tert*.-Butyl-1-[1-(4-chlor-3-nitrophenyl)-1-hydroxyethyl]cyclopropancarboxylat | LC-MS (Methode 5): |
| | | Rt = 1.21 min; m/z = 342 (M+H)⁺. |
| | 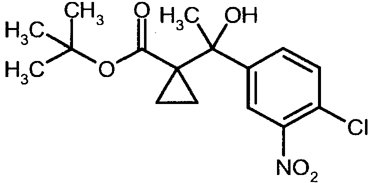 | ¹H-NMR (400 MHz, DMSO-*d₆*): δ [ppm] = 0.97-1.03 (m, 1H), 1.05-1.11 (m, 1H), 1.12-1.20 (m, 1H), 1.14 (s, 9H), 1.38-1.46 (m, 1H), 1.62 (s, 3H), 5.30 (s, 1H), 7.72 (d, 1H), 7.79 (dd, 1H), 8.05 (d, 1H). |
| | aus 1-(4-Chlor-3-nitrophenyl)ethanon und Cyclopropancarbonsäure-*tert*.-butylester | |

### Beispiel 228A

### tert.-Butyl-1-[(3-amino-4-chlorphenyl)(hydroxy)methyl]cyclopropancarboxylat

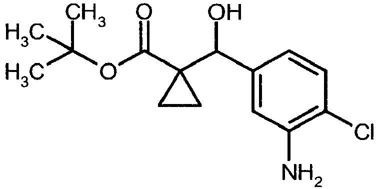

2.72 g (8.30 mmol) *tert*.-Butyl-1-[(4-chlor-3-nitrophenyl)(hydroxy)methyl]cyclopropancarboxylat wurden in 100 ml Ethylacetat gelöst und mit 177 mg Palladium auf Kohle (10%) versetzt. Die Reaktionsmischung wurde über drei Tage unter einer stationären Wasserstoffatmosphäre bei Normaldruck kräftig gerührt. Es wurde dann über Celite filtriert und das erhaltene Filtrat bis zur Trockene eingedampft. Das Rohprodukt wurde durch Chromatographie an Silicagel gereinigt (Laufmittel Cyclohexan/Ethylacetat 10:1). Es wurden 1.97 g der Zielverbindung erhalten (80% d. Th).

LC-MS (Methode 5): Rₜ = 1.02 min; m/z = 298 (M+H)⁺.

¹H-NMR (400 MHz, DMSO-*d₆*): δ [ppm] = 0.76-0.86 (m, 2H), 0.95-1.04 (m, 2H), 1.27 (s, 9H), 4.91 (d, 1H), 5.18 (d, 1H), 5.25 (br. s, 2H), 6.51 (dd, 1H), 6.81 (d, 1H), 7.08 (d, 1H).

Analog zu Synthesebeispiel 228A wurde die folgende Verbindung erhalten:

| **Beispiel** | **Name / Struktur / Edukt** | **Analytische Daten** |
|---|---|---|
| **229A** | *tert*.-Butyl-1-[1-(3-amino-4-chlorphenyl)-1-hydroxyethyl]cyclopropancarboxylat | LC-MS (Methode 7): |
| | | Rₜ = 1.13 min; m/z = 312 (M+H)⁺. |
| | 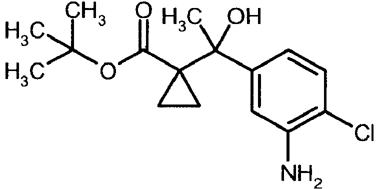 | ¹H-NMR (400 MHz, DMSO-*d*₆): δ [ppm] = 0.87-0.96 (m, 1 H), 0.98-1.08 (m, 2H), 1.15 (s, 9H), 1.29-1.37 (m, 1H), 1.52 (s, 3H), 4.67 (s, 1H), 5.21 (br. s, 2H), 6.66 (dd, 1H), 6.98 (d, 1H), 7.07 (d, 1H). |
| | aus *tert*.-Butyl-1-[1-(4-chlor-3-nitrophenyl)-1-hydroxyethyl]cyclopropancarboxylat | |

### Beispiel 230A

### tert.-Butyl-1-[(4-chlor-3- [(2S,3R)-2-(4-chlorphenyl)-4,4,4-trifluor-3-methylbutanoyl]amino}-phenyl)(hydroxy)methyl]cyclopropancarboxylat (Diastereomerengemisch)

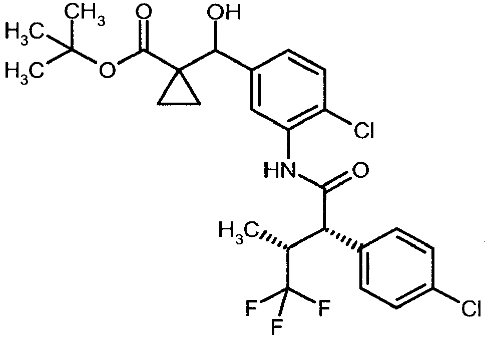

Eine Lösung von 2.65 g (9.92 mmol) (2*S*,3*R*)-2-(4-Chlorphenyl)-4,4,4-trifluor-3-methylbutansäure, 1.97 g (6.62 mmol) *tert*.-Butyl-1-[(3-amino-4-chlorphenyl)(hydroxy)methyl]cyclopropancarboxylat und 3.27 g (8.60 mmol) 2-(1*H*-7-Azabenzotriazol-1-yl)-1,1,3,3-tetramethyluronium-hexafluorophosphat (HATU) in 5 ml Pyridin und 10 ml DMF wurde über drei Tage bei Raumtemperatur gerührt. Der Ansatz wurde dann mit 100 ml Essigsäureethylester versetzt, und die erhaltene Lösung wurde mit 1 M Salzsäure und gesättigter Natriumchlorid-Lösung gewaschen. Nach Trocknen über Magnesiumsulfat wurde die Lösung filtriert und das Filtrat bis zur Trockene eingeengt. Das Rohprodukt wurde durch Chromatographie an Silicagel gereinigt (Laufmittel Cyclohexan/Ethylacetat 50:1 → 40:1 → 30:1). Das so erhaltene Produkt wurde in 100 ml Essigsäureethylester aufgenommen und die Lösung dreimal mit gesättigter Natriumhydrogencarbonat-Lösung extrahiert. Die organische Phase wurde noch einmal mit gesättigter Natriumchlorid-Lösung gewaschen, dann über Magnesiumsulfat getrocknet und schließlich im Vakuum bis zur Trockene eingeengt. Es wurden 2.75 g der Zielverbindung erhalten (76% d. Th).

LC-MS (Methode 5): Rₜ = 1.36 min; m/z = 544/546 (M-H)⁻.

¹H-NMR (400 MHz, DMSO-*d*₆): δ [ppm] = 0.77-0.85 (m, 5H), 0.93-1.08 (m, 2H), 1.18 (s, 4.5H), 1.22 (s, 4.5H), 3.27-3.44 (m, 1H, teilweise verdeckt durch H₂O-Signal), 4.08-4.14 (m, 1H), 4.94-4.98 (m, 1H), 5.40-5.45 (m, 1H), 7.12-7.17 (m, 1H), 7.36-7.41 (m, 1H), 7.41-7.50 (m, 4H), 7.53 (d, 0.5H), 7.55 (d, 0.5H), 9.85 (d, 1H).

Analog zu Synthesebeispiel 230A wurde die folgende Verbindung erhalten:

| **Beispiel** | **Name / Struktur / Edukte** | **Analytische Daten** |
|---|---|---|
| **231A** | *tert*.-Butyl-1-[1-(4-chlor-3-{[(2*S*,3*R*)-2-(4-chlor-phenyl)-4,4,4-trifluor-3-methylbutanoyl]amino}-phenyl)-1-hydroxyethyl]cyclopropancarboxylat (*Diastereomerengemisch*) | LC-MS (Methode 5): Rₜ = 1.42 min; m/z = 558/560 (M-H)⁻. |
| | | ¹H-NMR (400 MHz, DMSO-*d₆*): δ [ppm] = 0.79 (d, 3H), 0.86-0.95 (m, 1H), 0.98-1.12 (m, 2H), 1.07 (s, 4.5H), 1.09 (s, 4.5H), 1.28-1.36 (m, 1H), 1.56 (s, 3H), 3.29-3.45 (m, 1H, teilweise verdeckt durch H₂O-Signal), 4.12 (dd, 1H), 4.92 (d, 1H), 7.23-7.29 (m, 1H), 7.36 (d, 1H), 7.39-7.51 (m, 4H), 7.71-7.77 (m, 1H), 9.81 (s, 1H). |
| | 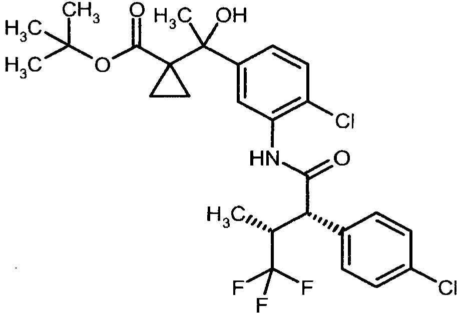 | |
| | aus *tert*.-Butyl-1-[1-(3-amino-4-chlorphenyl)-1-hydroxyethyl]cyclopropancarboxylat und (2*S*,3*R*)-2-(4-Chlorphenyl)-4,4,4-trifluor-3-methylbutansäure | |

### Beispiel 232A

### tert.-Butyl-1-[(4-chlor-3-{[(2S,3R)-2-(4-chlorphenyl)-4,4,4-trifluor-3-methylbutanoyl]amino}-phenyl)(vinyloxy)methyl]cyclopropancarboxylat (Diastereomerengemisch)

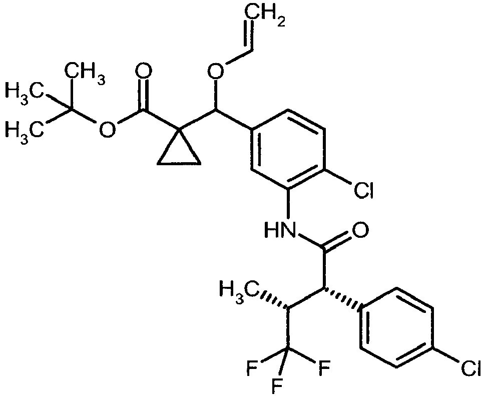

Unter Argon wurden 473 mg (0.87 mmol) *tert*.-Butyl-1-[(4-chlor-3-{[(2*S*,3*R*)-2-(4-chlorphenyl)-4,4,4-trifluor-3-methylbutanoyl]amino}phenyl)(hydroxy)methyl]cyclopropancarboxylat (als Diastereomerengemisch) in 2.4 ml Dichlormethan gelöst und bei Raumtemperatur mit 1.24 ml (13 mmol) Ethylvinylether und 11 mg (0.03 mmol) Bis(acetato)(1,10-phenanthrolin-*N*¹,*N*¹⁰)palladium *[*J. Org. Chem. 62, 1560-1562 (1997)] versetzt. Anschließend wurde die Reaktionslösung auf 50°C erhitzt und bei dieser Temperatur zwei Tage gerührt. Danach wurden nochmals 1.24 ml (13 mmol) Ethylvinylether und 11 mg (0.03 mmol) Bis(acetato)(1,10-phenanthrolin-*N*¹,*N*¹⁰)palladium hinzugefügt und das Reaktionsgemisch weiter über Nacht bei 50°C gerührt. Diese Prozedur wurde noch zweimal wiederholt. Danach wurde die Reaktionslösung auf Raumtemperatur abgekühlt und im Vakuum bis zur Trockene eingeengt. Das Rohprodukt wurde durch Chromatographie an Silicagel gereinigt (Laufmittel Cyclohexan/Dichlormethan 1:1). Es wurden 298 mg der Zielverbindung erhalten (60% d. Th).

LC-MS (Methode 7): Rₜ = 1.53 min; m/z = 570/572 (M-H)⁻.

¹H-NMR (400 MHz, DMSO-*d*₆): δ [ppm] = 0.48-0.59 (m, 1H), 0.80 (d, 3H), 0.82-0.90 (m, 1H), 0.92-0.99 (m, 1H), 1.00-1.08 (m, 1H), 1.30 (s, 4.5H), 1.32 (s, 4.5H), 3.28-3.46 (m, 1H, teilweise verdeckt durch H₂O-Signal), 3.94 (d, 1 H), 4.07-4.19 (m, 2H), 5.45 (s, 1 H), 6.34-6.44 (m, 1 H), 7.10 (d, 1H), 7.40-7.52 (m, 6H), 9.93 (s, 1H).

### Beispiel 233A

### tert.-Butyl-1-[(4-chlor-3-{[(2S,3R)-2-(4-chlorphenyl)-4,4,4-trifluor-3-methylbutanoyl]amino}-phenyl)(cyclopropyloxy)methyl]cyclopropancarboxylat (Diastereomerengemisch)

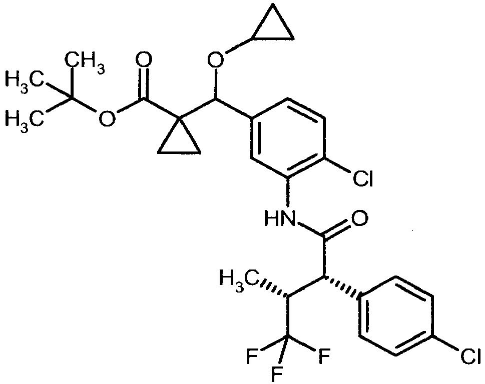

68 mg (0.52 mmol) Zink-Kupfer-Paar wurden in 5 ml abs. Diethylether aufgenommen und bei Raumtemperatur mit 41 µl (0.56 mmol) Chloriodmethan versetzt. Danach wurde zum Reaktionsgemisch eine Lösung von 200 mg (0.35 mmol) *tert*.-Butyl-1-[(4-chlor-3-{[(2*S*,3*R*)-2-(4-chlor-phenyl)-4,4,4-trifluor-3-methylbutanoyl]amino}phenyl)(vinyloxy)methyl]cyclopropancarboxylat (als Diastereomerengemisch) in 10 ml abs. Diethylether hinzugetropft. Die Reaktionslösung wurde dann auf Rückfluss erhitzt und über Nacht gerührt. Nach dem Abkühlen wurde die Lösung über Kieselgur filtriert und das Kieselgur mehrmals mit Diethylether nachgewaschen. Das erhaltene Filtrat wurde mit gesättigter wässriger Natriumhydrogencarbonat-Lösung und mit Wasser gewaschen, über Magnesiumsulfat getrocknet und im Vakuum bis zur Trockene eingeengt. Der Rückstand wurde durch präparative RP-HPLC gereinigt (Eluent Methanol/Wasser 80:20, isokratisch). Es wurden 54 mg (27% d. Th.) der Zielverbindung erhalten.

LC-MS (Methode 5): Rₜ = 1.52 min; m/z = 584/586 (M-H)⁻.

### Ausführungsbeispiele:

### Allgemeine Vorschrift 8: Spaltung von tert.-Butylestern zu den entsprechenden Carbonsäuren mit Trifluoressigsäure

Zu einer Lösung des betreffenden *tert*.-Butylesters in Dichlormethan (Konzentration ca. 0.1 bis 2.0 mol/l; zusätzlich optional ein Tropfen Wasser) wurde bei 0°C bis RT Trifluoressigsäure (TFA) hinzugefügt, bis ein Dichlormethan/TFA-Verhältnis von ca. 2:1 bis 1:2 (v/v) erreicht war. Die Mischung wurde 1-24 h bei RT gerührt; gegebenenfalls wurde die Mischung bis auf 40°C erwärmt, bis vollständiger Umsatz erreicht war. Danach wurde das Reaktionsgemisch bei RT im Vakuum eingeengt. Das Rohprodukt wurde durch Chromatographie an Silicagel (Elution mit Dichlormethan/Ethylacetat-, Cyclohexan/Ethylacetat- oder Dichlormethan/Methanol-Gemischen, gegebenenfalls unter Zusatz geringer Mengen Essigsäure), durch Kristallisation aus Diisopropylether, Acetonitril oder Acetonitril/Wasser-Gemischen, oder durch präparative RP-HPLC (Eluent: Acetonitril/Wasser-Gradient) gereinigt. Auch eine Kombination dieser Reinigungsmethoden kann verwendet werden, um das Zielprodukt in reiner Form zu isolieren.

Die folgenden Beispiele wurden gemäß der Allgemeinen Vorschrift 8 hergestellt:

| **Beispiel** | **Name / Struktur / Edukt** | **Analytische Daten** |
|---|---|---|
| **1** | (+/-)-1-(3-{[(4-Chlor-2-fluorphenyl)(cyclopentyl)-acetyl]amino}benzyl)cyclopropancarbonsäure | LC-MS (Methode 4): Rₜ = 1.46 min; m/z = 430 (M+H)⁺. |
| | 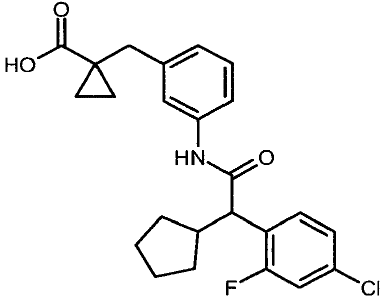 | ¹H-NMR (400 MHz, DMSO-*d₆*): δ [ppm] = 0.76-0.84 (m, 2H), 0.99 (dd, 1H), 1.11 (q, 2H), 1.32-1.61 (m, 5H), 1.62-1.79 (m, 2H), 2.48-2.58 (m, 1H, verdeckt), 2.83 (s, 2H), 3.78 (d, 1H), 6.93 (d, 1H), 7.16 (t, 1H), 7.29 (dd, 1H), 7.40 (dd, 1H), 7.43-7.49 (m, 2H), 7.70 (t, 1H), 10.14 (s, 1H), 12.09 (br. s, 1H). |
| | aus (+/-)-1-(3-{[(4-Chlor-2-fluorphenyl)-(cyclopentyl)acetyl]amino}benzyl)cyclopropan-carbonsäure-*tert*.-butylester | |
| **2** | (+/-)-1-(3-{[Cyclopentyl(2,4-dichlorphenyl)-acetyl]amino}benzyl)cyclopropancarbonsäure | LC-MS (Methode 4): Rₜ = 1.51 min; m/z = 446/447 (M+H)⁺. |
| | 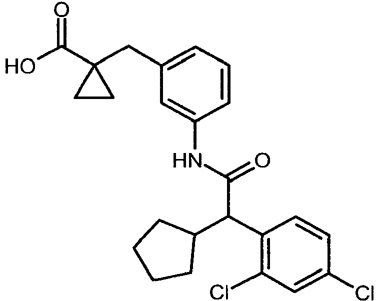 | ¹H-NMR (400 MHz, DMSO-*d₆*): δ [ppm] = 0.75-0.84 (m, 2H), 0.94-1.04 (m, 1H), 1.08-1.15 (m, 2H), 1.36-1.62 (m, 5H), 1.64-1.79 (m, 2H), 2.50-2.58 (m, 1H, verdeckt), 2.83 (s, 2H), 3.97 (d, 1H), 6.94 (d, 1H), 7.16 (t, 1H), 7.38-7.49 (m, 3H), 7.59 (d, 1H), 7.79 (d, 1H), 10.11 (s, 1H), 12.09 (s, 1H). |
| | aus (+/-)-1-(3-{[Cyclopentyl(2,4-dichlorphenyl)-acetyl]amino}benzyl)cyclopropancarbonsäure-*tert*.-butylester | |
| **3** | (+/-)-1-[3-({Cyclopentyl[3-fluor-4-(trifluormethyl)phenyl]acetyl}amino)benzyl]cyclopropancarbonsäure | LC-MS (Methode 4): Rₜ = 1.48 min; m/z = 464 (M+H)⁺. |
| | 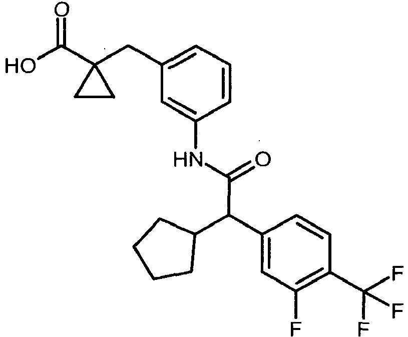 | ¹H-NMR (400 MHz, DMSO-*d₆*): δ [ppm] = 0.75-0.85 (m, 2H), 0.93-1.03 (m, 1H), 1.08-1.15 (m, 2H), 1.20-1.43 (m, 2H), 1.43-1.71 (m, 4H), 1.76-1.84 (m, 1H), 2.55-2.66 (m, 1H), 2.83 (s, 2H), 3.55 (d, 1H), 6.93 (d, 1H), 7.17 (t, 1H), 7.30-7.48 (m, 3H), 7.51 (d, 1H), 7.76 (t, 1H), 10.12 (s, 1H), 12.09 (s, 1H). |
| | aus (+/-)-1-[3-({Cyclopentyl[3-fluor-4-(trifluormethyl)phenyl]acetyl}amino)benzyl]cyclopropancarbonsäure-*tert*.-butylester | |
| **4** | (+/-)-1-[3-({Cyclopentyl[4-(trifluormethoxy)-phenyl]acetyl}amino)benzyl]cyclopropancarbonsäure | LC-MS (Methode 4): Rₜ = 1.47 min; m/z = 462 (M+H)⁺. |
| | 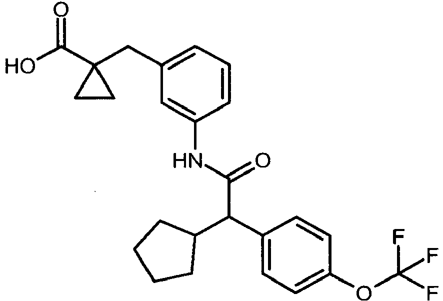 | ¹H-NMR (400 MHz, DMSO-*d₆*): δ [ppm] = 0.74-0.84 (m, 2H), 0.89-1.02 (m, 1H), 1.06-1.14 (m, 2H), 1.21-1.40 (m, 2H), 1.41-1.70 (m, 4H), 1.73-1.84 (m, 1H), 2.55-2.63 (m, 1H), 2.82 (s, 2H), 3.46 (d, 1H), 6.91 (d, 1H), 7.16 (t, 1H), 7.24-7.36 (m, 2H), 7.40-7.49 (m, 2H), 7.50-7.60 (m, 2H), 10.05 (s, 1H), 12.09 (s, 1H). |
| | aus (+/-)-1-[3-({Cyclopentyl[4-(trifluormethoxy)-phenyl]acetyl}amino)benzyl]cyclopropancarbonsäure-*tert*. -butylester | |
| **5** | (+/-)-1-(3-{[Cyclopentyl(3,4-dichlorphenyl)-acetyl]amino}benzyl)cyclopropancarbonsäure | LC-MS (Methode 4): Rₜ = 1.50 min; m/z = 446/447 (M+H)⁺. |
| | 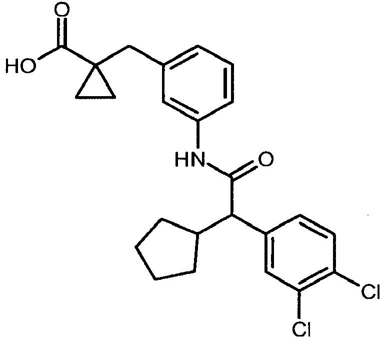 | ¹H-NMR (400 MHz, DMSO-d₆): δ [ppm] = 0.75-0.84 (m, 2H), 0.97 (dq, 1H), 1.07-1.15 (m, 2H), 1.19-1.31 (m, 1H), 1.31-1.42 (m, 1H), 1.42-1.70 (m, 4H), 1.71-1.85 (m, 1H), 2.54-2.62 (m, 1H), 2.83 (s, 2H), 3.42 (d, 1 H), 6.93 (d, 1H), 7.16 (t, 1H), 7.32-7.49 (m, 3H), 7.54-7.67 (m, 2H), 10.06 (s, 1H), 12.09 (br. s, 1H). |
| | aus (+/-)-1-(3-{[Cyclopentyl(3,4-dichlorphenyl)-acetyl]amino}benzyl)cyclopropancarbonsäure-*tert.* -butylester | |
| **6** | (+/-)-1-(3-{[(4-Chlorphenyl)(cyclopentyl)acetyl]-amino}-2-fluorbenzyl)cyclopropancarbonsäure | LC-MS (Methode 5): Rₜ = 1.25 min; m/z = 430 (M+H)⁺. |
| | 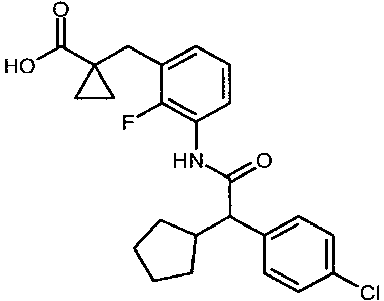 | ¹H-NMR (400 MHz, DMSO-*d₆*): δ [ppm] = 0.72-0.82 (m, 2H), 0.90-1.03 (m, 1H), 1.11-1.18 (m, 2H), 1.21-1.71 (m, 7H), 1.72-1.92 (m, 1H), 2.92 (s, 2H), 6.93-7.06 (m, 1H), 7.08-7.15 (m, 1H), 7.33-7.49 (m, 4H), 7.61 (t, 1H), 9.82 (s, 1H), 12.19 (br. s, 1H). |
| | aus (+/-)-1-(3-{[(4-Chlorphenyl)(cyclopentyl)-acetyl]amino}-2-fluorbenzyl)cyclopropancarbon-säure-*tert*. -butylester | |
| **7** | (+/-)-1-(3-{[{4-[(*E*)-2-Cyanovinyl]phenyl}-(cyclopentyl)acetyl]amino}-4-fluorbenzyl)-cyclopropancarbonsäure | LC-MS (Methode 5): Rₜ = 1.15 min; m/z = 447 (M+H)⁺. |
| | 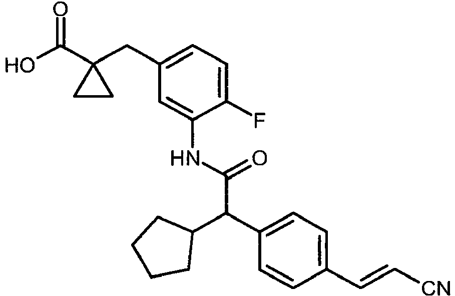 | ¹H-NMR (400 MHz, DMSO-*d₆*): δ [ppm] = 0.73-0.84 (m, 2H), 0.90-1.03 (m, 1H), 1.04-1.14 (m, 2H), 1.23-1.71 (m, 7H), 1.80 (dd, 1H), 2.80 (s, 2H), 6.42 (d, 1H), 6.97-7.05 (m, 1H), 7.06-7.16 (m, 1H), 7.48 (d, 2H), 7.58-7.70 (m, 4H), 9.76-9.88 (m, 1H), 12.12 (br. s, 1H). |
| | aus (+/-)-1-(3-{[{4-[(*E*)-2-Cyanovinyl]phenyl}-(cyclopentyl)acetyl]amino}-4-fluorbenzyl)-cyclopropancarbonsäure-*tert*.-butylester | |
| **8** | (+/-)-1-(3-{[(4-Chlorphenyl)(cyclopentyl)acetyl]-amino}-4-fluorbenzyl)cyclopropancarbonsäure | LC-MS (Methode 5): Rₜ = 1.24 min; m/z = 430 (M+H)⁺. |
| | 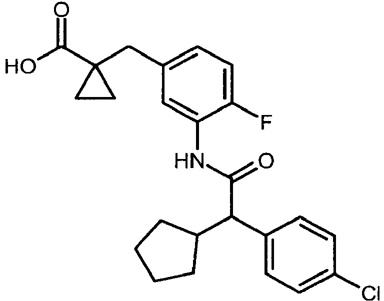 | ¹H-NMR (400 MHz, DMSO-*d₆*): δ [ppm] = 0.73-0.83 (m, 2H), 0.88-1.02 (m, 1H), 1.06-1.12 (m, 2H), 1.22-1.39 (m, 2H), 1.41-1.70 (m, 4H), 1.72-1.85 (m, 1H), 2.50-2.58 (m, 1H), 2.81 (s, 2H), 3.61 (d, 1H), 6.95-7.05 (m, 1H), 7.10 (dd, 1H), 7.33-7.41 (m, 2H), 7.41-7.46 (m, 2H), 7.66 (dd, 1H), 9.81 (s, 1H), 12.12 (s, 1H). |
| | aus (+/-)-1-(3-{[(4-Chlorphenyl)(cyclopentyl)-acetyl]amino}-4-fluorbenzyl)cyclopropancarbonsäure-*tert*.-butylester | |
| **9** | (+/-)-1-(3-{[2-(4-Ethylphenyl)-4,4,4-trifluor-3-methylbutanoyl]amino}-4-fluorbenzyl)-cyclopropancarbonsäure | LC-MS (Methode 5): Rₜ = 1.23 min; m/z = 452 (M+H)⁺. |
| | 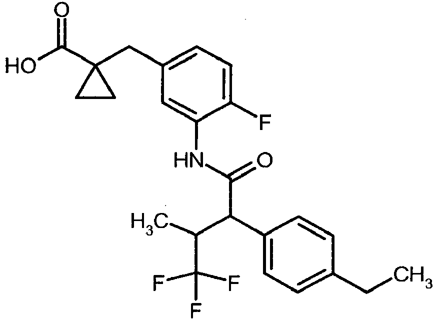 | ¹H-NMR (400 MHz, DMSO-*d₆*): δ [ppm] = 0.77 (d, 3H), 0.77-0.83 (m, 2H), 1.05-1.12 (m, 2H), 1.17 (t, 3H), 2.58 (q, 2H), 2.80 (s, 2H), 3.28-3.38 (m, 1H, verdeckt), 4.03 (d, 1H), 6.95-7.03 (m, 1H), 7.09 (dd, 1H), 7.17-7.23 (m, 2H), 7.31-7.35 (m, 2H), 7.69 (dd, 1H), 9.94 (s, 1H), 12.12 (br. s, 1H). |
| | aus (+/-)-1-(3-{[2-(4-Ethylphenyl)-4,4,4-trifluor-3-methylbutanoyl]amino}-4-fluorbenzyl)-cyclopropancarbonsäure-*tert.*-butylester | |
| **10** | (+/-)-1-(3-{[Cyclopentyl(4-fluorphenyl)acetyl]-amino}-4-fluorbenzyl)cyclopropancarbonsäure | LC-MS (Methode 5): Rₜ = 1.20 min; m/z = 414 (M+H)⁺. |
| | 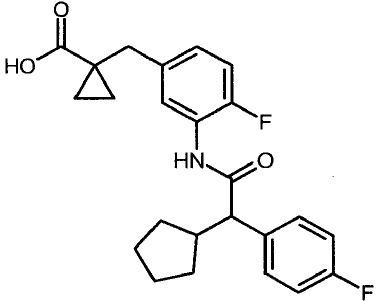 | ¹H-NMR (400 MHz, DMSO-*d₆*): δ [ppm] = 0.72-0.83 (m, 2H), 0.91-1.00 (m, 1H), 1.08-1.10 (m, 2H), 1.23-1.38 (m, 2H), 1.41-1.70 (m, 4H), 1.72-1.87 (m, 1H), 2.52-2.57 (m, 1H), 2.81 (s, 2H), 3.60 (d, 1H), 6.94-7.05 (m, 1H), 7.05-7.21 (m, 3H), 7.44 (dd, 2H), 7.67 (dd, 1H), 9.79 (s, 1H), 12.12 (s, 1H). |
| | aus (+/-)-1-(3-{[Cyclopentyl(4-fluorphenyl)-acetyl]amino}-4-fluorbenzyl)cyclopropan-carbonsäure-*tert*.-butylester | |
| **11** | (+/-)-1-(3-{[(4-Chlor-2-fluorphenyl)(cyclopentyl)-acetyl]amino}-4-fluorbenzyl)cyclopropan-carbonsäure | LC-MS (Methode 5): Rₜ = 1.27 min; m/z = 448 (M+H)⁺. |
| | 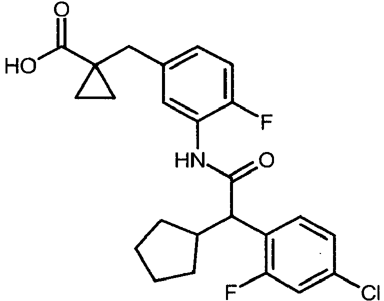 | ¹H-NMR (400 MHz, DMSO-*d₆*): δ [ppm] = 0.73-0.87 (m, 2H), 0.95-1.04 (m, 1H), 1.08-1.10 (m, 2H), 1.37-1.62 (m, 5H), 1.63-1.73 (m, 1H), 1.73-1.85 (m, 1H), 2.45-2.53 (m, 1H), 2.82 (s, 2H), 3.93 (d, 1H), 6.96-7.17 (m, 2H), 7.29 (dd, 1H), 7.40 (dd, 1H), 7.56 (dd, 1H), 7.68 (t, 1H), 9.94 (s, 1H), 12.13 (br. s, 1H). |
| | aus (+/-)-1-(3-{[(4-Chlor-2-fluorphenyl)-(cyclopentyl)acetyl]amino}-4-fluorbenzyl)-cyclopropancarbonsäure-*tert*.-butylester | |
| **12** | (+/-)-1-[3-({Cyclopentyl[4-(2,2-difluorvinyl)-phenyl]acetyl}amino)benzyl]cyclopropan-carbonsäure | LC-MS (Methode 5): Rₜ = 1.27 min; m/z = 440 (M+H)⁺. |
| | 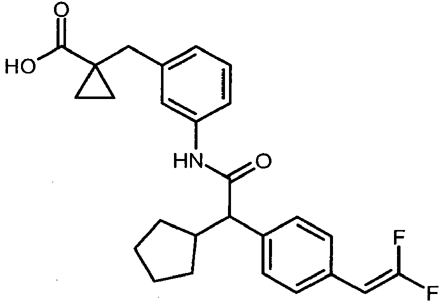 | ¹H-NMR (400 MHz, DMSO-*d₆*): δ [ppm] = 0.78-0.80 (m, 2H), 0.92-1.01 (m, 1H), 1.10-1.12 (m, 2H), 1.21-1.39 (m, 2H), 1.43-1.68 (m, 4H), 1.75-1.82 (m, 1H), 2.56-2.63 (m, 1H), 2.82 (s, 2H), 3.38 (d, 1H), 5.74 (dd, 1H), 6.90 (d, 1H), 7.15 (t, 1H), 7.30-7.34 (m, 2H), 7.40-7.47 (m, 4H), 9.99 (s, 1H), 12.09 (br. s, 1H). |
| | aus (+/-)-1-[3-({Cyclopentyl[4-(2,2-difluor-vinyl)phenyl]acetyl}amino)benzyl]cyclopropancarbonsäure-*tert*.-butylester | |
| **13** | (+/-)-1-(3-{[Cyclopentyl(4-vinylphenyl)acetyl]-amino}benzyl)cyclopropancarbonsäure | LC-MS (Methode 5): Rₜ = 1.24 min; m/z = 404 (M+H)⁺. |
| | 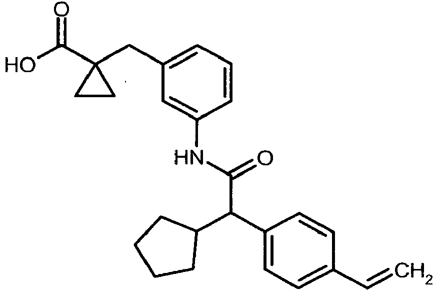 | ¹H-NMR (400 MHz, DMSO-*d₆*): δ [ppm] = 0.76-0.83 (m, 2H), 0.93-1.02 (m, 1H), 1.08-1.14 (m, 2H), 1.21-1.41 (m, 2H), 1.41-1.69 (m, 4H), 1.75-1.83 (m, 1H), 2.55-2.64 (m, 1H), 2.82 (s, 2H), 3.38 (d, 1H), 5.22 (d, 1H), 5.78 (d, 1H), 6.70 (dd, 1H), 6.90 (d, 1H), 7.15 (t, 1H), 7.34-7.51 (m, 6H), 9.98 (s, 1H), 12.08 (s, 1H). |
| | aus (+/-)-1-(3-{[Cyclopentyl(4-vinylphenyl)-acetyl]amino}benzyl)cyclopropancarbonsäure-*tert*.-butylester | |
| **14** | (+/-)-1-[3-({Cyclopentyl[4-(2,2-difluorethyl)-phenyl]acetyl}amino)benzyl]cyclopropancarbonsäure | LC-MS (Methode 4): Rt = 1.36 min; m/z = 442 (M+H)⁺. |
| | 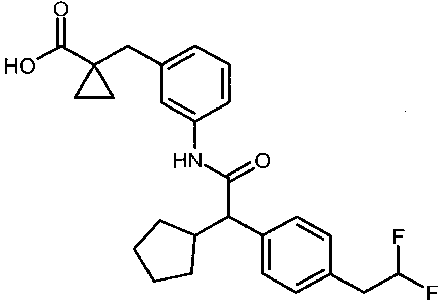 | ¹H-NMR (400 MHz, DMSO-*d₆*): δ [ppm] = 0.73-0.83 (m, 2H), 0.90-1.02 (m, 1H), 1.08-1.14 (m, 2H), 1.19-1.29 (m, 1H), 1.29-1.39 (m, 1H), 1.40-1.69 (m, 4H), 1.73-1.84 (m, 1H), 2.55-2.63 (m, 1H), 2.82 (s, 2H), 3.13 (td, 2H), 3.37 (d, 1H), 6.21 (dt, 1H), 6.90 (d, 1H), 7.14 (t, 1H), 7.21-7.27 (m, 2H), 7.32-7.40 (m, 2H), 7.40-7.49 (m, 2H), 9.98 (s, 1H), 12.08 (br. s, 1H). |
| | aus (+/-)-1-[3-({Cyclopentyl[4-(2,2-difluorethyl)-phenyl]acetyl}amino)benzyl]cyclopropancarbonsäure-*tert*.-butylester | |
| **15** | (+/-)-1-(3-{[(4-Cyanophenyl)(cyclopentyl)acetyl]-amino}-4-fluorbenzyl)cyclopropancarbonsäure | LC-MS (Methode 2): Rₜ = 2.37 min; m/z = 421 (M+H)⁺. |
| | 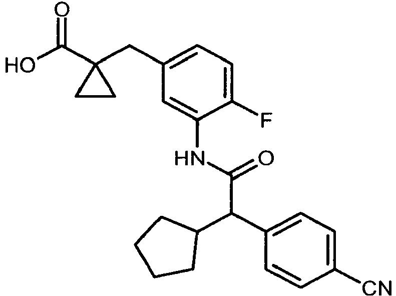 | ¹H-NMR (400 MHz, DMSO-*d₆*): δ [ppm] = 0.79 (q, 2H), 0.94 (dq, 1H), 1.05-1.14 (m, 2H), 1.23-1.74 (m, 7H), 1.74-1.89 (m, 1H), 2.80 (s, 2H), 3.72 (d, 1H), 6.98-7.05 (m, 1H), 7.06-7.16 (m, 1H), 7.57-7.69 (m, 3H), 7.76-7.85 (m, 2H), 9.89 (s, 1H), 12.12 (s, 1H). |
| | aus (+/-)-1-(3-{[(4-Cyanophenyl)(cyclopentyl)-acetyl]amino}-4-fluorbenzyl)cyclopropancarbonsäure-*tert*.-butylester | |
| **16** | (+/-)-1-(3-{[(4-Nitrophenyl)(cyclopentyl)acetyl]-amino}-4-fluorbenzyl)cyclopropancarbonsäure | LC-MS (Methode 4): Rₜ = 1.33 min; m/z = 441 (M+H)⁺. |
| | 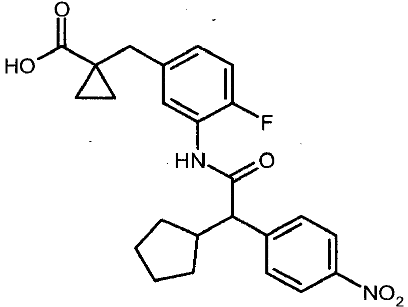 | ¹H-NMR (400 MHz, DMSO-*d₆*): δ [ppm] = 0.75-0.82 (m, 2H), 0.90-1.02 (m, 1H), 1.06-1.12 (m, 2H), 1.26-1.75 (m, 7H), 1.77-1.89 (m, 1H), 2.80 (s, 2H), 3.80 (d, 1H), 6.98-7.06 (m, 1H), 7.08-7.17 (m, 1H), 7.56-7.78 (m, 3H), 8.10-8.33 (m, 2H), 9.94 (s, 1H), 12.09 (br. s, 1H). |
| | aus (+/-)-1-(3-{[(4-Nitrophenyl)(cyclopentyl)-acetyl]amino}-4-fluorbenzyl)cyclopropan-carbonsäure-*tert*. -butylester | |
| **17** | 1-(3-{[(2*S*)-2-Cyclopentyl-2-(4-methylphenyl)-acetyl]amino}benzyl)cyclopropancarbonsäure | LC-MS (Methode 5): Rₜ = 1.22 min; m/z = 392 (M+H)⁺. |
| | 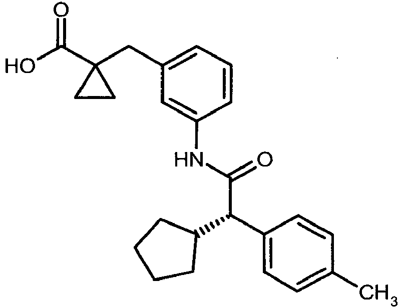 | ¹H-NMR (400 MHz, DMSO-*d₆*): δ [ppm] = 0.75-0.82 (m, 2H), 0.91-1.03 (m, 1H), 1.11 (q, 2H), 1.17-1.71 (m, 7H), 1.72-1.85 (m, 1H), 2.25 (s, 3H), 2.82 (s, 2H), 6.89 (d, 1H), 7.05-7.20 (m, 3H), 7.24-7.34 (m, 2H), 7.38-7.49 (m, 2H), 9.94 (s, 1H), 12.09 (br. s, 1H). |
| | aus 1-(3-{[(2*S*)-2-Cyclopentyl-2-(4-methyl-phenyl)acetyl]amino}benzyl)cyclopropancarbonsäure-*tert*. -butylester | |
| **18** | (+)-1-(3-{[(2*S*)-2-{4-[(*E*)-2-Cyanovinyl]phenyl}-2-cyclopentylacetyl]amino}-4-fluorbenzyl)-cyclopropancarbonsäure | LC-MS (Methode 5): Rₜ = 1.15 min; m/z = 447 (M+H)⁺. |
| | 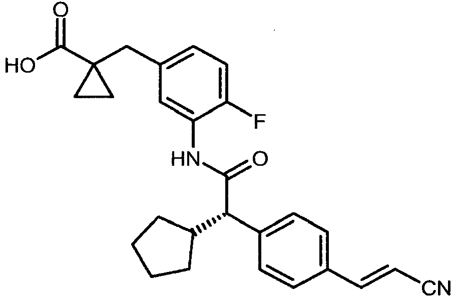 | ¹H-NMR (400 MHz, DMSO-*d₆*): δ [ppm] = 0.73-0.84 (m, 2H), 0.90-1.03 (m, 1H), 1.04-1.14 (m, 2H), 1.23-1.71 (m, 7H),1.80(dd, 1H), 2.80 (s, 2H), 6.42 (d, 1H), 6.97-7.05 (m, 1H), 7.06-7.16 (m, 1H), 7.48 (d, 2H), 7.58-7.70 (m, 4H), 9.76-9.88 (m, 1H), 12.12 (br. s, 1H). |
| | aus (+)-1-(3-{[(2*S*)-2-{4-[(*E*)-2-Cyanovinyl]-phenyl}-2-cyclopentylacetyl]amino}-4-fluorbenzyl)cyclopropancarbonsäure-*tert*.-butylester | [α]_{D}²⁰ = +94.1°, c = 0.505, Chloroform. |
| **19** | (+)-1-(3-{[(2*S*)-2-Cyclopentyl-2-(4-ethylphenyl)-acetyl]amino}benzyl)cyclopropancarbonsäure | LC-MS (Methode 5): Rt = 1.28 min; m/z = 406 (M+H)⁺. |
| | 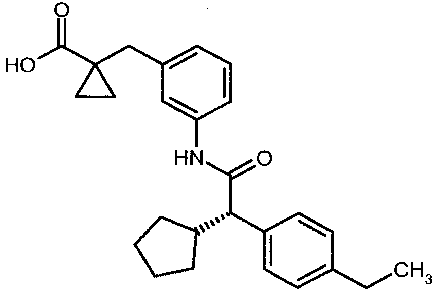 | ¹H-NMR (400 MHz, DMSO-*d₆*): δ [ppm] = 0.75-0.82 (m, 2H), 0.90-1.03 (m, 1H), 1.08-1.13 (m, 2H), 1.15 (t, 3H), 1.19-1.30 (m, 1H), 1.30-1.40 (m, 1H), 1.40-1.69 (m, 4H), 1.71-1.83 (m, 1H), 2.51-2.61 (m, 3H), 2.82 (s, 2H), 3.35 (d, 1H, verdeckt), 6.89 (d, 1H), 7.09-7.20 (m, 3H), 7.31 (d, 2H), 7.39-7.48 (m, 2H), 9.94 (s, 1H), 12.08 (br. s, 1H). |
| | aus (+)-1-(3-{[(2*S*)-2-Cyclopentyl-2-(4-ethylphenyl)acetyl]amino}benzyl)cyclopropancarbonsäure-*tert*. -butylester | |
| | | [α]_{D}²⁰ = +44.9°, c = 0.340, Chloroform. |
| **20** | (+)-1-(3-{[(2*S*)-2-Cyclopentyl-2-(4-ethylphenyl)-acetyl]amino}-4-fluorbenzyl)cyclopropancarbonsäure | LC-MS (Methode 4): Rₜ = 1.47 min; m/z = 424 (M+H)⁺. |
| | 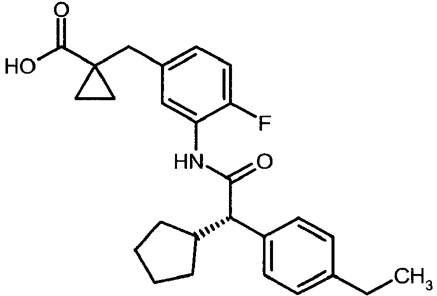 | ¹H-NMR (400 MHz, DMSO-*d₆*): δ [ppm] = 0.74-0.83 (m, 2H), 0.89-1.03 (m, 1H), 1.07-1.10 (m, 2H), 1.16 (t, 3H), 1.22-1.40 (m, 2H), 1.40-1.69 (m, 4H), 1.72-1.85 (m, 1H), 2.53-2.63 (m, 3H), 2.80 (s, 2H), 3.54 (d, 1H), 6.95-7.04 (m, 1H), 7.05-7.11 (m, 1H), 7.11-7.18 (m, 2H), 7.28-7.35 (m, 2H), 7.67 (dd, 1H), 9.73 (s, 1H), 12.12 (s, 1H). |
| | aus (+)-1-(3-{[(2*S*)-2-Cyclopentyl-2-(4-ethylphenyl)acetyl]amino}-4-fluorbenzyl)cyclopropancarbonsäure-*tert*.-butylester | [α]_{D}²⁰ = +80.9°, c = 0.505, Chloroform. |
| **21** | (+)-1-(3-{[(2*S*)-2-Cyclopentyl-2-(4-ethylphenyl)-acetyl]amino}-5-fluorbenzyl)cyclopropan-carbonsäure | LC-MS (Methode 3): Rₜ = 2.86 min; m/z = 424 (M+H)⁺. |
| | 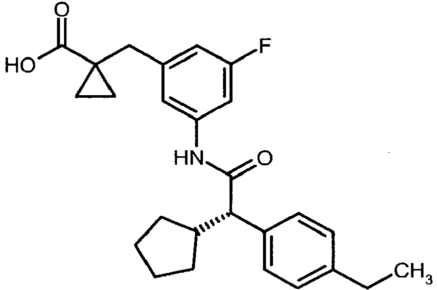 | ¹H-NMR (400 MHz, DMSO-*d₆*): δ [ppm] = 0.79-0.87 (m, 2H), 0.90-1.04 (m, 1H), 1.10- 1.16 (m, 2H), 1.15 (t, 3 H), 1.19-1.29 (m, 1H), 1.31-1.39 (m, 1H), 1.40-1.69 (m, 4H), 1.71-1.85 (m, 1H), 2.52-2.62 (m, 3H), 2.81 (s, 2H), 3.34 (d, 1H, verdeckt), 6.72 (d, 1H), 7.05-7.17 (m, 3H), 7.30 (d, 2H), 7.47 (d, 1H), 10.17 (s, 1H), 12.15 (br. s, 1H). |
| | aus (+)-1-(3-{[(2*S*)-2-Cyclopentyl-2-(4-ethyl-phenyl)acetyl]amino}-5-fluorbenzyl)cyclopropan-carbonsäure-*tert*.-butylester | [α]_{D}²⁰ = +42.5°, c = 0.305, Chloroform. |
| **22** | (+)-1-(4-Fluor-3-{[(2*S*,3*R*)-4,4,4-trifluor-2-(4-fluorphenyl)-3-methylbutanoyl]amino}-benzyl)cyclopropan carbonsäure | LC-MS (Methode 5): Rₜ = 1.12 min; m/z = 442 (M+H)⁺. |
| | 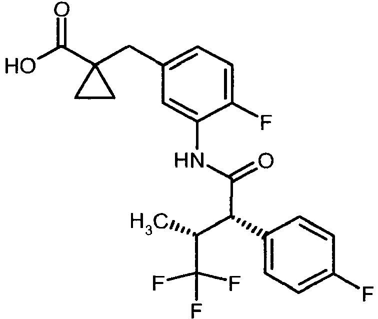 | ¹H-NMR (400 MHz, DMSO-*d₆*): δ [ppm] = 0.76-0.82 (m, 5H), 1.09 (br. s, 2H), 2.80 (s, 2H), 3.33-3.41 (m, 1H), 4.10 (d, 1H), 7.01 (br. s, 1H), 7.06-7.14 (m, 1H), 7.21 (t, 2H), 7.47 (t, 2H), 7.68 (d, 1H), 9.99 (s, 1H), 12.12 (br. s, 1H). |
| | aus (+)-1-(4-Fluor-3-{[(2*S*,3*R*)-4,4,4-trifluor-2-(4-fluorphenyl)-3-methylbutanoyl]amino}-benzyl)cyclopropancarbonsäure-*tert*.-butylester | [α]_{D}²⁰ = +113.3°, c = 0.53, Chloroform. |
| **23** | (+)-1-(3-{[(2*S*,3*R*)-2-(4-Chlorphenyl)-4,4,4-trifluor-3-methylbutanoyl]amino}-2-fluorbenzyl)-cyclopropancarbonsäure | LC-MS (Methode 5): Rₜ = 1.19 min; m/z = 458 (M+H)⁺. |
| | 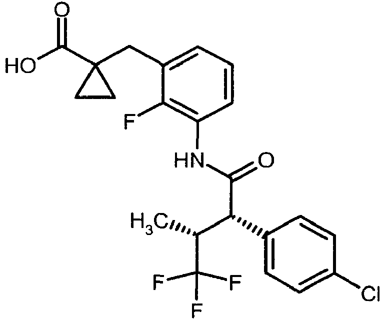 | ¹H-NMR (400 MHz, DMSO-*d₆*): δ [ppm] = 0.71-0.88 (m, 5H), 1.08-1.21 (m, 2H), 2.85-2.99 (m, 2H), 4.12 (d, 1H), 6.95-7.05 (m, 1H), 7.07-7.16 (m, 1H), 7.37-7.51 (m, 4H), 7.62 (td, 1H), 10.03 (s, 1H), 12.19 (s, 1H). |
| | aus (+)-1-(3-{[(2*S*,3*R*)-2-(4-Chlorphenyl)-4,4,4-trifluor-3-methylbutanoyl]amino}-2-fluorbenzyl)-cyclopropancarbonsäure-*tert*.-butylester | [α]_{D}²⁰ = +32.1°, c = 0.50, Chloroform. |
| **24** | (+)-1-(3- {[(2*S*,3*R*)-2-(4-Ethylphenyl)-4,4,4-trifluor-3-methylbutanoyl]amino}-2-fluorbenzyl)-cyclopropancarbonsäure | LC-MS (Methode 5): Rₜ = 1.23 min; m/z = 452 (M+EI)⁺. |
| | 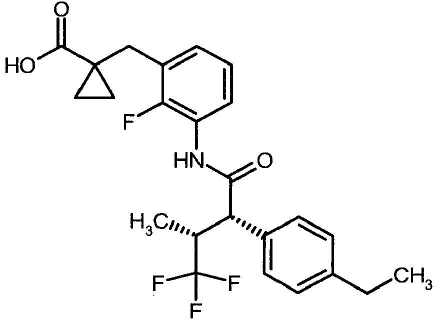 | ¹H-NMR (400 MHz, DMSO-d₆): δ [ppm] = 0.75-0.79 (m, 5H), 1.06-1.14 (m, 2H), 1.17 (t, 3H), 2.58 (q, 2H), 2.90 (s, 2H), 3.32-3.39 (m, 1H, verdeckt), 4.05 (d, 1 H), 6.96-7.06 (m, 1H), 7.06-7.12 (m, 1H), 7.16-7.23 (m, 2H), 7.31-7.37 (m, 2H), 7.64 (t, 1H), 9.96 (s, 1H), 12.19 (br. s, 1H). |
| | aus (+)-1-(3-{[(2*S*,3*R*)-2-(4-Ethylphenyl)-4,4,4-trifluor-3-methylbutanoyl]amino}-2-fluorbenzyl)-cyclopropancarbonsäure-*tert*.-butylester | [α]_{D}²⁰ = +40.4°, c = 0.375, Chloroform. |
| **25** | (+)-1-(3-{[(2*S*,3*R*)-2-(4-Ethylphenyl)-4,4,4-trifluor-3-methylbutanoyl]amino}-4-fluorbenzyl)-cyclopropancarbonsäure | LC-MS (Methode 5): Rₜ = 1.23 min; m/z = 452 (M+H)⁺. |
| | 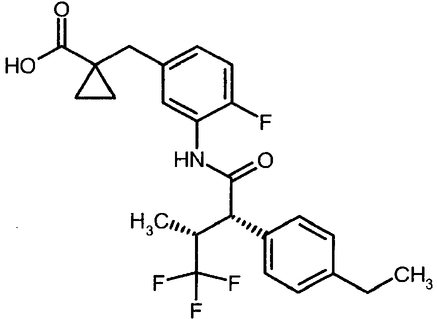 | ¹H-NMR (400 MHz, DMSO-*d₆*): δ [ppm] = 0.71-0.82 (m, 5H), 1.04-1.11 (m, 2H), 1.17 (t, 3H), 2.58 (q, 2H), 2.80 (s, 2H), 3.33-3.39 (m, 1H), 4.03 (d, 1H), 6.94-7.03 (m, 1H), 7.10 (dd, 1H), 7.17-7.24 (m, 2H), 7.29-7.38 (m, 2H), 7.69 (dd, 1H), 9.94 (s, 1H), 12.12 (s, 1H). |
| | aus (+)-1-(3-{[(2*S*,3*R*)-2-(4-Ethylphenyl)-4,4,4-trifluor-3-methylbutanoyl]amino}-4-fluorbenzyl)-cyclopropancarbonsäure-*tert*.-butylester | [α]_{D}²⁰ =+129.3°, c = 0.475, Chloroform. |
| **26** | (+)-1-(2-Chlor-5-{[(2*S*,3*R*)-2-(4-chlorphenyl)-4,4,4-trifluor-3-methylbutanoyl]amino}benzyl)-cyclopropancarbonsäure | LC-MS (Methode 4): Rₜ = 1.45 min; m/z = 474/475 (M+H)⁺. |
| | 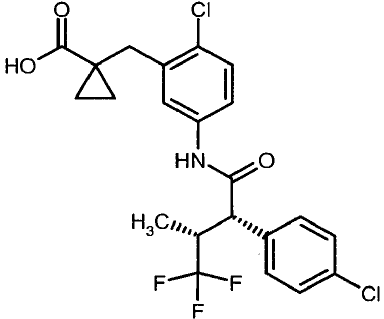 | ¹H-NMR (400 MHz, DMSO-*d₆*): δ [ppm] = 0.67-0.85 (m, 5H), 1.20-1.22 (m, 2H), 2.84-3.04 (m, 2H), 3.35-3.50 (m, 1H), 3.84 (d, 1H), 7.31 (d, 1H), 7.41-7.48 (m, 4H), 7.50 (d, 1H), 7.57 (dd, 1H), 10.37 (s, 1H), 12.22 (br. s, 1H). |
| | aus 1-(2-Chlor-5-{[(2*S*,3*R*)-2-(4-chlorphenyl)-4,4,4-trifluor-3-methylbutanoyl]amino}benzyl)-cyclopropancarbonsäure-*tert*.-butylester | [α]_{D}²⁰ = +72.7°, c = 0.510, Chloroform. |
| **27** | (+)-1-(2-Chlor-3-{[(2*S*,3*R*)-2-(4-chlorphenyl)-4,4,4-trifluor-3-methylbutanoyl]amino}benzyl)-cyclopropancarbonsäure | LC-MS (Methode 5): Rₜ = 1.20 min; m/z = 472 (M-H)⁻. |
| | 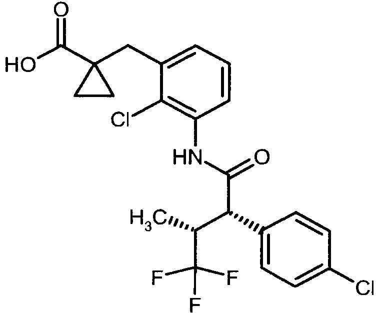 | ¹H-NMR (400 MHz, DMSO-d₆): δ [ppm] = 0.73 (br. s, 2H), 0.80 (d, 3H), 1.18 (br. s, 2H), 2.99 (br. s, 2H), 3.35-3.45 (m, 1H, verdeckt), 4.12 (d, 1H), 7.10-7.27 (m, 2H), 7.34 (d, 1H), 7.39-7.53 (m, 4H), 9.83 (s, 1H). |
| | aus (+)-1-(2-Chlor-3-{[(2*S*,3*R*)-2-(4-chlorphenyl)-4,4,4-trifluor-3-methylbutanoyl]amino}benzyl)-cyclopropancarbonsäure-*tert*.-butylester | [α]_{D}²⁰ = +1.5°, c = 0.415, Chloroform. |
| **28** | 1-(6-Chlor-3-{[(2*S*,3*R*)-2-(4-chlorphenyl)-4,4,4-trifluor-3-methylbutanoyl]amino}-2-fluorbenzyl)-cyclopropancarbonsäure | LC-MS (Methode 5): Rₜ = 1.24 min; m/z = 492/494 (M+H)⁺. |
| | 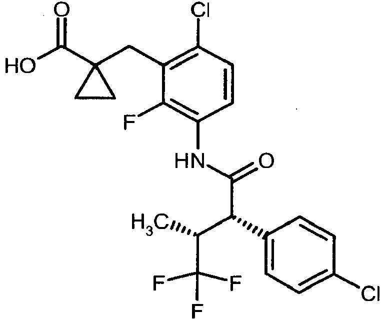 | ¹H-NMR (400 MHz, DMSO-*d₆*): δ [ppm] = 0.40 (br. s, 2H), 0.78 (d, 3H), 0.98-1.09 (m, 2H), 3.28 (s, 2H), 4.11 (d, 1H), 7.24 (d, 1H), 7.45 (s, 4H), 7.58-7.76 (m, 1H), 10.19 (s, 1H), 12.38 (br. s, 1H). |
| | aus 1-(6-Chlor-3-{[(2*S*,3*R*)-2-(4-chlorphenyl)-4,4,4-trifluor-3-methylbutanoyl]amino}-2-fluorbenzyl)cyclopropancarbonsäure-*tert*.-butylester | [α]_{D}²⁰ = +31.0°, c = 0.285, Chloroform. |
| 29 | (+)-1-(4,6-Dichlor-3-{[(2*S*,3*R*)-2-(4-chlorphenyl)-4,4,4-trifluor-3-methylbutanoyl]amino}-2-fluorbenzyl)cyclopropancarbonsäure | LC-MS (Methode 5): Rₜ = 1.22 min; m/z = 526/528 (M+H)⁺. |
| | 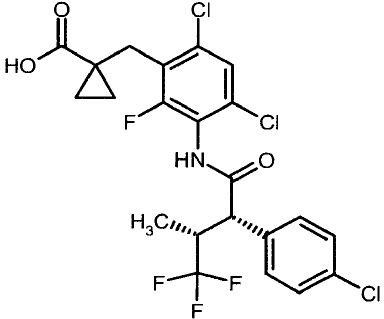 | ¹H-NMR (400 MHz, DMSO-d₆): δ [ppm] = 0.37 (br. s, 2H), 0.79 (d, 3H), 0.95-1.04 (m, 2H), 3.25 (s, 2H), 3.96 (d, 1H), 7.42-7.45 (m, 4H), 7.57 (d, 1H), 10.16 (s, 1H), 12.42 (br. s, 1H). |
| | aus 1-(4,6-Dichlor-3-{[(2*S*,3*R*)-2-(4-chlorphenyl)-4,4,4-trifluor-3-methylbutanoyl]amino}-2-fluorbenzyl)cyclopropancarbonsäure-*tert*.-butylester | [α]_{D}²⁰ = +27.0°, c = 0.210, Chloroform. |
| **30** | (+)-1-(4-Chlor-3-{[(2*S*,3*R*)-2-(4-chlor-3-fluorphenyl)-4,4,4-trifluor-3-methylbutanoyl]amino}-benzyl)cyclopropancarbonsäure | LC-MS (Methode 5): Rₜ = 1.19 min; m/z = 492/494 (M+H)⁺. |
| | 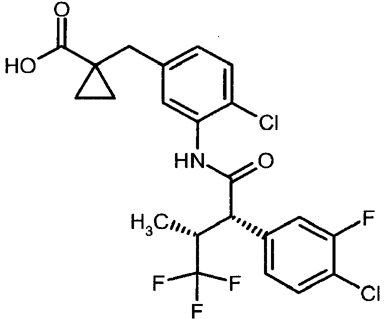 | ¹H-NMR(400MHz, DMSO-d₆): δ [ppm] = 0.69-0.87 (m, 5H), 1.05-1.14 (m, 2H), 2.82 (s, 2H), 3.36-3.45 (m, 1H), 4.04-4.19 (m, 1H), 7.09 (dd, 1H), 7.27-7.37 (m, 2H), 7.40-7.45 (m, 1H), 7.49 (dd, 1H), 7.62 (t, 1 H), 9.87 (s, 1H), 12.15 (br. s, 1H). |
| | aus (+)-1-(4-Chlor-3-{[(2*S*,3*R*)-2-(4-chlor-3-fluorphenyl)-4,4,4-trifluor-3-methylbutanoyl]amino}-benzyl)cyclopropancarbonsäure-*tert*.-butylester | [α]_{D}²⁰ = +73°, c = 0.300, Chloroform. |
| **31** | (+)-1-(2-Chlor-5-{[(2*S*,3*R*)-2-(4-chlorphenyl)-4,4,4-trifluor-3-methylbutanoyl]amino}-4-fluorbenzyl)cyclopropancarbonsäure | LC-MS (Methode 4): Rₜ = 1.44 min; m/z = 491/493 (M+H)⁺. |
| | 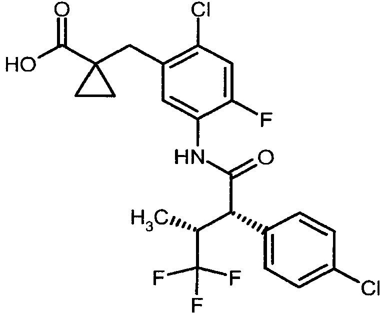 | ¹H-NMR (400 MHz, DMSO-*d₆*): δ [ppm] = 0.73 (q, 2H), 0.78 (d, 3H), 1.15 (q, 2H), 2.87-3.03 (m, 2H), 3.35-3.44 (m, 1H), 4.06-4.12(m, 1H), 7.36-7.48 (m, 5H), 7.90 (d, 1H), 10.15 (s, 1H), 12.26 (br. s, 1H). |
| | aus (+)-1-(2-Chlor-5-{[(2*S*,3*R*)-2-(4-chlorphenyl)-4,4,4-trifluor-3-methylbutanoyl]amino}-4-fluorbenzyl)cyclopropancarbonsäure-*tert*.-butylester | [α]_{D}²⁰ = +131.1°, c = 0.500, Chloroform. |
| **32** | (+)-1-(4-Chlor-3-{[(2*S*,3*R*)-2-(4-chlor-2-fluorphenyl)-4,4,4-trifluor-3-methylbutanoyl]amino}-benzyl)cyclopropancarbonsäure | LC-MS (Methode 5): Rₜ = 1.23 min; m/z = 490 (M-H)⁻. |
| | 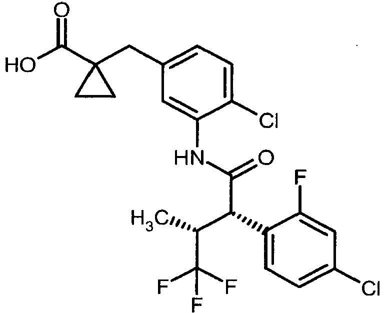 | ¹H-NMR (400 MHz, DMSO-*d₆*): δ [ppm] = 0.78-0.80 (m, 2H), 0.86 (d, 3H), 1.03-1.15 (m, 2H), 2.83 (s, 2H), 3.36-3.45 (m, 1H), 4.35 (d, 1H), 7.11 (dd, 1H), 7.30-7.38 (m, 3H), 7.52 (dd, 1H), 7.61 (t, 1H), 10.01 (s, 1H), 12.20 (br. s, 1H). |
| | aus (+)-1-(4-Chlor-3-{[(2*S*,3*R*)-2-(4-chlor-2-fluorphenyl)-4,4,4-trifluor-3-methylbutanoyl]amino}-benzyl)cyclopropancarbonsäure-*tert*.-butylester | [α]_{D}²⁰ = +24.1 °, c = 0.310, Chloroform. |
| **33** | 1-(3-{[(2*S*,3*R*)-2-(4-Chlorphenyl)-4,4,4-trifluor-3-methylbutanoyl]amino}-4-methylbenzyl)-cyclopropancarbonsäure | LC-MS (Methode 4): Rₜ = 1.34 min; m/z = 452/454 (M-H)-. |
| | 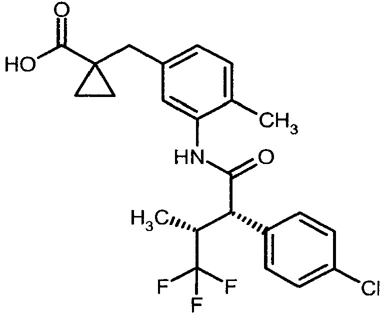 | ¹H-NMR (400 MHz, DMSO-*d₆*): δ [ppm] = 0.70-0.77 (m, 2H), 0.80 (d, 3H), 1.02-1.10 (m, 2H), 1.94 (s, 3H), 2.80 (s, 2H), 3.31-3.44 (m, 1H, teilweise verdeckt durch H₂O-Signal), 3.95 (d, 1H), 6.93-7.00 (m, 1H), 7.01.-7.08 (m, 2H), 7.38-7.52 (m, 4H), 9.59 (s, 1H), 12.06 (s, 1H). |
| | aus *tert*.-Butyl-1-(3-{[(2*S*,3*R*)-2-(4-chlorphenyl)-4,4,4-trifluor-3-methylbutanoyl]amino}-4-methylbenzyl)cyclopropancarboxylat | |
| **34** | 1-(3-{[(4-Chlorphenyl)(3,3-difluorcyclopentyl)-acetyl]amino}-4-fluorbenzyl)cyclopropancarbonsäure (*Diastereomerengemisch*) | LC-MS (Methode 4): Rₜ = 1.37 min; m/z = 464/466 (M-H)⁻. |
| | 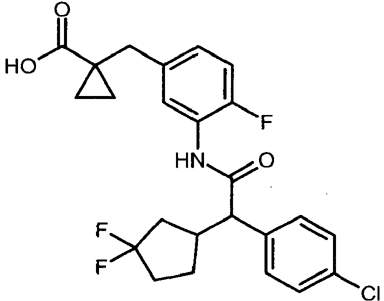 | ¹H-NMR (400 MHz, DMSO-*d₆*): δ [ppm] = 0.75-0.82 (m, 2H), 1.06-1.13 (m, 2H), 1.20-1.34 (m, 0.5H), 1.43-1.69 (m, 1.5H), 1.79-2.39 (m, 4H), 2.75-2.93 (m, 1H), 2.81 (s, 2H), 3.76 (t, 1H), 6.98-7.07 (m, 1H), 7.07-7.16 (m, 1H), 7.36-7.50 (m, 4H), 7.59-7.67 (m, 1H), 9.89 (s, 0.5H), 9.94 (s, 0.5H), 11.85-12.40 (br. s, 1H). |
| | aus *tert.*-Butyl-1-(3-{[(4-chlorphenyl)(3,3-difluorcyclopentyl)acetyl]amino}-4-fluorbenzyl)-cyclopropancarboxylat | |
| **35** | 1-(3-{[(4-Chlorphenyl)(3,3-difluorcyclopentyl)-acetyl]amino}-4-chlorbenzyl)cyclopropancarbonsäure (*Diastereomerengemisch*) | LC-MS (Methode 5): Rₜ = 1.21 min; m/z = 480/482 (M-H)⁻. |
| | 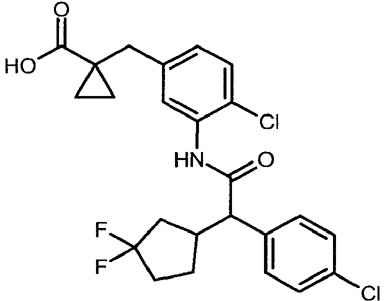 | |
| | aus *tert*.-Butyl-1-(3-{[(4-chlorphenyl)(3,3-difluorcyclopentyl)acetyl]amino}-4-chlorbenzyl)-cyclopropancarboxylat | |
| **36** | 1-[3-({4,4,4-Trifluor-3-methyl-2-[4-(trifluormethyl)phenyl]butanoyl}amino)benzyl]-cyclopropancarbonsäure | LC-MS (Methode 4): Rₜ = 1.40 min; m/z = 472 (M-H)-. |
| | 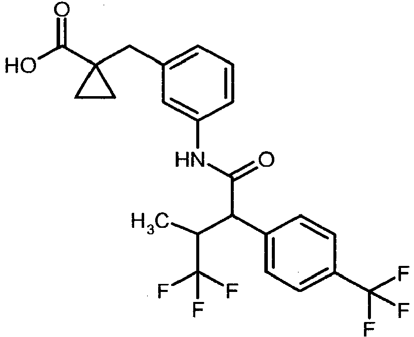 | ¹H-NMR (400 MHz, DMSO-*d₆*): δ [ppm] = 0.71-0.87 (m, 4.6H), 1.07-1.15 (m, 2H), 1.23 (d, 0.4H), 2.82 (s, 2H) 3.37-3.53 (m, 0.95H), 3.63-3.77 (m, 0.05H), 3.93-4.04 (m, 1H), 6.89-6.98 (m, 1H), 7.12-7.22 (m, 1H), 7.34-7.38 (m, 1H), 7.39-7.48 (m, 1H), 7.62-7.80 (m, 4H), 10.26 (s, 0.82H), 10.35 (s, 0.18H), 12.09 (br. s, 1H). |
| | aus *tert*.-Butyl-1-[3-({4,4,4-trifluor-3-methyl-2-[4-(trifluormethyl)phenyl]butanoyl}amino)-benzyl]cyclopropancarboxylat | |
| **37** | 1-(4-Fluor-3-{[(2*S*,3*R*)-4,4,4-trifluor-2-(4-isopropylphenyl)-3-methylbutanoyl]amino}-benzyl)cyclopropancarbonsäure | LC-MS (Methode 2): Rₜ = 2.70 min; m/z = 466 (M+H)⁺. |
| | 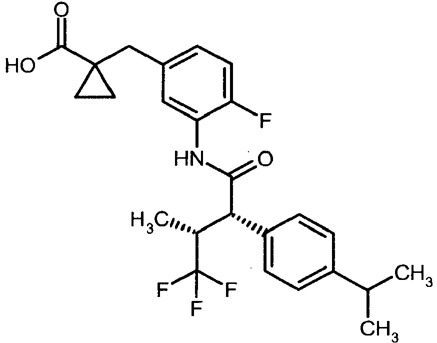 | ¹H-NMR (400 MHz, DMSO-*d₆*): δ [ppm] = 0.72-0.82 (m, 5H), 1.05-1.12 (m, 2H), 1.19 (d, 6H), 2.81 (s, 2H), 2.82-2.93 (m, 1H), 3.24-3.40 (m, 1H), 4.04 (d, 1H), 6.95-7.03 (m, 1H), 7.05-7.14 (m, 1H), 7.23 (d, 2H), 7.34 (d, 2H), 7.71 (d, 1H), 9.93 (s, 1H), 11.76-12.47 (br. s, 1H). |
| | aus *tert*.-Butyl-l-(4-fluor-3-{[(2*S*,3*R*)-4,4,4-trifluor-2-(4-isopropylphenyl)-3-methylbutanoyl]-amino}benzyl)cyclopropancarboxylat | |
| **38** | 1-[3-({(2*S*,3*R*)-2-[4-(1,1-Difluorethyl)phenyl]-4,4,4-trifluor-3-methylbutanoyl}amino)-4-fluorbenzyl]cyclopropancarbonsäure | LC-MS (Methode 5): Rₜ = 1.18 min; m/z = 488 (M+H)⁺. |
| | 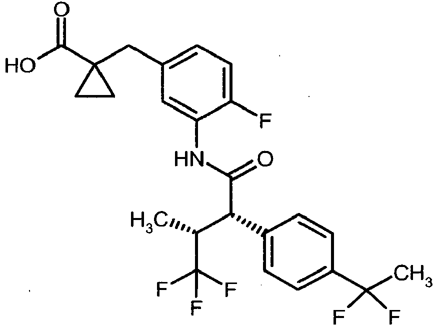 | ¹H-NMR (400 MHz, DMSO-*d₆*): δ [ppm] = 0.71-0.83 (m, 5H), 1.05-1.13 (m, 2H), 1.96 (t, 3H), 2.81 (s, 2H), 3.33-3.47 (m, 1H), 4.16 (d, 1H), 6.97-7.04 (m, 1H), 7.06-7.14 (m, 1H), 7.51-7.61 (m, 4H), 7.69 (dd, 1H), 10.02 (s, 1H), 12.10 (s, 1H). |
| | aus *tert*.-Butyl-1-[3-({(2*S*,3*R*)-2-[4-(1,1-difluorethyl)phenyl]-4,4,4-trifluor-3-methylbutanoyl}-amino)-4-fluorbenzyl]cyclopropancarboxylat | |
| **39** | 1-(3-{[(2*S*,3*R*)-2-(4-*tert*-Butylphenyl)-4,4,4-trifluor-3-methylbutanoyl]amino}-4-fluorbenzyl)-cyclopropancarbonsäure | LC-MS (Methode 5): Rₜ = 1.29 min; m/z = 480 (M+H)⁺. |
| | 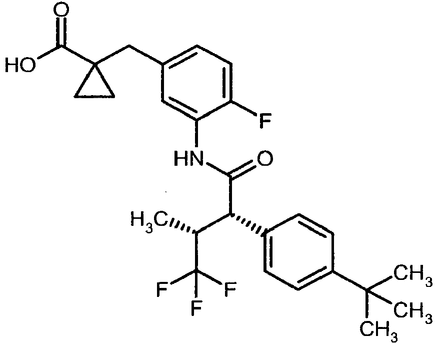 | ¹H-NMR (400 MHz, DMSO-*d₆*): δ [ppm] = 0.72-0.82 (m, 5H), 1.06-1.14 (m, 2H), 1.27 (s, 9H), 2.81 (s, 2H), 3.25-3.57 (m, 1H, teilweise verdeckt durch H₂O-Signal), 4.05 (d, 1H), 6.96-7.03 (m, 1H), 7.06-7.13 (m, 1H), 7.37 (q, 4H), 7.72 (dd, 1H), 9.94 (s, 1H), 11.84-12.42 (br. s, 1H). |
| | aus *tert.*-Butyl-1-(3-{[(2*S*,3*R*)-2-(4-*tert*.-butylphenyl)-4,4,4-trifluor-3-methylbutanoyl]amino}-4-fluorbenzyl)cyclopropancarboxylat | |
| **40** | 1-(3-{[(2*S*,3*R*)-2-(4-Cyclopropylphenyl)-4,4,4-trifluor-3-methylbutanoyl]amino}-4-fluorbenzyl)-cyclopropancarbonsäure | LC-MS (Methode 5): Rₜ = 1.23 min; m/z = 464 (M+H)⁺. |
| | 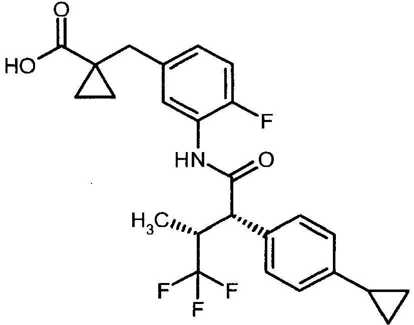 | ¹H-NMR (400 MHz, DMSO-*d₆*): δ [ppm] = 0.62-0.69 (m, 2H), 0.71-0.82 (m, 5H), 0.89-0.96 (m, 2H), 1.05-1.12 (m, 2H), 1.83-1.94 (m, 1H), 2.80 (s, 2H), 3.24-3.37 (m, 1H, teilweise verdeckt durch H₂O-Signal 4.01 (d, 1H), 6.96-7.02 (m, 1H), 7.03-7.13 (m, 3H), 7.29 (d, 2H), 7.68 (dd, 1H), 9.91 (s, 1H), 12.10 (s, 1H). |
| | aus *tert.*-Butyl-1-(3-{[(2*S*,3*R*)-2-(4-cyclopropylphenyl)-4,4,4-trifluor-3-methylbutanoyl]amino}-4-fluorbenzyl)cyclopropancarboxylat | |
| **41** | 1-[4-Chlor-3-({(2*S*,3*R*)-4,4,4-trifluor-3-methyl-2-[4-(trifluormethyl)phenyl]butanoyl}amino)-benzyl]cyclopropancarbonsäure | LC-MS (Methode 2): Rₜ = 2.62 min; m/z = 506/508 (M-H)⁻. |
| | 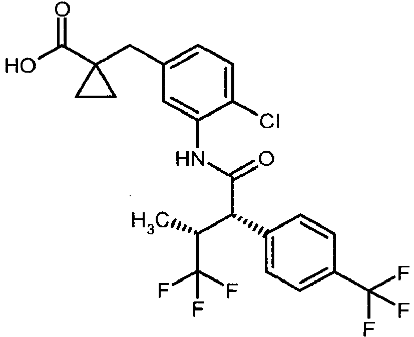 | ¹H-NMR (400 MHz, DMSO-*d₆*): δ [ppm] = 0.74-0.84 (m, 5H), 1.05-1.13 (m, 2H), 2.82 (s, 2H), 3.25-3.53 (m, 1H, teilweise verdeckt durch H₂O-Signal), 4.23 (d, 1H), 7.08 (dd, 1H), 7.34 (d, 1H), 7.43 (d, 1H), 7.68 (d, 2H), 7.77 (d, 2H), 9.88 (s, 1H), 11.95-12.34 (br. s, 1H). |
| | aus *tert*.-Butyl-1-[4-chlor-3-({(2*S*,3*R*)-4,4,4-trifluor-3-methyl-2-[4-(trifluormethyl)phenyl]-butanoyl}amino)benzyl]cyclopropancarboxylat | |
| **42** | 1-(3-{[(2*S*,3*R*)-2-(4-Chlor-3-methoxyphenyl)-4,4,4-trifluor-3-methylbutanoyl]amino}-4-fluorbenzyl)cyclopropancarbonsäure | LC-MS (Methode 5): Rₜ = 1.12 min; m/z = 486/488 (M-H)⁻. |
| | 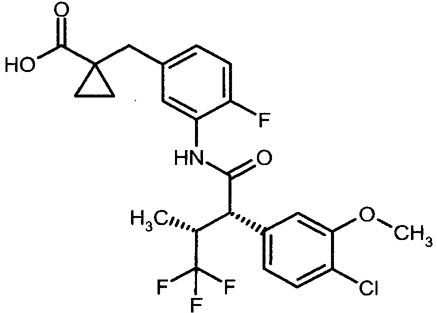 | ¹H-NMR (400 MHz, DMSO-*d₆*): δ [ppm] = 0.74-0.85 (m, 5H), 1.05-1.12 (m, 2H), 2.81 (s, 2H), 3.31-3.53 (m, 1H, verdeckt durch H₂O-Signal), 3.87 (s, 3H), 4.06 (d, 1H), 6.97-7.05 (m, 2H), 7.07-7.14 (m, 1H), 7.21 (d, 1H), 7.42 (d, 1H), 7.66 (dd, 1H), 9.99 (s, 1H), 11.91-12.37 (br. s, 1H). |
| | aus *tert.*-Butyl-1-(3-{[(2*S*,3*R*)-2-(4-chlor-3-methoxyphenyl)-4,4,4-trifluor-3-methylbutanoyl]-amino}-4-fluorbenzyl)cyclopropancarboxylat | |
| **43** | 1-(4-Chlor-3-{[(2*S*,3*R*)-2-(4-chlor-3-methoxyphenyl)-4,4,4-trifluor-3-methylbutanoyl]amino}-benzyl)cyclopropancarbonsäure | LC-MS (Methode 4): Rₜ = 1.39 min; m/z = 502/504 (M-H)⁻. |
| | 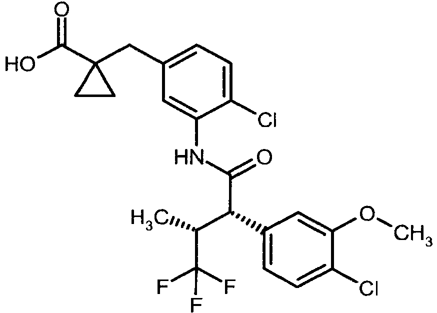 | ¹H-NMR (400 MHz, DMSO-*d₆*): δ [ppm] = 0.75-0.88 (m, 5H), 1.05-1.14 (m, 2H), 2.82 (s, 2H), 3.34-3.49 (m, 1H), 3.87 (s, 3H), 4.08 (d, 1H), 7.02 (dd, 1H), 7.07 (dd, 1H), 7.23 (d, 1H), 7.34 (d, 1H), 7.40-7.46 (m, 2H), 9.81 (s, 1H), 11.61-12.62 (br. s, 1H). |
| | aus *tert*.-Butyl-1-(4-chlor-3-{[(2*S*,3*R*)-2-(4-chlor-3-methoxyphenyl)-4,4,4-trifluor-3-methyl-butanoyl]amino}benzyl)cyclopropancarboxylat | |
| **44** | 1-[4-Chlor-3-({(2*S*,3*R*)-4,4,4-trifluor-3-methyl-2-[4-(1,1,1-trifluor-2-methylpropan-2-yl)phenyl]-butanoyl}amino)benzyl]cyclopropancarbonsäure | LC-MS (Methode 5): Rₜ = 1.29 min; m/z = 548/550 (M-H)⁻. |
| | 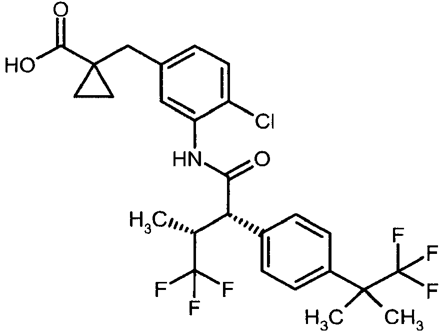 | |
| | aus *tert*.-Butyl-1-[4-chlor-3-({(2*S*,3*R*)-4,4,4-trifluor-3-methyl-2-[4-(1,1,1-trifluor-2-methylpropan-2-yl)phenyl]butanoyl}amino)benzyl]-cyclopropancarboxylat | |
| **45** | 1-(3-{[2-(4-Chlorphenyl)-4,4,4-trifluor-3-methyl-butanoyl]amino}benzyl)cyclopropancarbonsäure | LC-MS (Methode 4): Rₜ = 1.37 min; m/z = 438/440 (M-H)⁻. |
| | 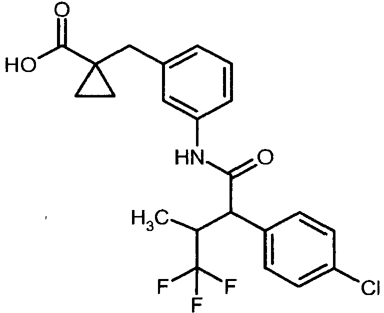 | ¹H-NMR (400 MHz, DMSO-*d₆*): δ [ppm] = 0.75-0.83 (m, 5H), 1.08-1.15 (m, 2H), 2.82 (s, 2H), 3.28-3.46 (m, 1H, verdeckt durch H₂O-Signal), 3.85 (d, 1H), 6.93 (d, 1H), 7.16 (t, 1H), 7.34-7.51 (m, 6H), 10.20 (s, 1H), 11.60-12.40 (br. s, 1H). |
| | aus *tert*.-Butyl-1-(3-{[2-(4-chlorphenyl)-4,4,4-trifluor-3-methylbutanoyl]amino}benzyl)-cyclopropancarboxylat | |
| **46** | 1-[4-Chlor-3-({4,4,4-trifluor-3-methyl-2-[4-(2,2,2-trifluorethyl)phenyl]butanoyl}amino)benzyl]-cyclopropancarbonsäure | LC-MS (Methode 5): Rₜ = 1.20 min; m/z = 520/522 (M-H)⁻. |
| | 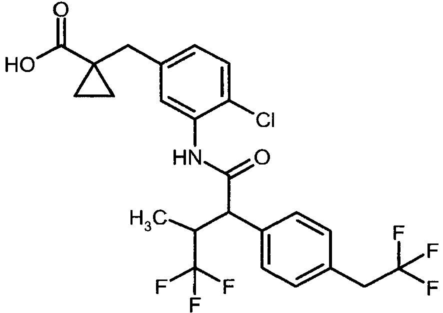 | ¹H-NMR (400 MHz, DMSO-*d₆*): δ [ppm] = 0.74-0.83 (m, 5H), 1.04-1.14 (m, 2H), 2.82 (s, 2H), 3.27-3.43 (m, 1H), 3.43-3.76 (q, 2H, teilweise verdeckt durch H₂O-Signal), 4.10 (d, 1H), 7.07 (dd, 1H), 7.30-7.39 (m, 3H), 7.41-7.48 (m, 3H), 9.79 (s, 1H), 11.60-12.60 (br. s, 1H). |
| | aus *tert*.-Butyl-1-[4-chlor-3-({4,4,4-trifluor-3-methyl-2-[4-(2,2,2-trifluorethyl)phenyl]-butanoyl}amino)benzyl]cyclopropancarboxylat | |
| **47** | 1-(3-{[4,4,4-Trifluor-3-methyl-2-(4-methyl-phenyl)butanoyl]amino}benzyl)cyclopropan-carbonsäure | LC-MS (Methode 4): Rₜ = 1.35 min; m/z = 418 (M-H)⁻. |
| | 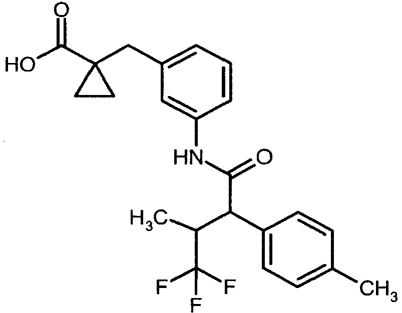 | |
| | aus *tert*.-Butyl-1-(3-{[4,4,4-trifluor-3-methyl-2-(4-methylphenyl)butanoyl]amino}benzyl)-cyclopropancarboxylat | |
| **48** | 1-[3-({4,4,4-Trifluor-3-methyl-2-[4-(trifluormethoxy)phenyl]butanoyl}amino)benzyl]-cyclopropancarbonsäure | LC-MS (Methode 5): Rₜ = 1.23 min; m/z = 488 (M-H)⁻. |
| | 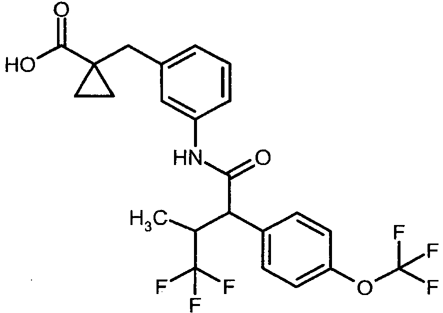 | ¹H-NMR (400 MHz, DMSO-*d₆*): δ [ppm] = 0.72-0.86 (m, 5H), 1.07-1.16 (m, 2H), 2.82 (s, 2H), 3.27-3.48 (m, 1H, teilweise verdeckt durch H₂O-Signal), 3.90 (d, 1H), 6.88-6.98 (m, 1H), 7.11-7.21 (m, 1H), 7.30-7.49 (m, 4H), 7.51.-7.64 (m, 2H), 10.22 (s, 1H), 12.08 (s, 1H). |
| | aus *tert*.-Butyl-1-[3-({4,4,4-trifluor-3-methyl-2-[4-(trifluormethoxy)phenyl]butanoyl}amino)-benzyl]cyclopropancarboxylat | |
| **49** | 1-(3-{[2-(4-Chlor-3-methylphenyl)-4,4,4-trifluor-3-methylbutanoyl]amino}benzyl)cyclopropancarbonsäure | LC-MS (Methode 5): Rₜ = 1.22 min; m/z = 452 (M-H)⁻. |
| | 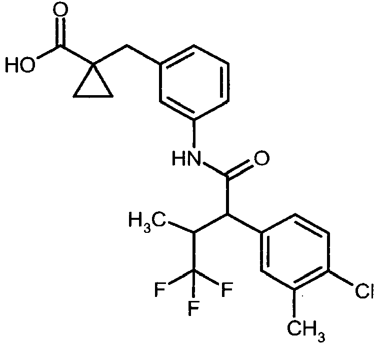 | ¹H-NMR (400 MHz, DMSO-*d₆*): δ [ppm] = 0.76-0.84 (m, 5H), 1.08-1.15 (m, 2H), 2.27-2.35 (m, 3H), 2.82 (s, 2H), 3.29-3.44 (m, 1H, teilweise verdeckt durch H₂O-Signal), 3.80 (d, 1H), 6.89-6.96 (m, 1H), 7.16 (t, 1H), 7.23-7.32 (m, 1H), 7.33-7.47 (m, 4H), 10.18 (s, 1H), 12.00-1.2.20 (br. s, 1H). |
| | aus *tert.*-Butyl-1-(3-{[2-(4-chlor-3-methylphenyl)-4,4,4-trifluor-3-methylbutanoyl]amino}benzyl)-cyclopropancarboxylat | |

### Beispiel 50

### (+)-1-(4-Chlor-3-{[(2S,3R)-2-(4-chlorphenyl)-4,4,4-trifluor-3-methylbutanoyl]amino}benzyl)-cyclopropancarbonsäure

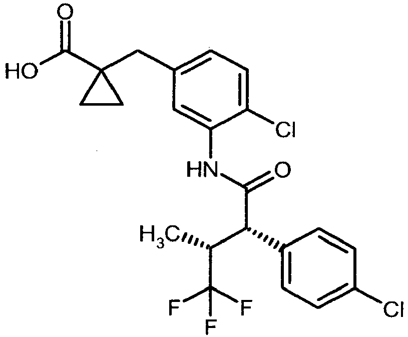

573 mg (1.08 mmol) *tert*.-Butyl-1-(4-chlor-3-{[(2*S*,3*R*)-2-(4-chlorphenyl)-4,4,4-trifluor-3-methyl-butanoyl]amino}benzyl)cyclopropancarboxylat wurden in 5 ml Dichlormethan gelöst und bei RT mit 2.5 ml TFA versetzt. Die Reaktionsmischung wurde 3 h bei RT gerührt und dann im Vakuum eingeengt. Das so erhaltene Rohprodukt wurde mit Wasser versetzt und 15 min bei RT gerührt. Anschließend wurden die erhaltenen Kristalle über eine Nutsche abgesaugt und im Hochvakuum getrocknet. Es wurden 445 mg (86% d. Th.) der Titelverbindung erhalten.

LC-MS (Methode 5): Rₜ = 1.23 min; m/z = 472/474 (M-H)⁻.

¹H-NMR (400 MHz, DMSO-*d₆*): δ [ppm] = 0.75-0.88 (m, 5H), 1.06-1.14 (m, 2H), 2.82 (s, 2H), 3.29-3.45 (m, 1H, teilweise verdeckt durch H₂O-Signal), 4.11 (d, 1H), 7.08 (dd, 1H), 7.34 (d, 1H), 7.40-7.50 (m, 5H), 9.83 (s, 1H), 12.16 (br. s, 1H).

[α]_{D}²⁰ = +95.4°, c = 0.40, Methanol.

### Beispiel 51

### (+)-1-(3-{[(2S,3R)-2-(4-Chlorphenyl)-4,4,4-trifluor-3-methylbutanoyl]amino}-4-fluorbenzyl)-cyclopropancarbonsäure

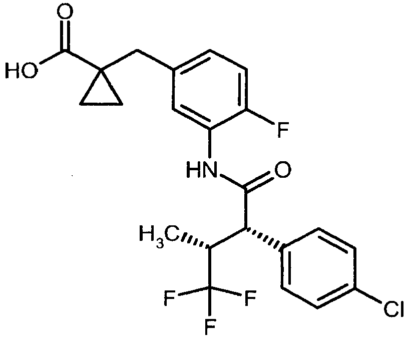

Entsprechend der Allgemeinen Vorschrift 8 wurden aus 1.59 g (3.1 mmol) (+)-1-(3-{[(2*S*,3*R*)-2-(4-Chlorphenyl)-4,4,4-trifluor-3-methylbutanoyl]amino}-4-fluorbenzyl)cyclopropancarbonsäure-*tert*.-butylester 1.36 g (96% d. Th.) der Titelverbindung erhalten.

LC-MS (Methode 5): Rₜ = 1.20 min; m/z = 458 (M+H)⁺.

¹H-NMR (400 MHz, DMSO-*d₆*): δ [ppm] = 0.73-0.84 (m, 5H), 1.05-1.12 (m, 2H), 2.81 (s, 2H), 4.06-4.14 (m, 1H), 6.95-7.04 (m, 1H), 7.11 (dd, 1H), 7.41-7.50 (m, 4H), 7.67 (dd, 1H), 10.01 (s, 1H), 12.12 (s, 1H).

[α]_{D}²⁰ = +125.6°, c = 0.545, Chloroform.

### Beispiel 52

### 1-(4-Chlor-3-{[(2S,3R)-2-(4-chlorphenyl)-4,4,4-trifluor-3-methylbutanoyl]amino}-2-fluorbenzyl)-cyclopropancarbonsäure

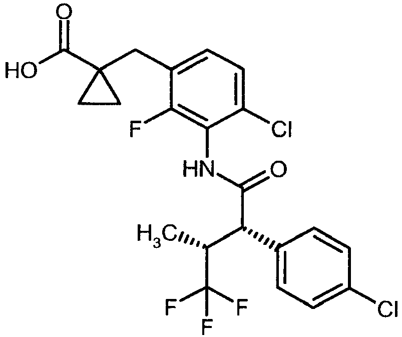

22.0 mg (0.040 mmol) 1-(4-Chlor-3-{[(2*S*,3*R*)-2-(4-chlorphenyl)-4,4,4-trifluor-3-methylbutanoyl]-amino}-2-fluorbenzyl)cyclopropancarbonsäure-*tert*.-butylester wurden in 0.19 ml Dichlormethan gelöst und bei RT mit 0.5 ml TFA versetzt. Die Reaktionsmischung wurde nach 1 h bei RT im Vakuum eingeengt und der Rückstand über Nacht im Hochvakuum getrocknet. Es wurden 17.8 mg (82.2% d. Th.) der Zielverbindung erhalten.

LC-MS (Methode 5): Rₜ = 1.14 min; m/z = 492 (M+H)⁺.

¹H-NMR (400 MHz, DMSO-*d₆*): δ [ppm] = 0.74-0.84 (m, 5H), 1.06-1.18 (m, 2H), 2.86 (s, 2H), 3.19-3.43 (m, 1H), 3.95 (d, 1H), 7.23-7.33 (m, 2H), 7.38-7.50 (m, 4H), 10.02 (s, 1H).

### Beispiel 53

### (+/-)-1-[3-({Cyclopentyl[4-(trifluormethyl)phenyl]acetyl}amino)benzyl]cyclopropancarbonsäure

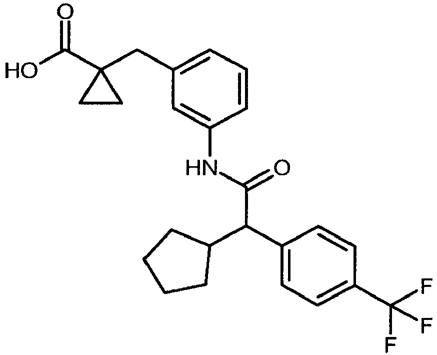

4.83 g (9.63 mmol) (+/-)-1-[3-({Cyclopentyl[4-(trifluormethyl)phenyl]acetyl}amino)benzyl]cyclopropancarbonsäure-*tert*.-butylester wurden in 25 ml Dichlormethan gelöst und nach Zugabe von einem Tropfen Wasser bei RT mit 7.5 ml TFA versetzt. Die Reaktionsmischung wurde 2 h bei RT gerührt und dann im Vakuum eingeengt. Das Rohprodukt wurde durch Chromatographie an Silicagel gereinigt (Laufmittel Cyclohexan/Ethylacetat 4:1 → 2:1). Es wurden 3.89 g (90.8% d. Th.) der Zielverbindung erhalten.

LC-MS (Methode 4): Rₜ = 1.46 min; m/z = 446 (M+H)⁺.

¹H-NMR (400 MHz, DMSO-*d₆*): δ [ppm] = 0.74-0.83 (m, 2H), 0.96 (dq, 1H), 1.07-1.14 (m, 2H), 1.24-1.40 (m, 2H), 1.41-1.71 (m, 4H), 1.74-1.87 (m, 1H), 2.61 (dt, 1H), 2.82 (s, 2H), 3.52 (d, 1H), 6.92 (d, 1H), 7.16 (t, 1H), 7.39-7.48 (m, 2H), 7.56-7.66 (m, 2H), 7.67-7.73 (m, 2H), 10.09 (s, 1H), 12.09 (br. s, 1H).

### Beispiel 54

### (+)-1-[3-({(2S)-2-Cyclopentyl-2-[4-(trifluormethyl)phenyl]acetyl}amino)benzyl]cyclopropancarbonsäure

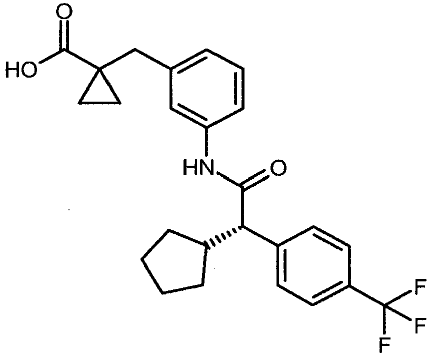

Aus dem oben erhaltenen Racemat der 1-[3-({Cyclopentyl[4-(trifluormethyl)phenyl]acetyl}amino)-benzyl]cyclopropancarbonsäure (Beispiel 53) wurde durch präparative HPLC an chiraler Phase das (+)-Enantiomer isoliert [Säule: Daicel Chiralpak AD-H, 5 µm, 250 mm x 20 mm; Injektionsvolumen: 0.5 ml; Temperatur: 30°C; Eluent: 70% Isohexan / 30% (Isopropanol + 0.2% TFA + 1% Wasser); Fluss: 15 ml/min; Detektion: 220 nm]. Ausgehend von 3.90 g Racemat wurden 1.69 g des (+)-Enantiomer erhalten.

LC-MS (Methode 5): Rₜ = 1.15 min; m/z = 446 (M+H)⁺.

¹H-NMR (400 MHz, DMSO-*d₆*): δ [ppm] = 0.75-0.85 (m, 2H), 0.90-1.03 (m, 1H), 1.08-1.15 (m, 2H), 1.22-1.39 (m, 2H), 1.41-1.71 (m, 4H), 1.75-1.88 (m, 1H), 2.61 (dt, 1H), 2.83 (s, 2H), 3.53 (d, 1H), 6.92 (d, 1H), 7.16 (t, 1H), 7.38-7.49 (m, 2H), 7.57-7.66 (m, 2H), 7.67-7.76 (m, 2H), 10.09 (s, 1H), 12.09 (br. s, 1H).

[α]_{D}²⁰ = +37.3°, c = 0.700, Chloroform.

### Beispiel 55

### (+/-)-1-(3-{[(4-Chlorphenyl)(cyclopentyl)acetyl]amino}benzyl)cyclopropancarbonsäure

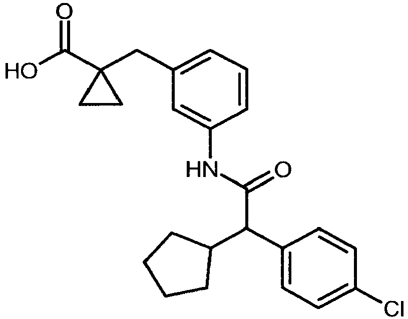

6.60 g (14.1 mmol) (+/-)-1-(3-{[(4-Chlorphenyl)(cyclopentyl)acetyl]amino}benzyl)cyclopropan-carbonsäure-*tert*.-butylester wurden in 41.8 ml Dichlormethan gelöst und nach Zugabe von einem Tropfen Wasser bei RT mit 10.9 ml TFA versetzt. Die Reaktionsmischung wurde 1 h bei RT gerührt und danach im Vakuum eingeengt. Der Rückstand wurde zunächst im Hochvakuum getrocknet und dann mit Diisopropylether verrührt. Der resultierende Feststoff wurde abgesaugt und im Hochvakuum getrocknet. Es wurden 4.52 g (77.8% d. Th.) der Zielverbindung erhalten.

LC-MS (Methode 4): Rₜ = 1.43 min; m/z = 412 (M+H)⁺.

¹H-NMR (400 MHz, DMSO-*d₆*): δ [ppm] = 0.73-0.82 (m, 2H), 0.90-1.01 (m, 1H), 1.08-1.17 (m, 2H), 1.21-1.39 (m, 2H), 1.41-1.70 (m, 4H), 1.71-1.85 (m, 1H), 2.56-2.63 (m, 1H), 2.82 (s, 2H), 3.40 (d, 1H), 6.91 (d, 1H), 7.15 (t, 1H), 7.32-7.50 (m, 6H), 10.02 (s, 1H), 12.10 (br. s, 1H).

### Beispiel 56

### (+)-1-(3-{[(2S)-2-(4-Chlorphenyl)-2-cyclopentylacetyl]amino}benzyl)cyclopropancarbonsäure

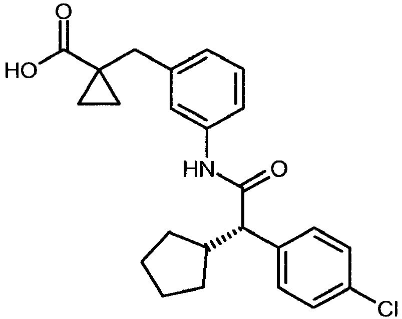

Aus dem oben erhaltenen Racemat der 1-(3-{[(4-Chlorphenyl)(cyclopentyl)acetyl]amino}benzyl)-cyclopropancarbonsäure (Beispiel 55) wurde durch präparative HPLC an chiraler Phase das (+)-Enantiomer isoliert [Säule: Daicel Chiralpak AD-H, 5 µm, 250 mm x 20 mm; Injektionsvolumen: 0.16 ml; Temperatur: 28°C; Eluent: 75% Isohexan / 25% (Isopropanol + 0.2% TFA + 1% Wasser); Fluss: 15 ml/min; Detektion: 220 nm]. Ausgehend von 4.52 g Racemat wurden 1.90 g des (+)-Enantiomer erhalten.

LC-MS (Methode 5): Rₜ = 1.23 min; m/z = 412 (M+H)⁺.

¹H-NMR (400 MHz, DMSO-*d₆*): δ [ppm] = 0.76-0.82 (m, 2H), 0.89-1.01 (m, 1H), 1.08-1.15 (m, 2H), 1.19-1.39 (m, 2H), 1.40-1.70 (m, 4H), 1.72-1.84 (m, 1H), 2.54-2.62 (m, 1H), 2.82 (s, 2H), 3.40 (d, 1H), 6.91 (d, 1H), 7.15 (t, 1H), 7.32-7.47 (m, 6H), 10.02 (s, 1H), 12.09 (br. s, 1H).

[α]_{D}²⁰ = +31.4°, c = 0.560, Chloroform.

### Beispiel 57 und Beispiel 58

Das oben erhaltene Racemat der 1-(3-{[(4-Chlor-2-fluorphenyl)(cyclopentyl)acetyl]amino}-benzyl)cyclopropancarbonsäure (Beispiel 1) wurde durch präparative HPLC an chiraler Phase in die Enantiomere aufgetrennt [Säule: Daicel Chiralpak AD-H, 5 µm, 250 mm x 20 mm; Injektionsvolumen: 0.35 ml; Temperatur: 30°C; Eluent: 80% Isohexan / 20% (Ethanol + 0.2% TFA + 1% Wasser); Fluss: 15 ml/min; Detektion: 220 nm]. Ausgehend von 150 mg Racemat wurden 67 mg von Enantiomer 1 (*Beispiel 57*) und 72 mg von Enantiomer 2 (*Beispiel 58*) erhalten.

### Beispiel 57 (Enantiomer 1):

### (+)-1-(3-{[(2S)-2-(4-Chlor-2-fluorphenyl)-2-cyclopentylacetyl]amino}benzyl)cyclopropancarbonsäure

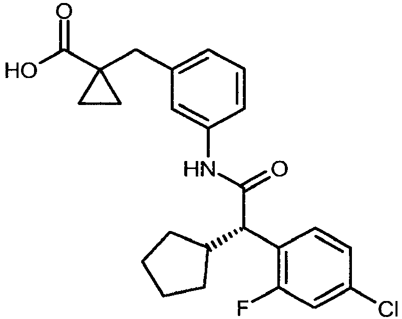

LC-MS (Methode 5): Rt = 1.28 min; m/z = 430 (M+H)⁺.

¹H-NMR (400 MHz, DMSO-*d₆*): δ [ppm] = 0.76-0.83 (m, 2H), 0.94-1.04 (m, 1H), 0.94-1.12 (m, 2H), 1.32-1.61 (m, 5H), 1.62-1.79 (m, 2H), 2.50-2.55 (m, 1H, verdeckt), 2.83 (s, 2H), 3.78 (d, 1H), 6.93 (d, 1H), 7.16 (t, 1H), 7.29 (dd, 1H), 7.40 (dd, 1H), 7.43-7.49 (m, 2H), 7.70 (t, 1H), 10.13 (s, 1H), 12.10 (br. s, 1H).

[α]_{D}²⁰ = +43.6°, c = 0.520, Chloroform.

### Beispiel 58 (Enantiomer 2):

### (-)-1-(3-{[(2R)-2-(4-Chlor-2-fluorphenyl)-2-cyclopentylacetyl]amino}benzyl)cyclopropancarbonsäure

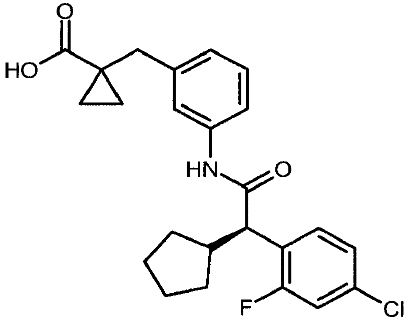

LC-MS (Methode 5): Rt = 1.28 min; m/z = 430 (M+H)⁺.

¹H-NMR (400 MHz, DMSO-*d₆*): δ [ppm] = 0.76-0.84 (m, 2H), 0.94-1.03 (m, 1H), 1.07-1.15 (m, 2H), 1.33-1.61 (m, 5H), 1.63-1.79 (m, 2H), 2.52-2.57 (m, 1H, verdeckt), 2.83 (s, 2H), 3.78 (d, 1H), 6.93 (d, 1H), 7.16 (t, 1H), 7.29 (dd, 1H), 7.40 (dd, 1H), 7.43-7.52 (m, 2H), 7.70 (t, 1H), 10.13 (s, 1H), 12.10 (br. s, 1 H).

[α]_{D}²⁰ = -39.7°, c = 0.540, Chloroform.

### Beispiel 59

### (+/-)-1-(3-{[2-(4-Ethylphenyl)-4,4,4-trifluor-3-methylbutanoyl]amino}benzyl)cyclopropancarbonsäure

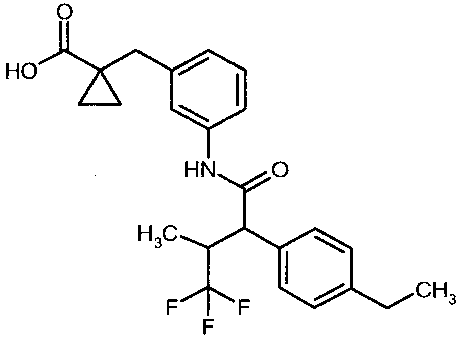

1.52 g (3.11 mmol) (+/-)-1-(3-{[2-(4-Ethylphenyl)-4,4,4-trifluor-3-methylbutanoyl]amino}benzyl)-cyclopropancarbonsäure-*tert*.-butylester wurden in 3 ml Dichlormethan gelöst und bei RT mit 12 ml TFA versetzt. Die Reaktionsmischung wurde 2 h bei RT gerührt, dann im Vakuum eingeengt und der Rückstand über Nacht im Hochvakuum getrocknet. Dieser Rückstand wurde mit Acetonitril verrührt, und der resultierende Feststoff wurde abgesaugt, mit wenig Acetonitril gewaschen und im Hochvakuum getrocknet. Es wurden 1.16 g (86.2% d. Th.) der Zielverbindung erhalten.

LC-MS (Methode 4): Rₜ = 1.42 min; m/z = 434 (M+H)⁺.

¹H-NMR (400 MHz, DMSO-*d₆*): δ [ppm] = 0.75-0.82 (m, 5H), 1.08-1.13 (m, 2H), 1.16 (t, 3H), 2.57 (q, 2H), 2.82 (s, 2H), 3.31-3.36 (m, 1H), 3.78 (d, 1H), 6.91 (d, 1H), 7.15 (t, 1H), 7.17-7.23 (m, 2H), 7.29-7.35 (m, 2H), 7.37 (s, 1H), 7.43 (d, 1H), 10.13 (s, 1H), 12.08 (br. s, 1H).

### Beispiel 60

### (+)-1-(3-{[(2S,3R)-2-(4-Ethylphenyl)-4,4,4-trifluor-3-methylbutanoyl]amino}benzyl)cyclopropancarbonsäure (Enantiomer 1)

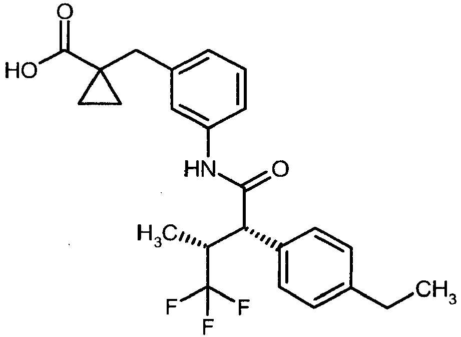

Das oben erhaltene Racemat der 1-(3-{[2-(4-Ethylphenyl)-4,4,4-trifluor-3-methylbutanoyl]amino}-benzyl)cyclopropancarbonsäure (Beispiel 59) wurde durch präparative HPLC an chiraler Phase weiter aufgetrennt [Säule: Daicel Chiralpak AD-H, 5 µm, 250 mm x 20 mm; Injektionsvolumen: 0.2 ml; Temperatur: 28°C; Eluent: 75% Isohexan / 25% (Isopropanol + 0.2% TFA + 1% Wasser); Fluss: 15 ml/min; Detektion: 220 nm]. Ausgehend von 1300 mg Racemat wurden 641 mg des Enantiomer 1 isoliert.

LC-MS (Methode 4): Rₜ = 1.41 min; m/z = 434 (M+H)⁺.

¹H-NMR (400 MHz, DMSO-*d₆*): δ [ppm] = 0.75-0.82 (m, 5H), 1.11 (q, 2H), 1.16 (t, 3H), 2.55-2.61 (m, 2H), 2.82 (s, 2H), 3.29-3.41 (m, 1H), 3.78 (d, 1H), 6.91 (d, 1H), 7.15 (t, 1H), 7.18-7.23 (m, 2H), 7.28-7.35 (m, 2H), 7.37 (s, 1H), 7.43 (d, 1H), 10.13 (s, 1H), 12.08 (br. s, 1H),

[α]_{D}²⁰ = +73.8°, c = 0.560, Chloroform.

### Beispiel 61 und Beispiel 62

Das oben erhaltene Racemat der 1-(3-{[(4-Chlorphenyl)(cyclopentyl)acetyl]amino}-4-fluorbenzyl)cyclopropancarbonsäure (Beispiel 8) wurde durch präparative HPLC an chiraler Phase in die Enantiomere aufgetrennt [Säule: Daicel Chiralpak AD-H, 5 µm, 250 mm x 20 mm; Injektionsvolumen: 0.35 ml; Temperatur: 35°C; Eluent: 30% Isohexan / 70% Isopropanol; Fluss: 15 ml/min; Detektion: 220 nm]. Ausgehend von 167 mg Racemat wurden 95 mg von Enantiomer 1 (*Beispiel 61*) und 89 mg von Enantiomer 2 (*Beispiel 62*) erhalten (beide noch Lösungsmittelreste enthaltend).

### Beispiel 61 (Enantiomer 1):

### (-)-1-(3-{[(2R)-2-(4-Chlorphenyl)-2-cyclopentylacetyl]amino}-4-fluorbenzyl)cyclopropancarbonsäure

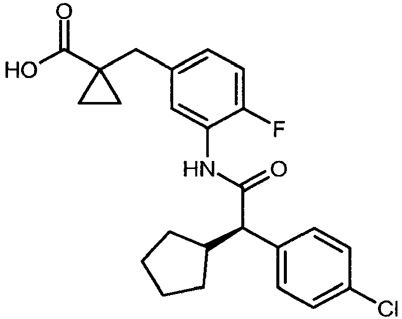

LC-MS (Methode 5): Rₜ = 1.26 min; m/z = 430 (M+H)⁺.

¹H-NMR (400 MHz, DMSO-*d₆*): δ [ppm] = 0.67-0.72 (m, 2H), 0.90-0.98 (m, 1H), 1.00-1.06 (m, 2H), 1.21-1.39 (m, 2H), 1.39-1.69 (m, 4H), 1.72-1.86 (m, 1H), 2.52-2.57 (m, 1H), 2.80 (s, 2H), 3.60 (d, 1H), 6.98-7.05 (m, 1H), 7.05-7.14 (m, 1H), 7.35-7.40 (m, 2H), 7.40-7.46 (m, 2H), 7.63 (dd, 1H), 9.80 (s, 1H).

[α]_{D}²⁰ = -65.7°, c = 0.360, Chloroform.

### Beispiel 62 (Enantiomer 2):

### (+)-1-(3-{[(2S)-2-(4-Chlorphenyl)-2-cyclopentylacetyl]amino}-4-fluorbenzyl)cyclopropancarbonsäure

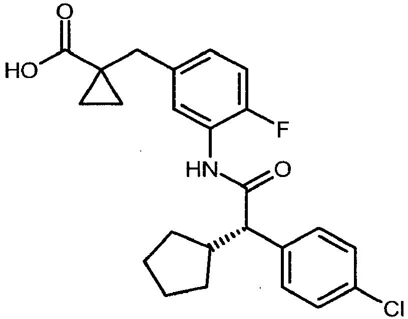

LC-MS (Methode 5): Rₜ = 1.26 min; m/z = 430 (M+H)⁺.

¹H-NMR (400 MHz, DMSO-*d₆*): δ [ppm] = 0.68 (br. s, 2H), 0.90-0.99 (m, 1H), 1.01-1.04 (m, 2H), 1.26-1.39 (m, 2H), 1.40-1.70 (m, 4H), 1.74-1.85 (m, 1H), 2.52-2.57 (m, 1H), 2.80 (s, 2H), 3.60 (d, 1H), 6.98-7.04 (m, 1H), 7.05-7.12 (m, 1H), 7.34-7.40 (m, 2H), 7.41-7.46 (m, 2H), 7.63 (dd, 1H), 9.80 (s, 1H).

[α]_{D}²⁰ = +63.5°, c = 0.550, Chloroform.

### Beispiel 63

### (+)-1-(4-Fluor-3-{[(2S,3R)-4,4,4-trifluor-3-methyl-2-(4-vinylphenyl)butanoyl]amino}benzyl)-cyclopropancarbonsäure

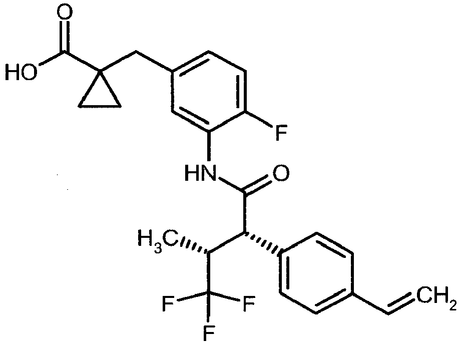

120 mg (0.237 mmol) (+)-1-(4-Fluor-3-{[(2*S*,3*R*)-4,4,4-trifluor-3-methyl-2-(4-vinylphenyl)-butanoyl]amino}benzyl)cyclopropancarbonsäure-*tert*.-butylester wurden bei RT mit 1.7 ml einer 4 M Lösung von Chlorwasserstoff in Dioxan behandelt. Nach Rühren über Nacht bei RT wurde die Reaktionsmischung mit Trockeneis eingefroren und anschließend im Hochvakuum lyophilisiert. Das erhaltene Produkt wurde in wenig Dichlormethan aufgenommen und im Hochvakuum aufgeschäumt. Es wurden 69 mg der Zielverbindung erhalten (65% d. Th.).

LC-MS (Methode 5): Rₜ = 1.19 min; m/z = 450 (M+H)⁺.

¹H-NMR (400 MHz, DMSO-*d₆*): δ [ppm] = 0.75-0.82 (m, 5H), 1.09 (q, 2H), 2.80 (s, 2H), 3.34-3.42 (m, 1H), 4.07 (d, 1H), 5.27 (d, 1H), 5.83 (d, 1H), 6.72 (dd, 1H), 6.95-7.03 (m, 1H), 7.10 (dd, 1H), 7.38-7.42 (m, 2H), 7.45-7.50 (m, 2H), 7.67 (dd, 1H), 9.98 (s, 1H), 12.12 (s, 1H).

[α]_{D}²⁰ = +37.7°, c = 0.385, Chloroform.

### Beispiel 64

### (+)-1-(3-{[(2S,3R)-2-(4-Acetylphenyl)-4,4,4-trifluor-3-methylbutanoyl]amino}-4-fluorbenzyl)-cyclopropancarbonsäure

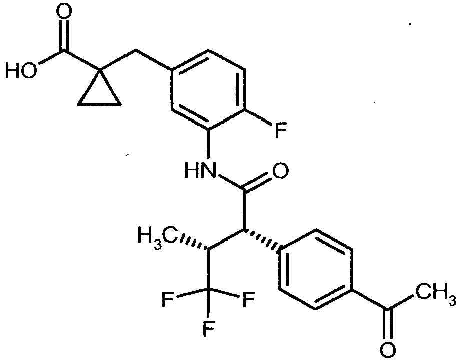

90 mg (0.172 mmol) (+)-1-[4-Fluor-3-({(2*S*,3*R*)-4,4,4-trifluor-2-[4-(1-fluorvinyl)phenyl]-3-methyl-butanoyl}amino)benzyl]cyclopropancarbonsäure-*tert*-butylester wurden in 5.2 ml einer 4 M Lösung von Chlorwasserstoff in Dioxan über Nacht bei RT gerührt. Die Reaktionsmischung wurde danach mit Trockeneis eingefroren und im Hochvakuum lyophilisiert. Der resultierende Rückstand wurde durch präparative RP-HPLC aufgereinigt (Acetonitril/Wasser-Eluent). Es wurden 27 mg (33.7% d. Th.) der Titelverbindung erhalten.

LC-MS (Methode 5): Rₜ = 1.05 min; m/z = 465 (M+H)⁺.

¹H-NMR (400 MHz, DMSO-*d₆*): δ [ppm] = 0.75-0.83 (m, 5H), 1.09 (q, 2H), 2.57 (s, 3H), 2.80 (s, 2H), 3.43 (dd, 1H), 4.19 (d, 1H), 6.96-7.04 (m, 1H), 7.10 (dd, 1H), 7.55-7.62 (m, 2H), 7.66 (dd, 1H), 7.94-8.00 (m, 2H), 10.06 (s, 1H), 12.12 (s, 1H).

[α]_{D}²⁰ = +121.8°, c = 0.49, Chloroform.

### Beispiel 65

### 1-(3-{[(4-Chlorphenyl)(2,2-difluorcyclopentyl)acetyl]amino}-4-fluorbenzyl)cyclopropancarbonsäure (racemisches Diastereomerengemisch)

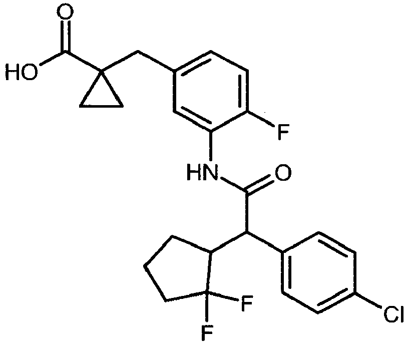

750 mg (1.44 mmol) 1-(3-{[(4-Chlorphenyl)(2,2-difluorcyclopentyl)acetyl]amino}-4-fluorbenzyl)-cyclopropancarbonsäure-*tert*.-butylester (als racemisches Diastereomerengemisch) wurden in 1.6 ml Dichlormethan gelöst und bei RT mit 5.5 ml TFA versetzt. Die Mischung wurde 2 h bei RT gerührt und dann im Vakuum eingeengt. Der Rückstand wurde im Hochvakuum getrocknet und anschließend mit Acetonitril verrührt. Der ausgefallene Feststoff wurde abfiltriert. Das Filtrat wurde eingeengt, der erhaltene Rückstand erneut mit Acetonitril verrührt und eine weitere Charge an Feststoff isoliert. Dieser Vorgang wurde noch einmal wiederholt und alle Feststoff-Chargen dann vereinigt. Es wurden insgesamt 553 mg (82.6% d. Th.) der Titelverbindung als Gemisch von vier Isomeren erhalten.

LC-MS (Methode 5): Rₜ = 1.18 min; m/z = 466 (M+H)⁺.

### Beispiele 66 - 68

Das oben erhaltene Gemisch der racemischen, diastereomeren 1-(3-{[(4-Chlorphenyl)(2,2-difluor-cyclopentyl)acetyl]amino}-4-fluorbenzyl)cyclopropancarbonsäuren (Beispiel 65) wurde durch präparative HPLC an chiraler Phase weiter aufgetrennt [Säule: Daicel Chiralpak AD-H, 5 µm, 250 mm x 20 mm; Injektionsvolumen: 10 µl; Temperatur: 40°C; Eluent: 80% Isohexan / 20% (Isopropanol + 0.2% TFA + 1% Wasser); Fluss: 15 ml/min; Detektion: 220 nm]. Ausgehend von 540 mg an Diastereomerengemisch wurden 163 mg an reinem Isomer 1 (*Beispiel 66*) erhalten. Isomer 2 und Isomer 3 wurden zunächst als Gemisch erhalten, das durch nochmalige präparative HPLC an der gleichen chiralen Phase aufgetrennt wurde [Injektionsvolumen: 10 µl; Temperatur: 40°C; Eluent: 85% Isohexan / 15% (Isopropanol + 0.2% TFA + 1% Wasser); Fluss: 15 ml/min; Detektion: 220 nm]. Es wurden 140 mg an reinem Isomer 2 (*Beispiel 67*) und 107 mg an reinem Isomer 3 (*Beispiel* 68) isoliert.

### Beispiel 66 (Isomer 1 = Enantiomer 1 von Diastereomer 2):

### (-)-1-(3-{[(2R)-2-(4-Chlorphenyl)-2-(2,2-difluorcyclopentyl)acetyl]amino}-4-fluorbenzyl)cyclopropancarbonsäure

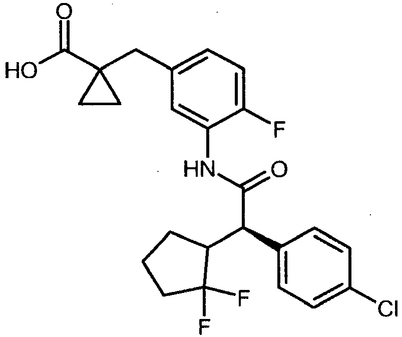

LC-MS (Methode 4): Rₜ = 1.33 min; m/z = 466 (M+H)⁺.

¹H-NMR (400 MHz, DMSO-*d₆*): δ [ppm] = 0.73-0.81 (m, 2H), 1.05-1.12 (m, 2H), 1.12-1.22 (m, 1H), 1.43-1.55 (m, 1H), 1.56-1.69 (m, 2H), 1.98-2.25 (m, 2H), 2.80 (s, 2H), 2.99-3.22 (m, 1H), 4.01 (d, 1H), 6.94-7.03 (m, 1H), 7.09 (dd, 1H), 7.37-7.43 (m, 2H), 7.43-7.51 (m, 2H), 7.65-7.75 (m, 1H), 9.84 (s, 1H), 12.10 (br. s, 1H).

[α]_{D}²⁰ = -79.1 °, c = 0.525, Chloroform.

### Beispiel 67 (Isomer 2 = Enantiomer 2 von Diastereomere 1):

### (+)-1-(3-{[(2S)-2-(4-Chlorphenyl)-2-(2,2-difluorcyclopentyl)acetyl]amino}-4-fluorbenzyl)cyclopropancarbonsäure

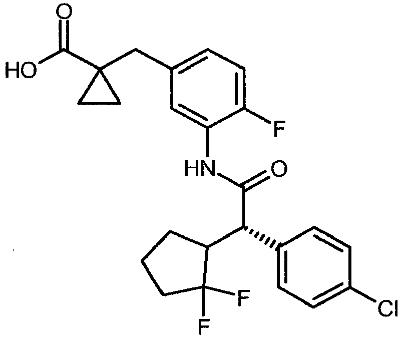

LC-MS (Methode 4): Rₜ = 1.33 min; m/z = 466 (M+H)⁺.

¹H-NMR (400 MHz, DMSO-*d₆*): δ [ppm] = 0.73-0.81 (m, 2H), 1.05-1.11 (m, 2H), 1.11-1.22 (m, 1H), 1.45-1.53 (m, 1H), 1.56-1.69 (m, 2H), 1.97-2.24 (m, 2H), 2.81 (s, 2H), 3.01-3.20 (m, 1H), 4.01 (d, 1H), 6.93-7.02 (m, 1H), 7.09 (dd, 1H), 7.36-7.43 (m, 2H), 7.44-7.50 (m, 2H), 7.66-7.73 (m, 1H), 9.84 (s, 1H), 12.08 (br. s, 1H).

[α]_{D}²⁰ = +89.6°, c = 0.480, Chloroform.

### Beispiel 68 (Isomer 3 = Enantiomer 2 von Diastereomer 2):

### (+)-1-(3-{[(2S)-2-(4-Chlorphenyl)-2-(2,2-difluorcyclopentyl)acetyl]amino}-4-fluorbenzyl)cyclopropancarbonsäure

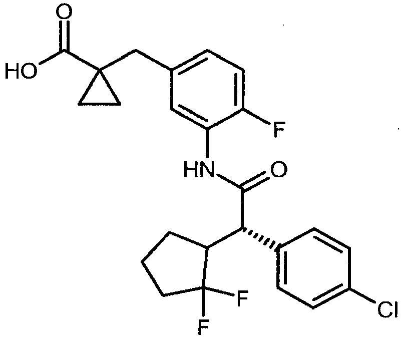

LC-MS (Methode 4): Rₜ = 1.32 min; m/z = 466 (M+H)⁺.

¹H-NMR (400 MHz, DMSO-*d₆*): δ [ppm] = 0.75-0.82 (m, 2H), 1.05-1.12 (m, 2H), 1.50-1.64 (m, 1H), 1.67-1.79 (m, 2H), 1.94-2.22 (m, 3H), 2.80 (s, 2H), 2.85-3.02 (m, 1H), 4.07 (d, 1H), 6.96-7.05 (m, 1H), 7.07-7.15 (m, 1H), 7.34-7.40 (m, 2H), 7.43-7.51 (m, 2H), 7.63 (dd, 1H), 9.98 (s, 1H).[α]_{D}²⁰ = +96.2°, c = 0.460, Chloroform.

### Beispiel 69

### 1-(4-Chlor-3-{[(2S,3R)-2-(4-chlorphenyl)-4,4,4-trifluor-3-methylbutanoyl]amino}benzyl)-2,2-difluorcyclopropancarbonsäure (Diastereomerengemisch)

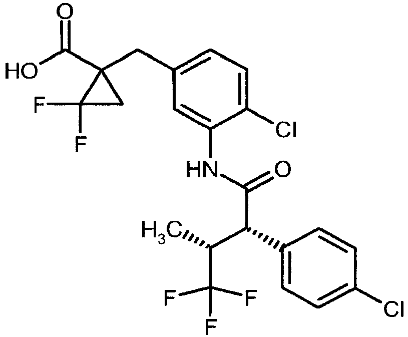

1.80 g (3.18 mmol) 1-(4-Chlor-3-{[(2*S*,3*R*)-2-(4-chlorphenyl)-4,4,4-trifluor-3-methylbutanoyl]-amino}benzyl)-2,2-difluorcyclopropancarbonsäure-*tert*.-butylester (als Diastereomerengemisch) wurden in 5 ml Dichlormethan gelöst und bei RT mit 4.9 ml TFA versetzt. Die Reaktionsmischung wurde 1 h bei RT gerührt und dann im Vakuum eingeengt. Der Rückstand wurde durch Chromatographie an Silicagel gereinigt (Laufmittel zunächst Dichlormethan, dann Dichlormethan/Ethylacetat 10:1 → 5:1). Es wurden 1.25 g der Zielverbindung erhalten (77.1% d. Th.).

LC-MS (Methode 5): Rt = 1.20 min; m/z = 510 (M+H)⁺.

¹H-NMR (400 MHz, DMSO-*d₆*): *beide Diastereomere:* δ [ppm] = 0.80 (d, 3H), 1.81-1.94 (m, 1H), 2.10-2.20 (m, 1H), 2.65-2.75 (m, 1H), 3.36-3.41 (m, 1H), 4.11 (d, 1H), 7.07 (dd, 1H), 7.35-7.61 (m, 6H), 9.85 (s, 1H), 13.25 (br. s, 1H).

### Beispiel 70 und Beispiel 71

Das oben erhaltene Diastereomerengemisch der 1-(4-Chlor-3-{[(2*S*,3*R*)-2-(4-chlorphenyl)-4,4,4-trifluor-3-methylbutanoyl]amino}benzyl)-2,2-difluorcyclopropancarbonsäure (Beispiel 69) wurde durch präparative HPLC an chiraler Phase weiter aufgetrennt [Säule: Daicel Chiralpak AD-H, 5 µm, 250 mm x 20 mm; Injektionsvolumen: 0.08 ml; Temperatur: 25°C; Eluent: 90% Isohexan / 10% Ethanol; Fluss: 15 ml/min; Detektion: 230 nm]. Ausgehend von 1.25 g Diastereomerengemisch wurden zunächst, in leicht verunreinigter Form, 298 mg von Diastereomer 1 und 400 mg von Diastereomer 2 isoliert. Nach weiterer Aufreinigung mittels präparativer RP-HPLC (Methanol/Wasser-Eluent) wurden 200 mg an reinem Diastereomer 1 (*Beispiel 70*) und 202 mg an reinem Diastereomer 2 (*Beispiel 71*) erhalten.

### Beispiel 70

### (+)-1-(4-Chlor-3-{[(2S,3R)-2-(4-chlorphenyl)-4,4,4-trifluor-3-methylbutanoyl]amino}benzyl)-2,2-difluorcyclopropancarbonsäure (Diastereomer 1)

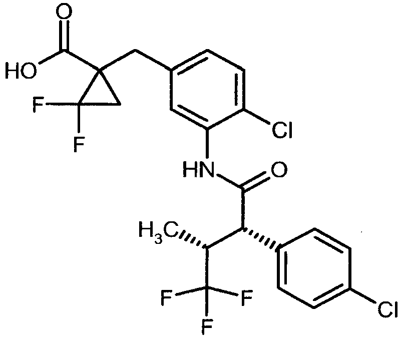

LC-MS (Methode 5): Rₜ = 1.20 min; m/z = 510 (M+H)⁺.

¹H-NMR (400 MHz, DMSO-*d₆*): δ [ppm] = 0.80 (d, 3H), 1.76-1.95 (m, 1H), 1.99-2.21 (m, 1H), 2.70 (d, 1H), 3.34-3.41 (m, 1H), 4.12 (d, 1H), 7.01-7.11 (m, 1H), 7.30-7.56 (m, 6H), 9.86 (s, 1H), 13.28 (br. s, 1H).

[α]_{D}²⁰ =+64.1°, c = 0.48, Chloroform.

### Beispiel 71

### (+)-1-(4-Chlor-3-{[(2S,3R)-2-(4-chlorphenyl)-4,4,4-trifluor-3-methylbutanoyl]amino}benzyl)-2,2-difluorcyclopropancarbonsäure (Diastereomer 2)

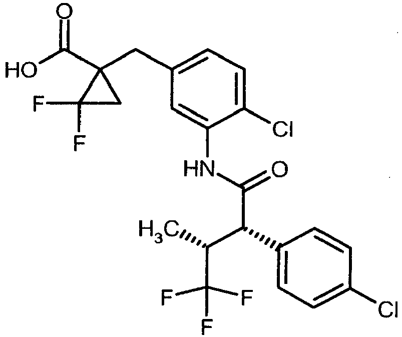

LC-MS (Methode 5): Rₜ = 1.20 min; m/z = 510 (M+H)⁺.

¹H-NMR (400 MHz, DMSO-*d₆*): δ [ppm] = 0.80 (d, 3H), 1.79-1.95 (m, 1H), 2.11-2.22 (m, 1H), 2.70 (d, 1H), 3.34-3.40 (m, 1H, verdeckt), 4.11 (d, 1H), 7.07 (dd, 1H), 7.39 (d, 1H), 7.42-7.54 (m, 5H), 9.86 (s, 1H), 13.25 (br. s, 1H).

[α]_{D}²⁰ = +32.3°, c = 0.530, Chloroform.

### Beispiele 72 - 75

### 1-(4-Chlor-3-{[(4-chlorphenyl)(3,3-difluorcyclopentyl)acetyl]amino}benzyl)cyclopropancarbonsäure (Isomer 1 - 4)

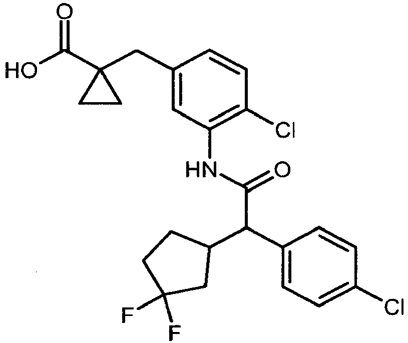

340 mg (0.63 mmol) des Diastereomerengemisches von 1-(4-Chlor-3-{[(4-chlorphenyl)(3,3-difluorcyclopentyl)acetyl]amino}benzyl)cyclopropancarbonsäure (Beispiel 35) wurden mittels präparativer HPLC an chiraler Phase weiter aufgetrennt [Säule: Daicel Chiralpak AD-H, 5 µm, 250 mm x 20 mm; Elutionsmittel: Isohexan/Ethanol 80:20 (v/v); Fluss: 15 ml/min; UV-Detektion: 220 nm; Temperatur: 35°C]. Es wurden vier verschiedene Isomere erhalten:

### Beispiel 72 (Isomer 1):

Ausbeute: 56 mg

Rₜ = 7.31 min; chemische Reinheit >99%; >99% ee; >99% de
[Säule: Daicel Chiralpak AD-H, 5 µm, 250 mm x 4.6 mm; Elutionsmittel: Isohexan/Ethanol 80:20 (v/v); Fluss: 1 ml/min; UV-Detektion: 220 nm; Temperatur: 30°C].

LC-MS (Methode 5): Rₜ = 1.22 min; m/z = 480/482 (M-H)⁻.

¹H-NMR (400 MHz, DMSO-*d₆*): δ [ppm] = 0.75-0.83 (m, 2H), 1.06-1.13 (m, 2H), 1.52-1.71 (m, 2H), 1.79-1.96 (m, 1H), 1.97-2.31 (m, 3H), 2.76-2.93 (m, 1H), 2.82 (s, 2H), 3.77 (d, 1H), 7.09 (d, 1H), 7.36 (d, 1H), 7.38-7.49 (m, 5H), 9.77 (s, 1H), 11.95-12.30 (br. s, 1H).

### Beispiel 73 (Isomere 2):

Ausbeute: 48 mg

Rₜ = 8.03 min, chemische Reinheit >98.5%; >99% ee; >98% de
[Säule: Daicel Chiralpak AD-H, 5 µm, 250 mm x 4.6 mm; Elutionsmittel: Isohexan/Ethanol 80:20 (v/v); Fluss: 1 ml/min; UV-Detektion: 220 nm; Temperatur: 30°C].

LC-MS (Methode 5): Rₜ = 1.23 min; m/z = 480/482 (M-H)⁻.

¹H-NMR (400 MHz, DMSO-*d₆*): δ [ppm] = 0.76-0.84 (m, 2H), 1.06-1.13 (m, 2H), 1.12-1.36 (m, 1H), 1.44-1.58 (m, 1H), 1.83-2.20 (m, 3H), 2.26-2.43 (m, 1H), 2.75-2.91 (m, 1H), 2.83 (s, 2H), 3.74 (d, 1H), 7.10 (d, 1H), 7.36 (d, 1H), 7.39-7.50 (m, 5H), 9.74 (s, 1H), 11.90-12.38 (br. s, 1H).

### Beispiel 74 (Isomer 3):

Ausbeute: 57 mg

Rₜ = 9.94 min; chemische Reinheit >99%; >99% ee; >99% de
[Säule: Daicel Chiralpak AD-H, 5 µm, 250 mm x 4.6 mm; Elutionsmittel: Isohexan/Ethanol 80:20 (v/v); Fluss: 1 ml/min; UV-Detektion: 220 nm; Temperatur: 30°C].

LC-MS (Methode 5): Rₜ = 1.23 min; m/z = 480/482 (M-H)⁻.

¹H-NMR: siehe Beispiel 73.

### Beispiel 75 (Isomer 4):

Ausbeute: 68 mg

Rₜ = 10.79 min; chemische Reinheit >99%; >99% ee; >99% de
[Säule: Daicel Chiralpak AD-H, 5 µm, 250 mm x 4.6 mm; Elutionsmittel: Isohexan/Ethanol 80:20 (v/v); Fluss: 1 ml/min; UV-Detektion: 220 nm; Temperatur: 30°C].

LC-MS (Methode 5): Rₜ = 1.22 min; m/z = 480/482 (M-H)⁻.

¹H-NMR: siehe Beispiel 72.

### Beispiele 76 - 79

### 1-(3-{[(4-Chlorphenyl)(3,3-difluorcyclopentyl)acetyl]amino}-4-fluorbenzyl)cyclopropancarbonsäure (Isomer 1 - 4)

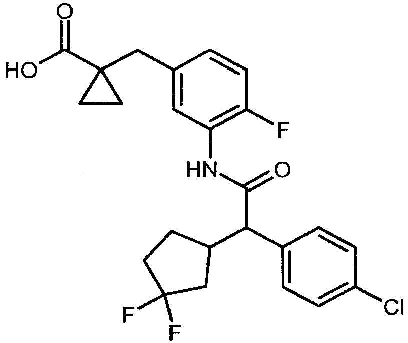

1310 mg (2.81 mmol) des Diastereomerengemisches von 1-(3-{[(4-Chlorphenyl)(3,3-difluorcyclo-pentyl)acetyl]amino}-4-fluorbenzyl)cyclopropancarbonsäure (Beispiel 34) wurden mittels präparativer HPLC an chiraler Phase weiter aufgetrennt [Säule: Daicel Chiralpak AD-H, 5 µm, 250 mm x 20 mm; Elutionsmittel: Isolzexan/(Isopropanol + 0.2% Trifluoressigsäure + 1% Wasser) 75:25 (v/v); Fluss: 15 ml/min; UV-Detektion: 220 nm; Temperatur: 30°C]. Peak 1 und Peak 2 wurden auf der gleichen Säule mit der Eluentenzusammensetzung Isohexan/(Ethanol + 0.2% Trifluoressigsäure + 1% Wasser) 75:25 (v/v) unter sonst gleichen Bedingungen nachgetrennt. Es wurden vier verschiedene Isomere erhalten:

### Beispiel 76 (Isomer 1):

Ausbeute: 245 mg

Rₜ = 5.93 min; chemische Reinheit >99%; >99% ee; >99% de
[Säule: Daicel Chiralpak AD-H, 5 µm, 250 mm x 4.6 mm; Elutionsmittel: Isohexan/(Isopropanol + 0.2% Trifluoressigsäure + 1% Wasser) 75:25 (v/v); Fluss: 1 ml/min; UV-Detektion: 220 nm; Temperatur: 40°C].

Rₜ = 6.39 min; chemische Reinheit >99%; >99% ee; >99% de [Säule: Daicel Chiralpak AD-H, 5 µm, 250 mm x 4.6 mm; Elutionsmittel: Isohexan/(Ethanol + 0.2% Trifluoressigsäure + 1% Wasser) 75:25 (v/v); Fluss: 1 ml/min; UV-Detektion: 220 nm; Temperatur: 40°C].

LC-MS (Methode 4): Rₜ = 1.35 min; m/z = 464/466 (M-H)⁻.

¹H-NMR (400 MHz, DMSO-*d₆*): δ [ppm] = 0.75-0.83 (m, 2H), 1.05-1.13 (m, 2H), 1.50-1.68 (m, 2H), 1.79-1.94 (m, 1H), 1.95-2.04 (m, 1H), 2.06-2.30 (m, 2H), 2.76-2.92 (m, 1H), 2.81 (s, 2H), 3.77 (d, 1H), 6.99-7.06 (m, 1H), 7.07-7.16 (m, 1H), 7.38-7.47 (m, 4H), 7.61-7.67 (m, 1H), 9.94 (s, 1H), 11.70-12.50 (br. s, 1H).

### Beispiel 77 (Isomer 2):

### Ausbeute: 210 mg

Rₜ = 6.09 min; chemische Reinheit >99%; >99% ee; >98.5% de
[Säule: Daicel Chiralpak AD-H, 5 µm, 250 mm x 4.6 mm; Elutionsmittel: Isohexan/(Isopropanol + 0.2% Trifluoressigsäure + 1% Wasser) 75:25 (v/v); Fluss: 1 ml/min; UV-Detektion: 220 nm; Temperatur: 40°C].

Rₜ = 6.93 min; chemische Reinheit >99%; >99% ee; >98.5% de
[Säule: Daicel Chiralpak AD-H, 5 µm, 250 mm x 4.6 mm; Elutionsmittel: Isohexan/(Ethanol + 0.2% Trifluoressigsäure + 1% Wasser) 75:25 (v/v); Fluss: 1 ml/min; UV-Detektion: 220 nm; Temperatur: 40°C].

LC-MS (Methode 4): Rₜ = 1.35 min; m/z = 464/466 (M-H)⁻.

¹H-NMR (400 MHz, DMSO-*d₆*): δ [ppm] = 0.75-0.83 (m, 2H), 1.05-1.13 (m, 2H), 1.20-1.35 (m, 1H), 1.43-1.56 (m, 1H), 1.79-2.20 (m, 3H), 2.23-2.39 (m, 1H), 2.75-2.89 (m, 1H), 2.81 (s, 2H), 3.74 (d, 1H), 7.00-7.06 (m, 1H), 7.07-7.15 (m, 1H), 7.38-7.48 (m, 4H), 7.59-7.66 (m, 1H), 9.89 (s, 1H), 11.44-12.68 (br. s, 1H).

### Beispiel 78 (Isomer 3):

Ausbeute: 224 mg

Rₜ = 6.65 min; chemische Reinheit >99%; >98.7% ee; >99% de
[Säule: Daicel Chiralpak AD-H, 5 µm, 250 mm x 4.6 mm; Elutionsmittel: Isohexan/(Isopropanol + 0.2% Trifluoressigsäure + 1% Wasser) 75:25 (v/v); Fluss: 1 ml/min; UV-Detektion: 220 nm; Temperatur: 40°C].

Rₜ = 6.35 min; chemische Reinheit >99%; >98.7% ee; >99% de
[Säule: Daicel Chiralpak AD-H, 5 µm, 250 mm x 4.6 mm; Elutionsmittel: Isohexan/(Ethanol + 0.2% Trifluoressigsäure + 1% Wasser) 75:25 (v/v); Fluss: 1 ml/min; UV-Detektion: 220 nm; Temperatur: 40°C].

LC-MS (Methode 4): Rₜ = 1.35 min; m/z = 464/466 (M-H)⁻.

¹H-NMR: siehe Beispiel 77.

### Beispiel 79 (Isomer 4):

Ausbeute: 276 mg

Rₜ = 8.81 min; chemische Reinheit >98.5%; >99% ee; >99% de
[Säule: Daicel Chiralpak AD-H, 5 µm, 250 mm x 4.6 mm; Elutionsmittel: Isohexan/(Isopropanol + 0.2% Trifluoressigsäure + 1% Wasser) 75:25 (v/v); Fluss: 1 ml/min; UV-Detektion: 220 nm; Temperatur: 40°C].

Rₜ = 7.20 min; chemische Reinheit 99%; >98.7% ee; >99% de
[Säule: Daicel Chiralpak AD-H, 5 µm, 250 mm x 4.6 mm; Elutionsmittel: Isohexan/(Ethanol + 0.2% Trifluoressigsäure + 1% Wasser) 75:25 (v/v); Fluss: 1 ml/min; UV-Detektion: 220 nm; Temperatur: 40°C].

LC-MS (Methode 4): Rt = 1.35 min; m/z = 464/466 (M-H)⁻.

¹H-NMR: siehe Beispiel 76.

### Beispiel 80 und Beispiel 81

### 1-[3-({4,4,4-Trilluor-3-methyl-2-[4-(trifluormethyl)phenyl]butanoyl}amino)benzyl]cyclopropan-carbonsäure (Isomer 1 und 2)

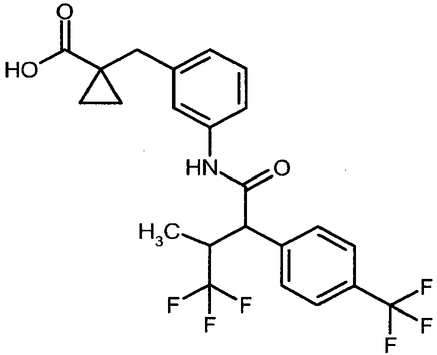

120 mg (0.25 mmol) des Diastereomerengemisches von 1-[3-({4,4,4-Trifluor-3-methyl-2-[4-(trifluormethyl)phenyl]butanoyl}amino)benzyl]cyclopropancarbonsäure (Beispiel 36) wurden mittels präparativer HPLC an chiraler Phase weiter aufgetrennt [Säule: Daicel Chiralcel OD-H, 5 µm, 250 mm x 20 mm; Elutionsmittel: Isohexan/(Isopropanol + 0.2% Trifluoressigsäure + 1% Wasser) 88:12 (v/v); Fluss: 15 ml/min; UV-Detektion: 220 nm; Temperatur: 30°C]. Es wurden zwei verschiedene Isomere erhalten:

### Beispiel 80 (Isomer 1):

Ausbeute: 40 mg

Rt = 6.10 min; chemische Reinheit >97%; >99% ee
[Säule: Daicel Chiralcel OD-H, 5 µm, 250 mm x 4.6 mm; Elutionsmittel: Isohexan/(Isopropanol + 0.2% Trifluoressigsäure + 1% Wasser) 90:10 (v/v); Fluss: 1 ml/min; UV-Detektion: 220 nm; Temperatur: 30°C].

LC-MS (Methode 5): Rₜ = 1.21 min; m/z = 474 (M+H)⁺.

### Beispiel 81 (Isomer 2):

Ausbeute: 42 mg

Rₜ = 6.95 min; chemische Reinheit >99%; >98% ee
[Säule: Daicel Chiralcel OD-H, 5 µm, 250 mm x 4.6 mm; Elutionsmittel: Isohexan/(Isopropanol + 0.2% Trifluoressigsäure + 1% Wasser) 90:10 (v/v); Fluss: 1 ml/min; UV-Detektion: 220 nm; Temperatur: 30°C].

LC-MS (Methode 5): Rₜ = 1.21 min; m/z = 474 (M+H)⁺.

### Beispiel 82 und Beispiel 83

### 1-[4-Chlor-3-({4,4,4-trifluor-3-methyl-2-[4-(2,2,2-trifluorethyl)phenyl]butanoyl}amino)benzyl]-cyclopropancarbonsäure (Enantiomere 1 und 2)

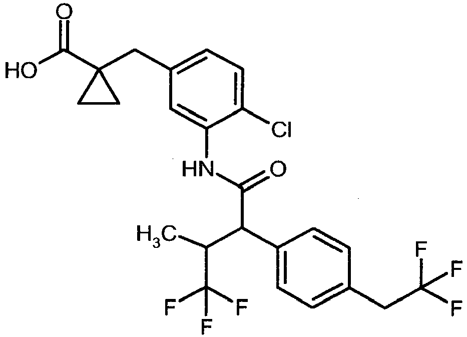

120 mg (0.23 mmol) des racemischen Gemisches von 1-[4-Chlor-3-({4,4,4-trifluor-3-methyl-2-[4-(2,2,2-trifluorethyl)phenyl]butanoyl}amino)benzyl]cyclopropancarbonsäure (Beispiel 46) wurden mittels präparativer HPLC an chiraler Phase in die Enantiomere aufgetrennt [Säule: Daicel Chiralpak AY-H, 5 µm, 250 mm x 20 mm; Elutionsmittel: Isohexan/Ethanol 85:15 (v/v); Fluss: 15 ml/ min; UV-Detektion: 220 nm; Temperatur: 45°C]:

### Beispiel 82 (Enantiomer 1):

Ausbeute: 55 mg

Rₜ = 4.23 min; chemische Reinheit 97.5%; 99% ee
[Säule: Daicel Chiralpak AY-H, 5 µm, 250 mm x 4.6 mm; Elutionsmittel: Isohexan/(Ethanol + 0.2% Trifluoressigsäure + 1% Wasser) 85:15 (v/v); Fluss: 1 ml/min; UV-Detektion: 220 nm; Temperatur: 45°C].

LC-MS (Methode 5): Rₜ = 1.20 min; m/z = 520/522 (M-H)⁻.

[α]_{D}²⁰ = +85.3°, c = 0.31, Methanol.

### Beispiel 83 (Enantiomer 2):

Ausbeute: 56 mg

Rₜ = 7.45 min; chemische Reinheit 99%; 99% ee
[Säule: Daicel Chiralpak AY-H, 5 µm, 250 mm x 4.6 mm; Elutionsmittel: Isohexan/(Ethanol + 0.2% Trifluoressigsäure + 1% Wasser) 85:15 (v/v); Fluss: 1 ml/min; UV-Detektion: 220 nm; Temperatur: 45°C].

LC-MS (Methode 5): Rₜ = 1.20 min; m/z = 520/522 (M-H)⁻.

[α]_{D}²⁰ = -78°, c = 0.255, Methanol.

### Beispiel 84 und Beispiel 85

### 1-(3-{[2-(4-Chlorphenyl)-4,4,4-trifluor-3-methylbutanoyl]amino}benzyl)cyclopropancarbonsäure (Enantiomer 1 und 2)

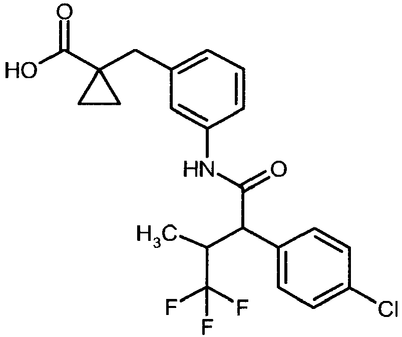

400 mg (0.91 mmol) des racemischen Gemisches von 1-(3-{[2-(4-Chlorphenyl)-4,4,4-trifluor-3-methylbutanoyl]amino}benzyl)cyclopropancarbonsäure (Beispiel 45) wurden mittels präparativer HPLC an chiraler Phase in die Enantiomere aufgetrennt [Säule: Daicel Chiralpak AD-H, 5 µm, 250 mm x 20 mm; Elutionsmittel: Isohexan/(Ethanol + 0.2% Trifluoressigsäure + 1% Wasser) 90:10 (v/v); Fluss: 15 ml/min; UV-Detektion: 220 nm; Temperatur: 30°C]:

### Beispiel 84 (Enantiomer 1):

Ausbeute: 247 mg (noch Lösungsmittel-Reste enthaltend)

Rₜ = 7.42 min; >99% ee
[Säule: Daicel Chiralpak AD-H, 5 µm, 250 mm x 4.6 mm; Elutionsmittel: Isohexan/(Ethanol + 0.2% Trifluoressigsäure + 1% Wasser) 90:10 (v/v); Fluss: 1 ml/min; UV-Detektion: 220 nm; Temperatur: 25°C].

LC-MS (Methode 5): Rₜ = 1.20 min; m/z = 438/440 (M-H)⁻.

[α]_{D}²⁰ = +60.8°, c = 0.35, Methanol.

### Beispiel 85 (Enantiomer 2):

Ausbeute: 288 mg (noch Lösungsmittel-Reste enthaltend)

Rt = 9.18 min; >99% ee
[Säule: Daicel Chiralpak AD-H, 5 µm, 250 mm x 4.6 mm; Elutionsmittel: Isohexan/(Ethanol + 0.2% Trifluoressigsäure + 1% Wasser) 90:10 (v/v); Fluss: 1 ml/min; UV-Detektion: 220 nm; Temperatur: 25°C].

LC-MS (Methode 5): Rₜ = 1.20 min; m/z = 438/440 (M-H)⁻.

[α]_{D}²⁰ =-58.1°, c = 0.37, Methanol.

### Beispiel 86 und Beispiel 87

### 1-(3-{[4,4,4-Trifluor-3-methyl-2-(4-methylphenyl)butanoyl]amino}benzyl)cyclopropancarbonsäure (Enantiomer 1 und 2)

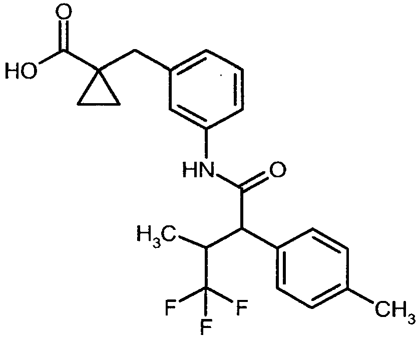

339 mg (0.81 mmol) des racemischen Gemisches von 1-(3-{[4,4,4-Trifluor-3-methyl-2-(4-methyl-phenyl)butanoyl]amino}benzyl)cyclopropancarbonsäure (Beispiel 47) wurden mittels präparativer HPLC an chiraler Phase in die Enantiomere aufgetrennt [Säule: Daicel Chiralpak AD-H, 5 µm, 250 mm x 20 mm; Elutionsmittel: Isohexan/(Isopropanol + 0.2% Trifluoressigsäure + 1% Wasser) 75:25 (v/v); Fluss: 15 ml/min; UV-Detektion: 220 nm; Temperatur: 40°C]:

### Beispiel 86 (Enantiomer 1):

Ausbeute: 192 mg (noch Lösungsmittel-Reste enthaltend)

Rₜ = 4.40 min; >99% ee
[Säule: Daicel Chiralpak AD-H, 5 µm, 250 mm x 4.6 mm; Elutionsmittel: Isohexan/(Isopropanol + 0.2% Trifluoressigsäure + 1% Wasser) 75:25 (v/v); Fluss: 1 ml/min; UV-Detektion: 220 nm; Temperatur: 25°C].

LC-MS (Methode 5): Rₜ = 1.16 min; m/z = 418 (M-H)⁻.

¹H-NMR (400 MHz, DMSO-d₆): δ [ppm] = 0.67-0.89 (m, 5H), 1.03-1.17 (m, 2H), 2.27 (s, 3H), 2.82 (s, 2H), 3.25-3.44 (m, 1H), 3.77 (d, 1H), 6.91 (d, 1H), 7.16 (d, 3H), 7.30 (d, 2H), 7.37 (s, 1H), 7.43 (d, 1H), 10.12 (s, 1H), 11.00-12.95 (br. s, 1H).

### Beispiel 87 (Enantiomer 2):

Ausbeute: 168 mg (noch Lösungsmittel-Reste enthaltend)

Rt = 5.10 min; >99% ee
[Säule: Daicel Chiralpak AD-H, 5 µm, 250 mm x 4.6 mm; Elutionsmittel: Isohexan/(.Isopropanol + 0.2% Trifluoressigsäure + 1% Wasser) 75:25 (v/v); Fluss: 1. ml/min; UV-Detektion: 220 nm; Temperatur: 25°C].

LC-MS (Methode 5): Rₜ = 1.16 min; m/z = 418 (M-H)⁻.

¹H-NMR: siehe Beispiel 86.

### Beispiel 88

### 1-[(4-Chlor-3-{[(2S,3R)-2-(4-chlorphenyl)-4,4,4-trifluor-3-methylbutanoyl]amino}phenyl)-(methoxy)methyl]cyclopropancarbonsäure (Diastereomerengemisch)

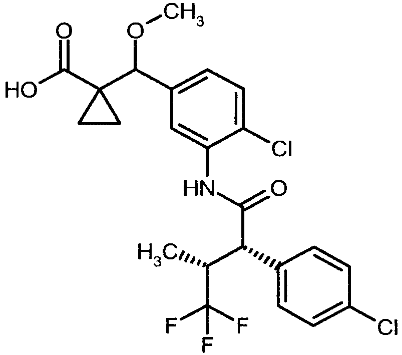

151 mg (0.269 mmol) *tert*.-Butyl-1-[(4-chlor-3-{[(2S,3R)-2-(4-chlorphenyl)-4,4,4-trifluor-3-methylbutanoyl]amino}phenyl)(methoxy)methyl]cyclopropancarboxylat (als Diastereomerengemisch) wurden in 2.9 ml Dichlormethan gelöst und bei RT mit 1.0 ml TFA versetzt. Die Reaktionsmischung wurde nach 30 min bei RT im Vakuum eingeengt und der Rückstand im Hochvakuum getrocknet. Das so erhaltene Rohprodukt wurde durch RP-HPLC gereinigt (Acetonitril/Wasser-Eluent). Es wurden 87 mg (64% d. Th.) der Zielverbindung erhalten.

LC-MS (Methode 4): Rₜ = 1.44 min; m/z = 521 (M+NH₄)⁺.

¹H-NMR (400 MHz, DMSO-*d₆*): *beide Diastereomere:* δ [ppm] = 0.37-0.45 (m, 1H), 0.80 (d, 3H), 0.82-1.05 (m, 3H), 3.13/3.14 (je s, zus. 3H), 4.13 (d, 1H), 4.87 (s, 1H), 6.98-7.16 (m, 1H), 7.36-7.55 (m, 6H), 9.91 (s, 1H).

### Beispiel 89 und Beispiel 90

(+)-1-[(4-Chlor-3-{[(2*S*,3*R*)-2-(4-chlorphenyl)-4,4,4-trifluor-3-methylbutanoyl]amino}phenyl)-(methoxy)methyl]cyclopropancarbonsäure *(Diastereomer 1 und 2)*

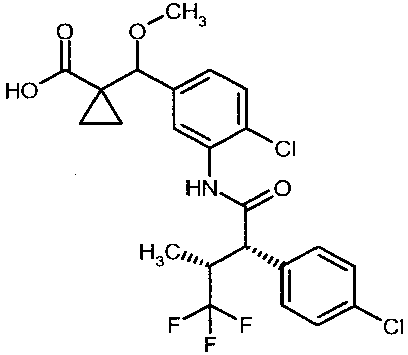

Das oben erhaltene Diastereomerengemisch der 1-[(4-Chlor-3-{[(2*S*,3*R*)-2-(4-chlorphenyl)-4,4,4-trifluor-3-methylbutanoyl]amino}phenyl)(methoxy)methyl]cyclopropancarbonsäure (Beispiel 88) wurde durch präparative HPLC an chiraler Phase weiter aufgetrennt [Säule: Daicel Chiralpak AD-H, 5 µm, 250 mm x 20 mm; Injektionsvolumen: 0.40 ml; Temperatur: 25°C; Eluent: 90% Isohexan / 10% Isopropanol; Fluss: 15 ml/min; Detektion: 220 nm]. Ausgehend von 63 mg Diastereomerengemisch wurden 26 mg an Diastereomer 1 *(Beispiel 89)* und 34 mg an Diastereomer 2 *(Beispiel 90)* isoliert.

### Beispiel 89 (Diastereomer 1)

LC-MS (Methode 5): Rₜ = 1.22 min; m/z = 502 (M-H)⁻.

¹H-NMR (400 MHz, DMSO-*d₆*): δ [ppm] = 0.35-0.50 (m, 1H), 0.80 (d, 3H), 0.83-1.05 (m, 3H), 3.13 (s, 3H), 4.13 (d, 1H), 4.86 (s, 1H), 7.10 (dd, 1H), 7.38-7.54 (m, 6H), 9.91 (s, 1H), 12.33 (br. s, 1H).

[α]_{D}²⁰ = +28°, c = 0.255, Chloroform.

### Beispiel 90 (Diastereomer 2):

LC-MS (Methode 5): Rₜ = 1.22 min; m/z = 502 (M-H)⁻.

¹H-NMR (400 MHz, DMSO-*d₆*): δ [ppm] = 0.36-0.52 (m, 1H), 0.80 (d, 3H), 0.82-1.04 (m, 3H), 3.14 (s, 3H), 4.13 (d, 1H), 4.86 (s, 1H), 7.09 (dd, 1H), 7.41 (d, 1H), 7.44-7.52 (m, 5H), 9.91 (s, 1H), 12.35 (br. s, 1H).

[α]_{D}²⁰ = +66°, c = 0.240, Chloroform.

### Beispiel 91

### 1-{(4-Chlor-3-{[(2S,3R)-2-(4-chlorphenyl)-4,4,4-trifluor-3-methylbutanoyl]amino}phenyl)-(trideuteromethoxy)methyl}cyclopropancarbonsäure (Diastereomerengemisch)

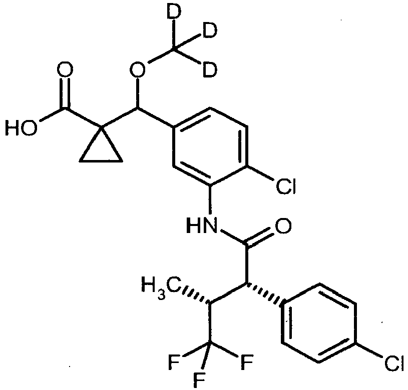

885 mg (1.57 mmol) *tert*.-Butyl-1-{(4-chlor-3-{[(2*S*,3*R*)-2-(4-chlorphenyl)-4,4,4-trifluor-3-methyl-butanoyl]amino}phenyl)(trideuteromethoxy)methyl}cyclopropancarboxylat (als Diastereomerengemisch) wurden in 2 ml Dichlormethan gelöst und bei RT mit 2.4 ml TFA versetzt. Die Reaktionsmischung wurde 2 h bei RT gerührt, dann im Vakuum eingeengt und der Rückstand im Hochvakuum getrocknet. Das so erhaltene Rohprodukt wurde durch RP-HPLC gereinigt (Methanol/ Wasser-Eluent). Es wurden 456 mg (57% d. Th.) der Zielverbindung erhalten.

LC-MS (Methode 5): Rₜ = 1.22 min; m/z = 505/507 (M-H)⁻.

¹H-NMR (400 MHz, DMSO-*d₆*): δ [ppm] = 0.41-0.49 (m, 1H), 0.80 (d, 3H), 0.83-1.04 (m, 3H), 3.29-3.45 (m, 1H, teilweise verdeckt durch H₂O-Signal), 4.13 (d, 1H), 4.86 (s, 1H), 7.07-7.12 (m, 1H), 7.39-7.51 (m, 6H), 9.91 (s, 1H), 12.21-12.51 (br. s, 1H).

Die folgenden Beispiele wurden gemäß der Allgemeinen Vorschrift 8 hergestellt:

| **Beispiel** | **Name / Struktur / Edukt** | **Analytische Daten** |
|---|---|---|
| **92** | 1-[(4-Chlor-3-{[(2*S*,3*R*)-2-(4-chlorphenyl)-4,4,4-trifluor-3-methylbutanoyl]amino}phenyl)-(ethoxy)methyl]cyclopropancarbonsäure *(Diastereomerengemisch)* | LC-MS (Methode 7): Rₜ = 1.29 min; m/z = 516/518 (M-H)⁻. |
| | | ¹H-NMR (400 MHz,, DMSO-*d₆*): δ [ppm] = 0.39-0.49 (m, 1H), 0.80 (d, 3H), 0.83-0.96 (m, 2H), 0.97-1.08 (m, 4H), 3.21-3.46 (m, 3H, teilweise verdeckt durch H₂O-Signal), 4.13 (d, 1H), 4.97 (d, 1H), 7.07-7.13 (m, 1H), 7.40 (d, 1H), 7.43-7.51 (m, 5H), 9.90 (d, 1H), 12.22-12.42 (br. s, 1H). |
| | 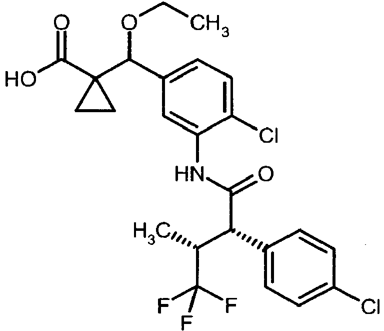 | |
| | aus *tert*.-Butyl-1-[(4-chlor-3-{[(2*S*,3*R*)-2-(4-chlor-phenyl)-4,4,4-trifluor-3-methylbutanoyl]amino}-phenyl)(ethoxy)methyl]cyclopropancarboxylat | |
| **93** | 1-[(4-Chlor-3-{[(2*S*,3*R*)-2-(4-chlorphenyl)-4,4,4-trifluor-3-methylbutanoyl]amino}phenyl)-(hydroxy)methyl]cyclopropancarbonsäure *(Diastereomerengemisch)* | LC-MS (Methode 7): Rₜ = 1.11 min; m/z = 488/490 (M-H)⁻. |
| | | ¹H-NMR (400 MHz, DMSO-*d₆*): δ [ppm] = 0.65-0.75 (m, 1H), 0.80 (d, 3H), 0.88-0.96 (m, 1H), 0.96-1.06 (m, 2H), 3.28-3.45 (m, 1H, teilweise verdeckt durch H₂O-Signal), 4.11 (d, 1H), 5.06 (s, 1H), 5.42 (d, 1H), 7.15 (dd, 1H), 7.36 (dd, 1H), 7.43-7.50 (m, 4H), 7.53 (dd, 1H), 9.85 (s, 1H), 12.18 (s, 1H). |
| | 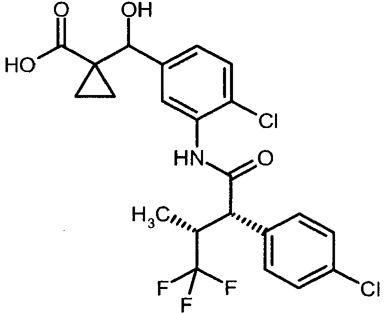 | |
| | aus *tert*.-Butyl-1-[(4-chlor-3-{[(2*S*,3*R*)-2-(4-chlor-phenyl)-4,4,4-trifluor-3-methylbutanoyl]amino}-phenyl)(hydroxy)methyl]cyclopropancarboxylat | |
| | oder als Nebenprodukt der Esterspaltung von *tert.-*Butyl-1-[(4-chlor-3-{[(2*S*,3*R*)-2-(4-chlorphenyl)-4,4,4-trifluor-3-methylbutanoyl]amino}phenyl)-(ethoxy)methyl]cyclopropancarboxylat | |
| **94** | 1-{(4-Chlor-3-{[(2*S*,3*R*)-2-(4-chlorphenyl)-4,4,4-trifluor-3-methylbutanoyl]amino}phenyl)-(trideuteromethoxy)methyl} cyclobutan-carbonsäure *(Diastereomerengemisch)* | LC-MS (Methode 5): Rₜ = 1.26 min; m/z = 519/521 (M-H)⁻. |
| | | ¹H-NMR (400 MHz, DMSO-*d₆*): δ [ppm] = 0.80 (d, 3H), 1.28-1.41 (m, 1H), 1.57-1.71 (m, 1H), 1.98-2.30 (m, 4H), 3.26-3.45 (m, 1H, teilweise verdeckt durch H₂O-Signal), 4.15 (d, 1H), 4.38 (d, 1H), 7.08 (dd, 1H), 7.41-7.51 (m, 5H), 7.54 (d, 1H), 9.90 (s, 1H), 12.33 (br. s, 1H). |
| | 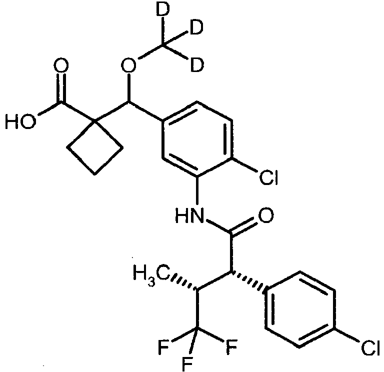 | |
| | aus *tert.* -Butyl-1-{(4-chlor-3-{[(2*S*,3*R*)-2-(4-chlor-phenyl)-4,4,4-trifluor-3-methylbutanoyl]amino}-phenyl)(trideuteromethoxy)methyl}-cyclobutancarboxylat | |
| **95** | 1-[(4-Chlor-3-{[(2*S*,3*R*)-2-(4-chlorphenyl)-4,4,4-trifluor-3-methylbutanoyl]amino}phenyl)-(methoxy)methyl]cyclobutancarbonsäure *(Diastereomerengemisch)* | LC-MS (Methode 5): Rₜ = 1.23 min; m/z = 516/518 (M-H)⁻. |
| | | ¹H-NMR (400 MHz, DMSO-*d₆*): δ [ppm] = 0.80 (d, 3H), 1.28-1.41 (m, 1H), 1.57-1.71 (m, 1H), 1.97-2.30 (m, 4H), 3.13 (s, 3H), 3.26-3.45 (m, 1H, teilweise verdeckt durch H₂O-Signal), 4.15 (d, 1H), 4.38 (d, 1H), 7.08 (dd, 1H), 7.40-7.51 (m, 5H), 7.54 (d, 1H), 9.90 (s, 1H), 12.12-12.53 (br. s, 1H). |
| | 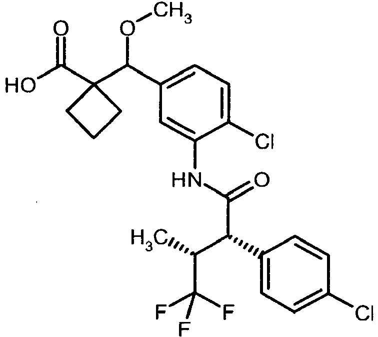 | |
| | aus *tert*.-Butyl-1-[(4-chlor-3-{[(2*S*,3*R*)-2-(4-chlor-phenyl)-4,4,4-trifluor-3-methylbutanoyl]amino}-phenyl)(methoxy)methyl]cyclobutancarboxylat | |
| **96** | 1-[(4-Chlor-3-{[(*2S*,3*R*)-2-(4-chlorphenyl)-4,4,4-trifluor-3-methylbutanoyl]amino}phenyl)-(ethoxy)methyl]cyclobutancarbonsäure *(Diastereomerengemisch)* | LC-MS (Methode 5): Rₜ = 1.32 min; m/z = 530/532 (M-H)⁻. |
| | | ¹H-NMR (400 MHz, DMSO-*d₆*): δ [ppm] = 0.80 (d, 3H), 1.03-1.10 (m, 3H), 1.27-1.41 (m, 1H), 1.56-1.70 (m, 1H), 1.97-2.29 (m, 4H), 3.23-3.46 (m, 3H, teilweise verdeckt durch H₂O-Signal), 4.14 (d, 1H), 4.49 (d, 1H), 7.09 (dd, 1H), 7.39-7.51 (m, 5H), 7.56 (dd, 1H), 9.89 (s, 1H), 12.27-12.39 (br. s, 1H). |
| | 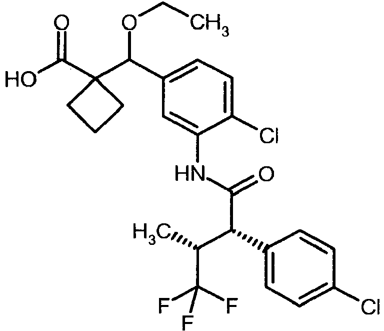 | |
| | aus *tert*.-Butyl-1-[(4-chlor-3-{[(2*S*,3*R*)-2-(4-chlor-phenyl)-4,4,4-trifluor-3 -methylbutanoyl]amino}-phenyl)(ethoxy)methyl]cyclobutancarboxylat | |
| **97** | 1-(3-{[(2*S*,3*R*)-2-(4-Chlorphenyl)-4,4,4-trifluor-3-methylbutanoyl]amino}-4-cyclopropyl-benzyl)cyclopropancarbonsäure | LC-MS (Methode 5): Rₜ = 1.20 min; m/z = 478/480 (M-H)⁻. |
| | | ¹H-NMR (400 MHz, DMSO-*d₆*): δ [ppm] = 0.28-0.35 (m, 1H), 0.39-0.47 (m, 1H), 0.54-0.68 (m, 2H), 0.70-0.78 (m, 2H), 0.81 (d, 3H), 1.02-1.11 (m, 2H), 1.60-1.70 (m, 1H), 2.79 (s, 2H), 3.28-3.45 (m, 1H, teilweise verdeckt durch H₂O-Signal), 4.01 (d, 1H), 6.80 (d, 1H), 6.96 (dd, 1H), 7.12 (d, 1H), 7.39-7.52 (m, 4H), 9.67 (s, 1H), 12.10 (br. s, 1H). |
| | 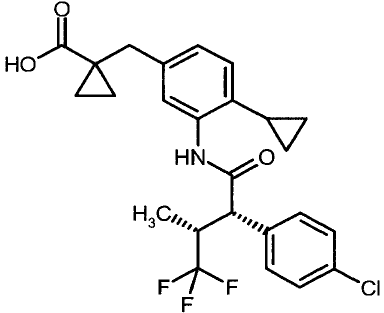 | |
| | aus *tert.*-Butyl-1-(3-{[(2S,3R)-2-(4-chlorphenyl)-4,4,4-trifluor-3-methylbutanoyl]amino}-4-cyclopropylbenzyl)cyclopropancarboxylat | |

### Beispiel 98

### 1-[1-(4-Chlor-3-{[(2S,3R)-2-(4-chlorphenyl)-4,4,4-tritluor-3-methylbutanoyl]amino}phenyl)-propyl]cyclopropancarbonsäure (Diastereomerengemisch)

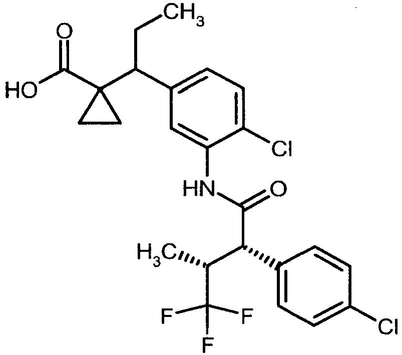

79 mg (0.15 mmol) Methyl-1-[1-(4-chlor-3-{[(2*S*,3*R*)-2-(4-chlorphenyl)-4,4,4-trifluor-3-methyl-butanoyl]amino}phenyl)propyl]cyclopropancarboxylat (als Diastereomerengemisch) wurden in 3 ml Essigsäure gelöst, mit 1.5 ml konzentrierter Salzsäure versetzt, auf 100°C erhitzt und 12 h bei dieser Temperatur gerührt. Anschließend wurde die Reaktionsmischung auf Raumtemperatur abgekühlt, mit Wasser verdünnt und dreimal mit Dichlormethan extrahiert. Die vereinigten organischen Phasen wurden über Magnesiumsulfat getrocknet und im Vakuum eingeengt. Der erhaltene Rückstand wurde durch präparative RP-HPLC gereinigt (Eluent Methanol/Wasser 7:3). Es wurden so 36 mg der Zielverbindung erhalten (43% d. Th.).

LC-MS (Methode 7): Rₜ = 1.33 min; m/z = 500/502 (M-H)⁻.

¹H-NMR (400 MHz, DMSO-*d₆*): *beide Diastereomere:* δ [ppm] = 0.47-0.58 (m, 1H), 0.68-0.77 (m, 4H), 0.80 (d, 3H), 0.93-1.06 (m, 2H), 1.65-1.89 (m, 2H), 2.65-2.76 (m, 1H), 3.27-3.45 (m, 1H, teilweise verdeckt durch H₂O-Signal), 4.12 (d, 1H), 7.10 (d, 1H), 7.34 (d, 1H), 7.39-7.50 (m, 5H), 9.84 (s, 1H), 12.00-12.23 (br. s, 1H).

### Beispiel 99 und Beispiel 100

### (+)-1-{(4-Chlor-3-{[(2S,3R)-2-(4-chlorphenyl)-4,4,4-trifluor-3-methylbutanoyl]amino}phenyl)-(trideuteromethoxy)methyl}cyclopropancarbonsäure (Diastereomer 1 und 2)

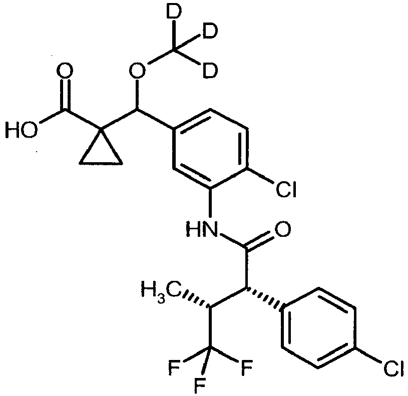

430 mg (0.76 mmol) des Diastereomerengemisches von 1-{(4-Chlor-3-{[(2*S*,3*R*)-2-(4-chlor-phenyl)-4,4,4-trifluor-3-methylbutanoyl]amino}phenyl)(trideuteromethoxy)methyl}cyclopropan-carbonsäure (Beispiel 91) wurden mittels präparativer HPLC an chiraler Phase weiter aufgetrennt [Säule: Daicel Chiralpak AD-H, 5 µm, 250 mm x 20 mm; Elutionsmittel: Isohexan/Isopropanol 90:10 (v/v); Fluss: 20 ml/min; UV-Detektion: 230 nm; Temperatur: 25°C]:

### Beispiel 99 (Diastereomer 1):

Ausbeute: 180 mg

Rₜ = 6.51 min; chemische Reinheit >99%; >99% de
[Säule: Daicel Chiralpak AD-H, 5 µm, 250 mm x 4 mm; Elutionsmittel: Isohexan/(Isopropanol + 0.2% Trifluoressigsäure) 90:10 (v/v); Fluss: 1 ml/min; UV-Detektion: 220 nm; Temperatur: 25°C].

LC-MS (Methode 5): Rₜ = 1.18 min; m/z = 505/507 (M-H)⁻.

¹H-NMR (400 MHz, DMSO-*d₆*): δ [ppm] = 0.42-0.49 (m, 1H), 0.80 (d, 3H), 0.83-0.90 (m, 1H), 0.90-0.97 (m, 1H), 0.97-1.04 (m, 1H), 3.30-3.47 (m, 1H, teilweise verdeckt durch H₂O-Signal), 4.13 (d, 1H), 4.85 (s, 1H), 7.10 (dd, 1H), 7.42 (d, 1H), 7.43-7.51 (m, 5H), 9.91 (s, 1H), 12.22-12.44 (br. s, 1H).

[α]_{D}²⁰ = +54.0°, c = 0.51, Chloroform.

### Beispiel 100 (Diastereomer 2):

Ausbeute: 215 mg

Rₜ = 7.68 min; chemische Reinheit >99%; >99% de
[Säule: Daicel Chiralpak AD-H, 5 µm, 250 mm x 4 mm; Elutionsmittel: Isohexan/(Isopropanol + 0.2% Trifluoressigsäure) 90:10 (v/v); Fluss: 1 ml/min; UV-Detektion: 220 nm; Temperatur: 25°C].

LC-MS (Methode 5): Rₜ = 1.18 min; m/z = 505/507 (M-H)⁻.

¹H-NMR (400 MHz, DMSO-*d₆*): δ [ppm] = 0.41-0.48 (m, 1H), 0.80 (d, 3H), 0.83-0.96 (m, 1H), 0.97-1.06 (m, 1H), 3.29-3.49 (m, 1H, teilweise verdeckt durch H₂O-Signal), 4.14 (d, 1H), 4.86 (s, 1H), 7.09 (dd, 1H), 7.41 (d, 1H), 7.43-7.52 (m, 5H), 9.91 (s, 1H), 12.08-12.54 (br. s, 1H).

[α]_{D}²⁰ = +96.4°, c = 0.47, Chloroform.

### Beispiel 101 und Beispiel 102

### 1-[(4-Chlor-3-{[(2S,3R)-2-(4-chlorphenyl)-4,4,4-trifluor-3-methylbutanoyl]amino}phenyl)-(ethoxy)methyl]cyclopropancarbonsäure (Diastereomer 1 und 2)

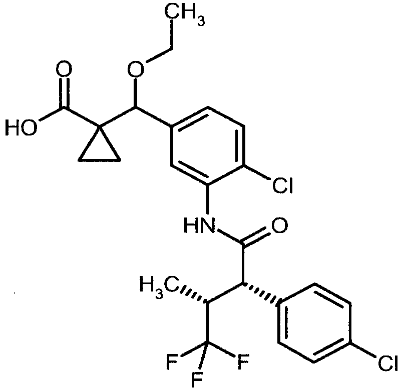

130 mg (0.25 mmol) des Diastereomerengemisches von 1-[(4-Chlor-3-{[(2*S*,3*R*)-2-(4-chlorphenyl)-4,4,4-trifluor-3-methylbutanoyl]amino}phenyl)(ethoxy)methyl]cyclopropancarbonsäure (Beispiel 92) wurden mittels präparativer HPLC an chiraler Phase weiter aufgetrennt [Säule: Daicel Chiralpak AD-H, 5 µm, 250 mm x 20 mm; Elutionsmittel: Isohexan/Isopropanol 93:7 (v/v); Fluss: 20 ml/min; UV-Detektion: 230 nm; Temperatur: 25°C]:

### Beispiel 101 (Diastereomer 1):

Ausbeute: 56 mg (noch Lösungsmittel-Reste enthaltend)

Rₜ = 5.79 min; >99% de
[Säule: Daicel Chiralpak AD-H, 5 µm, 250 mm x 4 mm; Elutionsmittel: Isohexan/(Isopropanol + 0.2% Trifluoressigsäure) 90:10 (v/v); Fluss: 1 ml/min; UV-Detektion: 220 nm; Temperatur: 25°C].

LC-MS (Methode 5): Rₜ = 1.27 min; m/z = 516/518 (M-H)⁻.

¹H-NMR (400 MHz, DMSO-*d₆*): δ [ppm] = 0.41-0.50 (m, 1H), 0.80 (d, 3H), 0.83-0.89 (m, 1H), 0.89-0.96 (m, 1H), 0.97-1.07 (m, 1H), 1.04 (t, 3H), 3.21-3.46 (m, 3H, teilweise verdeckt durch H₂O-Signal), 4.13 (d, 1H), 4.96 (s, 1H), 7.10 (dd, 1H), 7.40 (d, 1H), 7.43-7.51 (m, 5H), 9.89 (s, 1H), 12.29 (br. s, 1H).

[α]_{D}²⁰ = +54.0°, c = 0.42, Chloroform.

### Beispiel 102 (Diastereomer 2):

Ausbeute: 77 mg (noch Lösungsmittel-Reste enthaltend)

Rₜ = 6.17 min; >96% de
[Säule: Daicel Chiralpak AD-H, 5 µm, 250 mm x 4 mm; Elutionsmittel: Isohexan/(Isopropanol + 0.2% Trifluoressigsäure) 90:10 (v/v); Fluss: 1 ml/min; UV-Detektion: 220 nm; Temperatur: 25°C].

LC-MS (Methode 5): Rₜ = 1.27 min; m/z = 516/518 (M-H)⁻.

¹H-NMR (400 MHz, DMSO-*d₆*): 8 [ppm] = 0.40-0.47 (m, 1H), 0.80 (d, 3H), 0.83-0.95 (m, 2H), 0.97-1.10 (m, 1H), 1.05 (t, 3H), 3.21-3.46 (m, 3H, teilweise verdeckt durch H₂O-Signal), 4.13 (d, 1H), 4.97 (s, 1H), 7.09 (dd, 1H), 7.40 (d, 1H), 7.44-7.51 (m, 5H), 9.90 (s, 1H), 12.17-12.40 (br. s, 1H).

[α]_{D}²⁰ = +93.6°, c = 0.405, Chloroform.

### Beispiel 103 und Beispiel 104

### 1-[(4-Chlor-3-{[(2S,3R)-2-(4-chlorphenyl)-4,4,4-trifluor-3-methylbutanoyl]amino}phenyl)-(hydroxy)methyl]cyclopropancarbonsäure (Diastereomer 1 und 2)

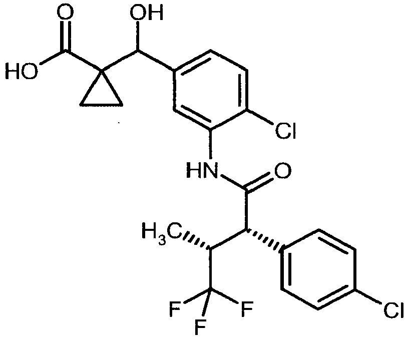

850 mg (1.73 mmol) des Diastereomerengemisches von 1-[(4-Chlor-3-{[(2*S*,3*R*)-2-(4-chlorphenyl)-4,4,4-trifluor-3-methylbutanoyl]amino}phenyl)(hydroxy)methyl]cyclopropancarbonsäure (Beispiel 93) wurden durch superkritische Flüssigchromatographie (SFC) an chiraler Phase weiter aufgetrennt [Säule: Daicel Chiralpak AD-H, 5 µm, 250 mm x 20 mm; Elutionsmittel: Kohlendioxid/Methanol 70:30 (v/v); Fluss: 100 ml/min; Druck: 120 bar; Temperatur: 40°C; UV-Detektion: 210 nm]:

### Beispiel 103 (Diastereomer 1):

Ausbeute: 271 mg

Rₜ = 11.44 min; chemische Reinheit >95%; >99% de
[Säule: Daicel Chiralpak AD-H, 5 µm, 250 mm x 4.6, mm; Elutionsmittel: Isohexan/(Methanol + 0.2% Trifluoressigsäure) 90:10 (v/v); Fluss: 1.5 ml/min; UV-Detektion: 210 nm; Temperatur: 30°C].

LC-MS (Methode 4): Rₜ = 1.32 min; m/z = 488/490 (M-H)⁻.

¹H-NMR (400 MHz, DMSO-*d₆*): δ [ppm] = 0.65-0.74 (m, 1H), 0.80 (d, 3H), 0.87-0.94 (m, 1H), 0.96-1.05 (m, 2H), 3.27-3.45 (m, 1H, teilweise verdeckt durch H₂O-Signal), 4.11 (d, 1H), 5.06 (s, 1H), 5.76 (s, ca. 1H), 7.15 (dd, 1H), 7.36 (d, 1H), 7.41-7.50 (m, 4H), 7.52 (d, 1H), 9.85 (s, 1H), 11.75-12.64 (br. s, ca. 1H).

[α]_{D}²⁰ = +96.1°, c = 0.47, Chloroform.

### Beispiel 104 (Diastereomer 2):

Ausbeute: 290 mg

Rₜ = 15.24 min; chemische Reinheit >96%; >99% de
[Säule: Daicel Chiralpak AD-H, 5 µm, 250 mm x 4.6 mm; Elutionsmittel: Isohexan/(Methanol + 0.2% Trifluoressigsäure) 90:10 (v/v); Fluss: 1.5 ml/min; UV-Detektion: 210 nm; Temperatur: 30°C].

LC-MS (Methode 4): Rₜ = 1.32 min; m/z = 488/490 (M-H)⁻.

¹H-NMR (400 MHz, DMSO-*d₆*): δ [ppm] = 0.64-0.73 (m, 1H), 0.80 (d, 3H), 0.88-0.96 (m, 1H), 0.96-1.05 (m, 2H), 3.26-3.44 (m, 1H, teilweise verdeckt durch H₂O-Signal), 4.11 (d, 1H), 5.05 (s, 1H), 5.76 (s, ca. 1H), 7.15 (dd, 1H), 7.36 (d, 1H), 7.42-7.50 (m, 4H), 7.54 (d, 1H), 9.85 (s, 1H), 11.52-1.2.80 (br. s, ca. 1H).

[α]_{D}²⁰ = +57.3°, c = 0.465, Chloroform.

### Beispiel 105 und Beispiel 106

### 1-{(4-Chlor-3-{[(2S,3R)-2-(4-chlorphenyl)-4,4,4-trifluor-3-methylbutanoyl]amino}phenyl)-(trideuteromethoxy)methyl}cyclobutancarbonsäure (Diastereomer 1 und 2)

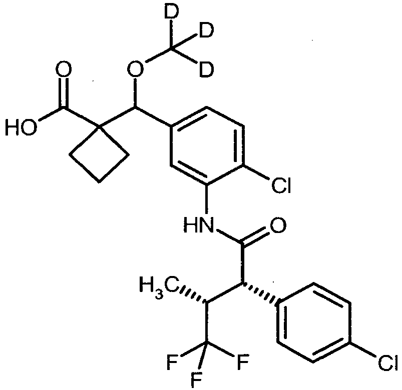

260 mg (0.50 mmol) des Diastereomerengemisches von 1-{(4-Chlor-3-{[(2S,3R)-2-(4-chlor-phenyl)-4,4,4-trifluor-3-methylbutanoyl]amino } phenyl)(trideuteromethoxy)methyl} cyclobutancarbonsäure (Beispiel 94) wurden mittels präparativer HPLC an chiraler Phase weiter aufgetrennt [Säule: Daicel Chiralpak AD-H, 5 µm, 250 mm x 20 mm; Elutionsmittel: Isohexan/Isopropanol 92:8 (v/v); Fluss: 11 ml/min; UV-Detektion: 220 nm; Temperatur: 23°C]:

### Beispiel 105 (Diastereomer 1):

Ausbeute: 127 mg

Rₜ = 8.28 min; chemische Reinheit >99%; >99% de
[Säule: Daicel Chiralpak AD-H, 5 µm, 250 mm x 4.6 mm; Elutionsmittel: Isohexan/(Isopropanol + 0.2% Trifluoressigsäure + 1% Wasser) 90:10 (v/v); Fluss: 0.8 ml/min; UV-Detektion: 220 nm; Temperatur: 25°C].

LC-MS (Methode 7): Rt = 1.29 min; m/z = 519/521 (M-H)⁻.

¹H-NMR (400 MHz, DMSO-*d₆*): δ [ppm] = 0.80 (d, 3H), 1.28-1.41 (m, 1H), 1.57-1.71 (m, 1H), 1.99-2.20 (m, 3H), 2.20-2.30 (m, 1H), 3.30-3.47 (m, 1H, teilweise verdeckt durch H₂O-Signal), 4.15 (d, 1H), 4.38 (d, 1H), 7.08 (dd, 1H), 7.43 (d, 1H), 7.44-7.51 (m, 4H), 7.54 (d, 1H), 9.91 (s, 1H), 12.24-12.45 (br. s, 1H).

[α]_{D}²⁰ = +25.4°, c = 0.41, Chloroform.

### Beispiel 1.06 (Diastereomere 2):

Ausbeute: 94 mg

Rₜ = 9.05 min; chemische Reinheit >99%; >98% de
[Säule: Daicel Chiralpak AD-H, 5 µm, 250 mm x 4.6 mm; Elutionsmittel: Isohexan/(Isopropanol + 0.2% Trifluoressigsäure + 1% Wasser) 90:10 (v/v); Fluss: 0.8 ml/min; UV-Detektion: 220 nm; Temperatur: 25°C].

LC-MS (Methode 7): Rₜ = 1.29 min; m/z = 519/521 (M-H)⁻.

¹H-NMR (400 MHz, DMSO-*d₆*): δ [ppm] = 0.80 (d, 3H), 1.27-1.40 (m, 1H), 1.57-1.71 (m, 1H), 1.98-2.19 (m, 3H), 2.19-2.29 (m, 1H), 3.27-3.50 (m, 1H, teilweise verdeckt durch H₂O-Signal), 4.15 (d, 1H), 4.38 (d, 1H), 7.09 (dd, 1H), 7.41-7.51 (m, 5H), 7.54 (d, 1H), 9.90 (s, 1H), 12.21-12.47 (br. s, 1H).

[α]_{D}²⁰ = +54.0°, c = 0.51, Chloroform.

### Beispiel 107 und Beispiel 108

### 1-[(4-Chlor-3-{[(2S,3R)-2-(4-chlorphenyl)-4,4,4-trifluor-3-methylbutanoyl]amino}phenyl)-(methoxy)methyl]cyclobutancarbonsäure (Diastereomer 1 und 2)

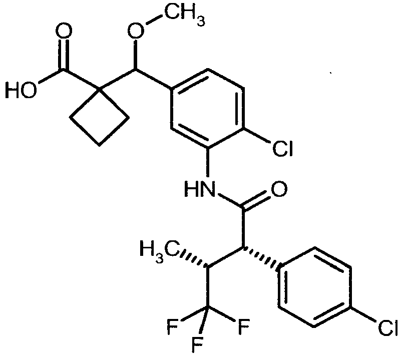

220 mg (0.44 mmol) des Diastereomerengemisches von 1-[(4-Chlor-3-{[(2*S*,3*R*)-2-(4-chlorphenyl)-4,4,4-trifluor-3-methylbutanoyl]amino}phenyl)(methoxy)methyl]cyclobutancarbonsäure (Beispiel 95) wurden mittels präparativer HPLC an chiraler Phase weiter aufgetrennt [Säule: Daicel Chiralpak IC, 5 µm, 250 mm x 20 mm; Elutionsmittel: Isohexan/Isopropanol 92:8 (v/v); Fluss: 15 ml/min; UV-Detektion: 220 nm; Temperatur: 30°C]:

### Beispiel 107 (Diastereomer 1):

Ausbeute: 113 mg

Rₜ = 5.59 min; chemische Reinheit >96.5%; >99% de
[Säule: Daicel Chiralpak IC, 5 µm, 250 mm x 4.6 mm; Elutionsmittel: Isohexan/(Isopropanol + 0.2% Trifluoressigsäure + 1% Wasser) 95:5 (v/v); Fluss: 1 ml/min; UV-Detektion: 220 nm; Temperatur: 25°C].

LC-MS (Methode 4): Rₜ = 1.49 min; m/z = 516/518 (M-H)⁻.

¹H-NMR (400 MHz, DMSO-*d₆*): δ [ppm] = 0.80 (d, 3H), 1.28-1.41 (m, 1H), 1.57-1.71 (m, 1H), 1.99-2.30 (m, 4H), 3.13 (s, 3H), 3.29-3.46 (m, 1H), 4.15 (d, 1H), 4.38 (d, 1H), 7.08 (dd, 1H), 7.41-7.51 (m, 5H), 7.54 (d, 1H), 9.90 (s, 1H), 12.03-12.68 (br. s, 1H).

[α]_{D}²⁰ = +25.4°, c = 0.41, Chloroform.

### Beispiel 108 (Diastereomer 2):

Ausbeute: 98 mg

Rₜ = 6.27 min; chemische Reinheit >99%; >99% de
[Säule: Daicel Chiralpak. IC, 5 µm, 250 mm x 4.6 mm; Elutionsmittel: Isohexan/(Isopropanol +

0.2% Trifluoressigsäure + 1% Wasser) 95:5 (v/v); Fluss: 1 ml/min; UV-Detektion: 220 nm; Temperatur: 25°C].

LC-MS (Methode 4): Rₜ = 1.49 min; m/z = 516/518 (M-H)⁻.

¹H-NMR (400 MHz, DMSO-*d₆*): δ [ppm] = 0.80 (d, 3H), 1.27-1.41 (m, 1H), 1.57-1.71 (m, 1H), 1.98-2.29 (m, 4H), 3.13 (s, 3H), 3.27-3.45 (m, 1H, teilweise verdeckt durch H₂O-Signal), 4.15 (d, 1H), 4.38 (d, 1H), 7.09 (dd, 1H), 7.41-7.51 (m, 5H), 7.54 (d, 1H), 9.90 (s, 1H), 12.02-12.62 (br. s, 1H).

[α]_{D}²⁰ = +54.0°, c = 0.51, Chloroform.

### Beispiel 109 und Beispiel 110

### 1-[(4-Chlor-3-{[(2S,3R)-2-(4-chlorphenyl)-4,4,4-trifluor-3-methylbutanoyl]amino}phenyl)-(ethoxy)methyl]cyclobutancarbonsäure (Diastereomere 1 und 2)

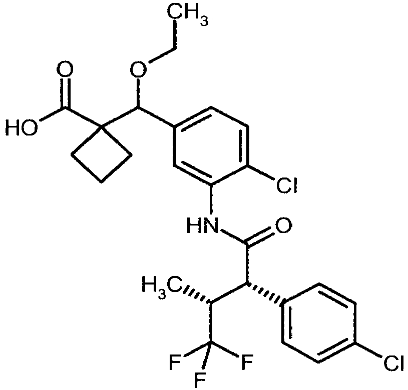

255 mg (0.48 mmol) des Diastereomerengemisches von 1-[(4-Chlor-3-{[(2*S*,3*R*)-2-(4-chlor-phenyl)-4,4,4-trifluor-3-methylbutanoyl]amino}phenyl)(ethoxy)methyl]cyclobutancarbonsäure (Beispiel 96) wurden mittels präparativer HPLC an chiraler Phase weiter aufgetrennt [Säule: Chiracel OZ-H, 5 µm, 250 mm x 20 mm; Elutionsmittel: Isohexan/Isopropanol 95:5 (v/v); Fluss: 15 ml/min; UV-Detektion: 220 nm; Temperatur: 25°C]:

### Beispiel 109 (Diastereomer 1):

Ausbeute: 77 mg

Rₜ = 5.35 min; chemische Reinheit >98%; >98.5% de
[Säule: Chiralcel OZ-H, 5 µm, 250 mm x 4.6 mm; Elutionsmittel: Isohexan/(Isopropanol + 0.2% Trifluoressigsäure + 1% Wasser) 95:5 (v/v); Fluss: 1 ml/min; UV-Detektion: 220 nm; Temperatur: 25°C].

LC-MS (Methode 5): Rₜ = 1.29 min; m/z = 530/532 (M-H)⁻.

¹H-NMR (400 MHz, DMSO-*d₆*): δ [ppm] = 0.80 (d, 3H), 1.06 (t, 3H), 1.28-1.41 (m, 1H), 1.56-1.70 (m, 1H), 1.97-2.29 (m, 4H), 3.28 (q, 2H, teilweise verdeckt durch H₂O-Signal), 3.33-3.45 (m, 1H, teilweise verdeckt durch H₂O-Signal), 4.14 (d, 1H), 4.48 (s, 1H), 7.09 (dd, 1H), 7.42 (d, 1H), 7.44-7.50 (m, 4H), 7.53-7.57 (m, 1H), 9.89 (s, 1H), 12.33 (br. s, 1H).

[α]_{D}²⁰ = +38.7°, c = 0.51, Chloroform.

### Beispiel 110 (Diastereomer 2):

Ausbeute: 60 mg

Rt = 5.77 min; chemische Reinheit >98%; >97.8% de
[Säule: Chiralcel OZ-H, 5 µm, 250 mm x 4.6 mm; Elutionsmittel: Isohexan/(Isopropanol + 0.2% Trifluoressigsäure + 1% Wasser) 95:5 (v/v); Fluss: 1 ml/min; UV-Detektion: 220 nm; Temperatur: 25°C].

LC-MS (Methode 5): Rₜ = 1.30 min; m/z = 530/532 (M-H)⁻.

¹H-NMR (400 MHz, DMSO-*d₆*): δ [ppm] = 0.80 (d, 3H), 1.07 (t, 3H), 1.27-1.40 (m, 1H), 1.56-1.69 (m, 1H), 1.96-2.29 (m, 4H), 3.28 (q, 2H, teilweise verdeckt durch H₂O-Signal), 3.32-3.47 (m, 1H, teilweise verdeckt durch H₂O-Signal), 4.14 (d, 1H), 4.49 (s, 1 H), 7.10 (dd, 1H), 7.42 (d, 1 H), 7.44-7.50 (m, 4H), 7.57 (d, 1H), 9.89 (s, 1H), 12.33 (br. s, 1H).

[α]_{D}²⁰ = +109.3°, c = 0.415, Chloroform.

Die folgenden beiden Beispiele wurden gemäß der Allgemeinen Vorschrift 8 hergestellt:

| **Beispiel** | **Name / Struktur / Edukt** | **Analytische Daten** |
|---|---|---|
| **111** | 1-[1-(4-Chlor-3-{[(2*S*,3*R*)-2-(4-chlorphenyl)-4,4,4-trifluor-3-methylbutanoyl]amino}phenyl)-1-hydroxyethyl]cyclopropancarbonsäure *(Diastereomerengemisch)* | LC-MS (Methode 5): Rₜ = 1.12 min; m/z = 502/504 (M-H)⁻. |
| | | ¹H-NMR (400 MHz, DMSO-*d₆*): δ [ppm] = 0.80 (d, 3H), 0.96-1.12 (m, 3H), 1.20-1.31 (m, 1H), 1.55 (s, 3H), 3.28-3.45 (m, 1H, teilweise verdeckt durch H₂O-Signal), 4.11 (d, 1H), 4.64-5.51 (br. s, ca. 1H), 7.25-7.36 (m, 2H), 7.42-7.51 (m, 4H), 7.70-7.75 (m, 1H), 9.83 (s, 1H), 11.40-12.40 (br. s, ca. 1H). |
| | 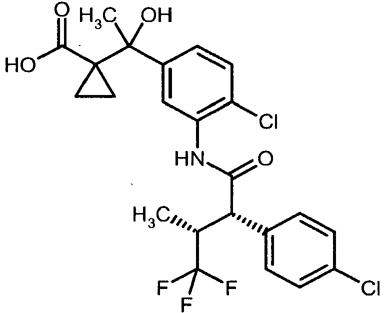 | |
| | aus *tert.*-Butyl-1-[1-(4-chlor-3-{[(2*S*,3*R*)-2-(4-chlorphenyl)-4,4,4-trifluor-3-methylbutanoyl]-amino}phenyl)-1-hydroxyethyl]cyclopropan-carboxylat *(Diastereomerengemisch)* | |
| **112** | 1-[(4-Chlor-3-{[(2*S*,3R)-2-(4-chlorphenyl)-4,4,4-trifluor-3-methylbutanoyl]amino}phenyl)-(cyclopropyloxy)methyl]cyclopropancarbonsäure *(Diastereomerengemisch)* | LC-MS (Methode 5): Rₜ = 1.28 min; m/z = 528/530 (M-H)⁻. |
| | 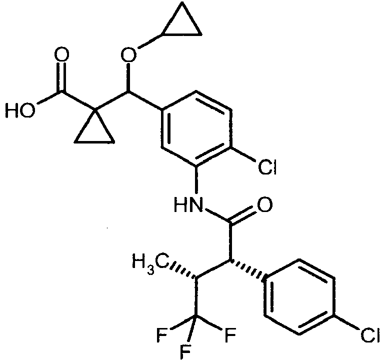 | |
| | aus tert.-Butyl-1-[(4-chlor-3-{[(2*S*,3*R*)-2-(4-chlor-phenyl)-4,4,4-trifluor-3-methylbutanoyl]amino}-phenyl)(cyclopropyloxy)methyl]cyclopropan-carboxylat *(Diastereomerengemisch)* | |

### Beispiel 113 und Beispiel 114

### 1-[(4-Chlor-3-{[(2S,3R)-2-(4-chlorphenyl)-4,4,4-trifluor-3-methylbutanoyl]amino}phenyl)-(cyclopropyloxy)methyl]cyclopropancarbonsäure (Diastereomer 1 und 2)

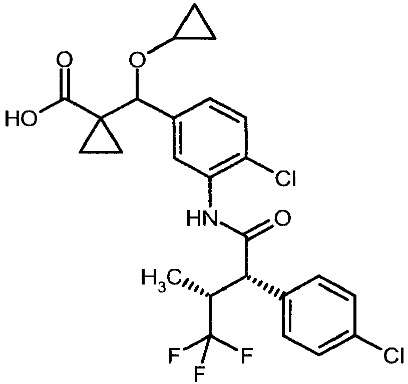

58 mg (0.11 mmol) des Diastereomerengemisches von 1-[(4-Chlor-3-{[(2S,3R)-2-(4-chlorphenyl)-4,4,4-trifluor-3-methylbutanoyl]amino}phenyl)(cyclopropyloxy)methyl]cyclopropancarbonsäure (Beispiel 112) wurden mittels präparativer HPLC an chiraler Phase weiter aufgetrennt [Säule: Daicel Chiracel OD-H, 5 µm, 250 mm x 20 mm; Elutionsmittel: Isohexan/(Ethanol + 0.2% Trifluoressigsäure + 1% Wasser) 95:5 (v/v); Fluss: 25 ml/min; UV-Detektion: 230 nm; Temperatur: 25°C]:

### Beispiel 113 (Diastereomer 1):

Ausbeute: 12 mg

Rₜ = 4.61 min; chemische Reinheit >99%; >99% de
[Säule: Daicel Chiralpak OD-H, 5 µm, 250 mm x 4.6 mm; Elutionsmittel: Isohexan/(Ethanol + 0.2% Trifluoressigsäure + 1% Wasser) 90:10 (v/v); Fluss: 1 ml/min; UV-Detektion: 220 nm; Temperatur: 25°C].

LC-MS (Methode 5): Rₜ = 1.26 min; m/z = 528/530 (M-H)⁻.

¹H-NMR (400 MHz, DMSO-*d₆*): δ [ppm] = 0.27-0.42 (m, 3H), 0.43-0.50 (m, 1H), 0.55-0.63 (m, 1H), 0.76-0.83 (m, 1H), 0.80 (d, 3H), 0.88-0.95 (m, 1H), 0.96-1.04 (m, 1H), 3.08-3.15 (m, 1H), 3.31-3.45 (m, 1H, teilweise verdeckt durch H₂O-Signal), 4.14 (d, 1H), 5.04 (s, 1H), 7.13 (dd, 1H), 7.42 (d, 1H), 7.44-7.51 (m, 5H), 9.92 (s, 1H), 12.10-12.55 (br. s, ca. 1H).

### Beispiel 114 (Diastereomer 2):

Ausbeute: 10 mg

Rt = 5.09 min; chemische Reinheit >99%; >98.5% de
[Säule: Daicel Chiralpak OD-H, 5 µm, 250 mm x 4.6 mm; Elutionsmittel: Isohexan/(Ethanol + 0.2% Trifluoressigsäure + 1% Wasser) 90:10 (v/v); Fluss: 1 ml/min; UV-Detektion: 220 nm; Temperatur: 25°C].

LC-MS (Methode 5): Rt = 1.26 min; m/z = 528/530 (M-H)⁻.

¹H-NMR (400 MHz, DMSO-*d₆*): δ [ppm] = 0.28-0.42 (m, 3H), 0.43-0.51 (m, 1H), 0.55-0.64 (m, 1H), 0.75-0.86 (m, 1H), 0.81 (d, 3H), 0.88-0.95 (m, 1H), 0.96-1.03 (m, 1H), 3.08-3.16 (m, 1H), 3.30-3.45 (m, 1H, teilweise verdeckt durch H₂O-Signal), 4.14 (d, 1H), 5.05 (s, 1H), 7.12 (dd, 1H), 7.42 (d, 1H), 7.44-7.53 (m, 5H), 9.92 (s, 1H), 12.32 (br. s, 1H).

### B. Bewertung der pharmakologischen Wirksamkeit

Die pharmakologische Wirkung der erfindungsgemäßen Verbindungen kann in folgenden Assays gezeigt werden:

### B-1. Stimulation der rekombinanten löslichen Guanylatcyclase (sGC) in vitro

Die Untersuchungen zur Stimulation der rekombinanten löslichen Guanylatcyclase (sGC) durch die erfindungsgemäßen Verbindungen mit und ohne Natriumnitroprussid sowie mit und ohne den Häm-abhängigen sGC-Inhibitor 1*H*-1,2,4-Oxadiazolo[4,3-a]chinoxalin-1-on (ODQ) werden nach der in folgender Literaturstelle im Detail beschriebenen Methode durchgeführt: M. Hoenicka, E.M. Becker, H. Apeler, T. Sirichoke, H. Schroeder, R. Gerzer und J.-P. Stasch, "Purified soluble guanylyl cyclase expressed in a baculovirus/Sf9 system: Stimulation by YC-1, nitric oxide, and carbon oxide", J. Mol. Med. 77 (1999), 14-23. Die Häm-freie Guanylatcyclase wird durch Zugabe von Tween 20 zum Probenpuffer (0.5% in der Endkonzentration) erhalten.

Die Aktivierung der sGC durch eine Prüfsubstanz wird als x-fache Stimulation der Basalaktivität angegeben. Das Ergebnis für Beispiel 50 ist in Tabelle 1A und das für Beispiel 99 in Tabelle 1B gezeigt:

**Tabelle 1A: Stimulation (x-fach) der rekombinanten löslichen Guanylatcyclase (sGC) in vitro durch Beispiel 50**

| **Konzentration Beispiel 50 [µM]** | **Häm-haltige sGC** | | | **Häm-freie sGC** |
|---|---|---|---|---|
| | **Basal (n=5)** | **+ 0.01 µM DEA/NO** | **+10 µM ODQ** | **Basal (n=5)** |
| 0 | 1.0 ± 0.0 | 6.6 ± 0.8 | 9.0 ± 1.4 | 1.0 ± 0.0 |
| 0.01 | 1.4 ± 0.1 | 7.5 ± 1.8 | 9.2 ± 1.4 | 2.6 ± 0.8 |
| 0.1 | 1.2 ± 0.1 | 7.6 ± 1.8 | 9.6 ± 1.5 | 7.1 ± 2.4 |
| 1.0 | 2.6 ± 0.4 | 9.0 ± 2.0 | 12.7 ± 1.5 | 27.2 ± 7.4 |
| 10 | 6.4 ± 0.6 | 13.3 ± 2.2 | 21.5 ± 2.2 | 41.2 ± 9.8 |
| 100 | 9.5 ± 0.9 | 14.6 ± 2.2 | 22.6 ± 2.6 | 39.0 ± 9.1 |

**Tabelle 1B: Stimulation (x-fach) der rekombinanten löslichen Guanylatcyclase (sGC) in vitro durch Beispiel 99**

| **Konzentration Beispiel 99 [µM]** | **Häm-haltige sGC** | | | **Häm-freie sGC** |
|---|---|---|---|---|
| | **Basal (n=3)** | **+ 0.01 µm DEA/NO** | **+ 10µM ODQ** | **Basal (n=3)** |
| 0 | 1.0 ± 0.0 | 4.2 ± 0.8 | 3.7 ± 0.3 | 1.0 ± 0.0 |
| 0.01 | 1.0 ± 0.0 | 4.0 ± 0.6 | 4.3 ± 0.4 | 2.0 ± 0.4 |
| 0.1 | 1.5 ± 0.2 | 4.4 ± 0.8 | 5.7 ± 0.3 | 5.2 ± 0.8 |
| 1.0 | 1..8 ± 0.3 | 5.0 ± 1.7 | 9.2 ± 0.9 | 14.9 ± 2.2 |
| 10 | 2.6 ± 0.4 | 5.3 ± 1.8 | 10.3 ± 1.3 | 19.4 ± 2.8 |

| | | | | |
|---|---|---|---|---|
| [DEA/NO = 2-(*N,N*-Diethylamino)diazenolat-2-oxid; ODQ = 1*H*-1,2,4-Oxadiazolo[4,3-a]chinoxa-lin-1-on]. | | | | |

Aus den Tabellen 1A und 1B ist ersichtlich, dass eine Stimulation sowohl des Häm-haltigen als auch des Häm-freien Enzyms erreicht wird. Weiterhin zeigt die Kombination von Beispiel 50 bzw. Beispiel 99 mit 2-(*N,N*-Diethylamino)diazenolat-2-oxid (DEA/NO), einem NO-Donor, keinen synergistischen Effekt, d.h. die Wirkung von DEA/NO wird nicht potenziert, wie dies bei einem über einen Häm-abhängigen Mechanismus wirkenden sGC-Aktivator zu erwarten wäre. Darüber hinaus wird die Wirkung des erfindungsgemäßen sGC-Aktivators durch 1*H*-1,2,4-Oxadiazolo[4,3-a]-chinoxalin-1-on (ODQ), einen Häm-abhängigen Inhibitor der löslichen Guanylatcyclase, nicht blockiert, sondern sogar gesteigert. Die Ergebnisse in den Tabellen 1A und 1B belegen somit den Wirkmechanismus der erfindungsgemäßen Verbindungen als Aktivatoren der löslichen Guanylatcyclase.

### B-2. Wirkung an rekombinanter Guanylatcyclase-Reporterzelllinie

Die zelluläre Wirkung der erfindungsgemäßen Verbindungen wird an einer rekombinanten Guanylatcyclase-Reporterzelllinie, wie in F. Wunder et al., Anal. Biochem. 339, 104-112 (2005) beschrieben, bestimmt.

Repräsentative Ergebnisse zu den erfindungsgemäßen Verbindungen sind in Tabelle 2 aufgeführt:

**Tabelle 2: sGC-aktivierende Wirkung in der CHO-Reporterzelle in vitro**

| **Beispiel Nr.** | **MEC [nM]** |
|---|---|
| 6 | 30 |
| 8 | 3 |
| 9 | 1 |
| 11 | 1 |
| 12 | 100 |
| 13 | 30 |
| 14 | 100 |
| 15 | 30 |
| 16 | 10 |
| 22 | 10 |
| 25 | 0.3 |
| 27 | 100 |
| 28 | 3 |
| 30 | 0.3 |
| 32 | 2.3 |
| 33 | 3 |
| 34 | 10 |
| 35 | 10 |
| 37 | 0.1 |
| 38 | 0.3 |
| 39 | 0.3 |
| 40 | 0.3 |
| 41 | 1 |
| 42 | 3 |
| 43 | 3 |
| 44 | 0.3 |
| 46 | 1 |
| 49 | 30 |
| 50 | 0.4 |
| 51 | 0.3 |
| 52 | 10 |
| 53 | 10 |
| 62 | 1 |
| 63 | 0.3 |
| 64 | 200 |
| 65 | 5.3 |
| 67 | 3 |
| 68 | 1 |
| 69 | 3 |
| 71 | 3 |
| 73 | 1.7 |
| 78 | 3 |
| 80 | 3 |
| 82 | 1.7 |
| 84 | 20 |
| 89 | 3 |
| 97 | 10 |
| 98 | 0.3 |
| 99 | 1 |
| 101 | 0.3 |
| 103 | 60 |
| 104 | 20 |
| 105 | 3 |
| 107 | 3 |
| 109 | 0.3 |
| 111 | 30 |
| 113 | 0.3 |
| 114 | 3 |

| | |
|---|---|
| (MEC = minimale effektive Konzentration). | |

Im Vergleich hierzu weisen die beiden aus dem Stand der Technik ausgewählten, dort als PPAR-Agonisten beschriebenen Verbindungen 1-(3-{[(2,4-Dichlorbenzoyl)amino]methyl}-4-methoxy-benzyl)cyclopropancarbonsäure und 2-(3-{[(2-Chlor-4-propoxybenzoyl)amino]methyl}-4-ethoxy-benzyl)tetrahydrofuran-2-carbonsäure [Example 11 bzw. Example 73 aus EP 1 452 521-A1] in diesem Test jeweils einen MEC-Wert von > 10 µM *auf (siehe auch Ergebnisse des weiter unten aufgeführten Tests B-6.*).

### B-3. Stimulation der sGC-Enzymaktivität

Lösliche Guanylatcyclase (sGC) setzt unter Stimulation GTP zu cGMP und Pyrophosphat (PPi) um. PPi wird mit Hilfe des nachfolgend beschriebenen Tests nachgewiesen. Das im Test entstehende Signal nimmt mit fortschreitender Umsetzung zu und dient als Maß für die sGC-Enzymaktivität unter der gegebenen Stimulation.

Zur Durchführung des Tests werden 29 µl Enzymlösung [0-10 nM lösliche Guanylatcyclase (hergestellt nach Hönicka et al., J. Mol. Med. 77, 14-23 (1999)) in 50 mM TEA, 2 mM MgCl₂, 0.1% BSA (Fraktion V), 0.005% Brij^{®}, pH 7.5] in eine Mikroplatte vorgelegt und 1 µl der zu testenden Substanz (als seriell verdünnte Lösung in DMSO) hinzugegeben. Der Ansatz wird 10 min bei Raumtemperatur inkubiert. Anschließend werden 20 µl Detektionsmix [1.2 nM Firefly-Luciferase (*Photinus pyralis*-Luciferase*,* Fa. Promega), 29 µM Dehydro-Luziferin (hergestellt nach Bitler & McElroy, Arch. Biochem. Biophys. 72, 358 (1957)), 122 µM Luziferin (Fa. Promega), 153 µM ATP (Fa. Sigma) und 0.4 mM DTT (Fa. Sigma) in 50 mM TEA, 2 mM MgCl₂, 0.1% BSA (Fraktion V), 0.005% Brij^{®}, pH 7.5] zugegeben. Die Enzymreaktion wird durch Zugabe von 20 µl Substratlösung [1.25 mM Guanosin-5'-triphosphat (Fa. Sigma) in 50 mM TEA, 2 mM MgCl₂, 0.1% BSA (Fraktion V), 0.005% Brij^{®}, pH 7.5] gestartet und kontinuierlich luminometrisch vermessen. Das Maß der Stimulation durch die zu testende Substanz kann relativ zum Signal der nicht stimulierten Reaktion bestimmt werden.

Durch Zugabe von 25 µM 1*H*-1,2,4-Oxadiazolo[4,3-a]chinoxalin-1-on (ODQ) zur Enzymlösung und anschließende 30-minütige Inkubation wird die Aktivierung der Häm-freien Guanylatcyclase untersucht und mit der Stimulation des nativen Enzyms verglichen.

Repräsentative Ergebnisse zu den erfindungsgemäßen Verbindungen sind in Tabelle 3 aufgeführt:

**Tabelle 3: Aktivierende Wirkung am sGC-Enzym in vitro**

| **Beispiel Nr.** | **MEC [nM]** | **EC₅₀ [nM]** |
|---|---|---|
| 30 | 0.6 | 7.6 |
| 32 | 0.9 | 7.3 |
| 43 | 3.4 | 24 |
| 50 | 0.7 | 7.3 |
| 51 | 3.0 | 21 |
| 62 | 2.2 | 30 |
| 68 | 2.6 | 45 |
| 73 | 3.8 | 83 |
| 82 | 3.4 | 44 |
| 89 | 3.9 | 70 |
| 99 | 4.1 | 53 |
| 101 | 0.7 | 4.6 |
| 105 | 36 | 240 |
| 107 | 22 | 330 |
| 109 | 0.7 | 20 |

| | | |
|---|---|---|
| (MEC = minimale effektive Konzentration; EC₅₀ = Konzentration bei 50% der maximalen Wirksamkeit). | | |

Im Vergleich hierzu weisen die beiden aus dem Stand der Technik ausgewählten, dort als PPAR-Agonisten beschriebenen Verbindungen 1-(3-{[(2,4-Dichlorbenzoyl)amino]methyl}-4-methoxybenzyl)cyclopropancarbonsäure und 2-(3-{[(2-Chlor-4-propoxybenzoyl)amino]methyl}-4-ethoxy-benzyl)tetrahydrofuran-2-carbonsäure [Example 11 bzw. Example 73 aus EP 1 452 521-A1] in diesem Test jeweils einen MEC-Wert von > 10 µM *auf (siehe auch Ergebnisse des weiter unten aufgeführten Tests B-6.).*

### B-4. Gefäßrelaxierende Wirkung in vitro

Kaninchen werden durch intravenöse Injektion von Thiopental-Natrium narkotisiert bzw. getötet (ca. 50 mg/kg) und entblutet. Die *Arteria Saphena* wird entnommen und in 3 mm breite Ringe geteilt. Die Ringe werden einzeln auf je einem triangelförmigen, am Ende offenen Häkchenpaar aus 0.3 mm starkem Spezialdraht (Remanium^{®}) montiert. Jeder Ring wird unter Vorspannung in 5 ml-Organbäder mit 37°C warmer, carbogenbegaster Krebs-Ilenseleit-Lösung folgender Zusammensetzung gebracht: NaCl 119 mM; KCl 4.8 mM; CaCl₂ x 2 H₂O 1 mM; MgSO₄ x 7 H₂O 1.4 mM; KH₂PO₄ 1.2 mM; NaHCO₃ 25 mM; Glucose 10 mM; Rinderserumalbumin 0.001%. Die Kontraktionskraft wird mit Statham UC2-Zellen erfasst, verstärkt und über A/D-Wandler (DAS-1802 HC, Keithley Instruments, München) digitalisiert sowie parallel auf Linienschreibern registriert. Kontraktionen werden durch Zugabe von Phenylephrin induziert.

Nach mehreren (allgemein 4) Kontrollzyklen wird die zu untersuchende Substanz in jedem weiteren Durchgang in steigender Dosierung zugesetzt und die Höhe der unter dem Einfluss der Testsubstanz erzielten Kontraktion mit der Höhe der im letzten Vordurchgang erreichten Kontraktion verglichen. Daraus wird die Konzentration errechnet, die erforderlich ist, um die in der Vorkontrolle erreichte Kontraktion auf 50% zu reduzieren (IC₅₀-Wert). Das Standard-Applikationsvolumen beträgt 5 µl. Der DMSO-Anteil in der Badlösung entspricht 0.1 %.

Repräsentative Ergebnisse zu den erfindungsgemäßen Verbindungen sind in Tabelle 4 aufgeführt:

**Tabelle 4: Gefäßrelaxierende Wirkung in vitro**

| **Beispiel Nr.** | **IC₅₀ [nM]** |
|---|---|
| 30 | 606 |
| 50 | 145 |
| 101 | 685 |
| 109 | 338 |

### B-5. Radiotelemetrische Messung von Blutdruck und Herzfrequenz an wachen SH-Ratten

Für die im Folgenden beschriebenen Messungen an wachen SH-Ratten wird ein im Handel erhältliches Telemetriesystem der Firma Data Sciences International DSI, USA, eingesetzt.

Das System besteht aus 3 Hauptkomponenten: (*1*) Implantierbare Sender, (2) Empfänger, die über einen Multiplexer mit einem (*3*) Datenakquisitionscomputer verbunden sind. Die Telemetrieanlage ermöglicht eine kontinuierliche Erfassung von Blutdruck und Herzfrequenz an wachen Tieren in ihrem gewohnten Lebensraum.

Die Untersuchungen werden an ausgewachsenen weiblichen, spontan-hypertensiven Ratten (SH-Ratten) mit einem Körpergewicht von >200 g durchgeführt. Die Versuchstiere werden nach Senderimplantation einzeln in Makrolon-Käfigen Typ 3 gehalten. Sie haben freien Zugang zu Standardfutter und Wasser. Der Tag/Nacht-Rhythmus im Versuchslabor wird per Raumbeleuchtung um 6:00 Uhr morgens und um 19:00 Uhr abends gewechselt.

Die eingesetzten Telemetriesender (TAM PA-C40, DSI) werden den Versuchstieren mindestens 14 Tage vor dem ersten Versuchseinsatz unter aseptischen Bedingungen chirurgisch implantiert. Die so instrumentierten Tiere sind nach Abheilen der Wunde und Einwachsen des Implantats wiederholt einsetzbar.

Zur Implantation werden die nüchternen Tiere mit Pentobarbital (Nembutal, Sanofi, 50 mg/kg i.p.) narkotisiert und an der Bauchseite weiträumig rasiert und desinfiziert. Nach Eröffnung des Bauchraumes entlang der Linea alba wird der flüssigkeitsgefüllte Messkatheter des Systems oberhalb der Bifurcation nach cranial in die Aorta descendens eingesetzt und mit Gewebekleber (VetBonD™, 3M) befestigt. Das Sendergehäuse wird intraperitoneal an der Bauchwandmuskulatur fixiert und die Wunde schichtweise verschlossen. Postoperativ wird zur Infektionsprophylaxe ein Antibiotikum verabreicht (Tardomyocel COMP, Bayer AG, 1 ml/kg s.c.).

### Versuchsablauf:

Die zu untersuchenden Substanzen werden jeweils einer Gruppe von Tieren (n = 6) per Schlundsonde oral verabreicht. Entsprechend einem Applikationsvolumen von 5 ml/kg Körpergewicht werden die Testsubstanzen in geeigneten Lösungsmittelgemischen gelöst oder in 0.5%-iger Tylose suspendiert. Eine Lösungsmittel-behandelte Gruppe von Tieren wird als Kontrolle eingesetzt.

Die Telemetrie-Messeinrichtung ist für 24 Tiere konfiguriert. Jeder Versuch wird unter einer Versuchsnummer registriert.

Den in der Anlage lebenden instrumentierten Ratten ist jeweils eine eigene Empfangsantenne zugeordnet (1010 Receiver, DSI). Die implantierten Sender sind über einen eingebauten Magnetschalter von außen aktivierbar und werden bei Versuchsvorlauf auf Sendung geschaltet. Die ausgestrahlten Signale können durch ein Datenakquisitionssystem (Dataquest™ A.R.T. for Windows, DSI) online erfasst und entsprechend aufgearbeitet werden. Die Ablage der Daten erfolgt jeweils in einem hierfür eröffneten Ordner, der die Versuchsnummer trägt.

Im Standardablauf werden über je 10 Sekunden Dauer gemessen: (*1*) systolischer Blutdruck (SBP), (2) diastolischer Blutdruck (DBP), (*3*) arterieller Mitteldruck (MAP) und (*4*) Herzfrequenz (HR).

Die Messwerterfassung wird rechnergesteuert in 5 Minuten-Abständen wiederholt. Die als Absolutwert erhobenen Quelldaten werden im Diagramm mit dem aktuell gemessenen Barometerdruck korrigiert und in Einzeldaten abgelegt. Weitere technische Details sind in der Dokumentation der Herstellerfirma (DSI) aufgeführt.

Die Verabreichung der Prüfsubstanzen erfolgt am Versuchstag um 9:00 Uhr. Im Anschluss an die Applikation werden die oben beschriebenen Parameter über 24 Stunden gemessen. Nach Versuchsende werden die erhobenen Einzeldaten mit der Analysis-Software (Dataquest™ A.R.T. Analysis) sortiert. Als Leerwert wird der Zeitpunkt 2 Stunden vor Substanz-Applikation angenommen, so dass der selektierte Datensatz den Zeitraum von 7:00 Uhr am Versuchstag bis 9:00 Uhr am Folgetag umfasst.

Die Daten werden über eine voreinstellbare Zeit durch Mittelwertbestimmung geglättet (15 Minuten-Mittelwert, 30 Minuten-Mittelwert) und als Textdatei auf einen Datenträger übertragen. Die so vorsortierten und komprimierten Messwerte werden in Excel-Vorlagen übertragen und tabellarisch dargestellt.

### B-6. Zellulärer Peroxisom-Proliferator-aktivierter Rezeptor (PPAR)-Transaktivierungsassay (zu Vergleichszwecken)

### a) Testprinzip:

Zur Überprüfung potentiell aktivierender Eigenschaften der erfindungsgemäßen Verbindungen bezüglich der drei humanen Isoformen des Peroxisom-Proliferator-aktivierten Rezeptors (PPARα, PPARγ und PPARδ) wird ein zellulärer Assay verwendet.

Da Säugetierzellen verschiedene endogene nukleäre Rezeptoren enthalten, die eine eindeutige Interpretation der Ergebnisse komplizieren könnten, wird ein etabliertes Chimärensystem eingesetzt, in dem die jeweilige Liganden-Bindungsdomäne der humanen PPAR-Isoformen an die DNA-Bindungsdomäne des Hefe-Transkriptionsfaktors GAL4 fusioniert wird. Die so entstehenden GAL4-PPARα-, γ- und δ-Chimären werden in CHO-Zellen mit einem Reporterkonstrukt co-transfiziert und stabil exprimiert.

### b) Klonierung:

Die GAL4-PPAR-Expressionskonstrukte enthalten die Ligandenbindungsdomänen von PPARα (Aminosäuren 167-468), PPARγ (Aminosäuren 203-506) bzw. PPARδ (Aminosäuren 138-442), welche PCR-amplifiziert und in den Vektor pcDNA3.1 hineinkloniert werden. Die jeweiligen Expressionsvektoren enthalten bereits die GAL4-DNA-Bindungsdomäne (Aminosäuren 1-147) des Vektors pFC2-dbd (Stratagene). Das Reporterkonstrukt, welches fünf Kopien der GAL4-Bindestelle, vorgeschaltet vor einem Thymidinkinase-Promoter, enthält, führt zur Expression der Firefly-Luciferase (*Pholintispyralis*) nach Aktivierung und Bindung von GAL4-PPARα, -γ oder -δ.

### c) Testablauf und Auswertung:

CHO-K1-Zellen (chinese hamster ovary; ATCC CCL-61), die jeweils eine der oben beschriebenen GAL4-PPAR-Chimären und das Luciferase-Reportergenkonstrukt stabil exprimieren, werden am Tag vor dem Test in Medium [Optimem (GIBCO) mit 2% Aktivkohle-gereinigtem fötalen Kälberserum (Hyclone), 1.35 mM Natriumpyruvat (GIBCO) und 0.2% Natriumbicarbonat (GIBCO)] in einer Dichte von 1 x 10³ Zellen in 96-Loch-Mikrotiterplatten (Greiner) ausplattiert und in einem Zellinkubator (96% Luftfeuchtigkeit, 5% v/v CO₂, 37°C) gehalten. Am Testtag werden die zu prüfenden Substanzen in oben genanntem Medium, allerdings ohne Zusatz von Kälberserum, aufgenommen und in verschiedenen Konzentrationen zu den Zellen gegeben. Nach einer Stimulationszeit von 6 h wird die Luciferase-Aktivität mit Hilfe einer Videokamera gemessen. Die gemessenen relativen Lichteinheiten ergeben in Abhängigkeit von der Substanzkonzentration eine sigmoide Stimulationskurve. Die Berechnung der EC₅₀-Werte erfolgt mit Hilfe des Computerprogramms GraphPad PRISM (Version 3.02). Der maximale Effekt einer Testsubstanz im jeweiligen PPAR-Assay wird als prozentuale Effektivität im Vergleich zu einer entsprechenden Referenzverbindung, deren maximaler Effekt als 100%-Wert definiert wird, bestimmt.

Als solche Referenzverbindung wurde hier die im Stand der Technik als potenter pan-PPAR-Agonist beschriebene Verbindung 2-(3-{[(2-Chlor-4-propoxybenzoyl)amino]methyl}-4-ethoxy-benzyl)tetrahydrofuran-2-carbonsäure [Example 73 aus EP 1 452 521-A1] gewählt. Eine zweite Vergleichssubstanz, 1-(3-{[(2,4-Dichlorbenzoyl)amino]methyl}-4-methoxybenzyl)cyclopropancarbonsäure [Example 11 aus EP 1 452 521-A1], erwies sich in diesem Test als nahezu inaktiv.

In der folgenden Tabelle 5 sind die EC₅₀- und Effektivitätswerte repräsentativer Beispielverbindungen sowie der beiden oben genannten Vergleichssubstanzen aufgeführt:

**Tabelle 5:**

| **Beispiel Nr.** | **PPARα ECso / Effektivität** | **PPARγ EC₅₀/ Effektivität** | **PPARδ EC₅₀ / Effektivität** |
|---|---|---|---|
| 30 | > 10 µM; 0% | 2.3 µM; 20% | > 10 µM; 0% |
| 32 | > 10 µM; 0% | 1.9 µM; 30% | > 10 µM; 0% |
| 33 | > 10 µM; 0% | 4.0 µM; 10% | > 10 µM; 0% |
| 38 | > 10 µM; 0% | > 10 µM; 0% | > 10 µM; 0% |
| 40 | > 10 µM; 0% | 0.12 µM; 10% | > 10 µM; 0% |
| 42 | > 10 µM; 0% | 2.0 µM; 10% | > 10 µM; 0% |
| 43 | > 10 µM; 0% | 1.8 µM; 10% | > 10 µM; 0% |
| 44 | > 10 µM; 0% | > 10 µM; 0% | > 10 µM; 0% |
| 50 | > 10 µM; 0% | 3.0 µM; 45% | > 10 µM; 0% |
| 51 | > 10 µM; 0% | 1.9 µM; 20% | > 10 µM; 0% |
| 68 | > 10 µM; 0% | > 10 µM; 0% | > 10 µM; 0% |
| 71 | > 10 µM; 0% | 0.46 µM; 20% | > 10 µM; 0% |
| 73 | > 10 µM; 0% | > 10 µM; 0% | > 10 µM; 0% |
| 82 | > 10 µM; 0% | 2.3 µM; 20% | > 10 µM; 0% |

| **Beispiel Nr.** | **PPARα ECso / Effektivität** | **PPARγ ECso / Effektivität** | **PPARδ EC₅₀ / Effektivität** |
|---|---|---|---|
| 89 | > 10 µM; 0% | > 10 µM; 0% | > 10 µM; 0% |
| 99 | > 10 µM; 0% | > 10 µM; 0% | > 10 µM; 0% |
| 101 | > 10 µM; 0% | > 10 µM; 0% | > 10 µM; 0% |
| 105 | > 10 µM; 0% | 1.0 µM; 10% | > 10 µM; 0% |
| 107 | > 10 µM; 0% | 2.0 µM; 10% | > 10 µM; 0% |
| Example 11 aus EP 1 452 521 | > 1 µM; 0% | > 1 µM; 0% | > 1 µM; 10% |
| Example 73 aus EP 1 452 521 | 0.0053 µM; 100% | 0.0089 µM; 100% | 0.0084 µM; 100% |

Die Verbindungen der vorliegenden Erfindung weisen nach diesen Untersuchungen somit keine agonistischen Eigenschaften bezüglich des humanen PPARα- und PPARδ-Rezeptors auf. Die in manchen Fällen zu beobachtende Aktivität bezüglich des humanen PPARγ-Rezeptors ist vergleichsweise als marginal einzustufen, und es ist davon auszugehen, dass diese keinen Beitrag zum erfindungsgemäßen pharmakologischen Wirkprofil der sGC-Aktivierung, insbesondere zur Gefäßwirkung, leistet.

### C. Ausführungsbeispiele für pharmazeutische Zusammensetzungen

Die erfindungsgemäßen Verbindungen können folgendermaßen in pharmazeutische Zubereitungen überführt werden:

### Tablette:

### Zusammensetzung:

100 mg der erfindungsgemäßen Verbindung, 50 mg Lactose (Monohydrat), 50 mg Maisstärke (nativ), 10 mg Polyvinylpyrrolidon (PVP 25) (Fa. BASF, Ludwigshafen, Deutschland) und 2 mg Magnesiumstearat.

Tablettengewicht 212 mg. Durchmesser 8 mm, Wölbungsradius 12 mm.

### Herstellung:

Die Mischung aus erfindungsgemäßer Verbindung, Lactose und Stärke wird mit einer 5%-igen Lösung (m/m) des PVPs in Wasser granuliert. Das Granulat wird nach dem Trocknen mit dem Magnesiumstearat 5 Minuten gemischt. Diese Mischung wird mit einer üblichen Tablettenpresse verpresst (Format der Tablette siehe oben). Als Richtwert für die Verpressung wird eine Presskraft von 15 kN verwendet.

### Oral applizierbare Suspension:

### Zusammensetzung:

1000 mg der erfindungsgemäßen Verbindung, 1000 mg Ethanol (96%), 400 mg Rhodigel^{®} (Xanthan gum der Firma FMC, Pennsylvania, USA) und 99 g Wasser.

Einer Einzeldosis von 100 mg der erfindungsgemäßen Verbindung entsprechen 10 ml orale Suspension.

### Herstellung:

Das Rhodigel wird in Ethanol suspendiert, die erfindungsgemäße Verbindung wird der Suspension zugefügt. Unter Rühren erfolgt die Zugabe des Wassers. Bis zum Abschluß der Quellung des Rhodigels wird ca. 6 h gerührt.

### Oral applizierbare Lösung:

### Zusammensetzung:

500 mg der erfindungsgemäßen Verbindung, 2.5 g Polysorbat und 97 g Polyethylenglycol 400. Einer Einzeldosis von 100 mg der erfindungsgemäßen Verbindung entsprechen 20 g orale Lösung.

### Herstellung:

Die erfindungsgemäße Verbindung wird in der Mischung aus Polyethylenglycol und Polysorbat unter Rühren suspendiert. Der Rührvorgang wird bis zur vollständigen Auflösung der erfindungsgemäßen Verbindung fortgesetzt.

### i.v.-Lösung:

Die erfindungsgemäße Verbindung wird in einer Konzentration unterhalb der Sättigungslöslichkeit in einem physiologisch verträglichen Lösungsmittel (z.B. isotonische Kochsalzlösung, Glucoselösung 5% und/oder PEG 400-Lösung 30%) gelöst. Die Lösung wird steril filtriert und in sterile und pyrogenfreie Injektionsbehältnisse abgefüllt.

## Patentansprüche

1. Verbindung der Formel (I) 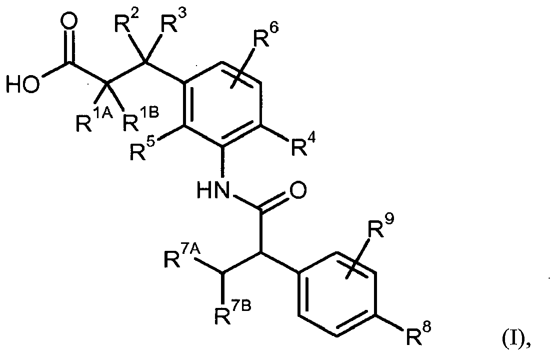 in welcher
R^{1A} und R^{1B} miteinander verknüpft sind und gemeinsam mit dem Kohlenstoffatom, an das sie gebunden sind, eine Cycloalkyl-Gruppe der Formel 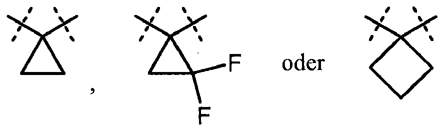 bilden,
R² für Wasserstoff, Methyl, Ethyl, Vinyl, Hydroxy, Methoxy, Trideuteromethoxy, Trifluormethoxy, Ethoxy oder Cyclopropyloxy steht,
R³ für Wasserstoff, Methyl, Ethyl, Isopropyl oder Cyclopropyl steht,
R⁴ für Wasserstoff, Fluor, Chlor, Brom, Cyano, Methyl, Trifluormethyl, Ethyl, Isopropyl, Cyclopropyl, Cyclobutyl, Methoxy oder Trifluormethoxy steht,
R⁵ für Wasserstoff, Fluor, Chlor, Methyl, Trifluormethyl oder Trifluormethoxy steht,
R⁶ für Wasserstoff, Fluor, Chlor, Methyl, Trifluormethyl oder Trifluormethoxy steht,
R^{7A} für Methyl oder Ethyl steht,
R^{7B} für Trifluormethyl steht,
oder
R^{7A} und R^{7B} miteinander verknüpft sind und gemeinsam mit dem Kohlenstoffatom, an das sie gebunden sind, einen optional difluor-substituierten Cyclopentyl-Ring der Formel 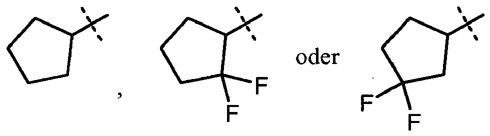 bilden,
R⁸ für Fluor, Chlor, Brom, Nitro, Cyano, Trifluormethoxy, Acetyl, 2-Cyanovinyl, (C₁-C₄)-Alkyl, (C₂-C₄)-Alkenyl, Cyclopropyl oder Cyclobutyl steht, wobei (C₁-C₄)-Alkyl und (C₂-C₄)-Alkenyl bis zu dreifach mit Fluor
und
Cyclopropyl und Cyclobutyl bis zu zweifach mit Fluor substituiert sein können,
und
R⁹ für Wasserstoff, Fluor, Chlor, Methyl, Trifluormethyl, Ethyl, Methoxy oder Trifluormethoxy steht,
sowie ihre Salze, Solvate und Solvate der Salze.

2. Verbindung der Formel (I) nach Anspruch 1, in welcher
R^{1A} und R^{1B} miteinander verknüpft sind und gemeinsam mit dem Kohlenstoffatom, an das sie gebunden sind, eine Cycloalkyl-Gruppe der Formel 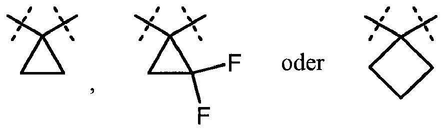 bilden,
R² für Wasserstoff, Methyl, Ethyl, Hydroxy, Methoxy, Trideuteromethoxy, Ethoxy oder Cyclopropyloxy steht,
R³ für Wasserstoff, Methyl oder Ethyl steht,
R⁴ für Wasserstoff, Fluor, Chlor, Methyl oder Cyclopropyl steht,
R⁵ für Wasserstoff, Fluor, Chlor oder Methyl steht,
R⁶ für Wasserstoff, Fluor, Chlor oder Methyl steht,
R^{7A} für Methyl steht,
R^{7B} für Trifluormethyl steht,
oder
R^{7A} und R^{7B} miteinander verknüpft sind und gemeinsam mit dem Kohlenstoffatom, an das sie gebunden sind, einen difluor-substituierten Cyclopentyl-Ring der Formel 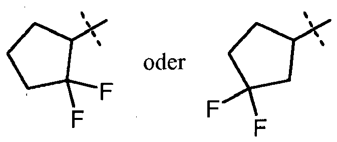 bilden,
R⁸ für Fluor, Chlor, Acetyl, 2-Cyanovinyl, (C₁-C₄)-Alkyl, (C₂-C₃)-Alkenyl, Cyclopropyl oder Cyclobutyl steht, wobei (C₁-C₄)-Alkyl und (C₂-C₃)-Alkenyl bis zu dreifach mit Fluor substituiert sein können,
und
R⁹ für Wasserstoff, Fluor, Chlor, Methyl, Trifluormethyl oder Methoxy steht,
sowie ihre Salze, Solvate und Solvate der Salze.

3. Verbindung der Formel (I) nach Anspruch 1 oder 2, in welcher
R^{1A} und R^{1B} miteinander verknüpft sind und gemeinsam mit dem Kohlenstoffatom, an das sie gebunden sind, einen optional difluor-substituierten Cyclopropyl-Ring der Formel 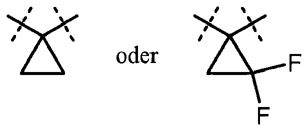 bilden,
R² für Wasserstoff oder Ethyl steht,
R³ für Wasserstoff steht,
R⁴ für Wasserstoff, Fluor oder Chlor steht,
R⁵ für Wasserstoff oder Fluor steht,
R⁶ für Wasserstoff steht,
R^{7A} für Methyl steht,
R^{7B} für Trifluormethyl steht,
oder
R^{7A} und R^{7B} miteinander verknüpft sind und gemeinsam mit dem Kohlenstoffatom, an das sie gebunden sind, einen difluor-substituierten Cyclopentyl-Ring der Formel 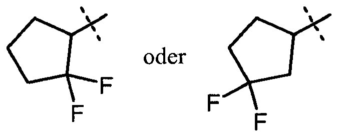 bilden,
R⁸ für Chlor, Methyl, Trifluormethyl, Ethyl, 1,1-Difluorethyl, 2,2,2-Trifluorethyl, Isopropyl, *tert.-Butyl,* 1,1,1-Trifluor-2-methylpropan-2-yl, Vinyl, 2,2-Difluorvinyl oder Cyclopropyl steht,
und
R⁹ für Wasserstoff, Fluor, Chlor oder Methoxy steht,
sowie ihre Salze, Solvate und Solvate der Salze.

4. Verbindung der Formel (I) nach Anspruch 1 oder 2, in welcher
R^{1A} und R^{1B} miteinander verknüpft sind und gemeinsam mit dem Kohlenstoffatom, an das sie gebunden sind, einen Cyclopropyl- oder Cyclobutyl-Ring der Formel 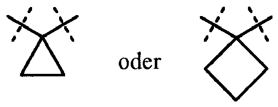 bilden,
R² für Hydroxy, Methoxy, Trideuteromethoxy, Ethoxy oder Cyclopropyloxy steht,
R³ für Wasserstoff steht,
R⁴ für Wasserstoff, Fluor oder Chlor steht,
R⁵ für Wasserstoff oder Fluor steht,
R⁶ für Wasserstoff steht,
R^{7A} für Methyl steht,
R^{7B} für Trifluormethyl steht,
oder
R^{7A} und R^{7B} miteinander verknüpft sind und gemeinsam mit dem Kohlenstoffatom, an das sie gebunden sind, einen difluor-substituierten Cyclopentyl-Ring der Formel 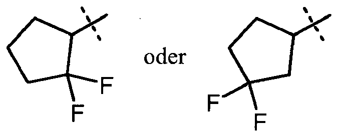 bilden,
R⁸ für Chlor, Methyl, Trifluormethyl, Ethyl, 1,1-Difluorethyl, 2,2,2-Trifluorethyl, Isopropyl, *tert*.-Butyl, 1,1,1-Trifluor-2-methylpropan-2-yl, Vinyl, 2,2-Difluorvinyl oder Cyclopropyl steht,
und
R⁹ für Wasserstoff, Fluor, Chlor oder Methoxy steht,
sowie ihre Salze, Solvate und Solvate der Salze.

5. Verfahren zur Herstellung einer Verbindung der Formel (I), wie in den Ansprüchen 1 bis 4 definiert, **dadurch gekennzeichnet, dass** man eine Carbonsäure der Formel (II) 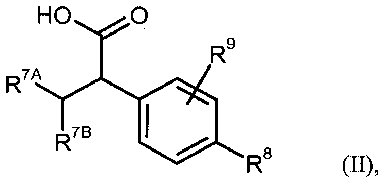 in welcher R^{7A}, R^{7B}, R⁸ und R⁹ die in den Ansprüchen 1 bis 4 angegebenen Bedeutungen haben,
in einem inerten Lösungsmittel mit Hilfe eines Kondensationsmittels oder über die Zwischenstufe des entsprechenden Carbonsäurechlorids in Gegenwart einer Base mit einem Amin der Formel (III) 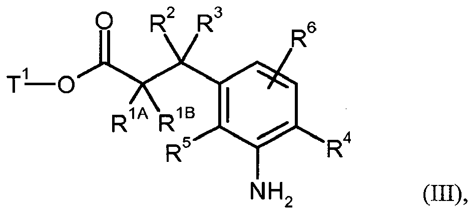 in welcher R^{1A}, R^{1B}, R², R³, R⁴, R⁵ und R⁶ die in den Ansprüchen 1 bis 4 angegebenen Bedeutungen haben
und
T¹ für (C₁-C₄)-Alkyl oder Benzyl steht,
zu einem Carbonsäureamid der Formel (IV) 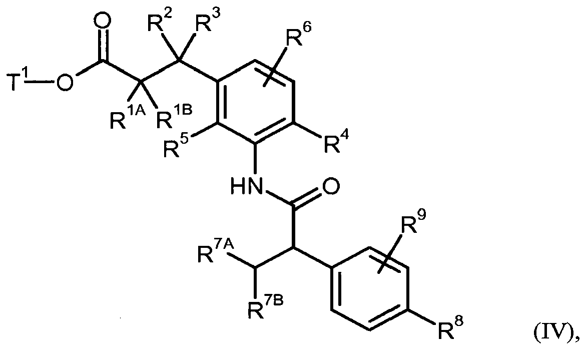 in welcher R^{1A}, R^{1B}, R², R³, R⁴, R⁵, R⁶, R^{7A}, R^{7B}, R⁸, R⁹ und T¹ die oben angegebenen Bedeutungen haben,
kuppelt und anschließend den Ester-Rest T¹ durch basische oder saure Solvolyse oder im Fall, dass T¹ für Benzyl steht, auch durch Hydrogenolyse unter Erhalt der Carbonsäure der Formel (I) abspaltet
und gegebenenfalls die Verbindungen der Formel (I) nach dem Fachmann bekannten Methoden in ihre Enantiomere und/oder Diastereomere trennt und/oder mit den entsprechenden (i) Lösungsmitteln und/oder (ii) Basen zu ihren Solvaten, Salzen und/oder Solvaten der Salze umsetzt.

6. Verbindung, wie in einem der Ansprüche 1 bis 4 definiert, zur Verwendung als Arzneimittel.

7. Verwendung einer Verbindung, wie in einem der Ansprüche 1 bis 4 definiert, zur Herstellung eines Arzneimittels zur Behandlung und/oder Prävention von Herzinsuffizienz, Angina pectoris, Hypertonie, pulmonaler Hypertonie, Ischämien, Gefäßerkrankungen, Mikrozirkulationsstörungen, thromboembolischen Erkrankungen, Niereninsuffizienz, fibrotischen Erkrankungen und Arteriosklerose.

8. Arzneimittel enthaltend eine Verbindung, wie in einem der Ansprüche 1 bis 4 definiert, in Kombination mit einem oder mehreren inerten, nicht-toxischen, pharmazeutisch geeigneten Hilfsstoffen.

9. Arzneimittel enthaltend eine Verbindung, wie in einem der Ansprüche 1 bis 4 definiert, in Kombination mit einem oder mehreren weiteren Wirkstoffen ausgewählt aus der Gruppe bestehend aus organischen Nitraten, NO-Donatoren, cGMP-PDE-Inhibitoren, Stimulatoren der Guanylatcyclase, antithrombotisch wirkenden Mitteln, den Blutdruck senkenden Mitteln sowie den Fettstoffwechsel verändernden Mitteln.

10. Arzneimittel nach Anspruch 8 oder 9 zur Verwendung bei der Behandlung und/oder Prävention von Herzinsuffizienz, Angina pectoris, Hypertonie, pulmonaler Hypertonie, Ischämien, Gefäßerkrankungen, Mikrozirkulationsstörungen, thromboembolischen Erkrankungen, Niereninsuffizienz, fibrotischen Erkrankungen und Arteriosklerose.

## Claims

1. Compound of the formula (I) 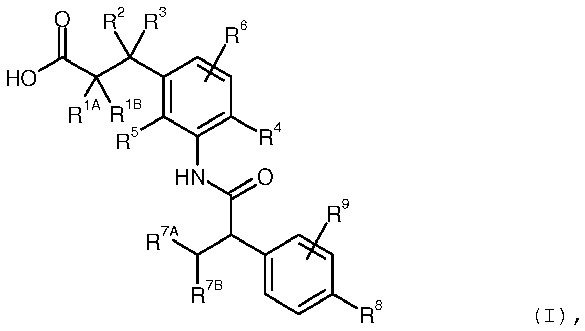 in which
R^{1A} and R^{1B} are attached to one another and together with the carbon atom to which they are attached form a cycloalkyl group of the formula 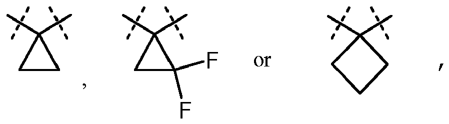
R² represents hydrogen, methyl, ethyl, vinyl, hydroxyl, methoxy, trideuteromethoxy, trifluoromethoxy, ethoxy or cyclopropyloxy,
R³ represents hydrogen, methyl, ethyl, isopropyl or cyclopropyl,
R⁴ represents hydrogen, fluorine, chlorine, bromine, cyano, methyl, trifluoromethyl, ethyl, isopropyl, cyclopropyl, cyclobutyl, methoxy or trifluoromethoxy,
R⁵ represents hydrogen, fluorine, chlorine, methyl, trifluoromethyl or trifluoromethoxy,
R⁶ represents hydrogen, fluorine, chlorine, methyl, trifluoromethyl or trifluoromethoxy,
R^{7A} represents methyl or ethyl,
R^{7B} represents trifluoromethyl,
or
R^{7A} and R^{7B} are attached to one another and together with the carbon atom to which they are attached form an optionally difluoro-substituted cyclopentyl ring of the formula 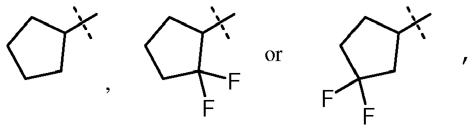
R⁸ represents fluorine, chlorine, bromine, nitro, cyano, trifluoromethoxy, acetyl, 2-cyanovinyl, (C₁-C₄)-alkyl, (C₂-C₄)-alkenyl, cyclopropyl or cyclobutyl, where
(C₁-C₄)-alkyl and (C₂-C₄)-alkenyl may be substituted up to three times by fluorine
and
cyclopropyl and cyclobutyl may be substituted up to two times by fluorine,
and
R⁹ represents hydrogen, fluorine, chlorine, methyl, trifluoromethyl, ethyl, methoxy or trifluoromethoxy,
and salts, solvates and solvates of the salts thereof.

2. Compound of the formula (I) according to Claim 1 in which
R^{1A} and R^{1B} are attached to one another and together with the carbon atom to which they are attached form a cycloalkyl group of the formula 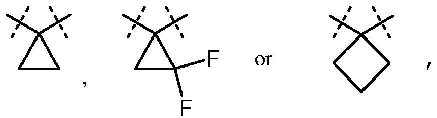
R² represents hydrogen, methyl, ethyl, hydroxyl, methoxy, trideuteromethoxy, ethoxy or cyclopropyloxy,
R³ represents hydrogen, methyl or ethyl,
R⁴ represents hydrogen, fluorine, chlorine, methyl or cyclopropyl,
R⁵ represents hydrogen, fluorine, chlorine or methyl,
R⁶ represents hydrogen, fluorine, chlorine or methyl,
R^{7A} represents methyl,
R^{7B} represents trifluoromethyl,
or
R^{7A} and R^{7B} are attached to one another and together with the carbon atom to which they are attached form an optionally difluoro-substituted cyclopentyl ring of the formula 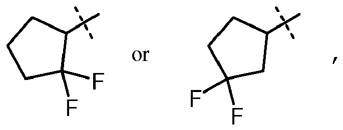
R⁸ represents fluorine, chlorine, acetyl, 2-cyanovinyl, (C₁-C₄)-alkyl, (C₂-C₃)-alkenyl, cyclopropyl or cyclobutyl, where (C₁-C₄)-alkyl and (C₂-C₃)-alkenyl may be substituted up to three times by fluorine,
and
R⁹ represents hydrogen, fluorine, chlorine, methyl, trifluoromethyl or methoxy,
and salts, solvates and solvates of the salts thereof.

3. Compound of the formula (I) according to Claim 1 or 2 in which
R^{1A} and R^{1B} are attached to one another and together with the carbon atom to which they are attached form an optionally difluoro-substituted cyclopropyl ring of the formula 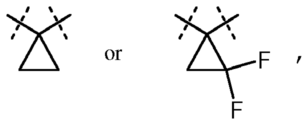
R² represents hydrogen or ethyl,
R³ represents hydrogen,
R⁴ represents hydrogen, fluorine or chlorine,
R⁵ represents hydrogen or fluorine,
R⁶ represents hydrogen,
R^{7A} represents methyl,
R^{7B} represents trifluoromethyl,
or
R^{7A} and R^{7B} are attached to one another and together with the carbon atom to which they are attached form a difluoro-substituted cyclopentyl ring of the formula 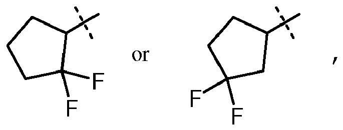
R⁸ represents chlorine, methyl, trifluoromethyl, ethyl, 1,1-difluoroethyl, 2,2,2-trifluoroethyl, isopropyl, tert-butyl, 1,1,1-trifluoro-2-methylpropan-2-yl, vinyl, 2,2-difluorovinyl or cyclopropyl,
and
R⁹ represents hydrogen, fluorine, chlorine or methoxy,
and salts, solvates and solvates of the salts thereof.

4. Compound of the formula (I) according to Claim 1 or 2 in which
R^{1A} and R^{1B} are attached to one another and together with the carbon atom to which they are attached form a cyclopropyl or cyclobutyl ring of the formula 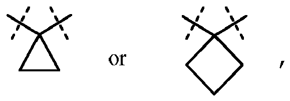
R² represents hydroxyl, methoxy, trideuteromethoxy, ethoxy or cyclopropyloxy,
R³ represents hydrogen,
R⁴ represents hydrogen, fluorine or chlorine,
R⁵ represents hydrogen or fluorine,
R⁶ represents hydrogen,
R^{7A} represents methyl,
R^{7B} represents trifluoromethyl,
or
R^{7A} and R^{7B} are attached to one another and together with the carbon atom to which they are attached form a difluoro-substituted cyclopentyl ring of the formula 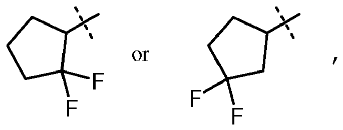
R⁸ represents chlorine, methyl, trifluoromethyl, ethyl, 1,1-difluoroethyl, 2,2,2-trifluoroethyl, isopropyl, tert-butyl, 1,1,1-trifluoro-2-methylpropan-2-yl, vinyl, 2,2-difluorovinyl or cyclopropyl,
and
R⁹ represents hydrogen, fluorine, chlorine or methoxy,
and salts, solvates and solvates of the salts thereof.

5. Process for preparing a compound of the formula (I) as defined in any of Claims 1 to 4, **characterized in that** a carboxylic acid of the formula (II) 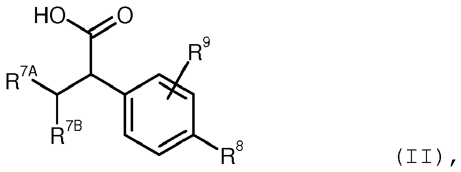 in which R^{7A}, R^{7B}, R⁸ and R⁹ have the meanings given in any of Claims 1 to 4,
is coupled in an inert solvent with the aid of a condensing agent or via the intermediate of the corresponding carbonyl chloride in the presence of a base with an amine of the formula (III) 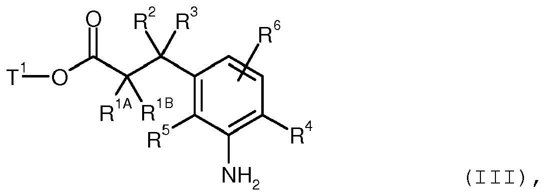 in which R^{1A}, R^{1B}, R², R³, R⁴, R⁵ and R⁶ have the meanings given in Claims 1 to 4
and
T¹ represents (C₁-C₄)-alkyl or benzyl,
to give a carboxamide of the formula (IV) 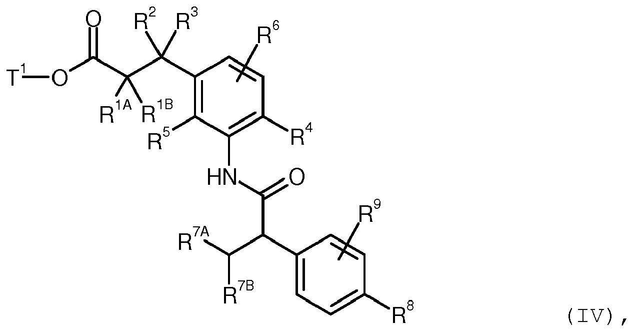 in which R^{1A}, R^{1B}, R², R³, R⁴, R⁵, R⁶, R^{7A}, R^{7B}, R⁸, R⁹ and T¹ have the meanings given above,
and the ester radical T¹ is then removed by basic or acidic solvolysis or, in the case that T¹ represents benzyl, also by hydrogenolysis to give the carboxylic acid of the formula (I)
and the compounds of the formula (I) are optionally separated by methods known to the person skilled in the art into their enantiomers and/or diastereomers and/or reacted with the appropriate (i) solvents and/or (ii) bases to give their solvates, salts and/or solvates of the salts.

6. Compound as defined in any of Claims 1 to 4 for use as a medicament.

7. Use of a compound as defined in any of Claims 1 to 4 for producing a medicament for the treatment and/or prevention of heart failure, angina pectoris, hypertension, pulmonary hypertension, ischaemias, vascular disorders, microcirculation impairments, thromboembolic disorders, renal insufficiency, fibrotic disorders and arteriosclerosis.

8. Medicament comprising a compound as defined in any of Claims 1 to 4 in combination with one or more inert, non-toxic, pharmaceutically suitable excipients.

9. Medicament comprising a compound as defined in any of Claims 1 to 4 in combination with one or more further active ingredients selected from the group consisting of organic nitrates, NO donors, cGMP-PDE inhibitors, stimulators of guanylate cyclase, agents having antithrombotic activity, agents lowering blood pressure, and agents altering lipid metabolism.

10. Medicament according to Claim 8 or 9 for use in the treatment and/or prevention of heart failure, angina pectoris, hypertension, pulmonary hypertension, ischaemias, vascular disorders, microcirculation impairments, thromboembolic disorders, renal insufficiency, fibrotic disorders and arteriosclerosis.

## Revendications

1. Composé de formule (I) 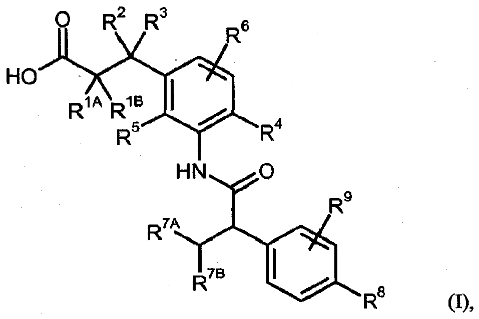 dans laquelle
R^{1A} et R^{1B} sont liés l'un à l'autre et forment, ensemble avec l'atome de carbone auquel ils sont liés, un groupe cycloalkyle de formule 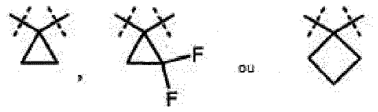
R² représente hydrogène, méthyle, éthyle, vinyle, hydroxy, méthoxy, trideutérométhoxy, trifluorométhoxy, éthoxy ou cyclopropyloxy,
R³ représente hydrogène, méthyle, éthyle, isopropyle ou cyclopropyle,
R⁴ représente hydrogène, fluor, chlore, brome, cyano, méthyle, trifluorométhyle, éthyle, isopropyle, cyclopropyle, cyclobutyle, méthoxy ou trifluorométhoxy,
R⁵ représente hydrogène, fluor, chlore, méthyle, trifluorométhyle ou trifluorométhoxy,
R⁶ représente hydrogène, fluor, chlore, méthyle, trifluorométhyle ou trifluorométhoxy,
R^{7A} représente méthyle ou éthyle,
R^{7B} représente trifluorométhyle, ou
R^{7A} et R^{7B} sont liés l'un à l'autre et forment, ensemble avec l'atome de carbone auquel ils sont liés, un cycle cyclopentyle éventuellement disubstitué par fluor de formule 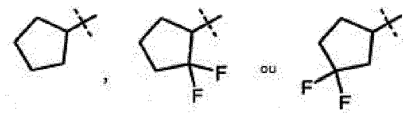
R⁸ représente fluor, chlore, brome, nitro, cyano, trifluorométhoxy, acétyle, 2-cyanovinyle, (C₁-C₄)-alkyle, (C₂-C₄)-alcényle, cyclopropyle ou cyclobutyle ;
(C₁-C₄)-alkyle et (C₂-C₄)-alcényle pouvant être jusqu'à trisubstitués par fluor et cyclopropyle et cyclobutyle pouvant être jusqu'à disubstitués par fluor,
et
R⁹ représente hydrogène, fluor, chlore, méthyle, trifluorométhyle, éthyle, méthoxy ou trifluorométhoxy,
ainsi que ses sels, solvates et solvates des sels.

2. Composé de formule (I) selon la revendication 1, dans laquelle
R^{1A} et R^{1B} sont liés l'un à l'autre et forment, ensemble avec l'atome de carbone auquel ils sont liés, un groupe cycloalkyle de formule 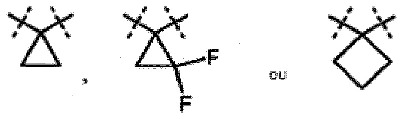
R² représente hydrogène, méthyle, éthyle, hydroxy, méthoxy, trideutérométhoxy, éthoxy ou cyclopropyloxy,
R³ représente hydrogène, méthyle ou éthyle,
R⁴ représente hydrogène, fluor, chlore, méthyle ou cyclopropyle,
R⁵ représente hydrogène, fluor, chlore ou méthyle,
R⁶ représente hydrogène, fluor, chlore ou méthyle,
R^{7A} représente méthyle,
R^{7B} représente trifluorométhyle, ou
R^{7A} et R^{7B} sont liés l'un à l'autre et forment, ensemble avec l'atome de carbone auquel ils sont liés, un cycle cyclopentyle disubstitué par fluor de formule 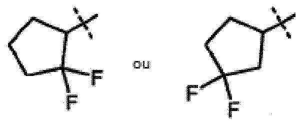
R⁸ représente fluor, chlore, acétyle, 2-cyanovinyle, (C₁-C₄)-alkyle, (C₂-C₃)-alcényle, cyclopropyle ou cyclobutyle ; (C₁-C₄)-alkyle et (C₂-C₃)-alcényle pouvant être jusqu'à trisubstitués par fluor, et
R⁹ représente hydrogène, fluor, chlore, méthyle, trifluorométhyle ou méthoxy,
ainsi que ses sels, solvates et solvates des sels.

3. Composé de formule (I) selon la revendication 1 ou 2, dans laquelle
R^{1A} et R^{1B} sont liés l'un à l'autre et forment, ensemble avec l'atome de carbone auquel ils sont liés, un cycle cyclopropyle éventuellement disubstitué par fluor de formule 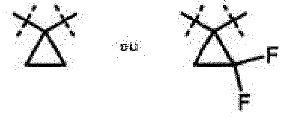
R² représente hydrogène ou éthyle,
R³ représente hydrogène,
R⁴ représente hydrogène, fluor ou chlore,
R⁵ représente hydrogène ou fluor,
R⁶ représente hydrogène,
R^{7A} représente méthyle,
R^{7B} représente trifluorométhyle, ou
R^{7A} et R^{7B} sont liés l'un à l'autre et forment, ensemble avec l'atome de carbone auquel ils sont liés, un cycle cyclopentyle disubstitué par fluor de formule 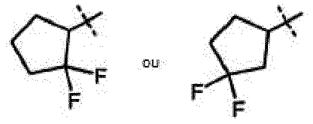
R⁸ représente chlore, méthyle, trifluorométhyle, éthyle, 1,1-difluoroéthyle, 2,2,2-trifluoroéthyle, isopropyle, tert-butyle, 1,1,1-trifluoro-2-méthylpropan-2-yle, vinyle, 2,2-difluorovinyle, ou cyclopropyle, et
R⁹ représente hydrogène, fluor, chlore ou méthoxy,
ainsi que ses sels, solvates et solvates des sels.

4. Composé de formule (I) selon la revendication 1 ou 2, dans laquelle
R^{1A} et R^{1B} sont liés l'un à l'autre et forment, ensemble avec l'atome de carbone auquel ils sont liés, un cycle cyclopropyle ou cyclobutyle de formule 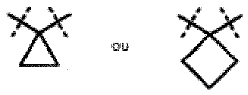
R² représente hydrogène, méthoxy, trideutérométhoxy, éthoxy ou cyclopropyloxy,
R³ représente hydrogène,
R⁴ représente hydrogène, fluor ou chlore,
R⁵ représente hydrogène ou fluor,
R⁶ représente hydrogène,
R^{7A} représente méthyle,
R^{7B} représente trifluorométhyle, ou
R^{7A} et R^{7B} sont liés l'un à l'autre et forment, ensemble avec l'atome de carbone auquel ils sont liés, un cycle cyclopentyle disubstitué par fluor de formule 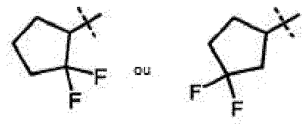
R⁸ représente chlore, méthyle, trifluorométhyle, éthyle, 1,1-difluoroéthyle, 2,2,2-trifluoroéthyle, isopropyle, tert-butyle, 1,1,1-trifluoro-2-méthylpropan-2-yle, vinyle, 2,2-difluorovinyle, ou cyclopropyle, et
R⁹ représente hydrogène, fluor, chlore ou méthoxy,
ainsi que ses sels, solvates et solvates des sels.

5. Procédé pour la préparation d'un composé de formule (I) tel que défini dans les revendications 1 à 4, **caractérisé en ce qu'**on couple un acide carboxylique de formule (II) 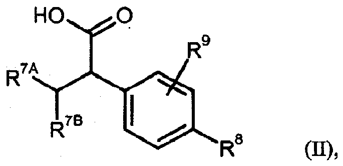 dans laquelle R^{7A}, R^{7B}, R⁸ et R⁹ présentent les significations indiquées dans les revendications 1 à 4,
dans un solvant inerte à l'aide d'un agent de condensation ou via l'étape intermédiaire du chlorure d'acide carboxylique correspondant en présence d'une base avec une amine de formule (III) 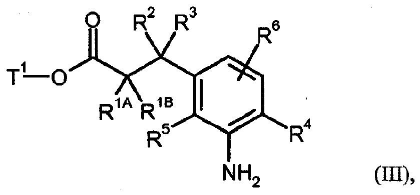 dans laquelle R^{1A}, R^{1B}, R², R³, R⁴, R⁵ et R⁶ présentent les significations indiquées dans les revendications 1 à 4 et
T¹ représente (C₁-C₄)-alkyle ou benzyle,
en un amide d'acide carboxylique de formule (IV) 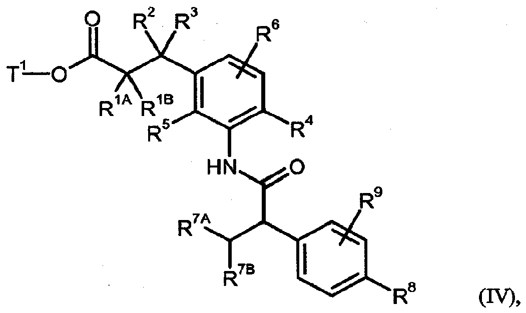 dans laquelle R^{1A}, R^{1B}, R², R³, R⁴, R⁵, R⁶, R^{7A}, R^{7B}, R⁸, R⁹ et T¹ présentent les significations indiquées ci-dessus,
puis on dissocie le radical ester T¹ par solvolyse basique ou acide ou, dans le cas où T¹ représente benzyle, également par hydrogénolyse avec obtention de l'acide carboxylique de formule (I)
et on sépare le cas échéant les composés de formule (I) selon des procédés connus par l'homme du métier en leurs énantiomères et/ou diastéréo-isomères et/ou on les transforme avec (i) des solvants et/ou (ii) des bases correspondant(e)s en leurs solvates, sels et/ou solvates des sels.

6. Composé tel que défini dans l'une quelconque des revendications 1 à 4 destiné à une utilisation comme médicament.

7. Utilisation d'un composé tel que défini dans l'une quelconque des revendications 1 à 4 pour la préparation d'un médicament destiné au traitement et/ou à la prévention de l'insuffisance cardiaque, de l'angine de poitrine, de l'hypertonie, de l'hypertonie pulmonaire, des ischémies, des maladies vasculaires, des troubles microcirculatoires, des maladies thrombotiques, de l'insuffisance rénale, des maladies fibrotiques et de l'artériosclérose.

8. Médicament contenant un composé, tel que défini dans l'une quelconque des revendications 1 à 4, en combinaison avec un ou plusieurs adjuvants inertes, non toxiques, pharmaceutiquement appropriés.

9. Médicament contenant un composé tel que défini dans l'une quelconque des revendications 1 à 4, en combinaison avec une ou plusieurs autres substances actives choisies dans le groupe constitué par les nitrates organiques, les donneurs de NO, les inhibiteurs de cGMP-PDE, les stimulateurs de la guanylate-cyclase, les agents à effet antithrombotique, les agents hypotenseurs ainsi que les agents modifiant le métabolisme des lipides.

10. Médicament selon la revendication 8 ou 9, destiné à une utilisation lors du traitement et/ou de la prévention de l'insuffisance cardiaque, de l'angine de poitrine, de l'hypertonie, de l'hypertonie pulmonaire, des ischémies, des maladies vasculaires, des troubles microcirculatoires, des maladies thrombotiques, de l'insuffisance rénale, des maladies fibrotiques et de l'artériosclérose.
